# EUROPEAN PATENT APPLICATION

(11) **EP 3 597 642 A1**
(43) Date of publication of application: **22.01.2020**
(21) Application number: 18184724.5
(22) Date of filing: 20.07.2018
(51) Int. Cl.: C07D 271/04, C07D 471/04, A61P 29/00, A61P 35/00, A61P 37/00, A61K 31/407

(54) **PHARMACEUTICAL 6,5 HETEROBICYCLIC RING DERIVATIVES**

(71) Applicant: UCB Biopharma SRL, 1070 Brussels (BE)
(72) Inventor: The designation of the inventor has not yet been filed
(74) Representative: UCB Intellectual Property

(57) **Abstract**

The invention relates to 6,5 heterobicyclic ring derivatives, processes for preparing them, pharmaceutical compositions containing them and their use for the treatment of inflammatory conditions driven by STING activation, such as, but not confined to, SLE and geographic atrophy.

## Description

### FIELD OF THE INVENTION

The invention relates to 6,5 heterobicyclic ring derivatives, processes for preparing them, pharmaceutical compositions containing them and their use for the treatment of inflammatory conditions driven by STING activation, such as, but not confined to, SLE and geographic atrophy. 6,5 heterobicyclic ring derivatives may be useful as STING (Stimulator of Interferon Genes) antagonists that inhibit the STING pathway.

### BACKGROUND OF THE INVENTION

The response of the body to tissue damage is to mount an inflammatory response. Usually this is self-limiting and functions to remove damaged tissue, counter any infectious microorganisms and restore the tissue to normal function. This innate immune response utilises pattern recognition receptors (PRRs) that can be divided into those that recognise specific microbial products (pathogen associated molecular patterns; PAMPs) and those that recognise host molecules (damage associated molecular patterns; DAMPs). The value in responding to PAMPs is clear but responding to DAMPs is equally important as it amplifies the inflammatory response and allows infections to be controlled early before they have a chance to overwhelm the host. DNA from viruses and bacteria and self DNA (nuclear or mitochondrial) can serve as PAMPs and DAMPs respectively (Paludan SR & Bowie AG (2013). Immune sensing of DNA. Immunity, 38:870-880). A number of DNA PRRs are recognised including cyclic GMP AMP synthase (cGAS) in the cytoplasmic compartment. cGAS appears to show little discrimination between microbial or self DNA although the extent of activation depends upon the length of the DNA, its' structure and whether or not it is oxidised (Andreeva L et al (2017). cGAS Senses Long and HMGB/TFAM-bound U-Turn DNA by Forming Protein-DNA Ladders. Nature 549:394-398). In the absence of infection or tissue damage, cGAS is silent, partly because self DNA is compartmentalised into the nucleus and mitochondria, and partly because of the activity of DNase enzymes that take care of physiological low levels of DNA leakage from cells and organelles.

When cGAS binds DNA it undergoes a conformational change and acquires enzyme activity utilising ATP and GTP as substrates and producing the cyclic dinucleotide 2',3'-cGAMP as a product. 2',3'-cGAMP engages the adapter protein STING that exists as a dimer on the endoplasmic reticulum. A conformational change to STING triggers a series of events including translocation to the trans Golgi network, recruitment of the tank binding kinase, TBK1, and phosphorylation of substrates IRF3, IKK and STAT 6 leading to the type I interferon response, proinflammatory cytokines and chemokines (Li, Y (2017). Regulating STING in health and disease. Journal of Inflammation 14:11). Activation is also linked to cell death pathways for example through inflammasome activation (Gaidt MM et al (2017). The DNA Inflammasome in Human Myeloid Cells Is Initiated by a STING-Cell Death Program Upstream of NLRP3. Cell 171:1110-1124).

There are instances where artificially stimulating the cGAS STING pathway may have value. For instance, strengthening the host immune response to infection or boosting immunological responses to tumour cells. STING agonists are being developed for these purposes (Mullard A (2018). Can innate immune system targets turn up the heat on 'cold' tumours? Nat Rev Drug Discov. 17:3-5).

The reverse is also true. For whilst activation of the cGAS STING pathway is an important response to tissue injury and infection, inflammatory changes induced by excessive activation of the pathway may be detrimental. Mutations in human STING that give rise to a constitutive activation of the protein cause SAVI (STING associated vasculopathy of infants) (Liu, Y (2014). Activated STING in a Vascular and Pulmonary Syndrome. The New England Journal of Medicine, 371:507-518) with affected children having skin rash, skin ulceration and interstitial lung disease (all symptoms that can be found in severe SLE). And in SLE, measurement of 2',3'-cGAMP in peripheral blood mononuclear cells was associated with patients with higher disease scores (An J et al (2017). Expression of Cyclic GMP-AMP Synthase in Patients With Systemic Lupus Erythematosus. Arthritis Rheumatol. 69:800-807) indicating the cGAS STING pathway to be active in patients with more severe SLE and suggesting the therapeutic potential of STING inhibitors.

There are other conditions where DNA sensing pathways and the cGAS STING pathway in particular, may drive pathological changes and where STING inhibitors may have utility. Extensive tissue damage, as seen in systemic inflammatory response syndrome (SIRS) and in critical care patients, is associated with symptoms similar to sepsis that may be explained by extensive engagement of innate immune receptors by DNA derived from tissue damage (Boyapati, RK et al. (2017). Advances in the understanding of mitochondrial DNA as a pathogenic factor in inflammatory diseases. F1000Research 6: 169). And in tissues where repair processes are limited, such as the heart and the brain, the inflammatory response to tissue injury can contribute to tissue damage. Experimental evidence suggests a damaging role for the cGAS STING pathway in myocardial infarction (King, KR et al (2017). IRF3 and type I interferons fuel a fatal response to myocardial infarction. Nat. Med. 23:1481-1487) and expression of cGAS and STING in CNS cells (Jeffries AM & Marriott I (2017). Human microglia and astrocytes express cGAS-STING viral sensing components. Neurosci Lett. 658:53-56) suggests similar mechanisms could be active in stroke.

Although the inflammatory response is essential to host defence, there are instances when the vigour of the host response to an infectious microorganism is itself a problem. For example the high mortality of N5N1 infection (avian flu) is caused by the local accumulation of exudate in the lungs from damaged cells attacked by the patient's own immune response (Short KR et al (2016). Influenza virus damages the alveolar barrier by disrupting epithelial cell tight junctions. Eur Respir J. 47:954-66). In such circumstances there may be a need to limit the extent of the innate immune response and inhibitors, that may include STING inhibitors, could have value in those circumstances.

Another area receiving attention relates to tissue degeneration. Many diseases are associated with mitochondrial stress and this has been linked to the release into the cytoplasm of mitochondrial DNA that can activate the cGAS STING pathway. This mechanism leads to the death of retinal pigmented epithelial cells and causes blindness in geographic atrophy (Kerur N et al (2018). cGAS drives noncanonical-inflammasome activation in age-related macular degeneration. Nat. Med. 24:50-61). The mechanism may also be active in other neurodegenerative diseases such as Parkinson's disease, amyotrophic lateral sclerosis, Alzheimer's disease and peripheral degenerative diseases such as chondrocyte death in osteoarthritis, loss of pancreatic islet cells etc.

Thus, there is a clear need to develop STING antagonists to explore their therapeutic potential in a range of human conditions with high unmet medical need.

### SUMMARY OF THE INVENTION

The present invention relates to 6,5 heterobicyclic ring derivatives, processes for preparing them, pharmaceutical compositions containing them and their use for the treatment of inflammatory conditions driven by STING activation, such as, but not confined to, SLE and geographic atrophy. A further aspect of the present invention consists of novel compounds that demonstrate the ability to functionally antagonise STING activation.

Further aspects of the invention will become apparent from the detailed specification.

### DESCRIPTION

In one aspect, the present invention relates to compounds of general formula I, or a pharmaceutically acceptable acid addition salt, a racemic mixture or its corresponding enantiomer and/or optical isomers thereof, 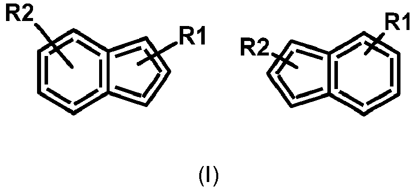 wherein
- the central core A which is the 6,5 heterobicyclic rings contains at least one heteroatom from O,N,S and C atoms can be optionally substituted by halogen, cyano, C1-6 alkyl, trifluoromethyl, difluromethyl, (C2-6)alkenyl, hydroxy, (C1-6)alkoxy, difluoromethoxy, trifluoromethoxy, trifluoroethoxy, (C1-6)alkylthio, (C1-6)alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6)alkylcarbonylamino, (C2-6)alkoxycarbonylamino, (C1-6)alkylsulphonylamino, formyl, (C2-6)alkylcarbonyl, carboxy, (C2-6) alkoxycarbonyl, aminocarbonyl, (C1-6)alkylaminocarbonyl, di(C1-6)alkylaminocarbonyl, aminosulphonyl, (C1-6)alkylaminosulphonyl or di(C1-6)alkylaminosulphonyl; (C3-7)heterocycloalkyl or (C3-7)spiroheterocycloalkyl, either of which groups may be optionally substituted by one or more substituents;
- R1 represents alkyl or cycloalkyl amines including fused and spirocycloalkyl amines optionally substituted including (C1-3)aminoalkyl, (C3-7)aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl; and
- R2 represents aryl, heteroaryl, heterobicyclic, (C4-7)aminocycloalkyl, cycloalkyl, heterocycloalkyl, (C6-8)diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6)alkenyl, hydroxy, (C1-6)alkoxy, difluoromethoxy, trifluoromethoxy, trifluoroethoxy, (C1-6) alkylthio, (C1-6)alkylsulphonyl, amino, (C1-6)alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6)alkoxycarbonylamino, (C1-6)alkylsulphonylamino, formyl, (C2-6) alkylcarbonyl, carboxy, (C2-6)alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.
- Examples of the central core A is selected from the group consisting of

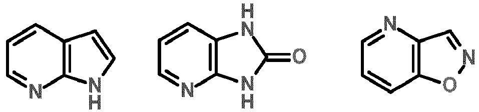

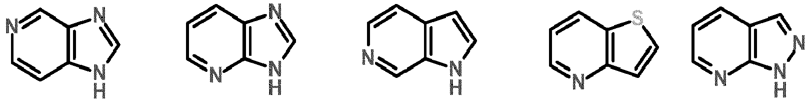

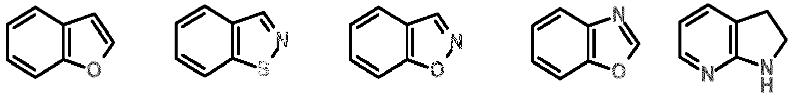

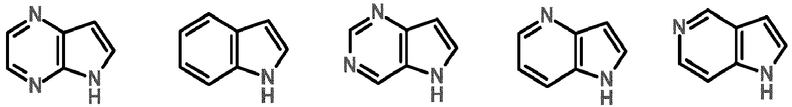

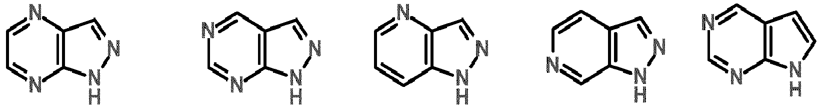

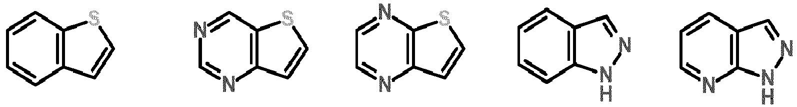

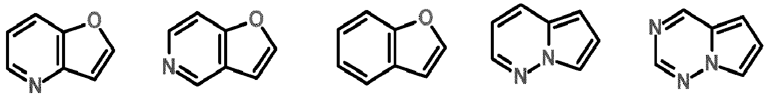

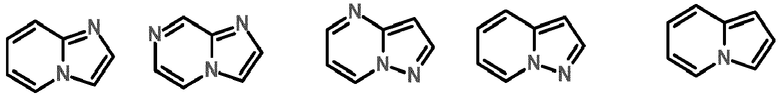

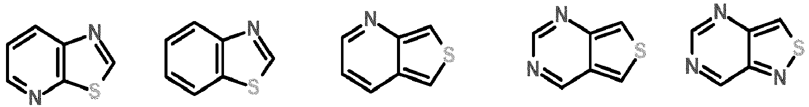

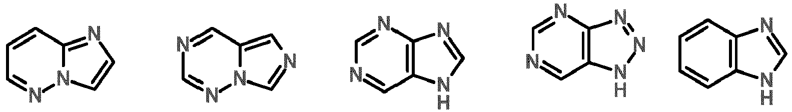

Preferrably, the central core A is selected from the group consisting of

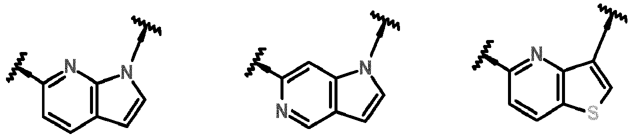

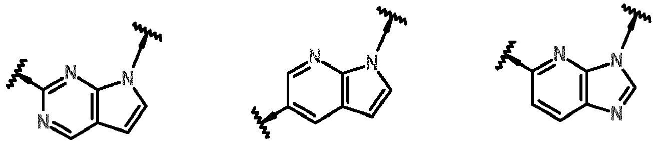

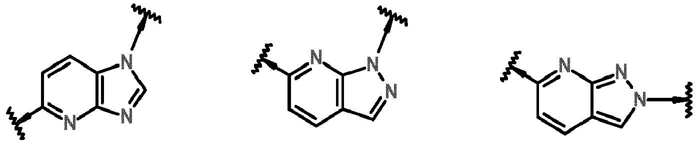

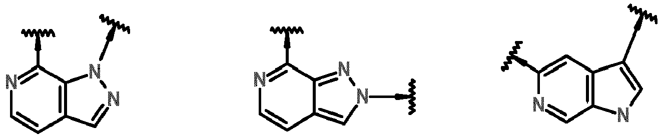

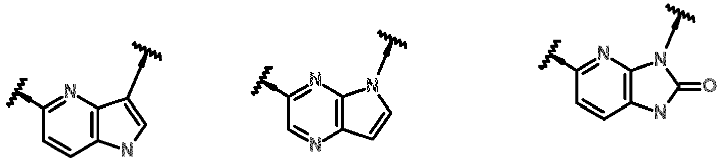

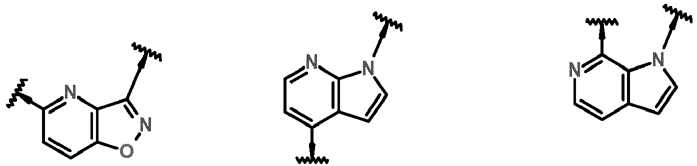

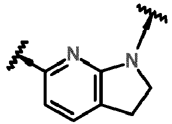

In a specific embodiment, the central core A is

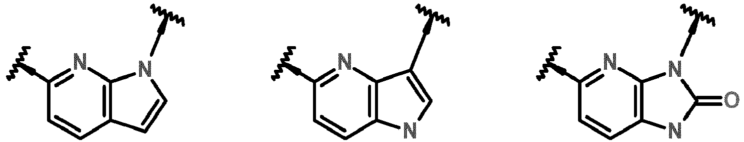

Usually, R1 is ethyl-azabicyclo[3.2.0]heptane; or 2-substituted-5-azaspiro[3.4.]octane; or ethyl-2-methyl-pyrrolidine or (C4-7) cycloalkylamines including 3-substituted-N-methyl-cyclobutanamine and piperidine.

Preferrably, R1 is 3-substituted-N-methyl-cyclobutanamine.

In another preferred embodiment, R1 is 2-substituted-5-azaspiro[3.4.]octane.

In another preferred embodiment, R1 is (C4-7) cycloalkylamines including 3-substituted-N-methyl-cyclobutanamine and piperidine.

Usually, R2 is 1-substituted-4-aryl-piperazine or 1-substituted-4-heteroaryl-piperazine or 1-(4-substituted-aryl)piperazine or 1-(4-substituted-heteroaryl)piperazine or 1-substituted-4-aryl-piperidine or 1-substituted4-heteroaryl-piperidine or 1-(4-substituted-aryl)piperidine or 1-(4-substituted-heteroaryl)piperidine with optional substitution on aryl, heteroaryl, piperazine or piperidine groups.

Preferrably, R2 is 1-substituted-4-aryl-piperazine or 1-substituted-4-heteroaryl-piperazine, 1-substituted-4-aryl-piperidine or 1-substituted-4-heteroaryl-piperidine.

In a further specific embodiment, compounds of formula I are those wherein
- the central core A is 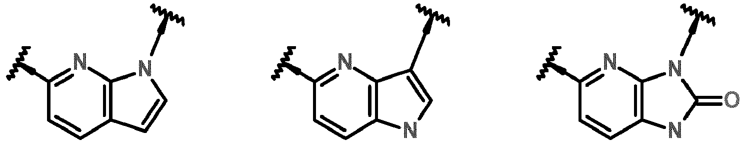
- R1 is ethyl-azabicyclo[3.2.0]heptane ; or 2 -substituted-5-azaspiro[3.4.]octane; or ethyl-2-methyl-pyrrolidine or (C4-7) cycloalkylamines including 3-substituted-N-methyl-cyclobutanamine and piperidine ;
- R2 is 1-substituted-4-aryl-piperazine or 1-substituted-4-heteroaryl-piperazine or 1-(4-substituted-aryl)piperazine or 1-(4-substituted-heteroaryl)piperazine or 1-substituted-4-aryl-piperidine or 1-substituted4-heteroaryl-piperidine or 1-(4-substituted-aryl)piperidine or 1-(4-substituted-heteroaryl)piperidine with optional substitution on aryl, heteroaryl, piperazine or piperidine groups.

In a further preferred specific embodiment, compounds of formula I are those wherein
- the central core A is 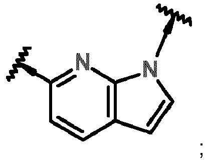
- R1 is 2-substituted-5-azaspiro[3.4.]octane or 3-substituted-N-methyl-cyclobutanamine or 4-substituted piperidine;
- R2 is 1-substituted-4-aryl-piperazine or 1-substituted-4-heteroaryl-piperazine, 1-substituted-4-aryl-piperidine or 1-substituted-4-heteroaryl-piperidine.

Specific compounds of the present invention are those selected from the group consisting of:
1-[4-[4-[3-(1-methylpyrazol-4-yl)-1-(4-piperidyl)pyrrolo[2,3-b]pyridin-6-yl]piperazin-1-yl]phenyl]ethanone,
1-[4-[4-[3-(2-methyl-3-pyridyl)-1-(4-piperidyl)pyrrolo[2,3-b]pyridin-6-yl]piperazin-1-yl]phenyl]ethanone,
6-[(2S)-2-methyl-4-(2-methylphenyl)piperazin-1-yl]-1-[2-(pyrrolidin-1-yl)ethyl]-1 H-pyrrolo[2,3-b]pyridine,
1-[4-[4-[1-(1-methylpyrazol-4-yl)-3-(4-piperidyl)pyrrolo[3,2-b]pyridin-5-yl]piperazin-1-yl]phenyl]ethanone,
6-[4-(4-acetylphenyl)piperazin-1-yl]-1-(5-azaspiro[3.4]octan-2-yl)pyrrolo[2,3-b]pyridine-3-carbonitrile,
1-[3-(methylamino)cyclobutyl]-6-[4-[4-(2,2,2-trifluoroacetyl)phenyl]piperazin-1-yl]pyrrolo[2,3-b]pyridine-3-carbonitrile,
6-[4-(4-acetylphenyl)piperazin-1-yl]-1-(4-piperidyl)pyrrolo[2,3-b]pyridine-3-carbonitrile, 6-[4-(4-acetyl-3-hydroxy-phenyl)piperazin-1-yl]-1-[3-(methylamino)cyclobutyl]pyrrolo[2,3-b]pyridine-3-carbonitrile,
6-[4-(4-acetyl-3-fluoro-phenyl)piperazin-1-yl]-1-[3-(methylamino)cyclobutyl]pyrrolo[2,3-b]pyridine-3-carbonitrile,
6-[(-4-(4-acetylphenyl)-2-methyl-piperazin-1-yl]-1-[3-(methylamino)cyclobutyl]pyrrolo[2,3-b]pyridine-3-carbonitrile,
6-[4-(4-acetylphenyl)piperazin-1-yl]-1-[3-(methylamino)cyclobutyl]pyrrolo[2,3-b]pyridine-3-carbonitrile,
5-[4-(4-acetylphenyl)piperazin-1-yl]-3-[3-(methylamino)cyclobutyl]-1-(1-methylpyrazol-4-yl)imidazo[4,5-b]pyridin-2-one,
6-[4-(4-acetylphenyl)-3-methyl-piperazin-1-yl]-1-[3-(methylamino)cyclobutyl]pyrrolo[2,3-b]pyridine-3-carbonitrile,
6-[4-(4-acetylphenyl)piperazin-1-yl]-2-methyl-1-[3-(methylamino)cyclobutyl]pyrrolo[2,3-b]pyridine-3-carbonitrile,
6-[4-(5-acetyl-2-pyridyl)piperazin-1-yl]-1-[3-(methylamino)cyclobutyl]pyrrolo[2,3-b]pyridine-3-carbonitrile,

The following paragraphs provide definitions of the various chemical moieties that make up the compounds according to the invention and are intended to apply uniformly throughout the specification and claims unless an otherwise expressly set out definition provides a broader definition.
As used herein, the term "C1-C6 alkyl" refers to a saturated, aliphatic hydrocarbon group including a straight or branched carbon chain with 1 - 6 carbon atoms. Examples for "alkyl" are methyl, ethyl, n-propyl, and isopropyl.
The term "C1-C6 alkoxy" refers to a group -O-R' wherein R' is C1-C6 alkyl as defined above. The term "halogen" refers to fluorine, chlorine, bromine or iodine.
The term "C1-C6 alkyl substituted by halogens or hydroxy or C1-C6 alkoxy" refers to an alkyl group as defined above, wherein at least one hydrogen atom is replaced by a halogen atom, a hydroxyl or a C1-C6 alkoxy.
The term "C1-C6 alkoxy substituted by halogens or hydroxy or C1-C6 alkoxy" refers to an alkoxy group as defined above, wherein at least one hydrogen atom is replaced by a halogen atom, a hydroxyl or a C1-C6 alkoxy.
The term "heterocyclyl" refers to a saturated ring, containing 1-3 heteroatoms, selected from N, O or S, for example morpholinyl, piperazinyl, piperidinyl or pyrrolidinyl or azetidinyl.
The term "pharmaceutically acceptable salt" or "pharmaceutically acceptable acid addition salt" according to the invention embraces therapeutically active, non-toxic acid or base salt forms which the compounds of formula I are able to form.
The acid addition salt form of a compound of formula I that occurs in its free form as a base can be obtained by treating the free base with an appropriate acid such as an inorganic acid, for example, a hydrohalic such as hydrochloric or hydrobromic, sulfuric, nitric, phosphoric and the like; or an organic acid, such as, for example, acetic, trifluoroacetic, oxalic, hydroxyacetic, propanoic, lactic, pyruvic, malonic, succinic, maleic, fumaric, malic, tartaric, citric, methanesulfonic, ethanesulfonic, benzenesulfonic, p-toluenesulfonic, cyclamic, salicylic, p-aminosalicylic, pamoic and the like.
The invention also relates to all stereoisomeric forms such as enantiomeric and diastereoisomeric forms of the compounds of formula I or mixtures thereof (including all possible mixtures of stereoisomers).
With respect to the present invention reference to a compound or compounds is intended to encompass that compound in each of its possible isomeric forms and mixtures thereof, unless the particular isomeric form is referred to specifically.
Some of the compounds of formula I may also exist in tautomeric forms. Such forms although not explicitly indicated in the above formula are intended to be included within the scope of the present invention.
It is to be understood that each individual atom present in formula (I), or in the formulae depicted hereinafter, may in fact be present in the form of any of its naturally occurring isotopes, with the most abundant isotope(s) being preferred. Thus, by way of example, each individual hydrogen atom present in formula (I), or in the formulae depicted hereinafter, may be present as a 1H, 2H (deuterium) or 3H (tritium) atom, preferably 1H. Similarly, by way of example, each individual carbon atom present in formula (I), or in the formulae depicted hereinafter, may be present as a 12C, 13C or 14C atom, preferably 12C.
Another embodiment of the present invention concerns a pharmaceutical composition comprising a detectable amount of a compound of formula I or a pharmaceutically acceptable salt thereof in combination with a pharmaceutically acceptable diluent or carrier.
In another embodiment, the present invention concerns a compound as listed above for use as a medicament.
In a specific embodiment, the present invention concerns a compound as listed above for use as a medicament in the treatment of inflammatory conditions driven by STING, such as SLE (Systemic lupus erythematosus) and geographic atrophy.
The invention concerns the compounds for use in the treatment of inflammatory conditions
driven by STING activation.
In another embodiment, the present invention concerns a pharmaceutical composition containing a compound as listed above as well as pharmaceutically acceptable excipients.

In another embodiment, the invention concerns the synthesis of intermediates, acid addition salt, a racemic mixture or its corresponding enantiomer and/or optical isomers thereof.
In another embodiment, the present invention concerns synthesis intermediates of general formula II 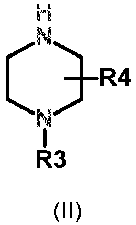 wherein R4 represents (C1-6)alkyl, (C1-6)alkoxy or oxo and R3 represents optionally substituted aryl or heteroaryl.

In another embodiment, the present invention concerns synthesis intermediates of general formula III 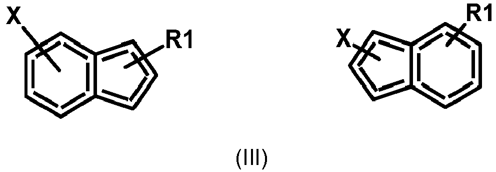 wherein
- X represents a halogen (Br, Cl, I) suitable for cross-coupling with intermediates of formula (II) or cross-coupling with an aryl or heteroaryl boronic acid derivative ; and
- R1 represents alkyl or cycloalkyl amines optionally substituted including (C1-3) aminoalkyl, (C3-7)aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl.

In another embodiment, the present invention concerns synthesis intermediates of general formula IV 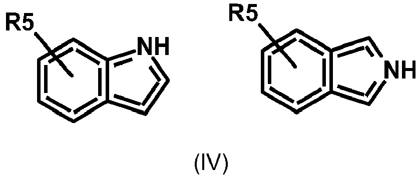 wherein R5 can be either X or R2.

In another embodiment, the present invention concerns synthesis intermediates of general formula V 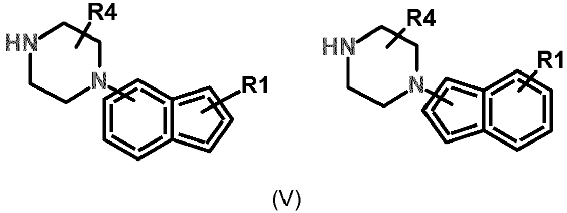 wherein
- R4 represents (C1-6)alkyl, (C1-6)alkoxy or oxo ; and
- R1 represents alkyl or cycloalkyl amines optionally substituted including (C1-3) aminoalkyl, (C3-7)aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl.

In another embodiment, the present invention concerns synthesis intermediates of general formula VI 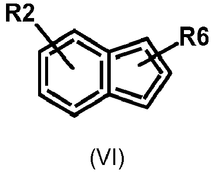 wherein
- R6 represents an alkyl or cycloalkyl substituent bearing a functional group suitable for displacement by an amine, for example 4-methylbenzenesulfonate; and
- R2 represents aryl, heteroaryl, heterobicyclic, (C4-7)aminocycloalkyl, cycloalkyl, heterocycloalkyl, (C6-8)diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6)alkenyl, hydroxy, (C1-6) alkoxy, difluoromethoxy, trifluoromethoxy, trifluoroethoxy, (C1-6)alkylthio, (C1-6) alkylsulphonyl, amino, (C1-6)alkylamino, di(C1-6)alkylamino, (C1-6)alkoxy(C1-6)alkylamino, N-[(C1-6)alkyl]-N-[hydroxy(C1-6)alkyl]amino, (C2-6) alkylcarbonylamino, (C2-6) alkoxycarbonylamino, (C1-6)alkylsulphonylamino, formyl, (C2-6)alkylcarbonyl, carboxy, (C2-6)alkoxycarbonyl, aminocarbonyl, (C1-6)alkylaminocarbonyl, di(C1-6)alkylaminocarbonyl, aminosulphonyl, (C1-6)alkylaminosulphonyl or di(C1-6)alkylaminosulphonyl; (C3-7)heterocycloalkyl or (C3-7)spiroheterocycloalkyl, either of which groups may be optionally substituted by one or more substituents.

In another embodiment, the present invention concerns synthesis intermediates of general formula VII 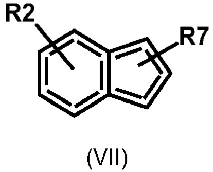 wherein
- R7 represents an primary or secondary amine ; and
- R2 represents aryl, heteroaryl, heterobicyclic, (C4-7)aminocycloalkyl, cycloalkyl, heterocycloalkyl, (C6-8)diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6)alkenyl, hydroxy, (C1-6) alkoxy, difluoromethoxy, trifluoromethoxy, trifluoroethoxy, (C1-6)alkylthio, (C1-6) alkylsulphonyl, amino, (C1-6)alkylamino, di(C1-6)alkylamino, (C1-6)alkoxy(C1-6)alkylamino, N-[(C1-6)alkyl]-N-[hydroxy(C1-6)alkyl]amino, (C2-6)alkylcarbonylamino, (C2-6) alkoxycarbonylamino, (C1-6)alkylsulphonylamino, formyl, (C2-6)alkylcarbonyl, carboxy, (C2-6)alkoxycarbonyl, aminocarbonyl, (C1-6)alkylaminocarbonyl, di(C1-6)alkylaminocarbonyl, aminosulphonyl, (C1-6)alkylaminosulphonyl or di(C1-6)alkylaminosulphonyl; (C3-7)heterocycloalkyl or (C3-7)spiroheterocycloalkyl, either of which groups may be optionally substituted by one or more substituents.

### SYNTHETIC METHODS

The compounds of formula I according to the invention can be prepared analogously to conventional methods as understood by the person skilled in the art of synthetic organic chemistry.

According to one embodiment, some compounds of general formula I may be prepared by reacting intermediate (II) with intermediate (III) under Buchwald cross-coupling conditions with a suitable transition metal catalyst and base. Compounds of general formula (I) may also be prepared by reacting intermediate (III) with an approporiate aryl or heteroaryl boronic acid or boronic ester under Suzuki cross-coupling conditions. Alternatively, some compounds of general formula (I) may be prepared by reacting intermediate (IV) with a suitable R1 group bearing either a halogen (CI, Br,I) for direct alkylation or an alcohol for Mitsunobu coupling. In this instance if R5 is a halogen in intermediate (IV), Buchwald or Suzuki cross-couping with a suitable R2 group bearing either an amine, boronic acid or boronic ester will also be required to yield compounds of formula (I).

Compounds of general formula (I) may also be prepared from intermediate (V) by Buchwald cross-coupling with a suitable arylhalide or heteroaryl halide.

Compounds of general formula (I) may also be prepared from intermediate (V) by amide bond formation or urea formation by reaction with a suitable acid, acid chloride or isocyanate under appropriate coupling conditions.
Alternatively, compounds of general formula (I) may also be prepared from intermediate (VI) by amine displacement of a leaving group on the R6 substituent.
Compounds of general formula (I) may also be prepared from intermediate (VII) by reductive amination involving condensation with an aldehyde or ketone followed by reduction of the resulting imine.

### EXAMPLES

The following examples illustrate how the compounds covered by formulas I and II may be synthesized. They are provided for illustrative purposes only and are not intended, nor should they be construed, as limiting the invention in any manner. Those skilled in the art will appreciate that routine variations and modifications of the following examples can be made without exceeding the spirit or scope of the invention.

All reactions involving air- or moisture-sensitive reagents were performed under a nitrogen atmosphere using dried solvents and glassware unless otherwise noted.

**Abbreviations**

| | |
|---|---|
| BOC: tert-butyloxycarbonyl | |
| DCM: dichloromethane | EtOAc: ethyl acetate |
| DMF: *N,N*-dimethylformamide | MeOH: methanol |
| DMSO: dimethylsulfoxide | EtOH: ethanol |
| Et₂O: diethyl ether | MeCN: acetonitrile |
| THF: tetrahydrofuran | DIPEA: *N,N*-diisopropylethylamine |
| H₂O: water | LDA: lithium diisopropylamide |
| NH₃: ammonia | Pd(OAc)₂: palladium(II) acetate |
| MgSO₄: magnesium sulphate | Na₂SO₄: sodium sulphate |
| K₂CO₃: potassium carbonate | NBS: N-bromosuccinimide |
| NIS: N-iodosuccinimide | DBU: 1,8-diazabicyclo[5.4.0]undec-7-ene |
| eq.: equivalents | SM: starting material |
| TFA: trifluoroacetic acid | DME: 1,2-dimethoxyethane |
| pTSA: *p*-toluenesulfonic acid | TBAF: tetra-n-butylammonium fluoride |
| min.: minutes | h: hour |
| r.t.: room temperature | RT: retention time |
| br: broad | M: molar |
| N: Normal | ES+: Electrospray Positive lonisation |

SCX: solid phase cation exchange N₂: nitrogen
HPLC: High Performance Liquid Chromatography
LCMS: Liquid Chromatography Mass Spectrometry
brine: saturated aqueous sodium chloride solution
PdCl₂(dppf)-DCM: [1,1'-bis(diphenylphosphino)ferrocene]dichloropalladium(II)
Pd-Ruphos G3: (2-Dicyclohexylphosphino-2',6'-diisopropoxy-1,1'-biphenyl)[2-(2'-amino-1,1'-biphenyl)]palladium(II) methanesulfonate
BINAP: (2,2'-bis(diphenylphosphino)-1,1'-binaphthyl)
Pd₂dba₃: Tris(dibenzylideneacetone)dipalladium(0)
HATU: 1-[Bis(dimethylamino)methylene]-1H-1,2,3-triazolo[4,5-b]pyridinium 3-oxid hexafluorophosphate
FCC: flash column chromatography
CMBP: (Cyanomethylene)trimethylphosphorane
CDI: 1,1'-carbonyldiimidazole

### Nomenclature

Compounds were named with the aid of ACD-ConsoleVersion 14.03 and/or Biovia Draw 2016.

### Analytical Conditions

All reactions involving air- or moisture-sensitive reagents were performed under a nitrogen atmosphere using dried solvents and glassware unless otherwise noted.

Except where otherwise stated, analytical LCMS data were obtained by using the below methods.

All quoted LCMS Retention Time (RT) values are in minutes.

### Method 1:

### pH 10

**Gradient:**

| Time (mins) | A% | B% |
|---|---|---|
| 0.00 | 95.00 | 5.00 |
| 4.00 | 5.00 | 95.00 |
| 5.00 | 5.00 | 95.00 |
| 5.10 | 95.00 | 5.00 |

Flow: 1 mL/min
Solvent A: 10 mM Ammonium Formate in water + 0.1 % Ammonia Solution
Solvent B: Acetonitrile + 5 % water + 0.1 % Ammonia Solution
Column: XBridge C18, 2.1 x 20 mm, 2.5 µm

### Method 2:

### pH 10

**Gradient:**

| Time (mins) | A% | B% |
|---|---|---|
| 0.00 | 95.00 | 5.00 |
| 1.50 | 5.00 | 95.00 |
| 2.25 | 5.00 | 95.00 |
| 2.50 | 95.00 | 5.00 |

Flow 1 mL/min
Solvent A: 10 mM Ammonium Formate in water + 0.1% Ammonia Solution
Solvent B: Acetonitrile + 5 % water + 0.1% Ammonia Solution
Column: XBridge C18, 2.1 x 20 mm, 2.5 µm

### Method 3:

### pH 3

**Gradient:**

| Time (mins) | A% | B% |
|---|---|---|
| 0.00 | 98.00 | 2.00 |
| 0.3 | 98.00 | 2.00 |
| 3.00 | 5.00 | 95.00 |
| 4.00 | 5.00 | 95.00 |
| 4.10 | 98.00 | 2.00 |
| 5.10 | 98.00 | 2.00 |

Flow 0.8 mL/min
Solvent A: Water + Acetonitrile + Formic Acid (95/5/750µl/L)
Solvent B: Water + Acetonitrile + Formic Acid (5/95/500µl/L)
Column: Acquity UPLC HSS T3 C18 column (1.8µm, 2.1 x 50 mm)

### Method 4:

### pH 10

**Gradient:**

| Time (mins) | A% | B% |
|---|---|---|
| 0.0 | 95 | 5 |
| 0.1 | 95 | 5 |
| 2.6 | 5 | 95 |
| 2.75 | 5 | 95 |
| 2.8 | 95 | 5 |
| 3.0 | 95 | 5 |

Flow: 1 mL/min
Solvent A = 10 mM Ammonium formate + 0.1 % Ammonia Solution (pH10)
Solvent B = MeCN + 5 % H2O + 0.1 % Ammonia Solution (pH10)
Column: Waters XBridge BEH C18 XP 2.5 µm 2.1 x 50 mm

### Method 5:

### pH 3

**Gradient:**

| Time (mins) | A% | B% |
|---|---|---|
| 0.0 | 94 | 6 |
| 1.5 | 5 | 95 |
| 2.25 | 5 | 95 |
| 2.5 | 94 | 6 |

Flow: 1 mL/min
Solvent A = 10 mM Ammonium formate in water + 0.1% Formic acid
Solvent B = MeCN + 5 % H2O + 0.1% Formic acid
Column: X-Bridge C18 Waters 2.1 x 20 mm, 2.5 µM column

### Method 6:

### pH 10

**Gradient:**

| Time (mins) | A% | B% |
|---|---|---|
| 0.0 | 94 | 6 |
| 1.5 | 5 | 95 |
| 2.25 | 5 | 95 |
| 2.5 | 94 | 6 |

Flow: 1 mL/min
Solvent A = 10 mM Ammonium formate in water + 0.1% ammonia solution
Solvent B = MeCN + 5 % H2O + 0.1% Formic acid
Column: X-Bridge C18 Waters 2.1 x 20 mm, 2.5 µM column

### Method 7:

**Gradient:**

| Time (mins) | A% | B% |
|---|---|---|
| 0.0 | 100 | 0 |
| 2.0 | 2 | 98 |
| 3.0 | 2 | 98 |
| 3.2 | 100 | 0 |
| 4.0 | 100 | 0 |

Flow: 1.2 mL/min
Solvent A = 5 mM Ammonium Bicarbonate Solution
Solvent B = MeCN
Column: Waters XBridge BEH C18 2.5 µm 3.0 x 50 mm

### Method 8:

**Gradient:**

| Time (mins) | A% | B% |
|---|---|---|
| 0.0 | 100 | 0 |
| 2.0 | 2 | 98 |
| 3.0 | 2 | 98 |
| 3.2 | 100 | 0 |
| 4.0 | 100 | 0 |

Flow: 1.2 mL/min
Solvent A = 0.05% Formic acid in water/MeCN (95:5)
Solvent B = 0.05% Formic acid in MeCN
Column: Waters X-Select CSH 2.5 µm 3.0 x 50 mm

### Method 9:

### pH 10

**Gradient:**

| Time (mins) | A% | B% |
|---|---|---|
| 0.0 | 95 | 5 |
| 4.0 | 5 | 95 |
| 5.0 | 5 | 95 |
| 5.1 | 95 | 5 |
| 6.5 | 95 | 5 |

Flow: 1 mL/min
Solvent A = 5 mM Ammonium formate + 0.1% Ammonia Solution
Solvent B = MeCN + 5% Solvent A + 0.1% Ammonia Solution
Column: Waters XBridge BEH C18 2.5 µm 2.1 x 50 mm

### Method 10:

### pH 3

**Gradient**

| Time (mins) | A% | B% |
|---|---|---|
| 0.0 | 95 | 5 |
| 1.2 | 0 | 100 |
| 1.30 | 0 | 100 |
| 1.31 | 95 | 5 |

Flow: 1.2 mL/min
Solvent A = Water + 0.1% Formic acid
Solvent B = MeCN + 0.1% Formic acid
Column: Kinetex Core-Shell C18, 2.1 x 50mm, 5µm column

### Method 11:

### pH 3

**Gradient**

| Time (mins) | A% | B% |
|---|---|---|
| 0.0 | 95 | 5 |
| 5.3 | 0 | 100 |
| 5.80 | 0 | 100 |
| 5.82 | 95 | 5 |
| 7.00 | 95 | 5 |

Flow: 0.6 mL/min
Solvent A = Water + 0.1% Formic acid
Solvent B = MeCN + 0.1% Formic acid
Column: Phenomenex Kinetix-XB C18, 2.1 x 100mm, 1.7µm column

### Method 12:

### pH 3

**Gradient**

| Time (mins) | A% | B% |
|---|---|---|
| 0.0 | 95 | 5 |
| 1.83 | 0 | 100 |
| 2.25 | 0 | 100 |
| 2.26 | 95 | 5 |

Flow: 1.2 ml/min
Solvent A = Water + 0.1% Formic acid
Solvent B = MeCN + 0.1% Formic acid
Column: Kinetex Core-Shell C8, 2.1 x 50mm, 5µm column

### Method 13:

### pH 3

**Gradient**

| Time (mins) | A% | B% |
|---|---|---|
| 0.0 | 95 | 5 |
| 5.00 | 0 | 100 |
| 5.40 | 0 | 100 |
| 5.42 | 95 | 5 |
| 7.00 | 95 | 5 |

Flow: 0.6 ml/min
Solvent A= Water + 0.1% Formic acid
Solvent B =: MeCN + 0.1% Formic acid
Column: Waters Atlantis dC18, 2.1 x 100mm, 3µm column

### General Procedure 1

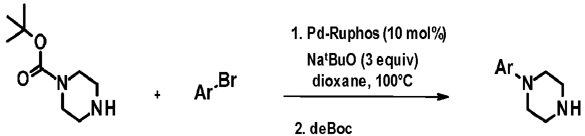

A mixture of 1-BOC-piperazine (1 equiv.), sodium tert-butoxide (1.5 - 3 equiv.), arylhalide (1.2 equiv.) and Pd-Ruphos G3 (0.1 equiv.) were dissolved in pre-degassed 1,4-dioxane (0.15 - 0.3 M). The reaction mixture was then heated at 100 °C until complete. The reaction mixture was cooled to ambient temperature and then either treated with an aqueous work up or with filtration through celite. The solvent was dried (using either MgSO₄, Na₂SO₄ or a phase separator) and subsequently removed under vacuum. The residue was purified by flash silica chromatography to give the product or used directly in the next step. The product was then dissolved in DCM (4-5 mL) and TFA (0.5 - 1 ml) added (either at 0 °C or at room temperature). The reaction mixture was stirred at ambient temperature until complete (LCMS monitoring). Then, the reaction was filtered through an SCX cartridge eluting first with methanol then with ammonia (2-7 M) in methanol. The ammonia in methanol solution was concentrated under reduced pressure to give the desired product.

### General Procedure 2

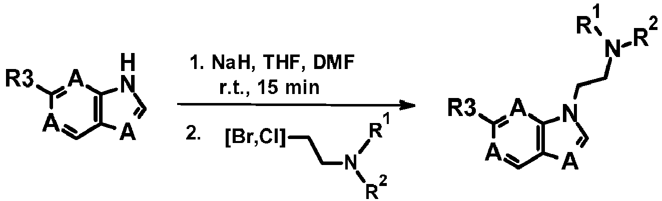

The appropriate heterocycle (1 equiv.) was dissolved in a mixture of DMF and THF (1:1 or 1:1.5). The solution was cooled to 0 °C and sodium hydride (1.2 or 2.2 equiv.) carefully added portion-wise. After approximately 5 min. the alkyl halide (1.2 equiv.) was added portion-wise. The reaction mixture was stirred at 0 °C or at rt for 15 min. to an hour then heated at 80 °C for a period ranging between 30 min to 6 h. The reaction mixture was cooled to 0 °C and quenched with water and treated with EtOAc and the organic layer separated. The organic solvent was dried (using either MgSO₄, Na₂SO₄ or a phase separator) and subsequently removed under vacuum. The residue was purified by flash silica column chromatography or reverse phase column chromatography to give the product.

### General Procedure 3

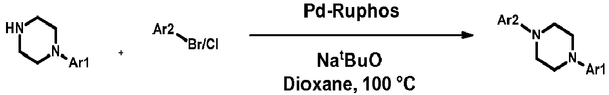

Heteroaryl or aryl halide (1 eq - 1.5 eq) and secondary amine typically a 4- substituted piperazine (1 eq.) were dissolved in pre-degassed anhydrous dioxane (0.1 - 0.2 M) and sodium tert-butoxide (3 eq.) was added followed by RuPhos Pd G3 (0.1 eq.). The reaction mixture was degassed and stirred at 80-100 °C in a sealed tube for 1-16 h. Small scale reactions (0.05 mmol) were filtered and diluted with DMF (0.6 mL) and purified directly by reverse phase column chromatography to yield the desired product. Larger scale reactions (> 0.05 mmol) were cooled to room temperature, filtered through a pad of celite and/or treated with an aqueous work up. The solvent was dried and concentrated in vacuo and the residue was purified by FCC or by a SCX catridge followed by reverse phase column chromatography.

### General Procedure 4

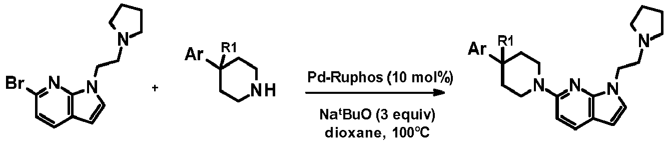

6-bromo-1-(2-pyrrolidin-1-ylethyl)pyrrolo[2,3-b]pyridine (100 mg, 0.3 mmol), sodium tert-butoxide (3 equiv., 98 mg, 1 mmol), Pd-Ruphos G3 (0.1 eq., 0.03 mmol), and the corresponding piperidine (1.2 eq., 0.34 mmol) were dissolved in degassed 1,4-dioxane (0.05 M, 7 mL). The reaction mixture was stirred at 100 °C overnight. The reaction was then cooled down to ambient temperature and filtered through a cartridge of celite, concentrated under vacuum, dissolved in DMSO and subjected to reverse phase column chromatography.

### General Procedure 5

A mixture of amine (1.0-1.5 equiv.), sodium tert-butoxide (1.4 equiv.), arylhalide (1.0 equiv.) and BINAP (0.15 equiv.) were dissolved in 1,4-dioxane (0.2 M) and the mixture was evacuated and backfilled with N₂ three times to degas the solvent. Pd₂dba₃ (0.05 equiv.) was then added and the reaction mixture heated at 80-100 °C until complete. The reaction mixture was cooled to r.t. and then either treated with an aqueous work up or with filtration through Celite. The solvent was dried (using either MgSO₄, Na₂SO₄ or a phase separator) and subsequently removed under vacuum. The residue was purified by flash silica chromatography to give the product or used directly in the next step.

### General Procedure 6

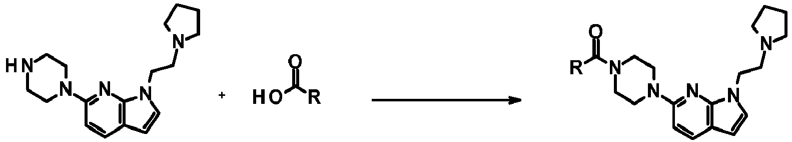

The appropriate carboxylic acid (0.05 mmol, 1 eq.) was added to a solution of Intermediate 7 (15 mg, 0.05 mmol, 1 eq.) and 2,4,6-tripropyl-1,3,5,2,4,6-trioxatriphosphorinane-2,4,6-trioxide (38 mg, 0.06 mmol, 1.2 eq) in DCM (0.5 mL). The reaction mixture was stirred at r.t. overnight. Reaction mixture filtered and diluted with DMF (0.45 mL) and purified by reverse phase column chromatography to yield the desired product.

### General Procedure 7

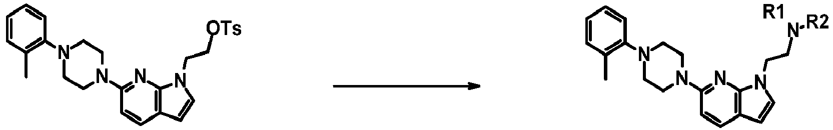

The appropriate amine (1.4 eq.), potassium carbonate (6 eq.) and Intermediate 17 (1.0 eq.) in 1,4-dioxane were stirred at 100 °C over a period of 6-12 hours. Small scale reactions (0.05 mmol) were filtered and purified by preparative HPLC to yield the desired product. Larger scale reactions (> 0.08 mmol) were passed through an SCX cartridge washing first with MeOH then eluting with approx. 2 M NH₃ in MeOH. The ammonia in methanol solution was concentrated under reduced pressure and the crude residue purified by reverse phase HPLC to yield the desired product.

### General Procedure 8

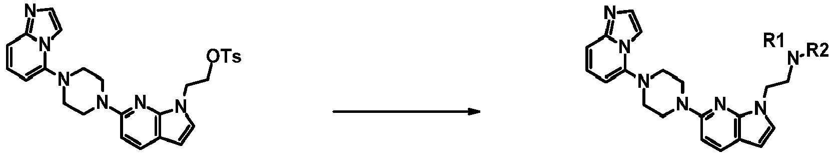

A mixture of the appropriate amine (1.5 eq.) and Intermediate 15 (20 mg, 0.03 mmol, 1 eq.) in acetonitrile and triethylamine (0.02 mL, 0.14 mmol, 4 eq.) were stirred at 80 °C overnight. The reaction mixture was filtered and purified by HPLC to yield the desired product.

### General Procedure 9

A mixture of the alcohol (1.5 eq.) and heterocycle (1 eq.) was dissolved in toluene (0.25 M). Cyanomethylenetributylphophorane (1.5 eq) was then added and the reaction mixture heated at 90-110 °C for 3-12 hours. The crude reaction mixture was loaded onto a silica column and eluted with a gradient of ethyl acetate in hexane to give the desired product.

### General Procedure 10

The corresponding arylbromide (1 eq.), boronic acid (1.5 eq.) and K₂CO₃ (2 eq.) were dissolved in 1,4-dioxane (0.1 M). H₂O (1 M) was added and the mixture degassed before PdCl₂(dppf)-DCM (0.01 eq.) was added and heated at 90 °C until the starting material was consumed. The reaction mixture was cooled to ambient temperature and EtOAc and H₂O were added. The layers were separated and the organic layer dried over Na₂SO₄. The solvent was removed under vacuum and the residue purified by flash silica column chromatography using hexane to EtOAc as eluent to afford the desired product.

### General Procedure 11

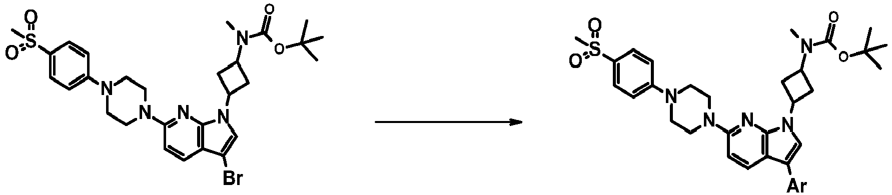

Intermediate 23 (70 mg, 0.11 mmol), boronic ester (2 eq., 0.22 mmol), K₂CO₃ (2 eq., 0.22 mmol) and PdCl₂(dppf)-DCM complex (0.1 equiv., 0.01 mmol) were dissolved in 1,4-dioxane (0.1 M, 1 mL). H₂O (0.1 mL) was added and the mixture was degassed for 1 min. The mixture was stirred overnight at 100°C. The mixture was then cooled to ambient temperature and DCM (3 mL) and H₂O (2 mL) were added and the layers separated. TFA (1 mL) was added to the DCM layer and the reaction mixture was stirred at room temperature for 2 h. reaction mixture was filtered through an SCX column, rinsed with MeOH and DCM, and the product eluted with NH₃ (7N in MeOH). The volatiles were removed under vacuum and the residue was purified by reverse phase HPLC to afford the desired product.

### General Procedure 12

The amine (1 eq.) was dissolved in a 1:1 mixture of tetrahydrofuran and ethanol (2 ml) or DCM (2 ml) or DMF (0.13 M). The carbonyl compound (1-10 eq.) was then added, followed by acetic acid (1-10 eq.) and sodium triacetoxyborohydride (3 - 4 eq.). The reaction mixture was stirred at room temperature until complete. The reaction mixture was then concentrated under reduced pressure, dissolved in DCM and washed with aq. sodium hydroxide (2 M). The organic solvent was separated, dried (Na₂SO₄) and concentrated under reduced pressure. Alternatively, the reaction mixture was diluted in EtOAc and the organic phase washed with water, brine, dried over Na₂SO₄ and concentrated to dryness in vacuo. The residue was optionally purified by reverse phase column chromatography to give the desired product as a free base or formic acid salt.

### General Procedure 13

Boc-protected amine (1 eq.) was treated according to Method A or Method B:
Method A: Amine was dissolved in DCM (4-5 mL) and TFA (0.5 - 1 mL or 20 eq.) added. The reaction mixture was stirred until completion. The reaction was diluted in DCM and washed with 2 M aqueous Na₂CO₃. The organic phase was then dried over Na₂SO₄ and concentrated to dryness in vacuo. Alternatively, the crude reaction mixture was purified by filtration through an SCX cartridge washing first with methanol then eluting with approx. 2 M ammonia in methanol. The ammonia in methanol solution was concentrated under reduced pressure to give the desired product. The product was optionally further purified by prep HPLC.
Method B: 4N HCI (10-50 eq.) in dioxane was added to a solution of Boc-protected amine (1 eq.) in DCM at room temperature. The reaction mixture was stirred for 1-16 h at room temperature. The reaction mixture was concentrated to dryness and the desired product was used as such in the next step as a HCI salt or purified by prep HPLC to afford the desired product as a formic acid salt or free base.

### General Procedure 14

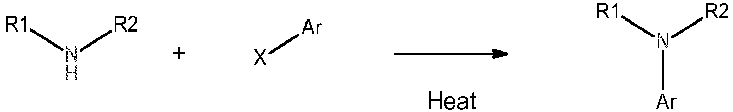

A solution of amine (1 eq.) and the corresponding halogen-substituted heterocycle (1 eq.) were heated (80-140 °C) with or without potassium carbonate (1.4 eq) in DMSO until reaction was complete. The mixture was diluted with EtOAc, washed with brine (5 times), dried (Na₂SO₄) and concentrated in vacuo. The product was optionally further purified by column chromatography or via trituration with an appropriate solvent.

### General Procedure 15

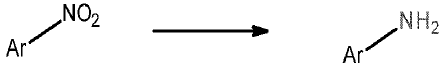

To a solution of nitroarene in MeOH was added zinc (6 equiv) and ammonium formate (5 equiv) at 0°C. After complete addition the resulting reaction mixture was stirred at rt. After complete consumption of starting material (∼15 mins), the methanol was evaporated under reduced pressure, the residue was then dissolved in ethyl acetate and filtered through Celite. The Celite was washed with ethyl acetate. The collected filtrate was washed with water (x 2), dried over sodium sulphate and evaporated under reduced pressure. The crude material was optionally purified by column chromatography.

### General Procedure 16

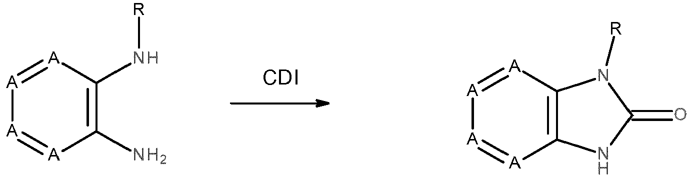

To a stirred solution of diamine in toluene was added CDI (2 equiv) at room temperature in a sealed tube. The reaction mixture was allowed to stir at 100 °C for 16 h. The reaction mixture was diluted with water and extracted with ethyl acetate (x 2). The organic layer was washed with brine, dried (Na₂SO₄) and concentrated under reduced pressure. The crude compound was optionally purified by column chromatography.

### General Procedure 17

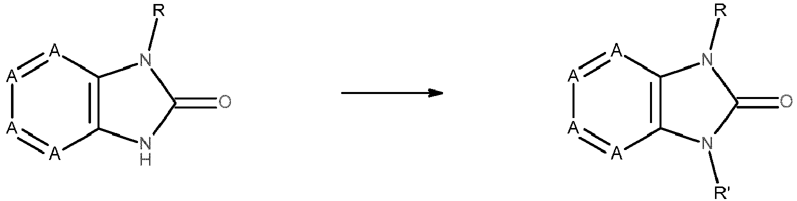

To a stirred solution of arylimidazol-2-one in MeCN was added aryl iodide (1.5 equiv), K₂CO₃ (3 equiv) and N,N'-dimethylethylenediamine (5 equiv) at rt. The mixture was degassed by using argon gas for 15 min. Cul (1.5 equiv) was then added and the reaction mixture was degassed for 15 min. The reaction was stirred at 100 °C for 16h. After cooling to rt, the mixture was diluted with water and extracted with ethyl acetate (x 3). The organic layer was washed with brine, dried (Na₂SO₄) and concentrated under reduced pressure. The crude product was optionally purified by column chromatography.

### General Procedure 18

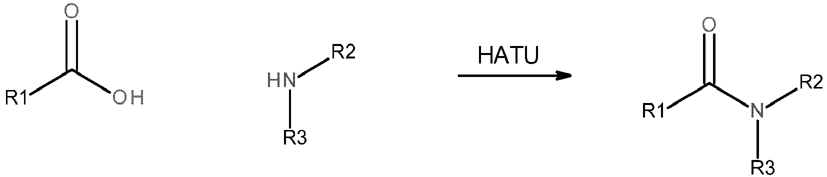

Carboxylic acid (1 equiv), amine (1.5 equiv) and HATU (1.3 equiv mmol) were dissolved in N,N-dimethylformamide and N,N-diisopropylethylamine. The mixture was then stirred at rt under N₂ for 1 hour. The mixture was diluted with EtOAc, water and brine then the layers were separated and the aqueous was further extracted with EtOAc. The combined organics were washed with brine, dried (Na₂SO₄) and concentrated in vacuo. The residue was optionally purified by column chromatography

### General Procedure 19

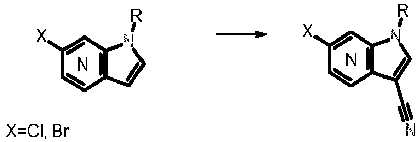

A 1-substituted -{6-bromo-1H-pyrrolo[2,3-b]pyridine (1 eq) was dissolved in anhydrous DMF (0.5 M) and anhydrous MeCN (0.5M) and cooled to 0 °C under nitrogen. Chlorosulfonyl isocyanate (1.15 eq.) was added and the reaction mixture was stirred at 0 °C under nitrogen for 10 min. The reaction mixture was allowed to warm to rt and stirred at rt for 30 min. The reaction was cooled to 0 °C and 1M NaOH aq. was added dropwise until the aqueous phase was at pH 10. The reaction suspension was extracted with EtOAc and the combined organic phase was washed with brine and dried over Na₂SO₄. The crude product was purified by FCC (Biotage Isolera, SNAP Ultra, gradient elution 10-100% EtOAc:Heptanes) to afford the desired 3-cyano product.

### General Procedure 20

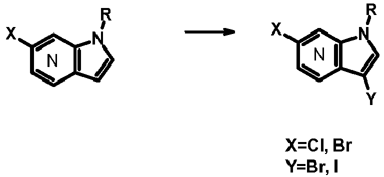

NIS or NBS (1 eq.) was added to a solution of 1-substituted -{6-halo-1H-pyrrolo[2,3-b]pyridine (1 eq) in anhydrous DMF (0.05M) at rt. The reaction mixture was stirred under nitrogen for 20 min. Water was added and the reaction mixture was extracted with EtOAc. The organic phase was washed successively with water, brine, dried over Na₂SO₄ and concentrated to dryness in vacuo to afford the desired 3-halogenated product.

### General Procedure 21

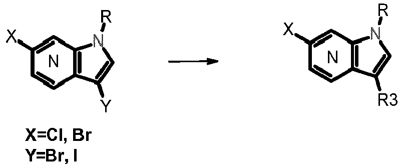

A mixture of aryl halide (1 eq.), aryl boronic aicd (1 eq.) and 2M sodium carbonate (3 eq.) in dioxane (0.12M) was degassed by N₂ bubbling for 10 min, then Tetrakis(triphenylphosphine)palladium(0) (0.05 - 0.1 eq) was added and the reaction was heated at 80-100 °C for 30 min in the CEM microwave 200W or thermally for 1-16 h. The reaction mixture was diluted in EtOAc and washed successively with water and brine. The aqueous phase was extracted with EtOAc, the combined organic phase was dried over Na₂SO₄ and concentrated to dryness in vacuo. The residue was purified by FCC (Biotage isolera, SNAP Ultra, gradient elution 10-100% EtOAc:Heptane to afford the desired product.

### INTERMEDIATE 1

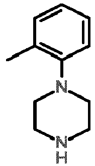

### 1-(o-tolyl)piperazine

1-(o-tolyl)piperazine was prepared following General Procedure 1 using 1-BOC-piperazine (6 g, 32 mmol) and 2-bromotoluene (5 mL, 41 mmol) to afford intermediate 1 (7.2 g, 82% yield)
LCMS (Method 2, ES⁺) 1.56 min, 177 *m*/*z* (M+H)⁺.

### INTERMEDIATE 2

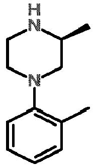

### (3S)-3-methyl-1-(o-tolyl)piperazine

(3S)-3-methyl-1-(o-tolyl)piperazine was prepared following General Procedure 1 using 2-bromotoluene (170 mg, 1 mmol) and (S)-4-N-Boc-2-methylpiperazine (240 mg, 1.2 eq., 1.2 mmol) to afford intermediate 2 (110 mg, 47% yield).
LCMS (Method 2, ES⁺) 1.1 min, 191 *m*/*z* (M+H)⁺.

### INTERMEDIATE 3

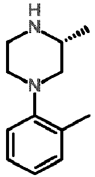

### (3R)-3-methyl-1-(o-tolyl)piperazine

(3R)-3-methyl-1-(o-tolyl)piperazine was prepared following General Procedure 1 using 2-bromotoluene (170 mg, 1 mmol) and (R)-4-N-Boc-2-methylpiperazine (240 mg, 1.2 eq., 1.2 mmol) to afford intermediate 3 (122 mg, 52% yield).
LCMS (Method 2, ES⁺) 1.1 min, 191 *m*/*z* (M+H)⁺.

### INTERMEDIATE 4

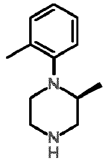

### (2S)-2-methyl-1-(o-tolyl)piperazine

(2S)-2-methyl-1-(o-tolyl)piperazine was prepared following General Procedure 1 using 2-bromotoluene (170 mg, 1 mmol) and (S)-1-N-Boc-2-methylpiperazine (240 mg, 1.2 eq., 1.2 mmol) to afford intermediate 4 (180 mg, 77% yield).
LCMS (Method 2, ES⁺) 1.08 min, 191 *m*/*z* (M+H)⁺.

### INTERMEDIATE 5

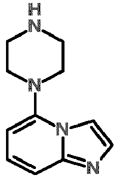

### 5-piperazin-1-ylimidazo[1,2-a]pyridine

5-piperazin-1-ylimidazo[1,2-a]pyridine was prepared following General Procedure 1 using 5-bromoimidazo[1,2-A]pyridine (5 g, 25 mmol) and 1-boc-piperazine (4.7 g, 25 mmol) to afford intermediate 5 (1.1 g, 55% yield).
LCMS (Method 2, ES⁺) 0.35 min, 203 *m*/*z* (M+H)⁺.

### INTERMEDIATE 6

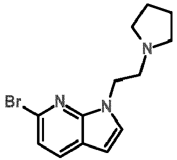

### 6-bromo-1-(2-pyrrolidin-1-ylethyl)pyrrolo[2,3-b]pyridine

6-bromo-1-(2-pyrrolidin-1-ylethyl)pyrrolo[2,3-b]pyridine was prepared following General Procedure 2 using 6-bromo-1H-pyrrolo[2,3-b]pyridine (10 g, 50 mmol) and 1-(2-chloroethyl)pyrrolidine hydrochloride (11 g, 63.4 mmol). The reaction was heated to 80 °C for 2 hours. The residue was purified by flash silica column chromatography using gradient elution from hexane to 1:1 hexane:EtOAc to afford the intermediate 6 (12 g, 82% yield).
LCMS (Method 2, ES⁺) 1.30 min, 295 & 297 *m*/*z* (M+H)⁺.

### INTERMEDIATE 7

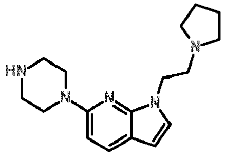

### 6-piperazin-1-yl-1-(2-pyrrolidin-1-ylethyl)pyrrolo[2,3-b]pyridine

Intermediate 6 (7 g, 23.8 mmol) was treated according to General Procedure 1 followed by General Procedure 13 to afford Intermediate 7 (4.5 g, 67% yield).
LCMS (Method 2, ES⁺) 1.02 min, 300 *m*/*z* (M+H)⁺.

### INTERMEDIATE 8

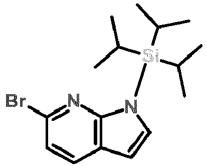

### (6-bromopyrrolo[2,3-b]pyridin-1-yl)-triisopropyl-silane

6-Bromo-1H-pyrrolo[2,3-b]pyridine (4 g, 20.3 mmol) was dissolved in 1,4-dioxane (100 mL) and DIPEA (11 mL, 63.1 mmol) was added followed by triisopropylsilyl trifluromethanesulfonate (6.5 mL, 24 mmol). The reaction mixture was then stirred at r.t. for 30 min. before it was heated at 80°C overnight. Triisopropylsilyl trifluromethanesulfonate (1.2 equiv., 24.4 mmol) was added and stirred at 80°C for an additional 3 hours. The reaction was cooled down to ambient temperature and NH₄Cl aq. solution and EtOAc were added. The layers were separated and the aq. phase was extracted with EtOAc. The combined organic layers were dried over MgSO₄ and the solvent was removed under vacuum. The residue was purified by flash chromatography using gradient elution from hexane to a 1:1 mixture hexane:EtOAc to yield Intermediate 8 (5.2 g, 72% yield).
LCMS (Method 2, ES⁺) 1.96 min, 353 & 355 *m*/*z* (M+H)⁺.

### INTERMEDIATE 9

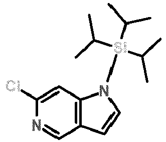

### (6-chloropyrrolo[3,2-c]pyridin-1-yl)-triisopropyl-silane

6-Chloro-5-azaindole (1 g, 6.42 mmol) was partially suspended in 1,4-dioxane (30 mL). DIPEA (3.5 mL, 20 mmol) was then added followed by triisopropylsilyl trifluoromethanesulfonate (2.5 mL, 9.3 mmol). The reaction mixture was stirred at room temperature for 5 h 30 min and then treated with an aq. work up. The organic solvent was dried (Na₂SO₄) and concentrated under reduced pressure to give a residue that was purified by flash silica column chromatography to give the title compound (1.68 g, 84%).

### INTERMEDIATE 10

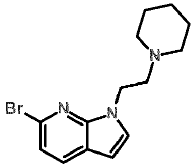

### 6-bromo-1-[2-(1-piperidyl)ethyl]pyrrol[2,3-b]pyridine

6-bromo-1-[2-(1-piperidyl)ethyl]pyrrolo[2,3-b]pyridine was prepared following General Procedure 2 using 6-bromo-1H-pyrrolo[2,3-b]pyridine (400 mg, 2 mmol) and 1-(2-bromoethyl)piperidine hydrobromide (1.2 equiv., 2.39 mmol). The product was purified by flash silica column chromatography using gradient elution from hexane to hexane:EtOAc 1:1 to give intermediate 10 (420 mg, 69% yield).
LCMS (Method 2, ES⁺) 1.45 min, 308 & 310 *m*/*z* (M+H)⁺.

### INTERMEDIATE 11

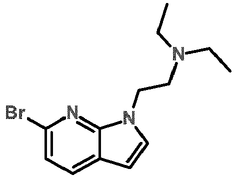

### 2-(6-bromopyrrolo[2,3-b]pyridin-1-yl)-N,N-diethyl-ethanamine

2-(6-bromopyrrolo[2,3-b]pyridin-1-yl)-N,N-diethyl-ethanamine was prepared following General Procedure 2 using 6-bromo-1H-pyrrolo[2,3-b]pyridine (400 mg, 2 mmol) and 2-bromo-N,N-diethylamine hydrobromide (1.2 equiv., 2.4 mmol). The crude product was purified by flash silica column chromatography using gradient elution from hexane to EtOAc to afford Intermediate 11 (360 mg, 61% yield).
LCMS (Method 2, ES⁺) 1.42 min, 296 &298 *m*/*z* (M+H)⁺.

### INTERMEDIATE 12

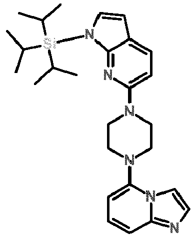

### [6-(4-imidazo[1,2-a]pyridin-5-yl)piperazin-1-yl)pyrrolo[2,3-b]pyridin-1-yl]-triisopropyl-silane

[6-(4-imidazo[1,2-a]pyridin-5-ylpiperazin-1-yl)pyrrolo[2,3-b]pyridin-1-yl]-triisopropyl-silane was prepared following General Procedure 3 using Intermediate 8 (1.2 eq., 13 mmol) and Intermediate 5 (2.2 g, 11 mmol). The residue was purified by flash silica column chromatography with gradient elution from hexane to EtOAc to yield Intermediate 12 (3.2 g, 61% yield).
LCMS (Method 2, ES⁺) 1.90 min, 475 *m*/*z* (M+H)⁺.

### INTERMEDIATE 13

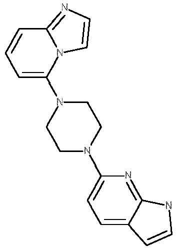

### 5-[4-(1H-pyrrolo[2,3-b]pyridin-6-yl)piperazin-1-yl]imidazo[1,2-a]pyridine

Intermediate 12 (3.15 g, 6.64 mmol) was dissolved in THF (0.25 M, 26 mL) and TBAF (8 mL, 1.2 eq., 7.96 mmol, 1 mmol/mL) added. The reaction mixture was stirred at ambient temperature for 3 hours and then H₂O and Et₂O were added. The layers were separated and the aqueous phase extracted with Et₂O. The solvent was removed under vacuum and the residue was redissolved in MeOH and filtered through an SCX column and washed with MeOH and then eluted with NH₃ (7N in MeOH) to give Intermediate 13 (1.0 g, 44%).
LCMS (Method 2, ES⁺) 1.13 min, 319 *m*/*z* (M+H)⁺.

### INTERMEDIATE 14

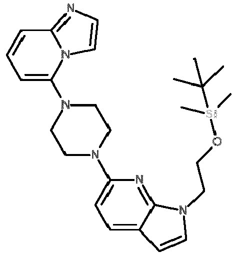

### tert-butyl-[2-[6-(4-imidazo[1,2-a]pyridin-5-ylpiperazin-1-yl)pyrrolo[2,3-b]pyridin-1-yl]ethoxyl-dimethyl-silane

Intermediate 13 (2 g, 6.3 mmol) was dissolved in a mixture of THF (0.25 M) and DMF (0.25 M) and cooled at 0 °C before sodium hydride (1.5 eq., 400 mg, 9.95 mmol) was added in portions. The mixture was stirred at ambient temperature for 30 min. before (2-bromoethoxy)-tert-butyldimethylsilane (1.2 eq., 8 mmol) was added. The mixture was warmed to 60 °C and stirred until the reaction was complete. The reaction mixture was quenched with H₂O and EtOAc added. The layers were separated and the aq. phase extracted with EtOAc. The combined organic layers were dried over Na₂SO₄ and the solvent removed under vacuum. The residue was purified by column chromatography, gradient elution from EtOAc to 1:1 MeOH:EtOAc to afford Intermediate 14 as a white solid (1 g, 44% yield).
LCMS (Method 2, ES⁺) 1.78 min, 477 *m*/*z* (M+H)⁺.

### INTERMEDIATE 15

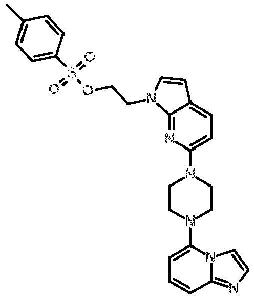

### 2-[6-(4-imidazo[1,2-a]pyridin-5-ylpiperazin-1-yl)pyrrolo[2,3-b]pyridin-1-yl]ethyl 4-methylbenzenesulfonate

Intermediate 14 (1 g, 2.098 mmol) was dissolved in THF (0.25 M, 102.3 mmol) and TBAF (3.1 mL, 3.15 mmol, 1 mmol/mL) was added. The reaction mixture was stirred at ambient temperature for 3 hours and then H₂O and Et₂O added. The layers were separated and the aqeous phase was extracted with Et₂O. The combined organic layers were dried over Na₂SO₄. The solvent was removed under vacuum and the residue was dissolved in MeOH and filtered through an SCX column and washed with MeOH and then eluted with NH₃ (7 N) in MeOH. The resulting residue was dissolved in DCM (0.25 M) and triethylamine (1.5 eq., 3 mmol) added. The solution was cooled to 0 °C, and p-toluensulfonyl chloride (1.2 eq., 2 mmol) was added and the reaction mixture stirred at ambient temperature overnight. The reaction mixture was quenched with H₂O and DCM and the layers separated. The solvent was removed under vacuum and the residue purified by flash silica column chromatography using gradient elution from hexane to EtOAc, and then to 20% MeOH in EtOAc to afford Intermediate 15 (900 mg, 69% yield)
LCMS (Method 2, ES⁺) 1.50 min, 517 *m*/*z* (M+H)⁺.

### INTERMEDIATE 16

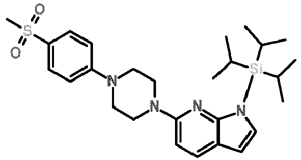

### triisopropyl-[6-[4-(4-methylsulfonylphenyl)piperazin-1-yl]pyrrolo[2,3-b]pyridin-1-yl]silane

Triisopropyl-[6-[4-(4-methylsulfonylphenyl)piperazin-1-yl]pyrrolo[2,3-b]pyridin-1-yl]silane was prepared following General Procedure 3 using Intermediate 8 (5 g, 14.2 mmol) and 1-[4-(methylsulfonyl)phenyl]piperazine (1.2 eq., 17 mmol). The residue was purified by flash column chromatography with gradient elution from hexane to EtOAc and then until 30% MeOH in EtOAc to give Intermediate 16 (5.3 g, 73% yield).
LCMS (Method 2, ES⁺) 1.98 min, 513 *m*/*z* (M+H)⁺

### INTERMEDIATE 17

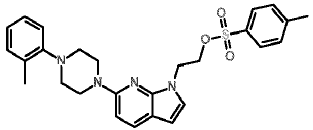

### 2-[6-[4-(o-tolyl)piperazin-1-yl]pyrrolo[2,3-b]pyridin-1-yl]ethyl 4-methylbenzenesulfonate

Intermediate 21 (0.3 g, 0.89 mmol) was dissolved in dichloromethane (5 mL) and triethylamine (0.2 mL, 1 mmol) was added followed by p-toluenesulfonylchloride (171 mg, 0.88 mmol). After 2 h a second portion of triethylamine (0.2 mL, 1 mmol) was added followed by a second portion of intermediate 21 (0.3 g, 0.89 mmol) in dichloromethane (5 mL) and a second portion of p-toluenesulfonylchloride (171 mg, 0.88 mmol). The reaction mixture was then stirred at rt overnight and treated with an aqueous work up. The organic solvent was dried (Na₂SO₄) and concentrated under reduced pressure. The crude residue was then purified by flash silica column chromatography to give the title compound (0.51 g, quant.)
LCMS (Method 2, ES⁺) 1.63 min, 491 *m*/*z* (M+H)⁺.

### INTERMEDIATE 18

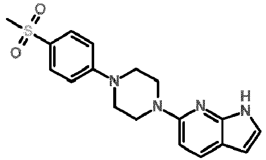

### 6-[4-(4-methylsulfonylphenyl)piperazin-1-yl]-1H-pyrrolo[2,3-b]pyridine

Intermediate 16 (3 g, 5.9 mmol) was dissolved in THF (0.5 M) and TBAF (8.8 mL, 1.5 eq., 8.8 mmol, 1 mmol/mL) was added at ambient temperature. The mixture was stirred for 1 hour then H₂O and Et₂O were added. The layers were separated and the aq. phase was extracted with Et₂O. The solvent was removed under vacuum and the residue purified by flash chromatography gradient elution from hexane to EtOAc and then to a mixture of 1:1 EtOAc:MeOH to yield intermediate 18 (950 mg, 46% yield).
LCMS (Method 2, ES⁺) 1.20 min, 357 *m*/*z* (M+H)⁺.

### INTERMEDIATE 19

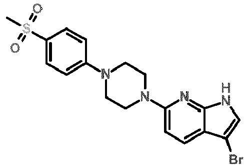

### 3-bromo-6-[4-(4-methylsulfonylphenyl)piperazin-1-yl]-1H-pyrrolo[2,3-b]pyridine

Intermediate 16 (730 mg, 1.4 mmol) was dissolved in DCM (30 mL) and cooled to 0 °C. N-bromosuccinimide (165 mg, 0.92 mmol) was added portion-wise over 20 min. The reaction was stirred at that temperature for an additional 10 minutes before it was quenched with saturated NaHCO₃ and the layers separated. The aqueous phase was then extracted with DCM. The solvent was removed under vacuum and the residue directly dissolved in THF (10 mL). TBAF (1.6 mL, 1.2 eq., 1.6 mmol, 1 mmol/mL) was added and the mixture was stirred at ambient temperature for 20 minutes. The crude reaction mixture was quenched with saturated aq. NH₄Cl solution and EtOAc added. The layers were separated. The aq. phase was then extracted with EtOAc. The combined organic layers were combined, dried over Na₂SO₄ and the solvent removed under vacuum. The residue was purified by chromatography gradient elution from DCM to 30% MeOH in DCM to yield Intermediate 19 (500 mg, 32% yield).
LCMS (Method 2, ES⁺) 1.34 min, 434 & 436 *m*/*z* (M+H)⁺.

### INTERMEDIATE 20

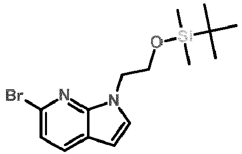

### 2-(6-bromopyrrolo[2,3-b]pyridin-1-yl)ethoxy-tert-butyl-dimethyl-silane

2-(6-bromopyrrolo[2,3-b]pyridin-1-yl)ethoxy-tert-butyl-dimethyl-silane was prepared following General Procedure 2 using 6-bromo-1H-pyrrolo[2,3-b]pyridine (2 g, 9.9 mmol) and (2-bromoethoxy)-tert-butyldimethylsilane (2.7 mL, 12 mmol) to afford intermediate 20 (3.13 g, 88% yield).
LCMS (Method 2, ES⁺) 1.78 min, 355 & 357 *m*/*z* (M+H)⁺.

### INTERMEDIATE 21

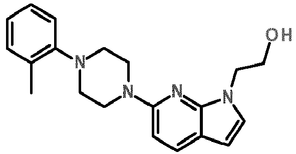

### 2-[6-[4-(o-tolyl)piperazin-1-yl]pyrrolo[2,3-b]pyridin-1-yl]ethanol

Using general procedure 3, intermediate 1 (1.2 g, 6.8 mmol), intermediate 20 (2.7 g, 7.6 mmol) gave tert-butyl-dimethyl-[2-[6-[4-(o-tolyl)piperazin-1-yl]pyrrolo[2,3-b]pyridin-1-yl]ethoxy]silane, which was then dissolved in THF (20 mL). TBAF (14 mL, 14 mmol) was then added and the reaction mixture stirred at room temperature for 5 days. The reaction mixture was concentrated under reduced pressure and loaded onto an SCX cartridge washing first with methanol then eluting with 2 M (approx.) ammonia in methanol. The ammonia in methanol solution was concentrated under reduced pressure and the residue purified by flash silica column chromatography to give the title compound (1.37 g, 60% yield).
LCMS (Method 2, ES⁺) 1.47 min, 337 *m*/*z* (M+H)⁺.

### INTERMEDIATE 22

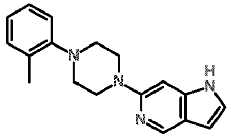

### 6-[4-(o-tolyl)piperazin-1-yl]-1H-pyrrolo[3,2-c]pyridine

Using general procedure 3, intermediate 9 (455 mg, 1.47 mmol) and intermediate 1 (236 mg, 1.33 mmol) gave triisopropyl-[6-[4-(o-tolyl)piperazin-1-yl]pyrrolo[3,2-c]pyridin-1-yl]silane, which was dissolved in THF (5 mL). TBAF (4 mL, 4 mmol) was then added and the reaction mixture stirred at r.t. overnight. The reaction mixture was treated with an aqueous work up and the organic layer dried (Na₂SO₄) and concentrated under reduced pressure. The resulting residue was passed through an SCX cartridge washing first with methanol then eluting with approx. 2 M ammonia in methanol. The solution was concentrated under reduced pressure to give the title compound (46 mg, 12% yield over two steps).
LCMS (Method 2, ES⁺) 1.31 min, 293 *m*/*z* (M+H)⁺.

### INTERMEDIATE 23

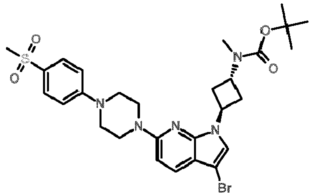

### Trans-tert-butyl N-[3-[3-bromo-6-[4-(4-methylsulfonylphenyl)piperazin-1-yl]pyrrolo[2,3-b]pyridin-1-yl]cyclobutyl]-N-methyl-carbamate

Intermediate 19 (500 mg, 1.15 mmol) and cis-tert-butyl N-(3-hydroxycyclobutyl)-N-methylcarbamate (1.5 eq., 1.7 mmol) were reacted following General Procedure 9 and purified by flash column chromatography eluting from hexane to 70% EtOAc in hexane to give intermediate 23 (410 mg, 78% yield).
LCMS (Method 2, ES⁺) 1.73 min, 618 & 620 *m*/*z* (M+H)⁺.

### INTERMEDIATE 24

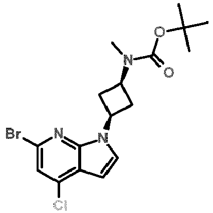

### Cis-tert-butyl N-[3-(6-bromo-4-chloro-pyrrolo[2,3-b]pyridin-1-yl)cyclobutyl]-N-methyl-carbamate

6-Bromo-4-chloro-1H-pyrrolo[2,3-b]pyridine (250 mg, 1.08 mmol, 100 mass%) and trans-tert-butyl N-(3-hydroxycyclobutyl)-N-methyl-carbamate (1.5 eq., 1.62 mmol) were reacted following General Procedure 9 to yield Intermediate 24 (430 mg, 96% yield).
LCMS (Method 2, ES⁺) 1.84 min, 414 & 416 *m*/*z* (M+H)⁺.

### INTERMEDIATE 25

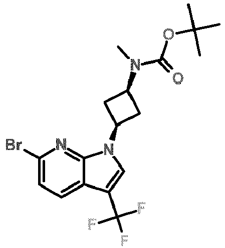

### Cis-tert-butyl N-[3-[6-bromo-3-(trifluoromethyl)pyrrolo[2,3-b]pyridin-1-yl]cyclobutyl]-N-methylcarbamate

6-Bromo-3-(trifluoromethyl)-1H-pyrrolo[2,3-b]pyridine (100 mg, 0.38 mmol) and trans-tert-butyl N-(3-hydroxycyclobutyl)-N-methyl-carbamate (1.5 eq., 0.6 mmol) were reacted following General Procedure 9 to yield intermediate 25 (160 mg, 86% yield).
LCMS (Method 2, ES⁺) 1.84 min, 448 & 450 *m*/*z* (M+H)⁺.

### INTERMEDIATE 26

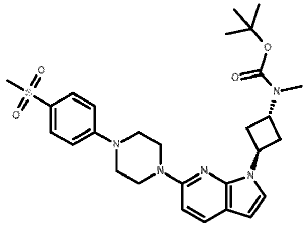

### Trans-tert-butyl N-methyl-N-[3-[6-[4-(4-methylsulfonylphenyl)piperazin-1-yl]pyrrolo[2,3-b]pyridin-1-yl]cyclobutyl]carbamate

6-[4-(4-methylsulfonylphenyl)piperazin-1-yl]-1H-pyrrolo[2,3-b]pyridine (Intermediate 18, 650 mg, 1.8 mmol) and cis-tert-butyl N-(3-hydroxycyclobutyl)-N-methyl-carbamate (1.5 eq., 2.7 mmol) were reacted following the General Procedure 9. The reaction mixture was concentrated and the residue was directly purified by flash chromatography gradient elution from hexane to EtOAc to afford Intermediate 26 (610 mg, 62% yield).
LCMS (Method 2, ES⁺) 1.61 min, 640 *m*/*z* (M+H)⁺.

### INTERMEDIATE 27

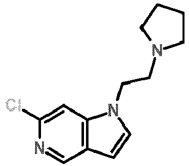

### 6-chloro-1-(2-pyrrolidin-1-ylethyl)pyrrolo[3,2-c]pyridine

6-Chloro-5-azaindole (750 mg, 4.8 mmol) and 1-(2-chloroethyl)pyrrolidine hydrochloride (1 g, 5.76 mmol) were reacted using general procedure 2 to give the title compound (860 mg, 71%).
LCMS (Method 2, ES⁺) 1.06 min, 250 *m*/*z* (M+H)⁺.

### INTERMEDIATE 28

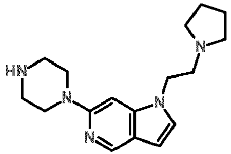

### 6-piperazin-1-yl-1-(2-pyrrolidin-1-ylethyl)pyrrolo[3,2-c]pyridine

Using general procedure 1 and intermediate 27 (250 mg, 1.00 mmol) the title compound was obtained (250 mg, 83%).
LCMS (Method 2, ES⁺) 0.94 min, 300 *m*/*z* (M+H)⁺.

### INTERMEDIATE 29-32

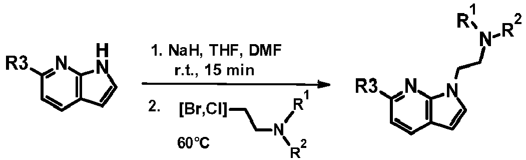

Intermediates 29-32 can be synthesized from commercially available 6-bromo-1H-pyrrolo[2,3-b]pyridine and the appropriate alkyl halide using General Procedure 2. Intermediates 29-32 analysed by LCMS Method 2.

| Interm. Number | Structure | Alkyl halide | Compound Name | LCMS (Method 2) RT (min) | *m*/*z* (M+H)⁺ |
|---|---|---|---|---|---|
| 29 | 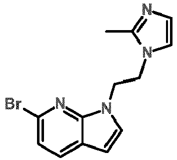 | 1-(2-Chloroethyl)-2-methyl-1H-imidazole hydroch loride | 6-bromo-1-[2-(2-methylimidazol-1-yl)ethyl]pyrrolo[2, 3-b]pyridine | 1.07 | 305 & 307 |
| 30 | 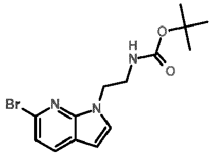 | N-Boc-2-chloethylamine | tert-butyl N-[2-(6-bromopyrrolo[2,3-b]pyridin-1-yl)ethyl]carbamat e | 1.40 | 340 & 242 |
| 31 | 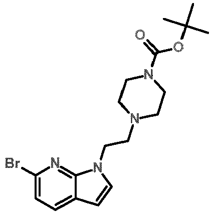 | tert-butyl 4-(2-bromoethyl)pip erazine-1-carboxylate | tert-butyl 4-[2-(6-bromopyrrolo[2,3-b]pyridin-1-yl)ethyl]piperazin e-1-carboxylate | 1.50 | 409 & 411 |
| 32 | 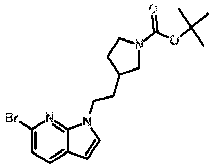 | tert-butyl 3-(2-bromoethyl)pyr rolidine-1-carboxylate | tert-butyl 3-[2-(6-bromopyrrolo[2,3-b]pyridin-1-yl)ethyl]pyrrolidin e-1-carboxylate | 1.54 | 338 & 340 [M-tBu+H] |

### INTERMEDIATE 33

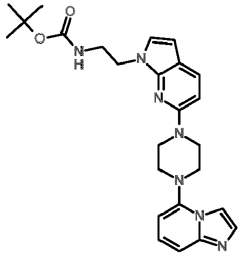

### tert-butyl N-[2-[6-(4-imidazo[1,2-a]pyridin-5-ylpiperazin-1-yl)pyrrolo[2,3-b]pyridin-1-yl]ethyl]carbamate

General Procedure 3, using intermediate 30 (384 mg, 1.13 mmol) and intermediate 5 (240 mg, 1.18 mmol) gave the title compound (252 mg, 48%).
LCMS (Method 2, ES⁺) 1.41 min, 462 *m*/*z* (M+H)⁺.

### INTERMEDIATE 34-42

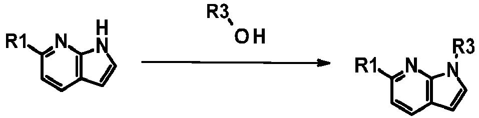

Intermediates 34-42 were synthesized according to General Procedure 9 from an appropriate heterocycle and alcohol tabulated below.

Intermediate 33 was purified by filtration through an SCX cartridge washing first with methanol then eluting with approx. 2 M ammonia in methanol. The ammonia in methanol solution was concentrated under reduced pressure to give the desired product.

Intermediates 34-42 were analysed by LCMS Method 2.

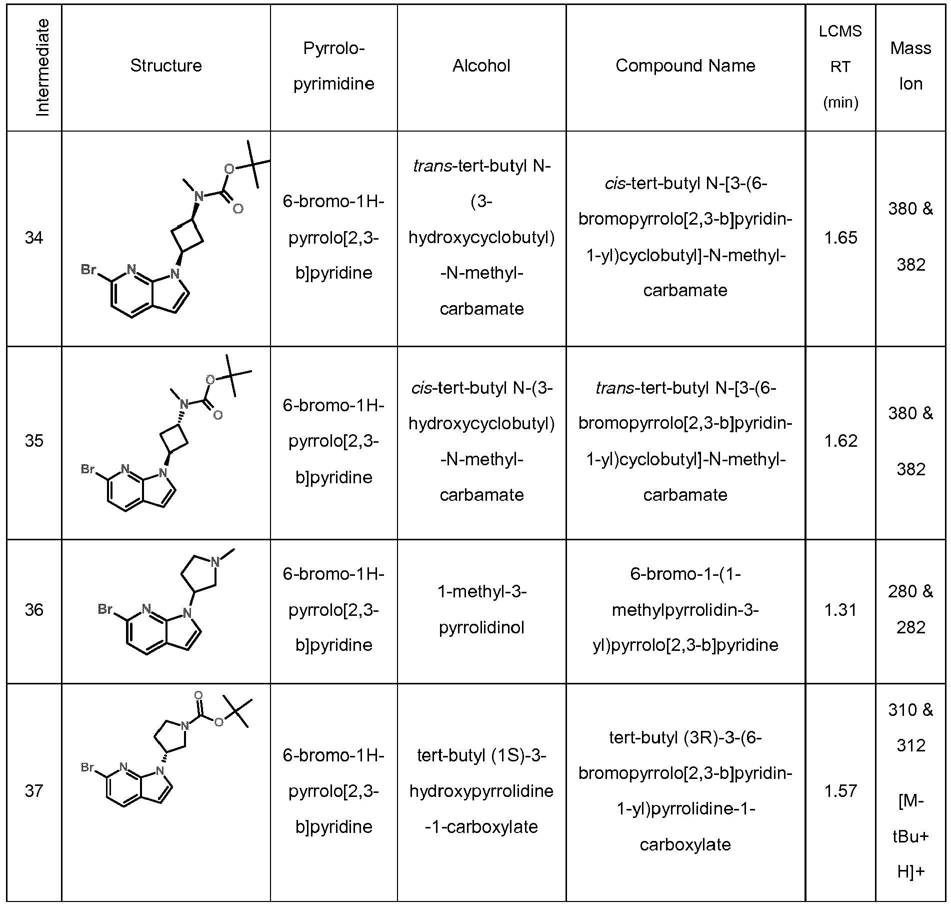

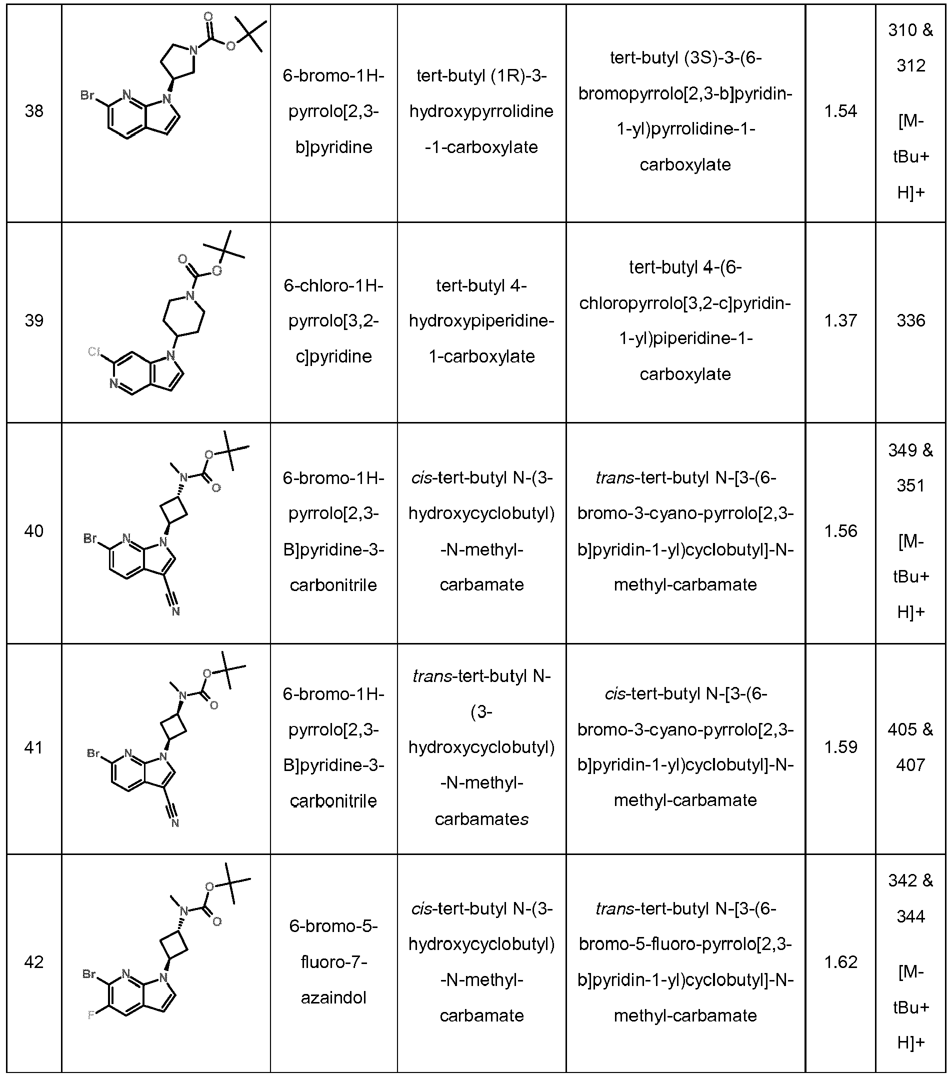

### INTERMEDIATE 43

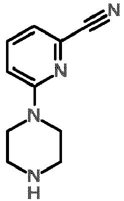

### 6-Piperazin-1-ylpyridine-2-carbonitrile

Intermediate 43 was prepared using General Procedure 1, from 6-bromo-2-pyridinecarbonitrile (500 mg, 2.7 mmol) and tert-butyl piperazine-1-carboxylate (610 mg, 3.2 mmol), followed by General Procedure 13 to give the title compound after reverse phase chromatography (120 mg, 23%)
LCMS (Method 2, ES⁺) 0.51 min, 189 m/z (M+H)⁺.

### INTERMEDIATE 44

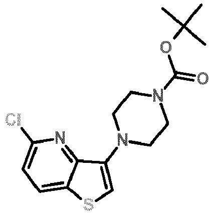

### tert-butyl 4-(5-Chlorothieno[3,2-b]pyridin-3-yl)piperazine-1-carboxylate

General Procedure 5, using 3-bromo-5-chlorothieno[3,2-B]pyridine (200 mg, 0.79 mmol) and 1-boc-piperazine (180 mg, 0.95 mmol) at 80 °C gave the title compound (24 mg, 9%).
LCMS (Method 2, ES⁺) 1.53 min, 354 & 356 m/z (M+H)⁺.

### INTERMEDIATE 45

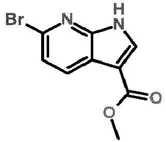

### Methyl 6-bromo-1H-pyrrolo[2,3-b]pyridine-3-carboxylate

6-bromo-1H-pyrrolo[2,3-b]pyridine (1 g, 5.1 mmol) was dissolved in DCM (40 mL) and AlCl₃ (3.5 equiv., 17.8 mmol) was added at r.t. portion-wise. The mixture was stirred for 30 minutes before trichloroacetyl chloride (1 equiv., 5.1 mmol) was added drop-wise. The mixture was stirred at r.t. for 2 hours. H₂O and DCM were added and the layers were separated. The solvent was removed under vacuum. The solid was dissolved in MeOH (20 mL) and KOH (200 mg, 3.6 mmol) was added. The mixture was stirred at 60°C for 3 hours until complete consumption of the starting material. Then the reaction was cooled to r.t. and EtOAc and HCI (2 M) aq. solution was added. The layers were separated and the organic layer was dried over Na₂SO₄. The volatiles were removed under vacuum and the residue purified by flash chromatography with gradient elution from hexane to EtOAc, and then 10% MeOH in EtOAc to yield Intermediate 45 (420 mg, 24% yield).
LCMS (Method 2, ES⁺) 1.01 min, 255 & 257 m/z.

### INTERMEDIATE 46

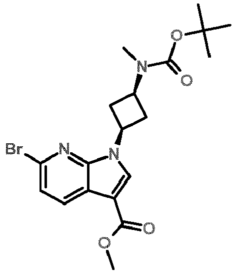

### cis-Methyl 6-bromo-1-[3-[tert-butoxycarbonyl(methyl)amino]cyclobutyl]pyrrolo[2,3-b]pyridine-3-carboxylate

Intermediate 45 (400 mg, 1.6 mmol) was reacted with trans-tert-butyl-N-(trans-3-hydroxycyclobutyl)-N-methylcarbamate (1.5 equiv., 2.3 mmol) following the General Procedure 9. The product was purified by flash column chromatography gradient elution from hexane to 70% EtOAc in hexane to yield Intermediate 46 (550 mg, 80% yield).
LCMS (Method 2, ES⁺) 1.66 min, 438 & 440 m/z (M+H)⁺.

### INTERMEDIATE 47

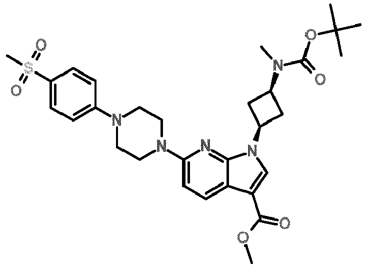

### cis-Methyl 1-[3-[tert-butoxycarbonyl(methyl)amino]cyclobutyl]-6-[4-(4-methylsulfonylphenyl) piperazin-1-yl]pyrrolo[2,3-b]pyridine-3-carboxylate

Intermediate 46 (315 mg, 0.7 mmol)and 1-[4-methylsulfonyl)phenyl]piperazine were reacted following the General Procedure 3 at 60 °C. The mixture was cooled to r.t. and H₂O and EtOAc added. The layers were separated and the aq. phase was extracted with EtOAc. The combined organic layers were dried over Na₂SO₄ and the volatiles were removed under vacuum. The residue was purified by flash column chromatography using gradient elution from hexane to EtOAc and then EtOAc to 30% of MeOH in EtOAc to afford Intermediate 47 (140 mg, 19% yield) and Intermediate 48.
LCMS (Method 2, ES⁺) 1.54 min, 598 *m*/*z* (M+H)⁺.

### INTERMEDIATE 48

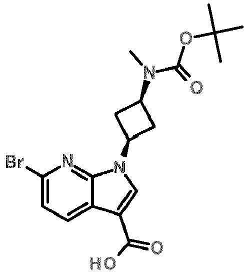

### cis-6-bromo-1-[3-[tert-butoxycarbonyl(methyl)amino]cyclobutyl]pyrrolo[2,3-b]pyridine-3-carboxylic acid

Intermediate 48 was obtained (180 mg, 34% yield) as a by-product from the synthesis of Intermediate 47.
LCMS (Method 2, ES⁺) 1.14 min, 424 & 426 m/z

### INTERMEDIATE 49

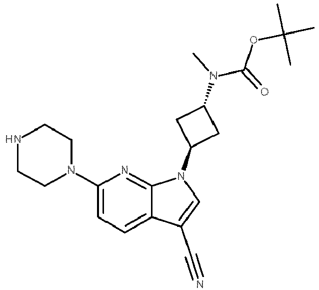

### trans-tert-butyl N-[3-(3-cyano-6-piperazin-1-yl-pyrrolo[2,3-b]pyridin-1-yl)cyclobutyl]-N-methylcarbamate

A solution of Intermediate 50 (3.4 g, 9.3 mmol) and piperazine (4.0 g, 46.0 mmol) was dissolved in a mixture of dimethyl sulfoxide (15 mL), 2-propanol (5 mL) and ethanol (3 mL). The mixture was heated at 120 °C overnight. After cooling to rt, the reaction mixture was diluted with diethyl ether (200 mL) and 50% saturated bicarbonate solution (200 mL). The organic layer was washed with water (100 mL) and brine (200 mL), dried (Na₂SO₄) and concentrated under reduced pressure to give an oil which solidified on standing to give the product as a yellow solid (3.4 g, 89%).
LCMS (Method 2, ES⁺) 1.23 min, 411 *m*/*z* (M+H)⁺

### INTERMEDIATE 50

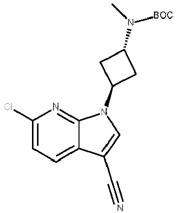

### trans-tert-butyl N-[3-(6-chloro-3-cyano-pyrrolo[2,3-b]pyridin-1-yl)cyclobutyl]-N-methyl-carbamate

6-chloro-1H-pyrrolo[2,3-b]pyridine-3-carbonitrile (2.0 g, 10.7 mmol) and cis-tert-butyl N-(3-hydroxycyclobutyl)-N-methyl-carbamate (2.6 g, 13.0 mmol) were used in General Procedure 9 to give the product (3.4 g, 9.3 mmol, 86%).
LCMS (Method 2, ES⁺) 1.46 min, 305 & 307 *m*/*z* (M-^{t}Bu+H)⁺

### INTERMEDIATE 51

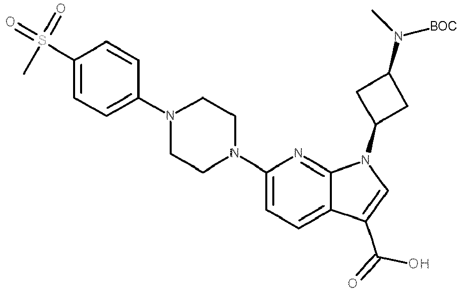

### trans-1-[3-[tert-butoxycarbonyl(methyl)amino]cyclobutyl]-6-[4-(4-methylsulfonylphenyl)piperazin-1-yl]pyrrolo[2,3-b]pyridine-3-carboxylic acid

General procedure 3 with Intermediate 46 and 1-[4-(methylsulfonyl)phenyl]piperazine gave the product in 15% yield.
LCMS (Method 2, ES⁺) 1.19 min, 584 *m*/*z* (M+H)⁺

### INTERMEDIATE 52

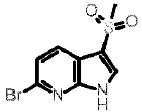

### 6-bromo-3-methylsulfonyl-1H-pyrrolo[2,3-b]pyridine

6-Bromo-1H-pyrrolo[2,3-b]pyridine (1 g, 5.1 mmol, 1 equiv.) was dissolved in THF (0.05 M, 100 mL), and cooled at -10 °C before potassium *tert*-butoxide (626 mg, 1.1 equiv., 5.6 mmol) was added. The reaction mixture was stirred at that temperature for 30 min before triethylborane (5.6 mL, 1.1 equiv., 5.6 mmol, 1 mol/L) was added. The reaction was stirred at that temperature for an additional 30 minutes. Then, methanesulfonyl chloride (0.45 mL, 1.2 equiv., 5.8 mmol) was added dropwise at -10°C and was stirred over the weekend at room temperature. The reaction was quenched by addition of NH₄Cl aq. sat solution and EtOAc. The layers were separated, and the aqueous phase was extracted again with EtOAc. The combined organic layers were dried over Na₂SO₄. The volatiles were removed under vacuum and the residue was purified from hexane to 1:1 hexane:EtOAc to give Intermediate 52 as a white solid (180 mg, 13% yield).
LCMS (Method 2, ES⁺) 0.86 min, 275 & 277 *m*/*z* (M+H)⁺

### INTERMEDIATE 53

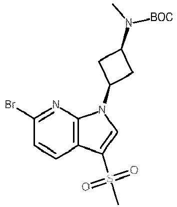

### cis-tert-butyl N-[3-(6-bromo-3-methylsulfonyl-pyrrolo[2,3-b]pyridin-1-yl)cyclobutyl]-N-methylcarbamate

Intermediate 52 (180 mg, 0.7 mmol) was reacted with trans-tert-butyl-N-(trans-3-hydroxycyclobutyl)-N-carbamate (208 mg, 1.5 equiv., 1 mmol) following General Procedure 9 to give Intermediate 53 as a yellowish oil (220 mg, 69% yield).
LCMS (Method 2, ES⁺) 1.50 min, 458 & 460 *m*/*z* (M+H)⁺.

### INTERMEDIATE 54

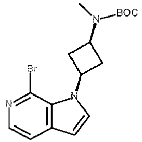

### cis-tert-butyl N-[3-(7-bromopyrrolo[2,3-c]pyridin-1-yl)cyclobutyl]-N-methyl-carbamate

7-Bromo-1H-pyrrolo[2,3-c]pyridine (200 mg, 1 mmol) and trans-tert-butyl-N-(trans-3-hydroxycyclobutyl)-N-methylcarbamate (306 mg, 1.5 equiv., 1.45 mmol) were reacted following General Procedure 9 to give Intermediate 54 as a yellow syrup (330 mg, 90% yield).
LCMS (Method 2, ES⁺) 1.45 min, 380 & 382 *m*/*z* (M+H)⁺.

### INTERMEDIATE 55

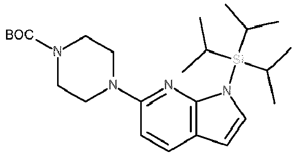

### tert-butyl 4-(1-triisopropylsilylpyrrolo[2,3-b]pyridin-6-yl)piperazine-1-carboxylate

Intermediate 8 (8.5 g, 24 mmol), 1-boc-piperazine (1.8 equiv., 43 mmol), sodium tert-butoxide (3 equiv., 72 mmol) and (2-dicyclohexylphosphino-2',6'-diisopropoxy-1,1'-biphenyl)[2-(2'-amino-1,1'-biphenyl)]palladium(II) methanesulfonate (0.1 equiv., 2.4 mmol) were dissolved in degassed 1,4-dioxane (0.5 M). The mixture was heated to 80°C overnight and then cooled down to ambient temperature. EtOAc and H₂O were added and the layers were separated. The aqueous phase was then further extracted with EtOAc. The combined organic layers were dried over Na₂SO₄ and the solvent was removed under vacuum. The residue was purified by column chromatography from DCM to 20% MeOH in DCM to afford Intermediate 55 as yellowish solid (11 g, 94% yield).
LCMS (Method 2, ES⁺) 2.12 min, 459 *m*/*z* (M+H)⁺.

### INTERMEDIATE 56

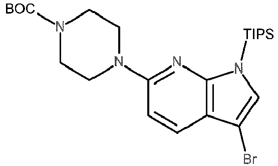

### tert-butyl 4-(3-bromo-1-triisopropylsilyl-pyrrolo[2,3-b]pyridin-6-yl)piperazine-1-carboxylate

Intermediate 55 (4 g, 8.7 mmol) was dissolved in DCM (30 mL) and cooled to 0 °C. NBS (1.1 g, 0.75 equiv.) was added portionwise over 30 minutes. The reaction mixture was stirred in the presence of the cooling bath for an additional 10 mins before it was quenched with saturated bicarb (20 mL) and diluted with DCM (20 mL). The aqueous layer was extracted with DCM, the combined organic layer was dried over Na₂SO₄ and concentrated under reduced pressure. The residue was purified from hexane to EtOAc affording a syrup (4.1 g, 87% yield).
LCMS (Method 2, ES⁺) 2.27 min, 537 & 539 *m*/*z* (M+H)⁺.

### INTERMEDIATE 57

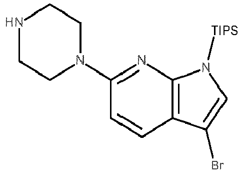

### (3-bromo-6-piperazin-1-yl-pyrrolo[2,3-b]pyridin-1-yl)-triisopropyl-silane

Intermediate 56 (800 mg, 1.5 mmol) was reacted following the General Procedure 13 to give Intermediate 57 (420 mg, 65% yield).
LCMS (Method 2, ES⁺) 1.63 min, 437 & 439 *m*/*z* (M+H)⁺.

### INTERMEDIATE 58

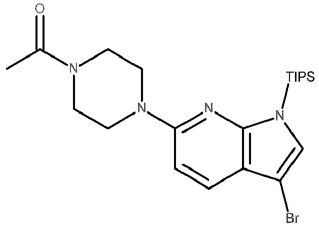

### 1-[4-(3-bromo-1-triisopropylsilyl-pyrrolo[2,3-b]pyridin-6-yl)piperazin-1-yl]ethenone

Intermediate 57 (300 mg, 0.7 mmol) were reacted with acetic acid (1.5 equiv., 1 mmol) analogously to General Procedure 6. H₂O was added and the layers were separated. The aq. phase was washed again with DCM. The combined organic layers were dried under vacuum and the residue was purified by flash chromatography from hexane to EtOAc to give Intermediate 58 as a white solid (220 mg, 67% yield)
LCMS (Method 2, ES⁺) 1.88 min, 479 & 481 *m*/*z* (M+H)⁺.

### INTERMEDIATE 59

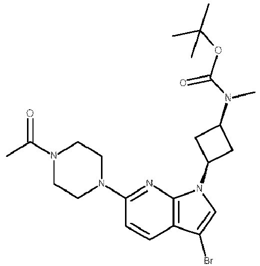

### trans-tert-butvl N-[3-[6-(4-acetylpiperazin-1-yl)-3-bromo-pyrrolo[2,3-b]pyridin-1-yl]cyclobutyl]-N-methyl-carbamate

Intermediate 58 (200 mg, 0.42 mmol) was dissolved in THF (0.1 M) and TBAF (1.3 equiv., 0.5 mmol, 1 mmol/mL in THF) was added. The mixture was stirred at ambient temperature for 30 minutes until starting material was consumed. EtOAc and H₂O were added to the reaction mixture and the layers were separated. The organic phase was dried over Na₂SO₄ and the solvent was removed under vacuum. The residue was then reacted with tert-butyl-N-(3-hydroxycyclobutyl)-N-methyl-carbamate following General Procedure 9 to give Intermediate 59 as a brownish solid (100 mg, 47% yield over two steps).
LCMS (Method 2, ES⁺) 1.54 min, 506 & 508 *m*/*z* (M+H)⁺.

### INTERMEDIATE 60

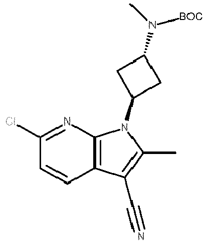

### trans-tert-butyl N-[3-(6-chloro-3-cyano-2-methyl-pyrrolo[2,3-b]pyridin-1-yl)cyclobutyl]-N-methyl-carbamate

6-chloro-2-methyl-1H-pyrrolo[2,3-b]pyridine was reacted with cis-tert-butyl N-(3-hydroxycyclobutyl)-N-methyl-carbamate using General Procedure 9. The resulting trans-tert-butyl N-[3-(6-chloro-2-methyl-pyrrolo[2,3-b]pyridin-1-yl)cyclobutyl]-N-methyl-carbamate was dissolved in 1:1 MeCN/DMF, cooled to 0 °C, then chlorosulphonyl isocyanate (5 equiv) was added. The mixture was stirred at 40 °C for 2 hours. After cooling to rt, the mixture was diluted with water, extracted with EtOAc (2 x), washed with brine, dried (Na₂SO₄) and concentrated in vacuo. The residue was then purified by column chromatography (10% EtOAc/hexane) to give the product (35% over 2 steps).
LCMS (Method 7, ES⁺) 2.34 min, 319 & 321 *m*/*z* (M-^{t}Bu+H)⁺

### INTERMEDIATE 61

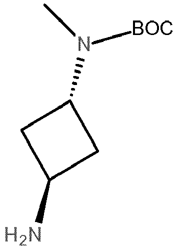

### trans-tert-butvl N-(3-aminocyclobutyl)-N-methyl-carbamate

To a stirred solution of cis-tert-butyl N-(3-hydroxycyclobutyl)-N-methyl-carbamate (3.00 g, 14.9 mmol) in DCM (50 mL) was added triethylamine (5.02 mL, 44.7 mmol) and methanesulfonyl chloride (1.50 mL, 19.4 mmol) at 0°C, and maintained for 30 min for 0°C. After complete addition, the resulting reaction mixture was stirred at rt for 1 h. After complete consumption of starting material, reaction mixture was quenched by ice cold water (100 mL). Aqueous layer extracted with DCM (100 mL x 2). The collected organic layer was dried over sodium sulphate and evaporated under reduced pressure to give crude cis-[3-[tert-butoxycarbonyl(methyl)amino]cyclobutyl] methanesulfonate (crude weight: 4.2 g).

To a stirred solution of cis-[3-[tert-butoxycarbonyl(methyl)amino]cyclobutyl] methanesulfonate (4.20 g, 14.2 mmol) in DMF (30 mL), was added NaN₃ (4.61 g, 70.9 mmol) at 0 °C. After complete addition, the resulting reaction mixture was stirred at 70 °C for 16 h. After complete consumption of starting material, the reaction mixture was quenched by ice cold water (100 mL) and extracted with ethyl acetate (100 mL x 2). The organic layer washed with saturated sodium bicarbonate solution (100 mL), and brine (100 mL), the organic layer was collected and dried over sodium sulphate, then filtered and evaporated under reduced pressure to get crude trans-tert-butyl N-(3-azidocyclobutyl)-N-methyl-carbamate (crude weight: 4.3 g)

To a stirred solution of trans-tert-butyl N-(3-azidocyclobutyl)-N-methyl-carbamate (4.30 g, 17.6 mmol) in methanolic ammonia (30 mL) was added 20% palladium on carbon (1 g). The reaction mixture was stirred under hydrogen atmosphere for 36 hour at room temperature. After complete consumption of starting material, the reaction mixture was filtered on celite and washed with methanol (100 mL). The filtrate was concentrated under reduced pressure and purified by column chromatography (15% methanol in DCM) to obtain the product (2.0 g, 9.57 mmol, 64 % over 3 steps).
LCMS (Method 7, ES⁺) 1.36 min, 201 *m*/*z* (M+H)⁺

### INTERMEDIATE 62

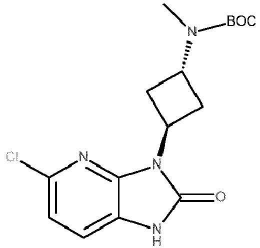

### trans-tert-butyl N-[3-(5-chloro-2-oxo-1H-imidazo[4,5-b]pyridin-3-yl)cyclobutyl]-N-methyl-carbamate

To a stirred solution of 2,6-dichloro-3-nitro-pyridine (1.00 g, 5.18 mmol) in ethanol (20 mL) was added Intermediate 61 (1.30 g, 6.22 mmol) followed by sodium carbonate (1.63 g, 15.5 mmol) at rt. The mixture was stirred at rt for 16 hours, then diluted with water (50 mL) and extracted with EtOAc (50 mL x 2). The collected organic layer was washed with brine (50 mL), dried (Na2SO4) and concentrated under reduced pressure. The crude compound was purified by column chromatography (1:1 EtOAc/hexane) to give trans-tert-butyl N-[3-[(6-chloro-3-nitro-2-pyridyl)amino]cyclobutyl]-N-methyl-carbamate (1.30 g, 3.61 mmol, 70%).
trans-tert-butyl N-[3-[(6-chloro-3-nitro-2-pyridyl)amino]cyclobutyl]-N-methyl-carbamate (1.30 g, 3.61 mmol) was submitted to General Procedure 15, followed by General Procedure 16 to give the product trans-tert-butyl N-[3-(5-chloro-2-oxo-1H-imidazo[4,5-b]pyridin-3-yl)cyclobutyl]-N-methyl-carbamate (0.65 g, 1.84 mmol, 51% over two steps).
LCMS (Method 8, ES⁺) 1.87 min, 297 & 299 *m*/*z* (M-^{t}Bu+H)⁺

### INTERMEDIATE 63

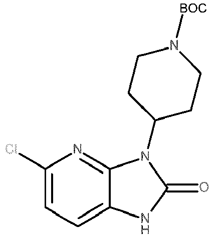

### tert-butyl 4-(5-chloro-2-oxo-1H-imidazo[4,5-b]pyridin-3-yl)piperidine-1-carboxylate

To a stirred solution of 2,6-dichloro-3-nitro-pyridine (5.00 g, 25.9 mmol) in ethanol (50 mL) was added tert-butyl 4-aminopiperidine-1-carboxylate (7.78 g, 38.9 mmol) followed by sodium carbonate (8.16 g, 77.7 mmol) at rt. The mixture was stirred at rt for 16 hours then diluted with ethyl acetate (300 mL) and washed with water (300 mL x 2). The organic layer was separated, washed with brine (500 mL), dried over sodium sulfate and concentrated under reduced pressure. The crude compound was purified by column chromatography (70% EtOAc in hexane) to give tert-butyl 4-[(6-chloro-3-nitro-2-pyridyl)amino]piperidine-1-carboxylate (6.50 g, 18.2 mmol, 70%). tert-butyl 4-[(6-chloro-3-nitro-2-pyridyl)amino]piperidine-1-carboxylate (4.0 g, 11.2 mmol) was submitted to General Procedure 15, followed by General Procedure 16 to give the product tert-butyl 4-(5-chloro-2-oxo-1H-imidazo[4,5-b]pyridin-3-yl)piperidine-1-carboxylate (1.0 g, 2.71 mmol, 24% over two steps).
LCMS (Method 7, ES⁺) 1.85 min, 297 & 299 [55%] *m*/*z* (M-^{t}Bu+H)⁺, 253 & 255 [100%] *m*/*z* (M-^{t}BuCO₂+H)⁺

### INTERMEDIATE 64

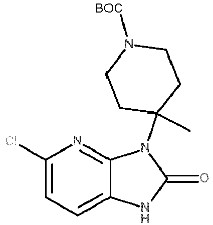

### tert-butyl 4-(5-chloro-2-oxo-1H-imidazo[4,5-b]pyridin-3-yl)-4-methyl-piperidine-1-carboxylate

Sodium carbonate (3.26 g, 31.1 mmol) and tert-butyl 4-amino-4-methyl-piperidine-1-carboxylate (2.67 g, 12.4 mmol) were added to a stirred solution of 2,6-dichloro-3-nitro-pyridine (2 g, 10.4 mmol) in ethanol (35 mL) at rt under argon. The resulting reaction mixture was heated at 70 °C for 24 h. The reaction mixture was diluted with ethyl acetate (100 mL) and washed with water (100 mL X 2). The organic layer was separated, washed with brine (100 mL), dried over sodium sulfate and concentrated under reduced pressure. The crude material was purified by column chromatography using 50% ethyl acetate in hexane to give tert-butyl 4-[(6-chloro-3-nitro-2-pyridyl)amino]-4-methyl-piperidine-1-carboxylate (2.50 g, 5.76 mmol, 56%).
tert-butyl 4-[(6-chloro-3-nitro-2-pyridyl)amino]-4-methyl-piperidine-1-carboxylate (2.50 g, 5.76 mmol) was submitted to General Procedure 15 then General Procedure 16 to give tert-butyl 4-(5-chloro-2-oxo-1H-imidazo[4,5-b]pyridin-3-yl)-4-methyl-piperidine-1-carboxylate (0.70 g, 1.84 mmol, 32% over 2 steps).
LCMS (Method 7, ES⁺) 2.07 min, 311 & 313 *m*/*z* (M-^{t}Bu+H)⁺

### INTERMEDIATE 65

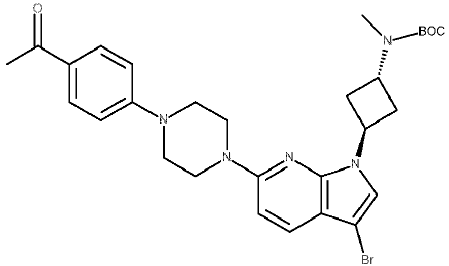

### trans-tert-butyl N-[3-[6-[4-(4-acetylphenyl)piperazin-1-yl]-3-bromo-pyrrolo[2,3-b]pyridin-1-yl]cyclobutyl]-N-methyl-carbamate

3-Bromo-6-chloro-7-azaindole (4.0 g, 16.8 mmol) and cis-tert-butyl N-(3-hydroxycyclobutyl)-N-methyl-carbamate (4.1 g, 20.1 mmol) were reacted according to General Procedure 9 to give trans- tert-butyl N-[3-(3-bromo-6-chloro-pyrrolo[2,3-b]pyridin-1-yl)cyclobutyl]-N-methylcarbamate (7.6 g, quant).
tert-butyl N-[3-(3-bromo-6-chloro-pyrrolo[2,3-b]pyridin-1-yl)cyclobutyl]-N-methyl-carbamate (3.0 g, 7.2 mmol) and 1-(4-acetylphenyl)piperazine (7.4 g, 36 mmol) were reacted according to General Procedure 14 to give 0.6 g product (14%).
LCMS (Method 2, ES⁺) 1.52 min, 582 & 584 m/z (M+H)⁺.

### INTERMEDIATE 66

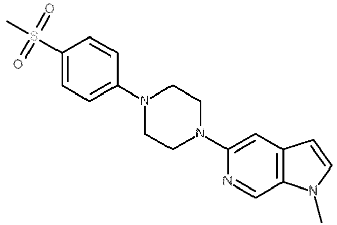

### 1-methyl-5-[4-(4-methylsulfonylphenyl)piperazin-1-yl]pyrrolo[2,3-c]pyridine

A solution of 5-bromo-1h-pyrrolo[2,3-c]pyridine (2.5 g, 13 mmol) in tetrahydrofuran (25 mL) was degassed (3 x evacuate for 5 minutes then backfill with N2) then cooled with an ice/brine bath. sodium hydride (760 mg, 19 mmol) was then added in three portions and the mixture stirred for 30 minutes. Iodomethane (1.04 mL, 16.5 mmol) was then added, the mixture stirred for 5 minutes then removed from the ice bath and allowed to warm to rt. After 1 hour, the reaction was quenched with water (30 ml) and the product was extracted with EtOAc (2 x 30 ml). The combined organics were washed with brine (20 ml), dried (Na₂SO₄) and concentrated in vacuo. The product was then purified by column chromatography to give 5-bromo-1-methyl-1h-pyrrolo[2,3-c]pyridine (2.5 g, 11.3 mmol, 89%) as an orange oil which crystallised on standing.
5-Bromo-1-methyl-1h-pyrrolo[2,3-c]pyridine (1.0 g, 4.5 mmol) was reacted with 1-[4-(methylsulfonyl)phenyl]piperazine (1.2 g, 5.0 mmol) according to General Procedure 5 to give the product (1.3 g, 3.6 mmol, 80%).
LCMS (Method 2, ES⁺) 1.12 min, 371 m/z (M+H)⁺.

### INTERMEDIATES 67-69

Intermediates 67-69 can be synthesised from commercially available 6-bromo-1H-pyrrolo[2,3-b]pyridine and the appropriate alkyl halide using General Procedure 2

| Interm. Number | Structure | Alkyl halide | Compound Name | LCMS (Method 2) RT (min) | *m*/*z* (M+H)⁺ |
|---|---|---|---|---|---|
| 67 | 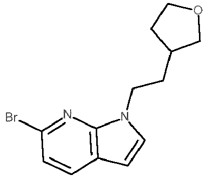 | 3-(2-bromoethyl)tetr ahydrofuran | 6-bromo-1-(2-tetrahydrofuran-3-ylethyl)pyrrolo[2,3 -b]pyridine | 1.29 | 295 & 297 |
| 68 | 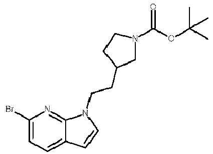 | tert-butyl 3-(2-bromoethyl)pyr rolidine-1-carboxylate | tert-butyl 3-[2-(6-bromopyrrolo[2,3-b]pyridin-1-yl)ethyl]pyrrolidin e-1-carboxylate | 1.54 | 338 & 340 |
| 69 | 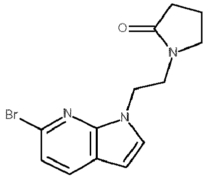 | 1-(2-bromoethyl)pyr rolidin-2-one | 1-[2-(6-bromopyrrolo[2,3-b]pyridin-1-yl)ethyl]pyrrolidin-2-one | 1.07 | 308 & 310 |

### INTERMEDIATE 70

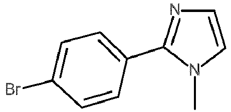

### 2-(4-bromophenyl)-1-methyl-imidazole

2-(4-Bromo-phenyl)-1H-imidazole (215 mg, 0.94 mmol) was dissolved in a mixture of tetrahydrofuran (5 mL) and N,N-dimethylformamide (1 mL). The solution was cooled to 0 °C and sodium hydride (45 mg, 1.12 mmol, 60 mass%) added. After approx. 5 mins iodomethane (0.1 mL, 2 mmol) was added and the reaction mixture (suspension) stirred at RT for approx. 3 hours. The suspension was cooled to 0 °C and water added. The clear solution was stirred in the presence of the cooling bath for 1 h then diluted with 50% saturated ammonium chloride (20 mL) and diethyl ether (20 mL). The organic layer was washed with water (2 x 20 mL), dried (Na₂SO₄) and concentrated under reduced pressure to give the title intermediate (215 mg, 96%).
LCMS (Method 2, ES⁺) 1.02 min, 239 *m*/*z* (M+H)⁺.

### INTERMEDIATE 71

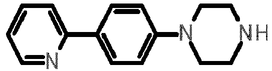

### 1-[4-(2-pyridyl)phenyl]piperazine

2-(4-Bromophenyl)pyridine (529 mg, 2.15 mmol) and (S)-4-N-boc-2-methylpiperazine (400 mg, 2.15 mmol) were used in general procedure 3 to afford tert-butyl 4-[4-(2-pyridyl)phenyl]piperazine-1-carboxylate (736 mg, quantitative). tert-Butyl 4-[4-(2-pyridyl)phenyl]piperazine-1-carboxylate (185 mg, 0.54 mmol) was then used in procedure 13 to afford the title intermediate (139 mg, quantitative)
LCMS (Method 2, ES⁺) 0.84 min, 240 *m*/*z* (M+H)⁺.

### INTERMEDIATES 72-73

Intermediates 72-73 were synthesized according to General Procedure 9 from an appropriate heterocycle and alcohol tabulated below and analysed by LCMS Method 5.

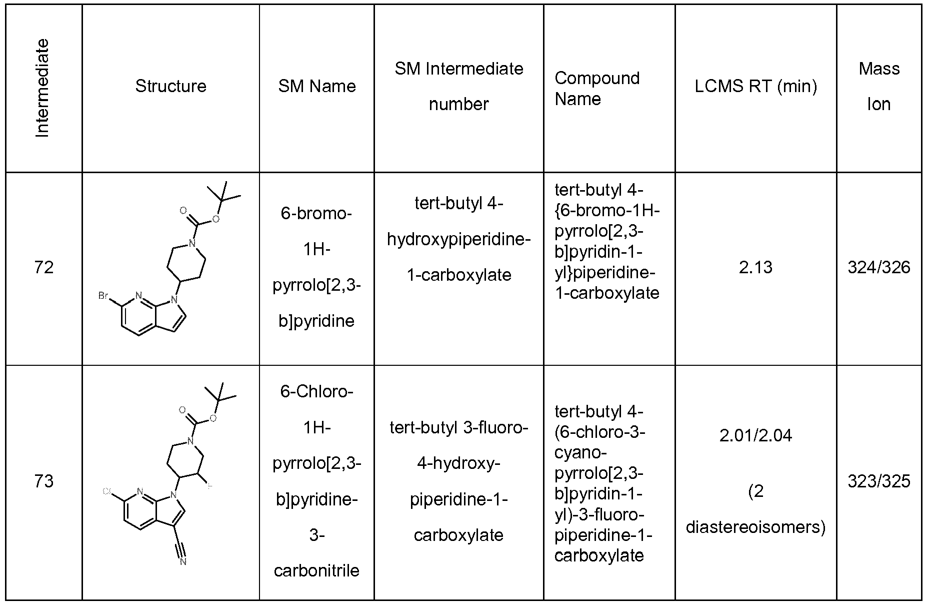

### INTERMEDIATES 74-77

Intermediates 74-77 were prepared according to General Procedure 19 and LCMS method 5. Except for intermiedate 76 which was analyszed using LCMS method 10.

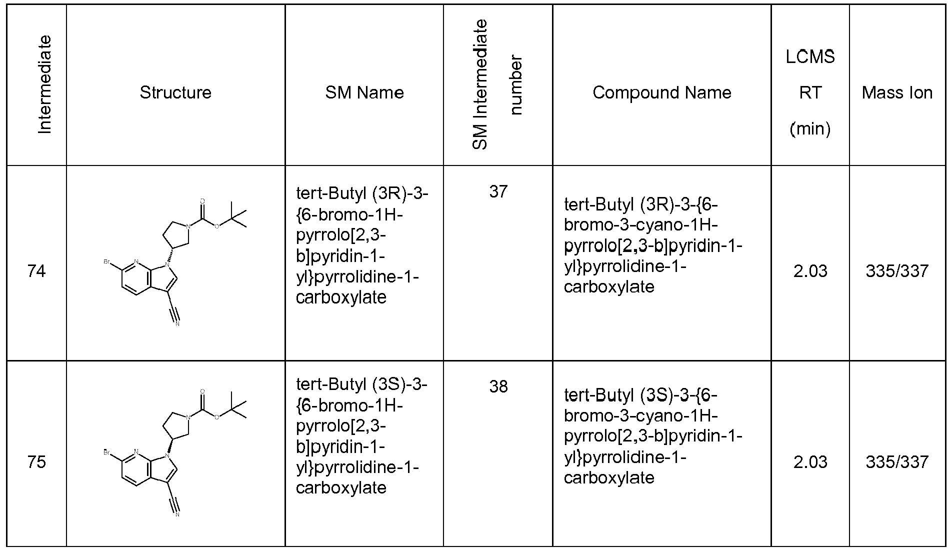

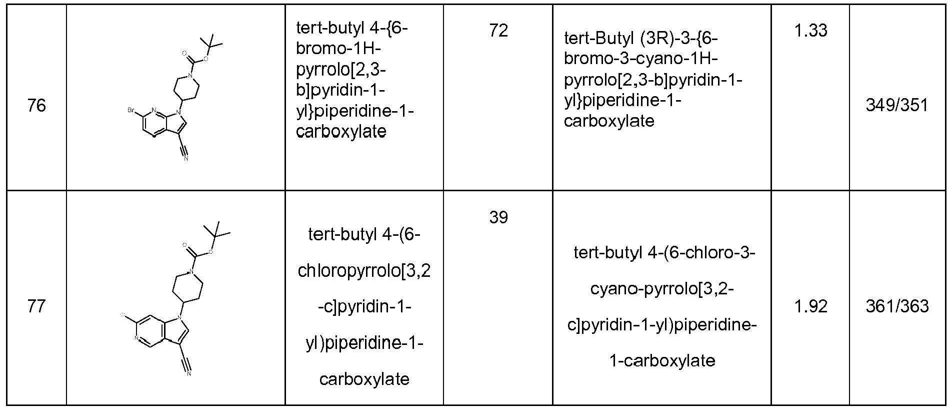

### INTERMEDIATES 78-81

Intermediates 78-81 were prepared via general procedure 14 followed by gerenal procedure 13, from 1-(4-fluorophenyl)ethanone and the appropriate N-Boc piperazine. The intermediates were analysed by LCMS method 6.

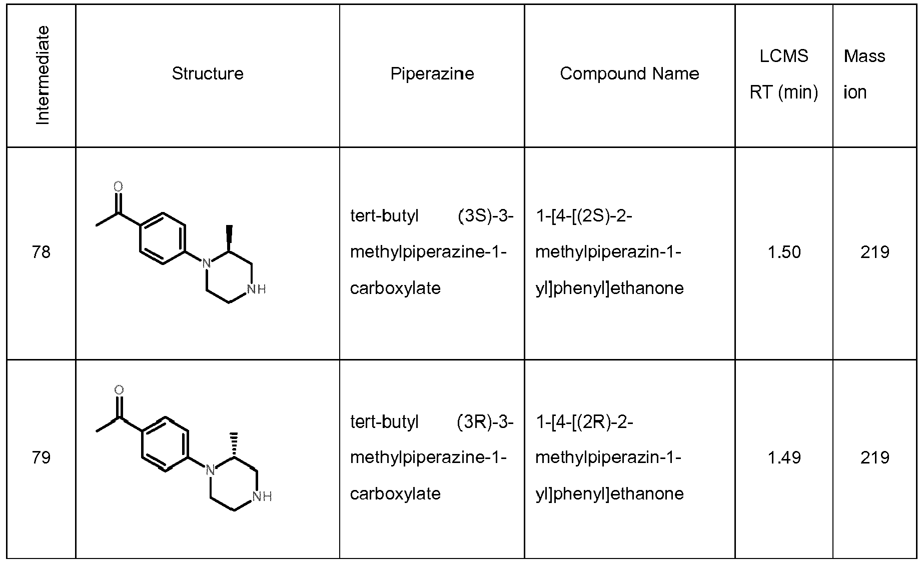

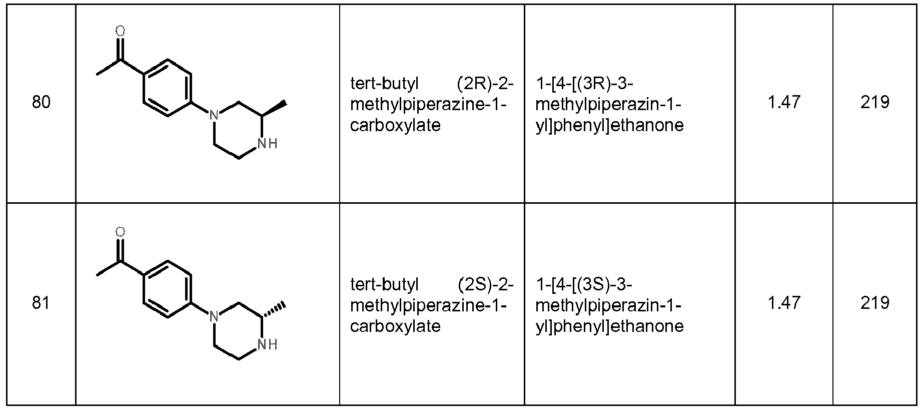

### INTERMEDIATES 82-85

Intermediates 82-85 were synthesized from 6-Chloro-1H-pyrrolo[2,3-b]pyridine, according to General Procedure 9 from an appropriate heterocycle and alcohol tabulated below and analysed by LCMS Method 5 except for intermediate 85 which was analysed by LCMS Method 12

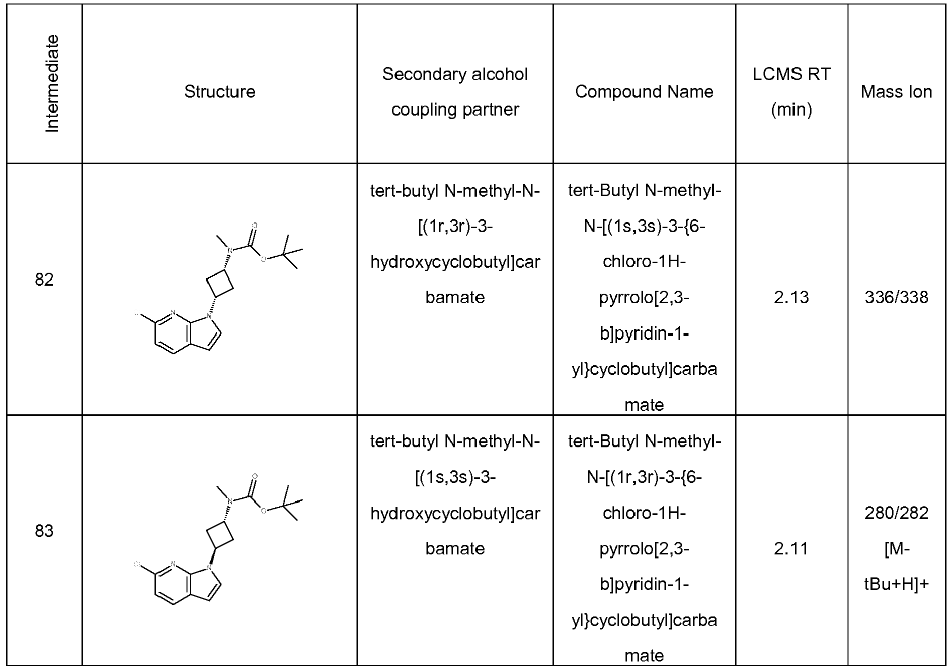

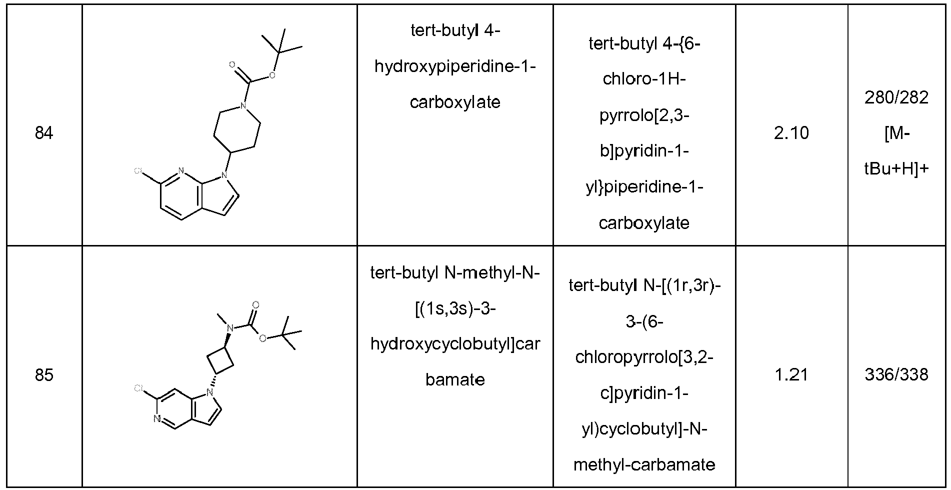

### INTERMEDIATES 86-89

Intermediates 86-89 were prepared from the appropriate aza-indole according to general procedures 20.

Intermediates 86 & 87 were analysed using LCMS method 5

Intermediates 88 & 89 were analysed using LCMS method 10

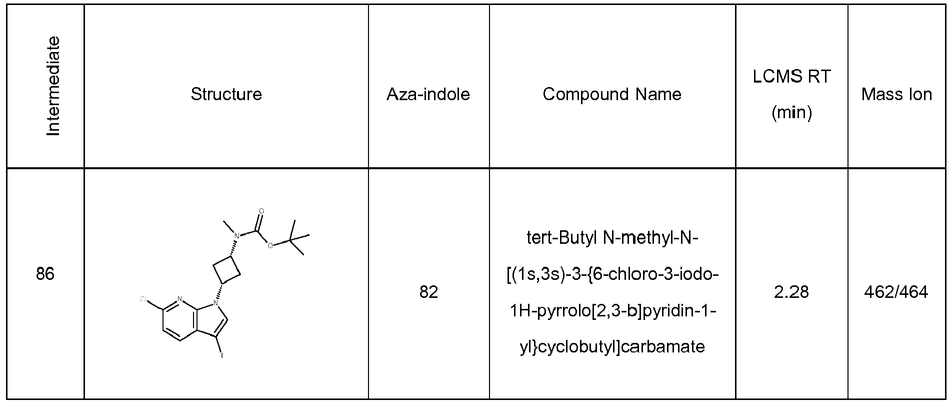

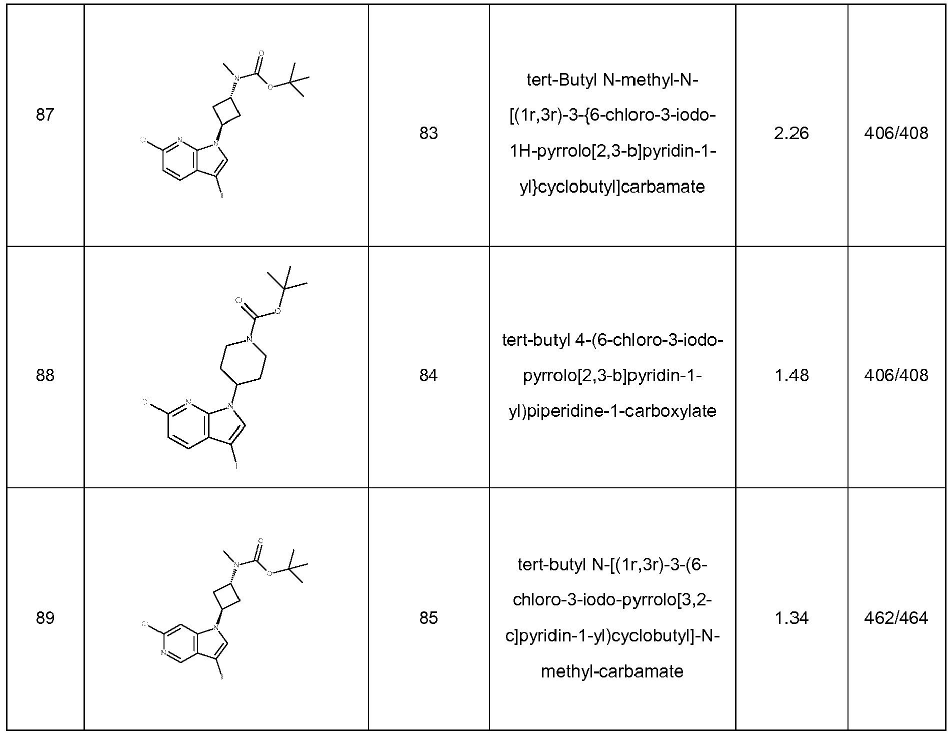

### INTERMEDIATES 90-95

Intermediates 90-95 were prepared from the corresponding iodo aryl intermeidate and commercial boronic ester / acid according to general procedures 21.

Intermediates 90 & 93-95 were analysed by LCMS method 10.

Intermediates 91-92 were analysed by LCMS method 5.

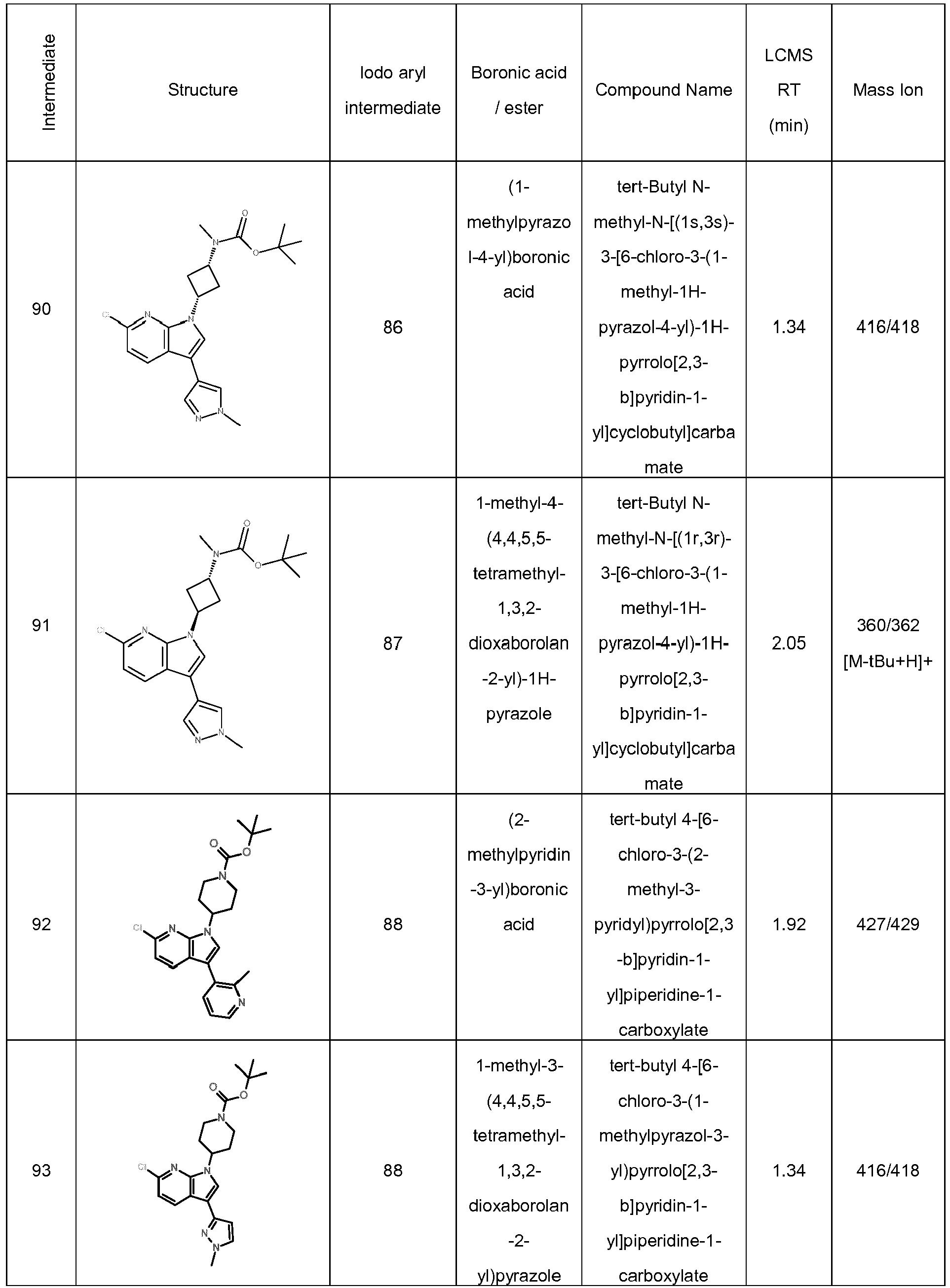

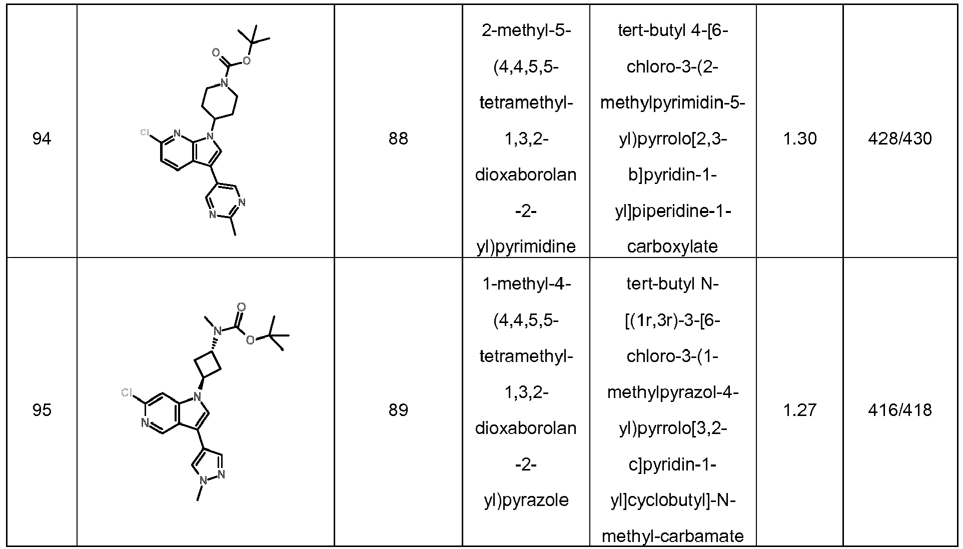

### INTERMEDIATE 96

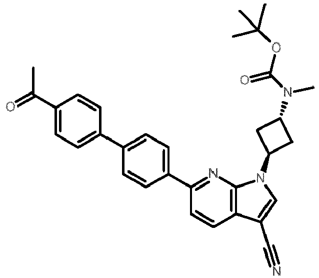

### tert-butyl N-methyl-N-[(1r,3r)-3-(6-{4'-acetyl-[1,1'-biphenyl]-4-yl}-3-cyano-1H-pyrrolo[2,3-b]pyridin-1-yl)cyclobutyl]carbamate

The mixture of tert-butyl N-[3-(6-chloro-3-cyano-pyrrolo[2,3-b]pyridin-1-yl)cyclobutyl]-N-methylcarbamate (Intermediate 50) (84 mg, 0.23 mmol), [4-(4-acetylphenyl)phenyl]boronic acid (50.0 mg, 0.21 mmol) and disodium;carbonate (45 mg, 0.42 mmol) in 1,4-dioxane (3 mL) and was degassed by flushing with N₂ and then 1,1'-bis(diphenylphosphino)ferrocene palladium(II)chloride dichloromethane complex (17.4 mg, 0.02 mmol) was added.. The reaction mixture was stirred at 100 °C overnight and then allowed to cool to RT, diluted with DCM (5 mL) and filtered through celite. The solids were washed with DCM (2 x 5 mL) and the combined filtrates were concentrated to dryness under vacuum. The residue obtained was purified by FCC (wet loaded in DCM to Biotage SNAP Ultra 10g cartridge, eluting 5% to 49% EtOAc in heptane) to yield 120 mg (84%) of the title compound as an off-white solid.
LCMS (Method 10, ES+) 1.49 min, 521 *m*/*z* [M+H]⁺

### INTERMEDIATE 97

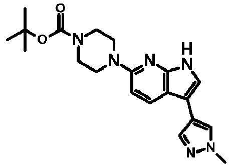

### tert-butyl 4-[3-(1-methyl-1H-pyrazol-4-yl)-1H-pyrrolo[2,3-b]pyridin-6-yl]piperazine-1-carboxylate

A mixture of tert-butyl 4-{3-bromo-1-[tris(propan-2-yl)silyl]-1H-pyrrolo[2,3-b]pyridin-6-yl}piperazine-1-carboxylate (2.2 g, 4.09 mmol), intermediate 56 (936.59 mg, 4.5 mmol) (and 2M sodium carbonate (2M aq) (6.14 mL) in dioxane (12 mL) was degassed by N₂ bubbling for 10 min then Tetrakis(triphenylphosphine)palladium(0) (472. mg, 0.41 mmol) was added and reaction was heated at 100°C for 16h. The reaction mixture was diluted in EtOAc and washed successively with water and brine. The aqueous phase was extracted with EtOAc (3 x 30mL). The combined organic phase was dried over Na₂SO₄ and concentrated to dryness in vacuo. Purification by FCC (Biotage Isolera, 100g SNAP KP-Sil, gradient elution 10-100% EtOAc:Heptanes, then 0-20% MeOH:EtOAc) gave the title compound 440 mg (27.3%) as a yellow solid.
LCMS (Method 12, ES+) 1.32 min, 383 *m*/*z* [M+H]⁺

### INTERMEDIATE 98

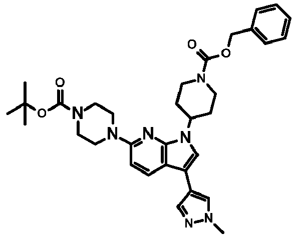

### tert-butyl 4-(1-{1-[(benzyloxy)carbonyl]piperidin-4-yl}-3-(1-methyl-1H-pyrazol-4-yl)-1H-pyrrolo[2,3-b]pyridin-6-yl)piperazine-1-carboxylate

The title compound was prepared from Intermediate_97 and benzyl 4-hydroxypiperidine-1-carboxylate via Mitsunobu, according to General Procedure 9.
LCMS (Method 10, ES+) 1.41min, 600 *m*/*z* [M+H]⁺

### INTERMEDIATE 99

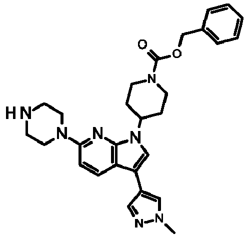

### benzyl 4-[3-(1-methyl-1H-pyrazol-4-yl)-6-(piperazin-1-yl)-1H-pyrrolo[2,3-b]pyridin-1-yl]piperidine-1-carboxylate

The title compound was prepared from Intermediate 98 according to General Procedure 13.
LCMS (method 5, ES+) 1.69 min, 500 *m*/*z* [M+H]⁺

### INTERMEDIATE 100

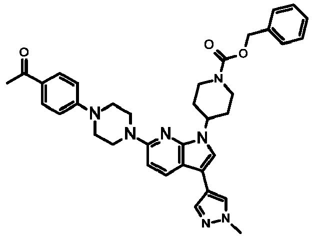

### benzyl 4-[6-[4-(4-acetylphenyl)piperazin-1-yl]-3-(1-methylpyrazol-4-yl)pyrrolo[2,3-b]pyridin-1-yl]piperidine-1-carboxylate

The title compound was prepared from Intermediate 99 and 1-(4-bromophenyl)ethenone according to General Procedure 14.
LCMS (method 10, ES+) 1.38 min, 618 *m*/*z* [M+H]⁺

**INTERMEDIATE 101**

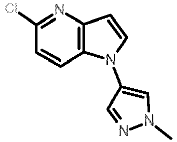

### 5-chloro-1-(1-methylpyrazol-4-yl)pyrrolo[3,2-b]pyridine

The mixture of 5-chloro-1H-pyrrolo[3,2-b]pyridine (350 mg, 2.29 mmol), 4-bromo-1-methyl-1H-pyrazole (480 mg, 2.98 mmol), copper(1+) iodide (87 mg, 0.46 mmol), quinolin-8-ol (67 mg, 0.46 mmol) and dipotassium carbonate (476 mg, 3.44 mmol) was suspended in DMSO (6 mL) and was stirred at 130 °C for 16 hours under microwave irradiation. The reaction mixture was coolled to r.t and diluted with 10% aqueous ammonium hydroxide (20 mL), and then extracted with EtOAc (3 × 25 mL). The combined organic was dried over Na₂SO₄, filtered and evaporated in vacuo to dryness. The residue was purified by flash silica column chromatography, gradient elution from 15% to 100% EtOAc in heptane to yield the title compound as off-white solid (320 mg, 56% yield).
LCMS (Method 10, ES⁺) 1.01 min, 233 & 235 *m*/*z* [M+H]⁺

### INTERMEDIATE 102

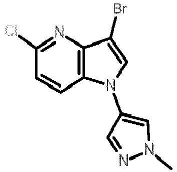

### 3-bromo-5-chloro-1-(1-methylpyrazol-4-yl)pyrrolo[3,2-b]pyridine

To the solution of Intermediate 101 (320 mg, 1.38 mmol) in DCM (10 mL) was added NBS (269 mg, 1.51 mmol) portionwise over 5 min at r.t and then stirred for 90 mins. The reaction mixture diluted with DCM (10 mL), water (10 mL) and aqueous saturated Na₂CO₃ (10 mL). The organic phase was separated and the aqueous was extracted with DCM (2 × 10mL). The combined organic was dried over MgSO₄, filtered and evaporated to dryness. The residue was purified by flash silica column chromatography, gradient elution from 15% to 100% EtOAc in heptane to yield of the title compound as off-white solid (400 mg, 89% yield).
LCMS (Method 10, ES⁺) 1.08 min, 311 & 313 *m*/*z* [M+H]⁺

### INTERMEDIATE 103

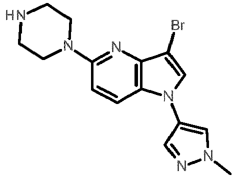

### 3-bromo-1-(1-methylpyrazol-4-yl)-5-piperazin-1-yl-pyrrolo[3,2-b]pyridine

To the mixture of Intermediate 102 (260 mg, 0.83 mmol) and piperazine (1.08 g, 12.52 mmol) in DMSO (3 mL) was added 4*N* HCl in dioxane (0.21 mL) and then stirred at 136 °C for 16 hours under microwave irradiation. The reaction mixture was diluted with water (20 mL) and then extracted with EtOAc (4 × 25 mL). The combined organic was dried over MgSO₄, filtered and evaporated in vacuo. The residue was purified by flash NH-silica column chromatography, gradient elution from EtOAc to 11% MeOH in EtOAc to yield the title compound as brown gum (235 mg, 70% yield).
LCMS (Method 10, ES⁺) 0.87 min, 361 & 363 *m*/*z* [M+H]⁺

### INTERMEDIATE 104

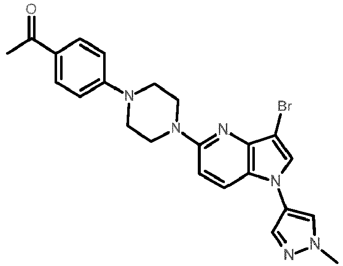

### 1-[4-[4-[3-bromo-1-(1-methylpyrazol-4-yl)pyrrolo[3,2-b]pyridin-5-yl]piperazin-1-yl]phenyl]ethanone

The mixture of Intermediate 103 (90% purtiy, 230 mg, 0.57 mmol), 1-(4-fluorophenyl)ethanone (158 mg, 1.15 mmol) and dipotassium carbonate (207 mg, 1.5 mmol) in DMSO (3 mL) was stirred at 130°C for 19 hours under microwave irradiation. The reaction mixture was cooled to r.t and diluted with DCM (25 mL) and water (25 mL). The organic layer was separated and the aqueous was extracted with DCM (2 × 25 mL). The combined organic layers were dried over MgSO₄, filtered and evaporated in vacuo to yield a beige solid residue. This solid was triturated with 50% EtOAc in heptane (5 mL) and the solid was collected by filtration, washed with 50% EtOAc in heptane (2*3 mL) to give the title compound as beige solid (250 mg, 82% yield).
LCMS (Method 10, ES⁺) 1.21 min, 479, 481 *m*/*z* [M+H]⁺

### INTERMEDIATE 105

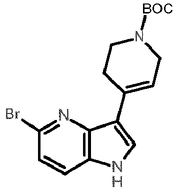

### tert-butyl 4-(5-bromo-1H-pyrrolo[3,2-b]pyridin-3-yl)-3,6-dihydro-2H-pyridine-1-carboxylate

To the solution of 5-bromo-1H-pyrrolo[3,2-b]pyridine (400 mg, 2.03 mmol) and tert-butyl 4-oxopiperidine-1-carboxylate (607 mg, 3.05 mmol) in MeOH (10 mL) was added potassium hydroxide (167 µL, 6.09 mmol) and then stirred at reflux for 20 hours. The reaction mixture was cooled to r.t, diluted with water (40 mL) and extracted with EtOAc (3 × 30 mL). The combined organic was dried over MgSO₄, filtered and evaporated in vacuo to dryness. The residue was purified by flash silica column chromatography, gradient elution from 12% to 100% EtOAc in heptane to yield the title compound as off-white solid (560 mg, 65% yield).
LCMS (Method 10, ES⁺) 1.28 min, 378 & 380 *m*/*z* [M+H]⁺

### INTERMEDIATE 106

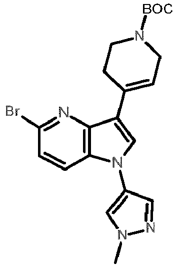

### tert-butyl 4-[5-bromo-1-(1-methylpyrazol-4-yl)pyrrolo[3,2-b]pyridin-3-yl]-3,6-dihydro-2H-pyridine-1-carboxylate

The mixture of intermediate 105 (460 mg, 1.09 mmol), 4-iodo-1-methyl-1H-pyrazole (342 mg, 1.64 mmol), copper(1+) iodide (21 mg, 0.11 mmol), quinolin-8-ol (16 mg, 0.11 mmol) and K₂CO₃ (227 mg, 1.64 mmol) in DMSO (6 mL) was stirred at 110°C for 36 min under microwave irradiation. The mixture was then cooled to r.t , diluted with 10% ammonium hydroxide (20 mL), extracted with EtOAc (4 × 20 mL), dried MgSO₄, filtered and evaporated in vacuo to dryness. The residue was purified by flash silica column chromatography, gradient elution from 17% to 59% EtOAc in heptane to yield the title compound as yellow solid (265 mg, 48% yield).
LCMS (Method 10, ES⁺) 1.35 min, 458 & 460 *m*/*z* [M+H]⁺

### INTERMEDIATE 107

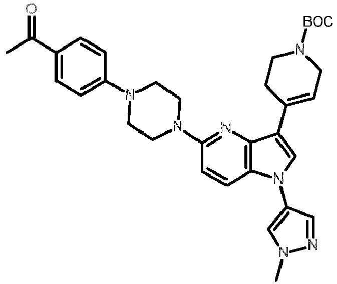

### tert-butyl 4-[5-[4-(4-acetylphenyl)piperazin-1-yl]-1-(1-methylpyrazol-4-yl)pyrrolo[3,2-b]pyridin-3-yl]-3,6-dihydro-2H-pyridine-1-carboxylate

This intermediate was prepared following General Procedure 21 using intermediate 106 and 1-(4-piperazin-1-ylphenyl)ethanone.
LCMS (Method 10, ES⁺) 1.37 min, 582 *m*/*z* [M+H]⁺

### INTERMEDIATE 108

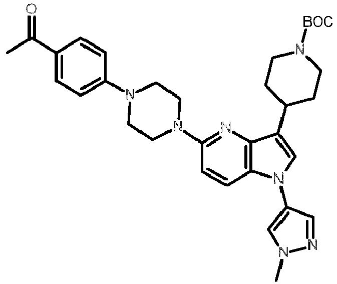

### tert-butyl 4-[5-[4-(4-acetylphenyl)piperazin-1-yl]-1-(1-methylpyrazol-4-yl)pyrrolo[3,2-b]pyridin-3-yl]piperidine-1-carboxylate

The solution of intermediate 107 (105 mg, 0.16 mmol) in MeOH/EtOAc (1:1, 15 mL) was passed through a 10% Pd/C Cartridge in H-cube system (20 °C, H₂ pressure 5 bar) for 2 cycles. The solution was then evaporated in vacuo and the crude residue was purified by flash NH-silica column chromatography, gradient elution from 18% to 100% EtOAc in heptane] to yield the title compound as brown solid (40 mg, 31% yield).
LCMS (Method 10, ES⁺) 1.29 min, 584 *m*/*z* [M+H]⁺

### INTERMEDIATE 109

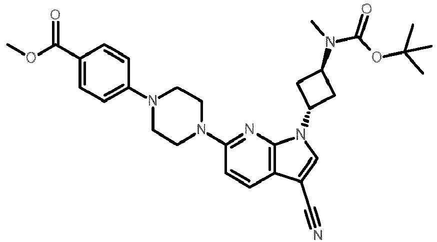

### methyl 4-[4-[1-[3-[tert-butoxycarbonyl(methyl)amino]cyclobutyl]-3-cyano-pyrrolo[2,3-b]pyridin-6-yl]piperazin-1-yl]benzoate

This intermediate was prepared following General Procedure 3 using intermediate 40 and methyl 4-(piperazin-1-yl)benzoate.
LCMS (Method 2, ES⁺) 1.56 and 1.62 min, 545 *m*/*z* [M+H]⁺

### INTERMEDIATE 110

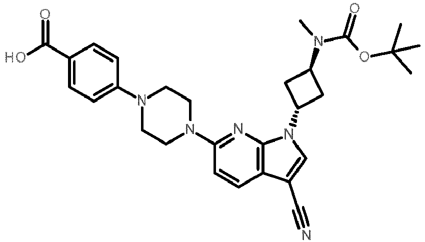

### 4-[4-[1-[3-[tert-butoxycarbonyl(methyl)amino]cyclobutyl]-3-cyano-pyrrolo[2,3-b]pyridin-6-yl]piperazin-1-yl]benzoic acid

Intermediate 109 (117 mg, 0.2 mmol) was dissolved in THF (5 mL) and a solution of lithium hydroxide monohydrate (45 mg, 1.1 mmol) in WATER (2 mL) added. The reaction mixture was stirred at RT for 2 hours, and a second portion of of lithium hydroxide monohydrate (45 mg, 1.1 mmol) in water (0.5 mL) followed by 1,4-DIOXANE (1 mL). The reaction mixture was stirred at RT for 1 h then heated at 50 °C for ∼ 2 hours. Then left to stand at RT for 2 days. The reaction mixture was diluted with pH 4 buffer and ethyl acetate. The aqueous layer was extracted with ethyl acetate three times. Organic layers combined, dried (Na2SO4) and concentrated under reduced pressure. The residue was then purified by flash column chromatography to give the title compound (110 mg, 96%).
LCMS (Method 2, ES⁺) 1.12 min, 531 *m*/*z* [M+H]⁺

### Examples 1-16

The following compounds were synthesised using General Procedure 3 from heteroaryl halide and the appropriate amine as noted in the table below.
Examples 1-8, Example 10, and Examples 15-16: LCMS Method 1
Example 9: LCMS Method 2
Examples 11-14: LCMS Method 4

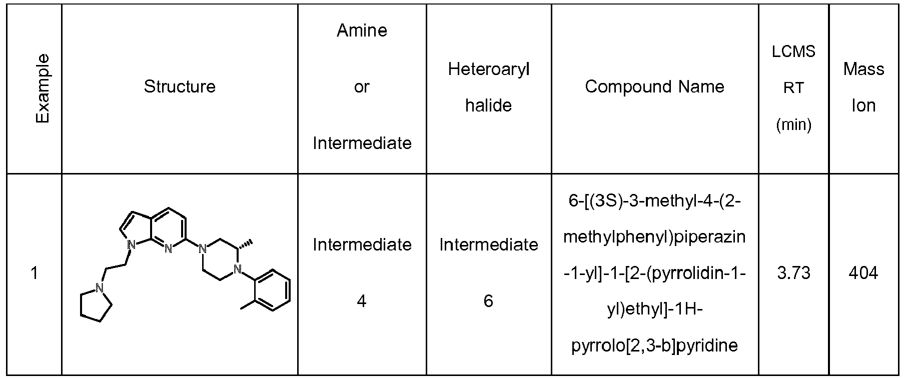

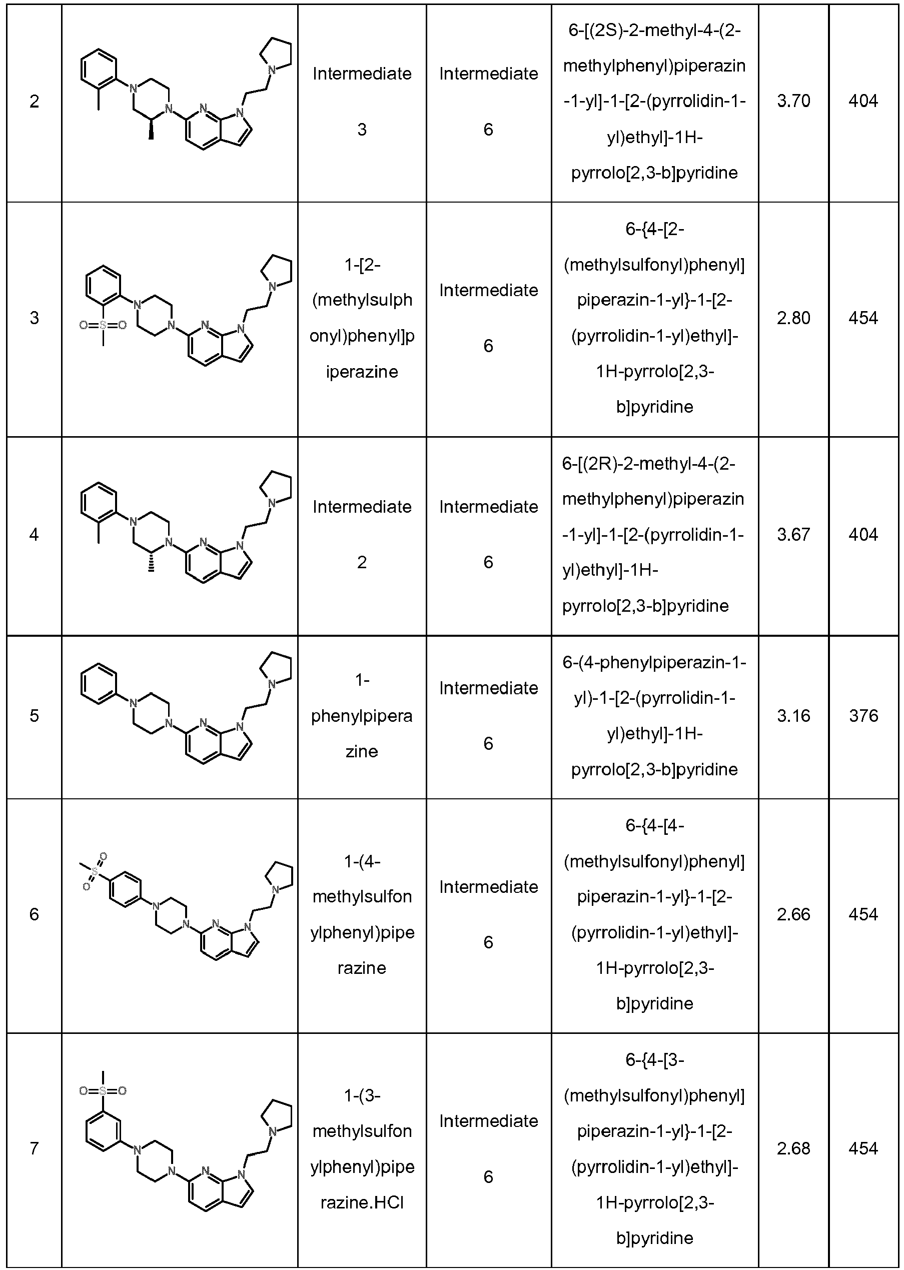

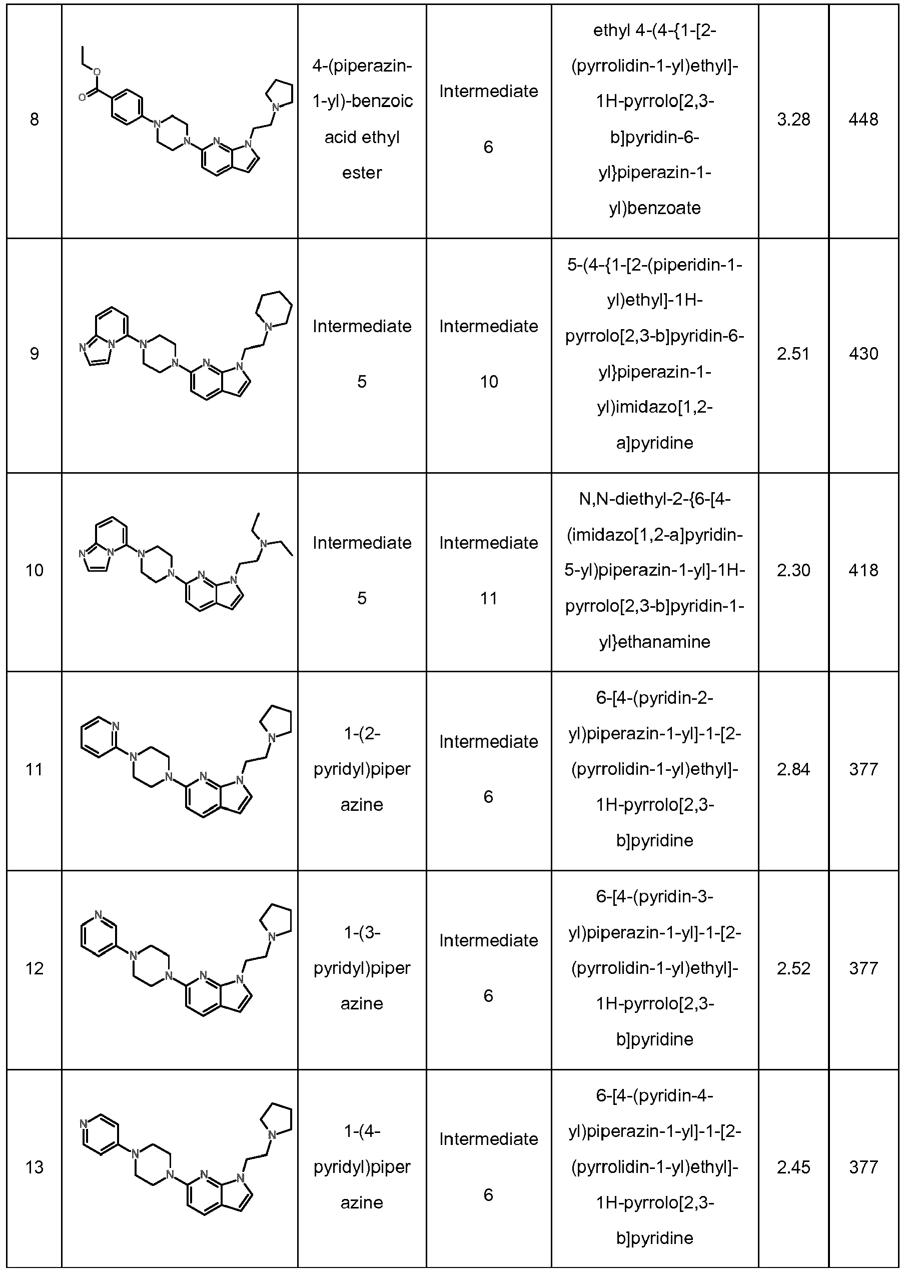

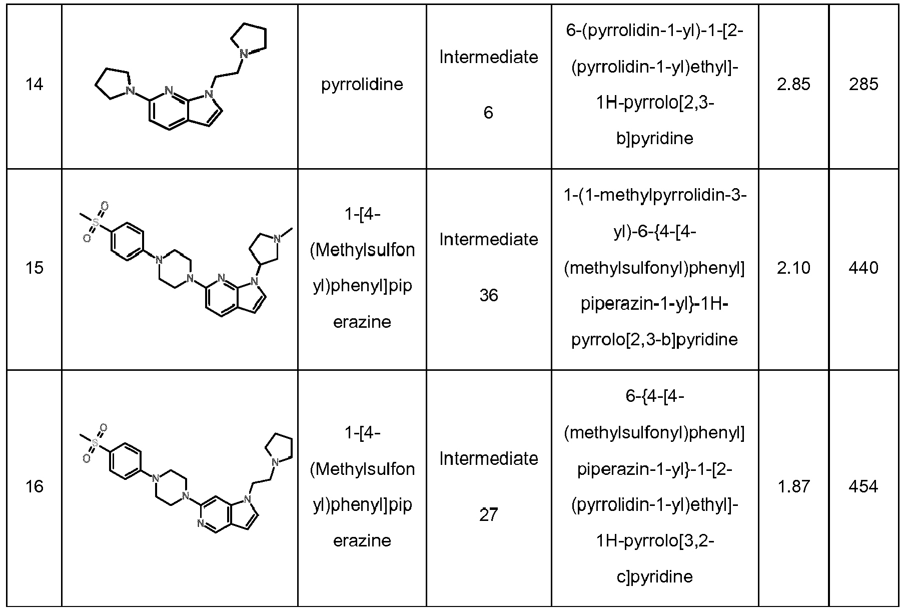

### Examples 17-29

Examples 17-29 were prepared in two steps according to General Procedure 3 followed by General Procedure 13 and analysed by LCMS Method 1.

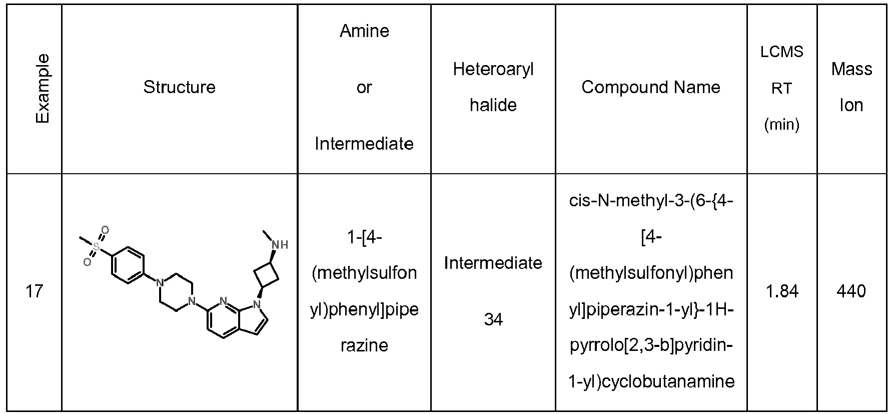

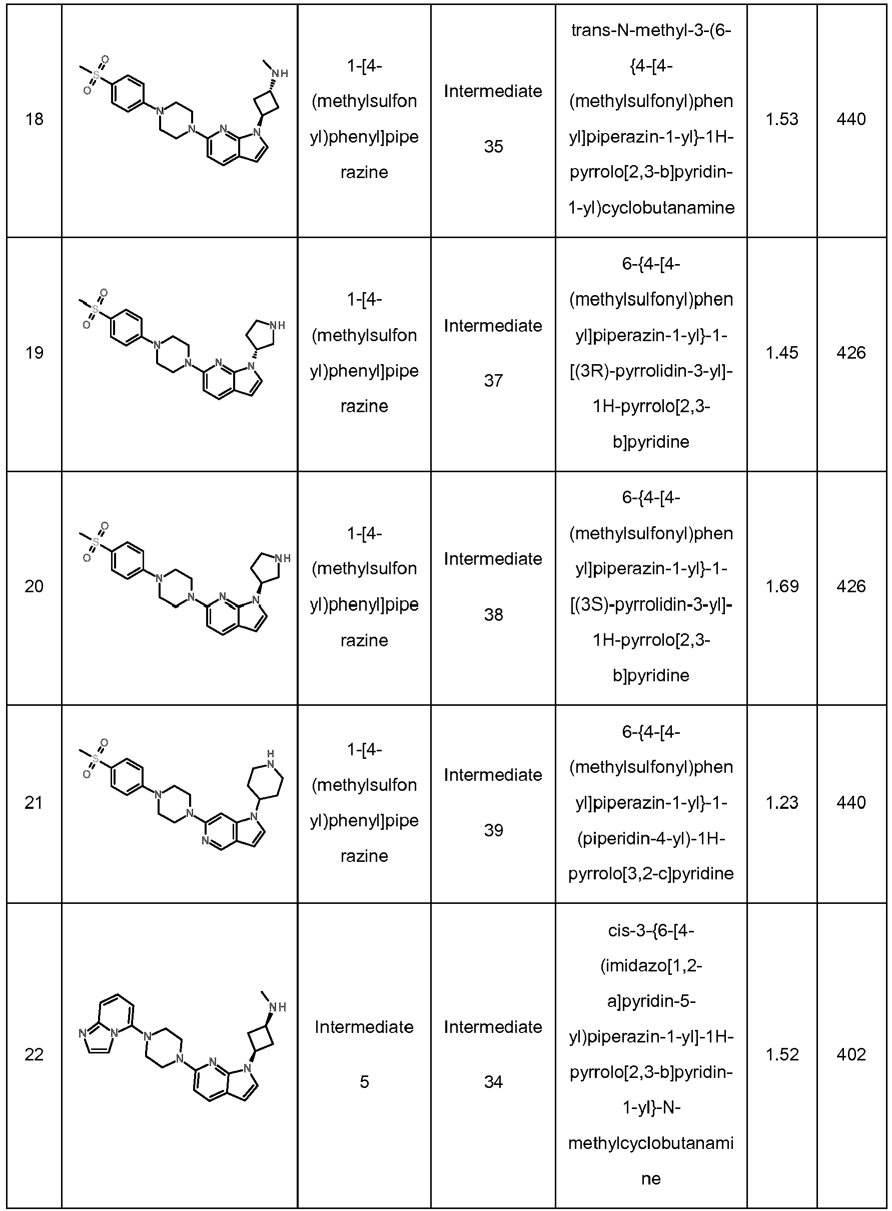

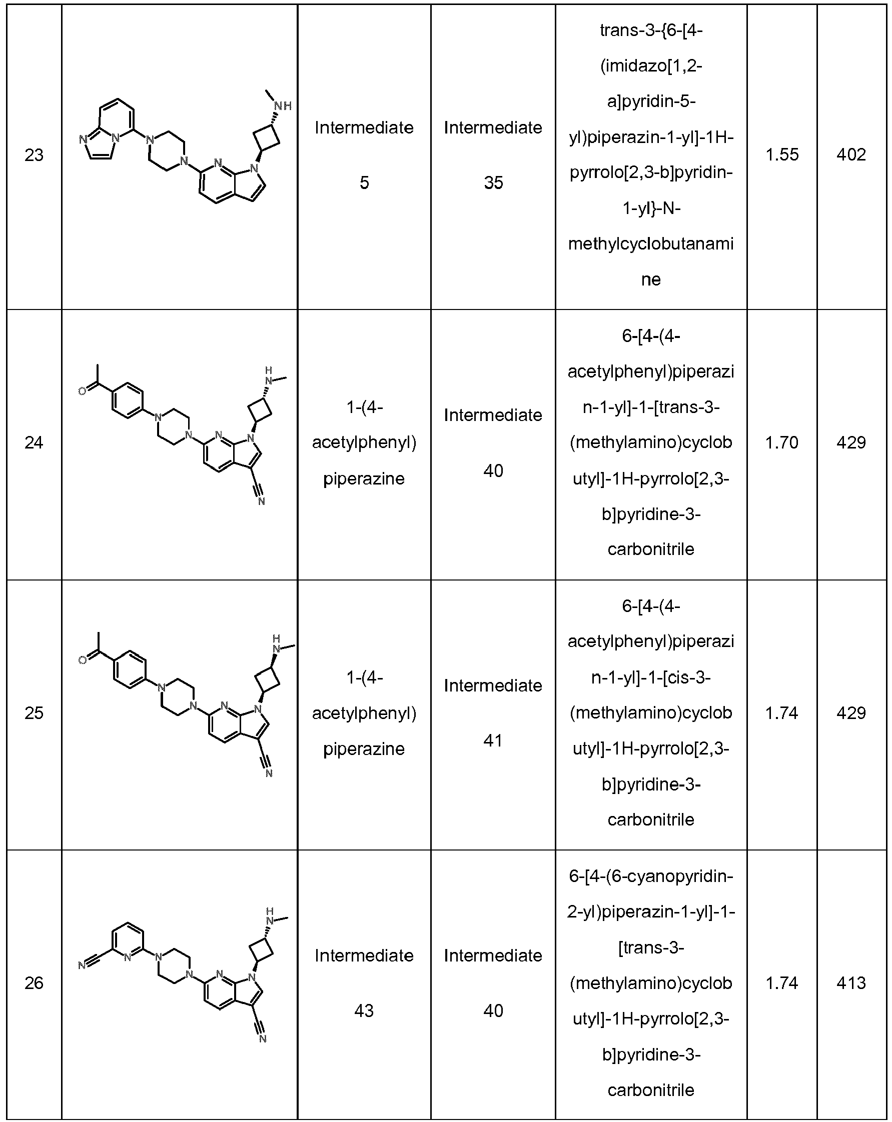

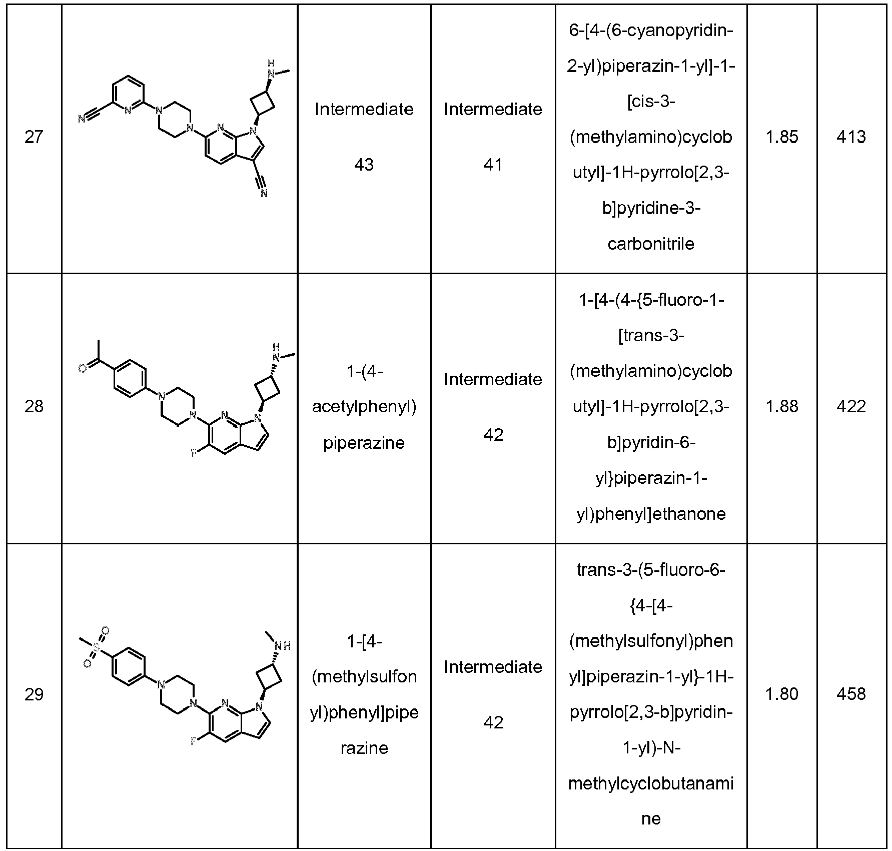

### Examples 30-33

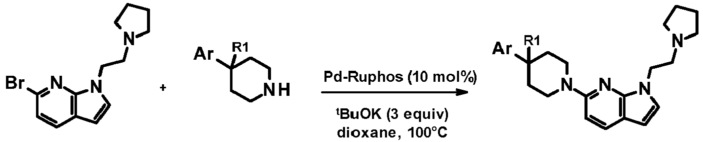

The following compounds were synthesized from Intermediate 6 and the appropriate piperidine in accordance with General Procedure 4.

Examples were analysed by LCMS Method 1.

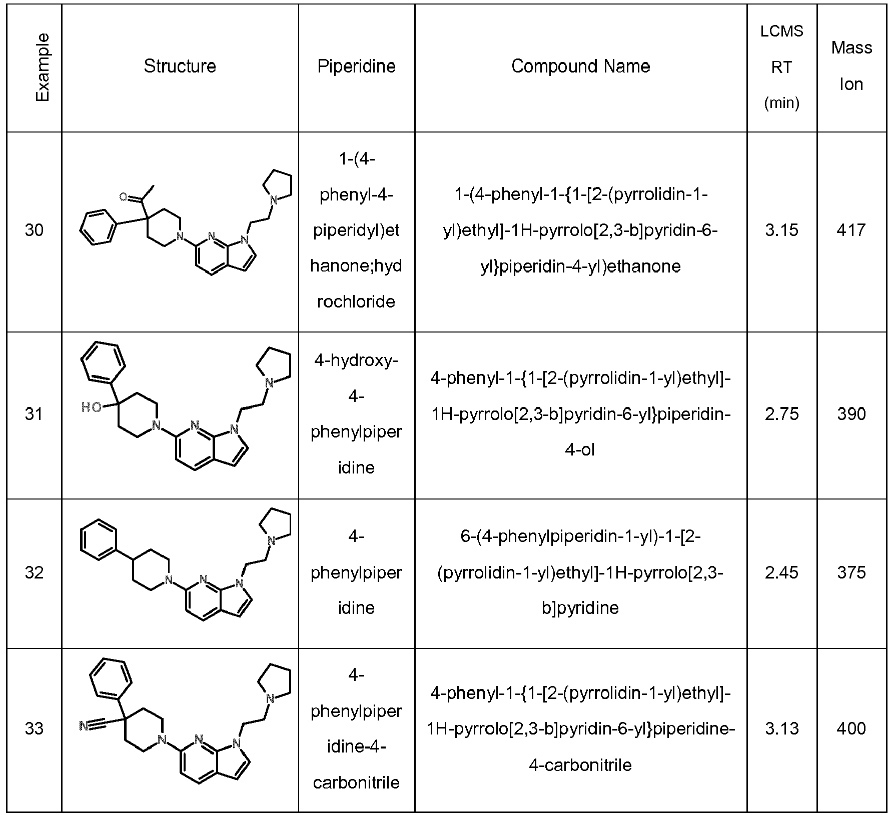

### Examples 34-51

The following compounds were synthesized from Intermediate 7 and the appropriate aryl bromide in accordance with General Procedure 3.

Examples 34-45 were analysed by LCMS Method 3.

Examples 46-51 were analysed by LCMS Method 1.

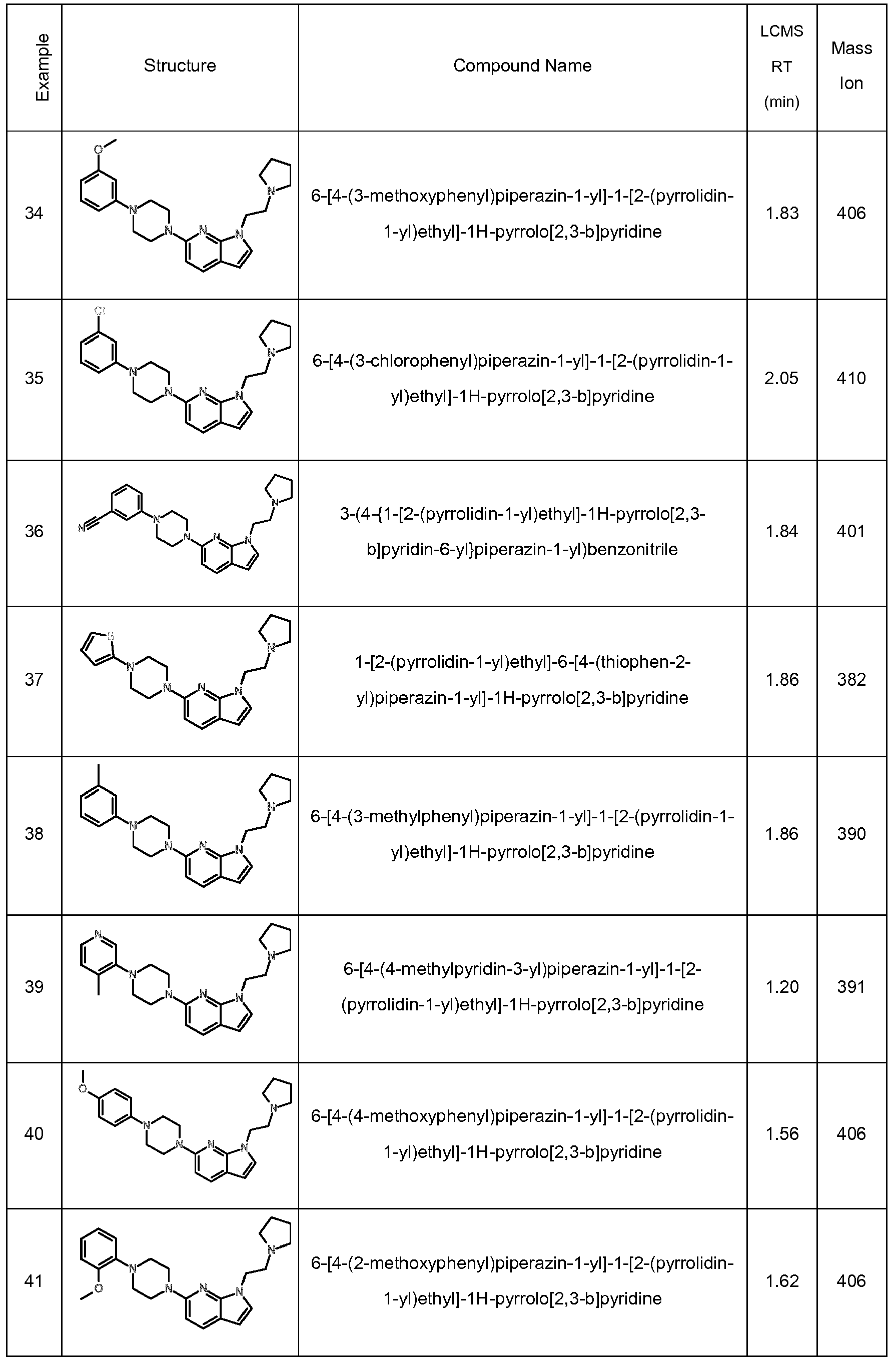

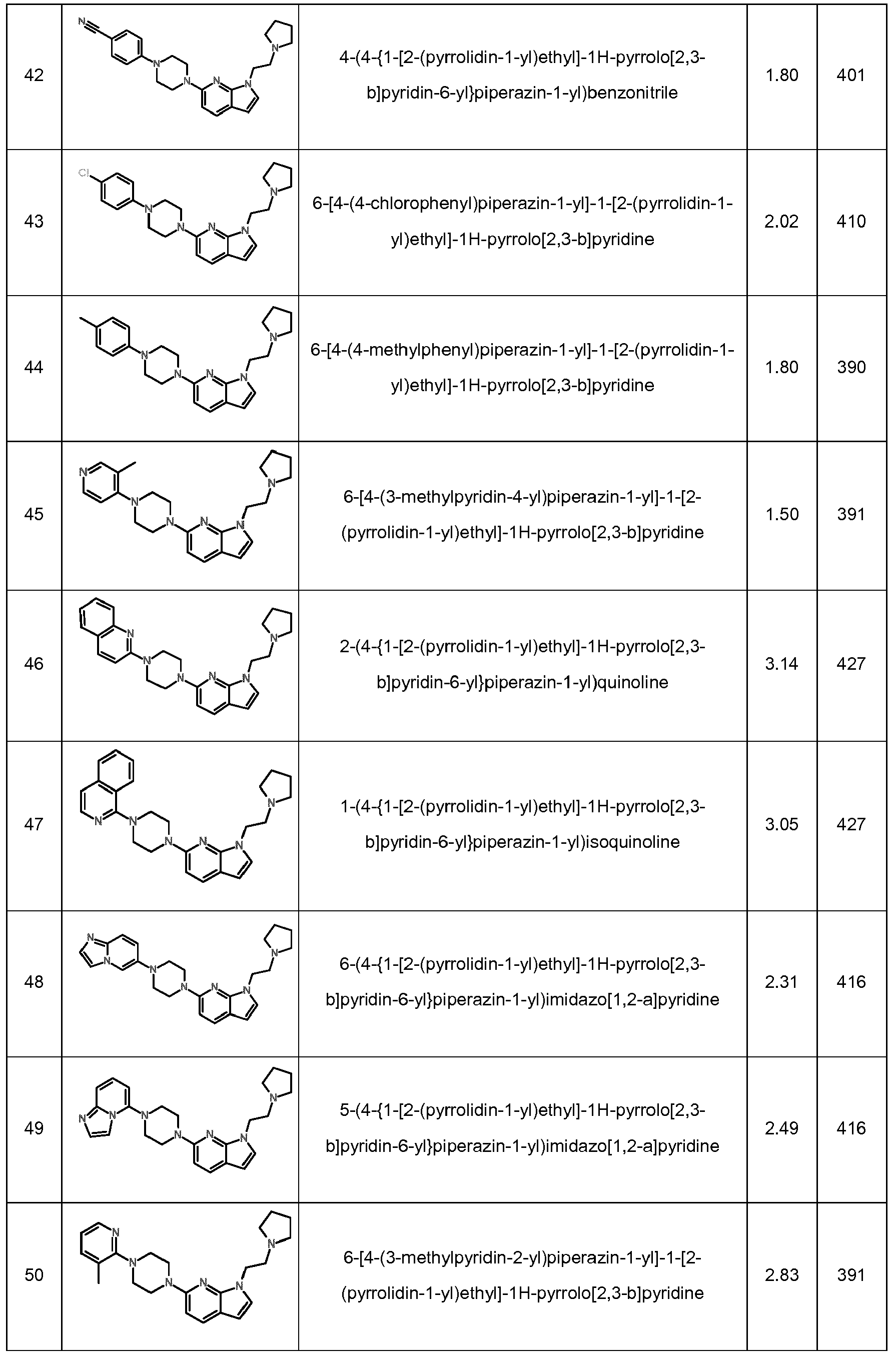

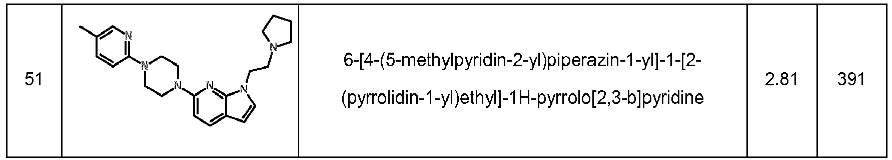

### Examples 52-54

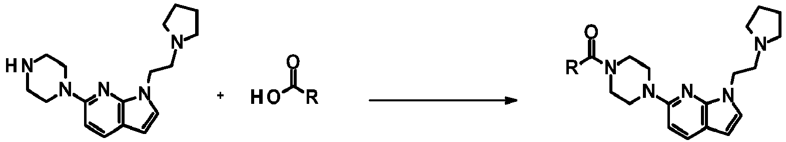

Examples 52-54 were synthesized from Intermediate 7 and the appropriate carboxylic acid in accordance with General Procedure 6 and analysed by LCMS Method 3.

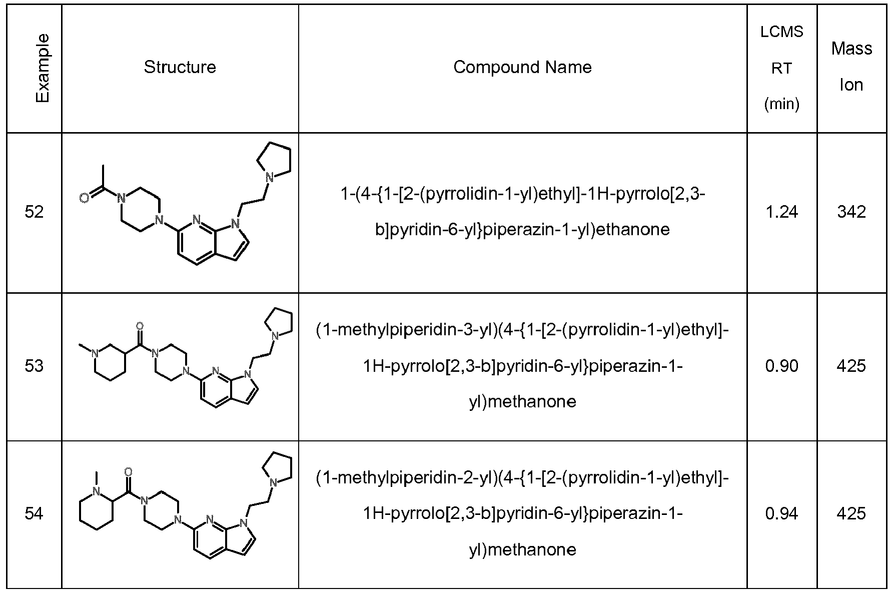

### Examples 55-66

The following compounds were synthesised from Intermediate 17 and the appropriate amine in accordance with General Procedure 7.
Examples 55-65: LCMS Method 3
Example 66: LCMS Method 1

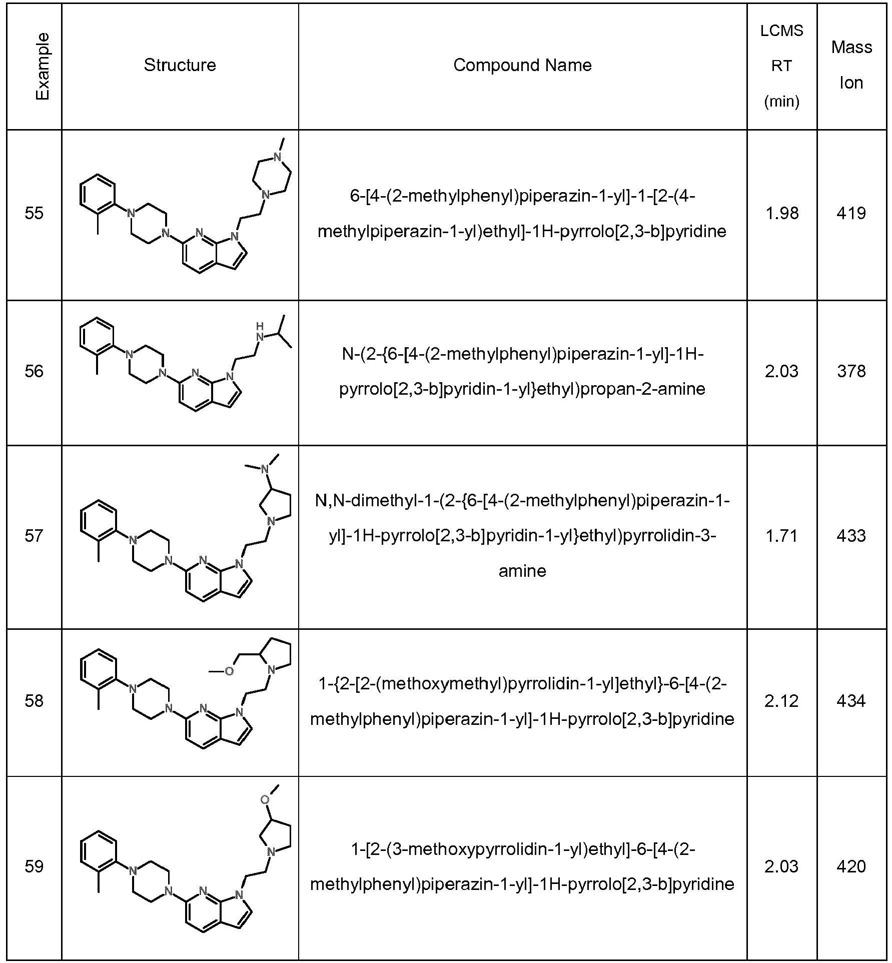

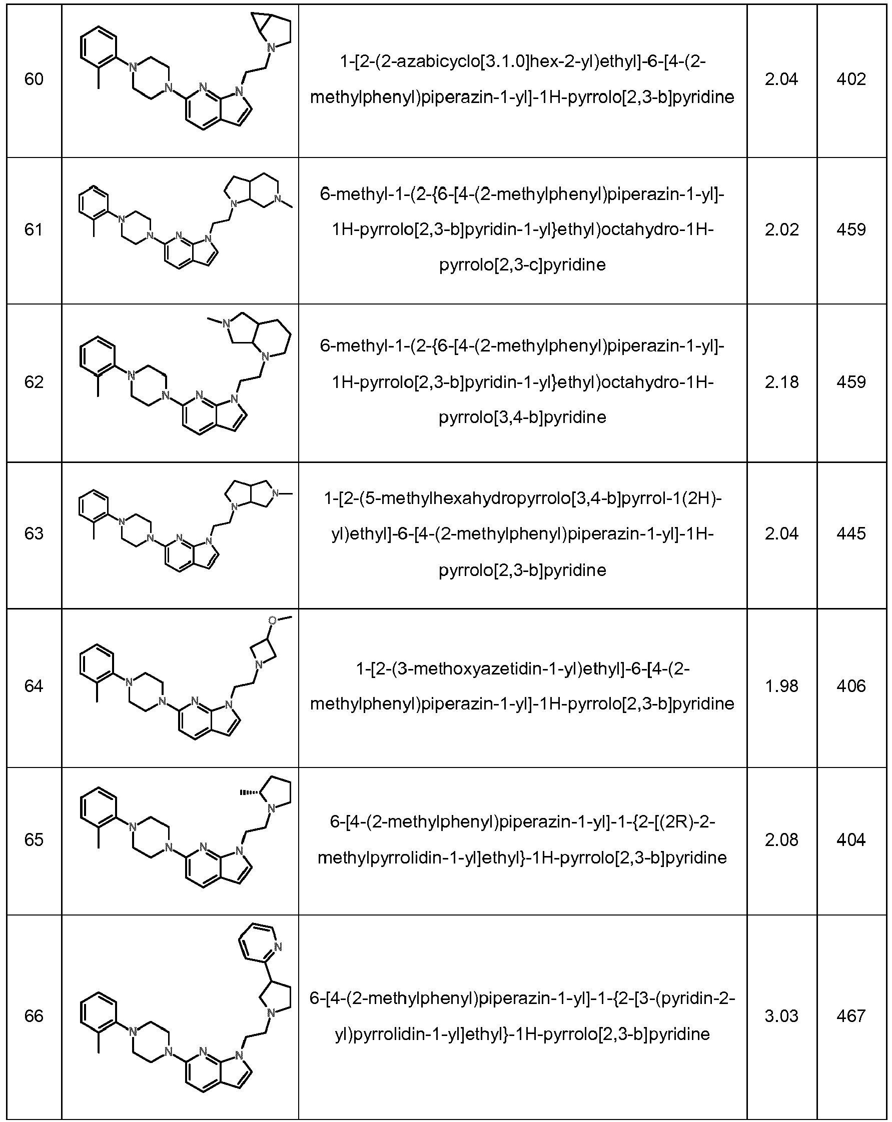

### Examples 67-81

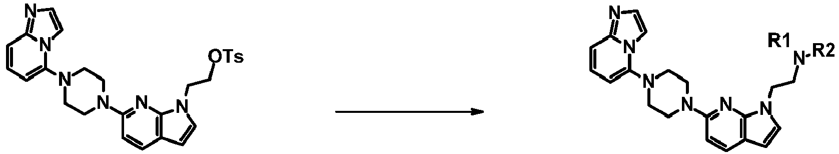

The following compounds were synthesised from Intermediate 15 and the appropriate amine in accordance with General Procedure 8.

Examples 67-81 were analysed by LCMS Method 3.

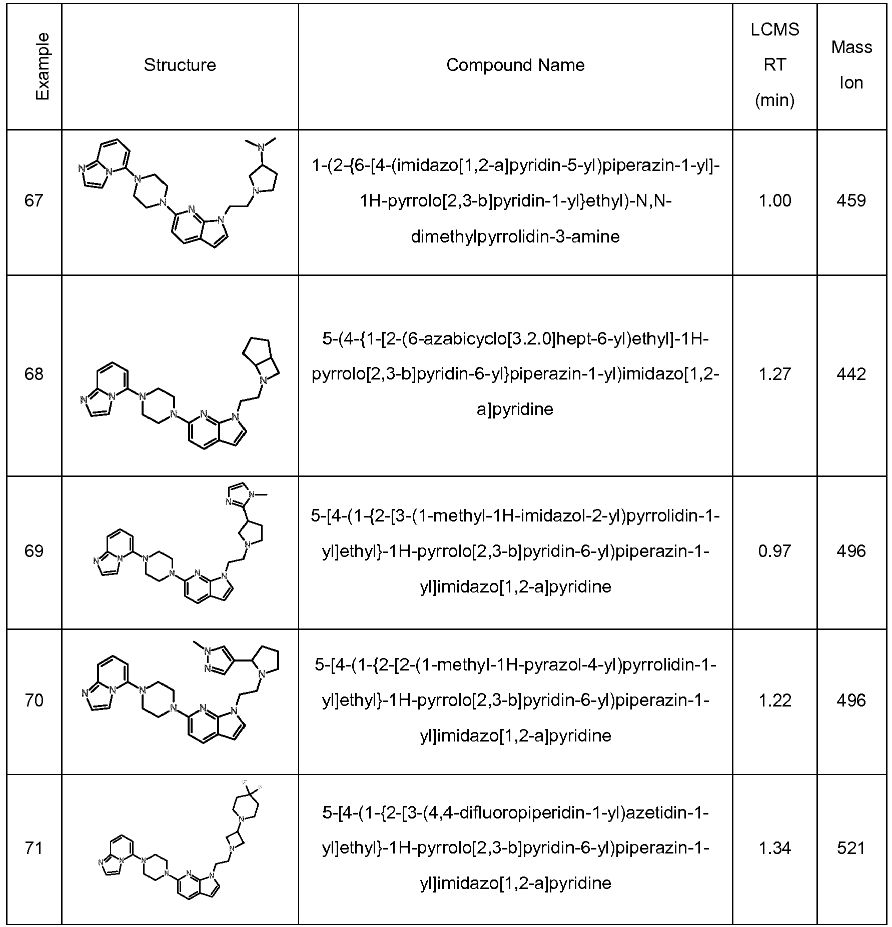

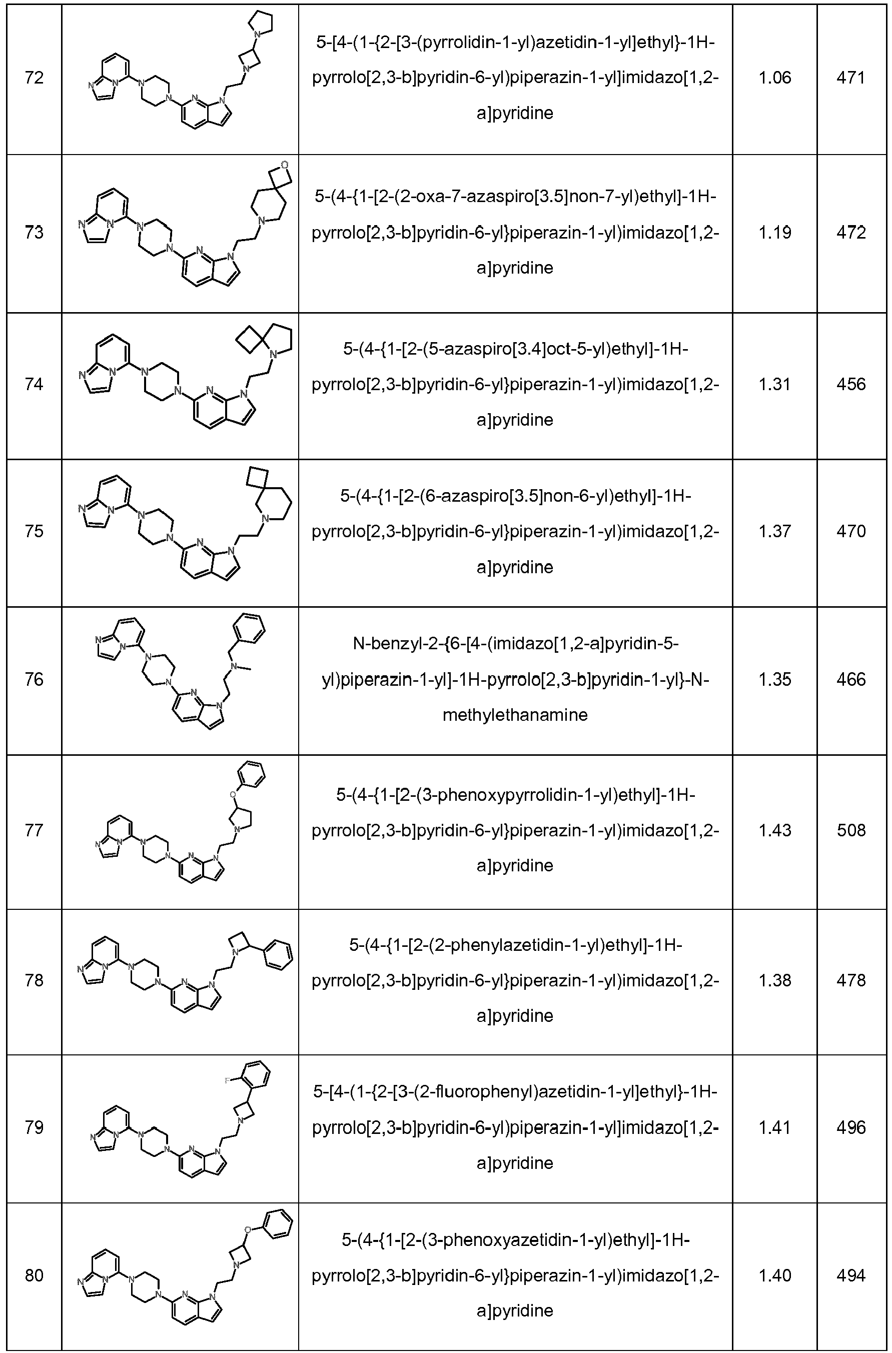

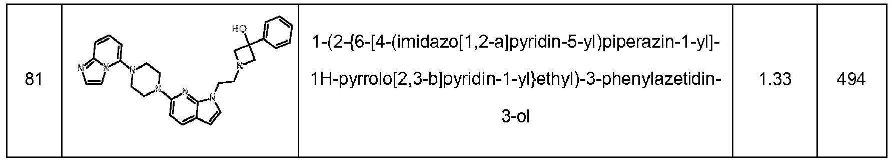

### Examples 82-85

Intermediate 23 (70 mg, 0.11 mmol), boronic ester (2 eq., 0.23 mmol), K₂CO₃ (2 eq., 0.23 mmol) and PdCl₂(dppf)-DCM complex (0.1 equiv., 0.01 mmol) were dissolved in 1,4-dioxane (0.1 M, 1 mL). H₂O (1 M, 0.1 mL) was added and the mixture degassed for 1 min. The mixture was stirred overnight at 100 °C. The mixture was cooled to ambient temperature and DCM (3 mL) and H₂O (2 mL) were added and the layers separated. TFA (1 mL) was added to the DCM solution and the reaction mixture was stirred at r.t. for 2 hours. The residue was filtered through an SCX column, washing with MeOH and DCM, and then eluting the product with NH₃ (7 N) in MeOH. The volatiles were removed under vacuum and the residue purified by reverse phase column chromatography.

Examples 82-85 were synthesised from Intermediate 23 and the appropriate commercially available boronic ester in accordance with the foregoing procedure.

Examples were analysed by LCMS Method 1.

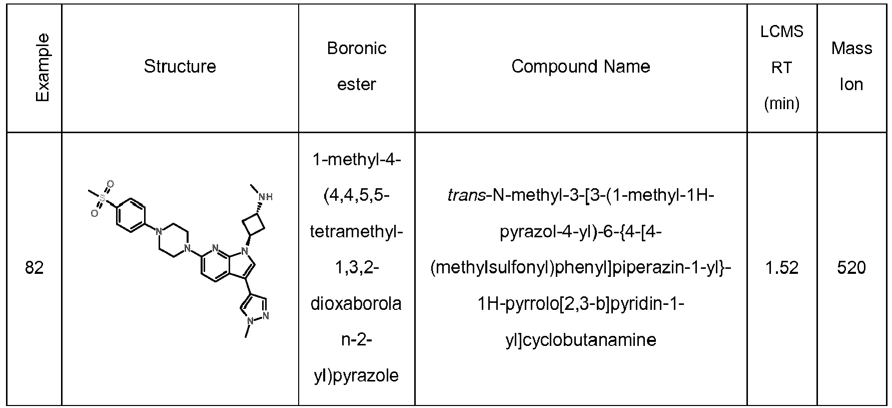

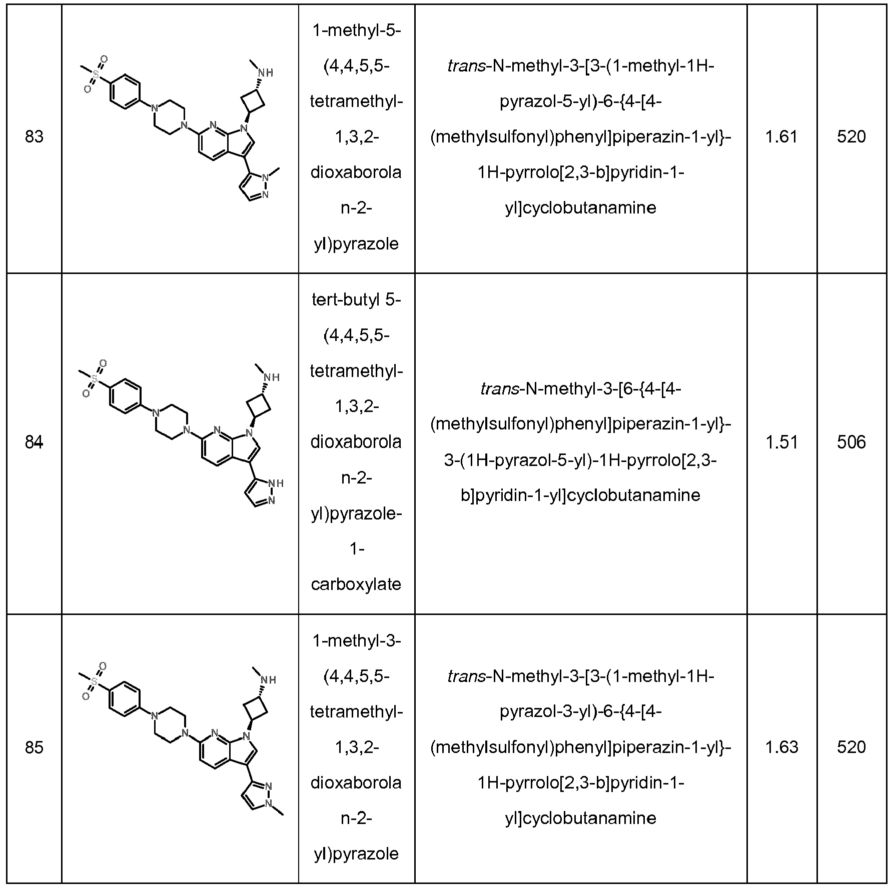

### Examples 86-97

Examples 86-97 were synthesised from the appropriate amine and aldehyde in accordance with General Procedure 12.

Examples 86-97 were analysed by LCMS Method 1.

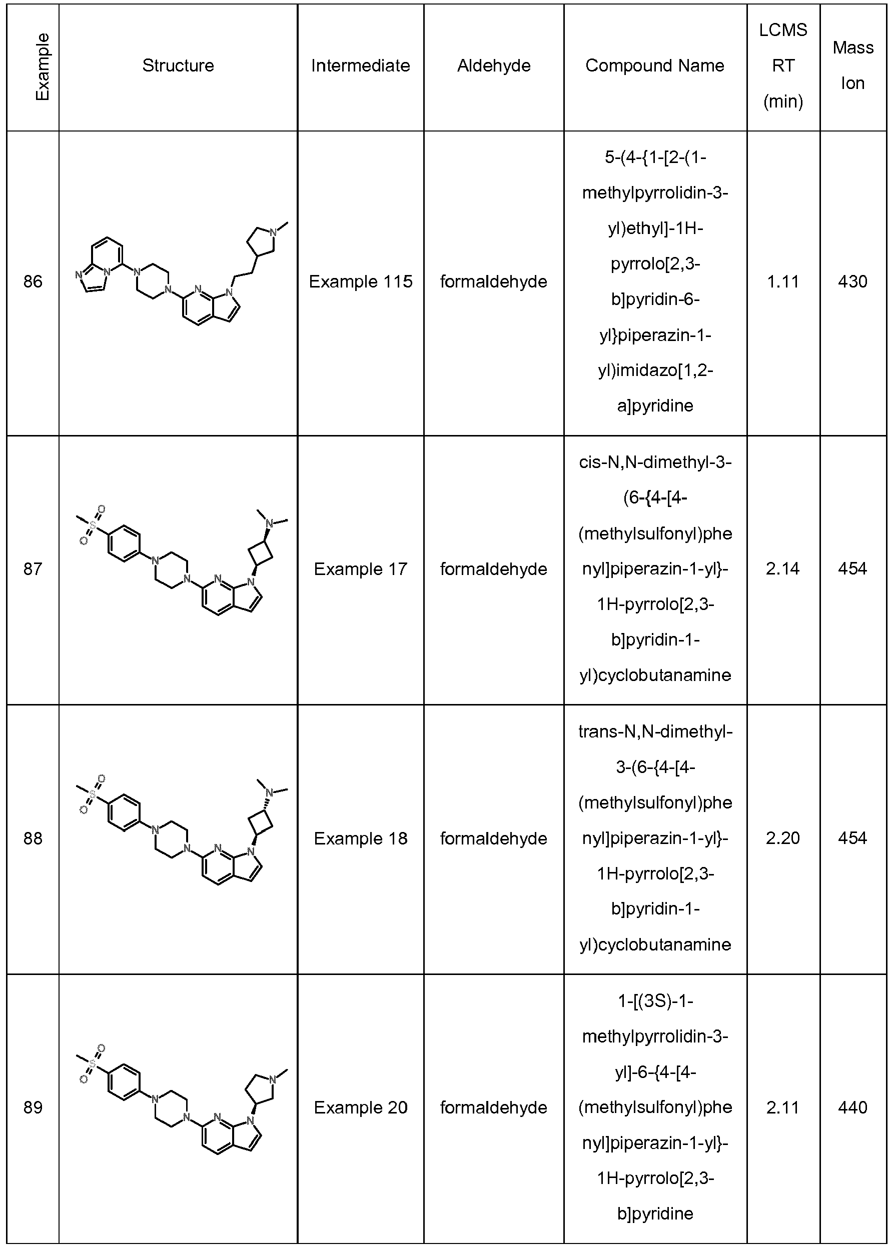

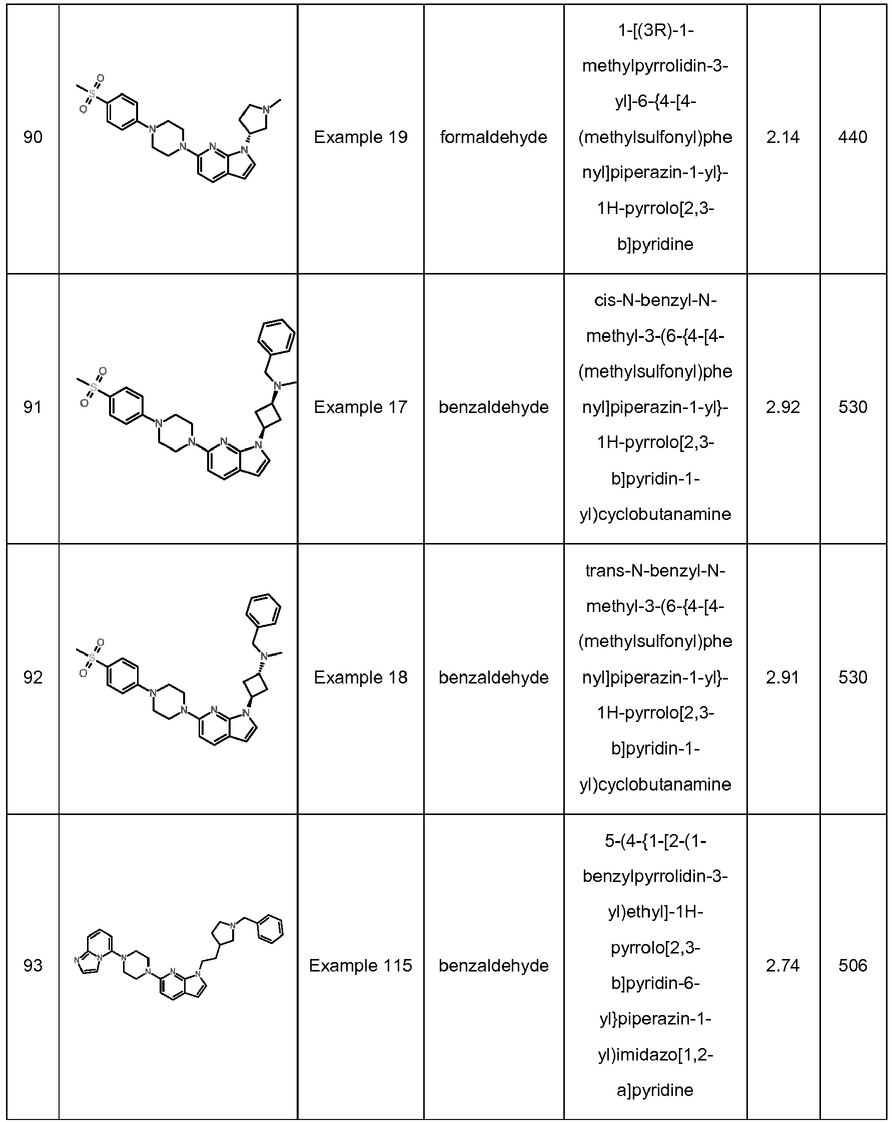

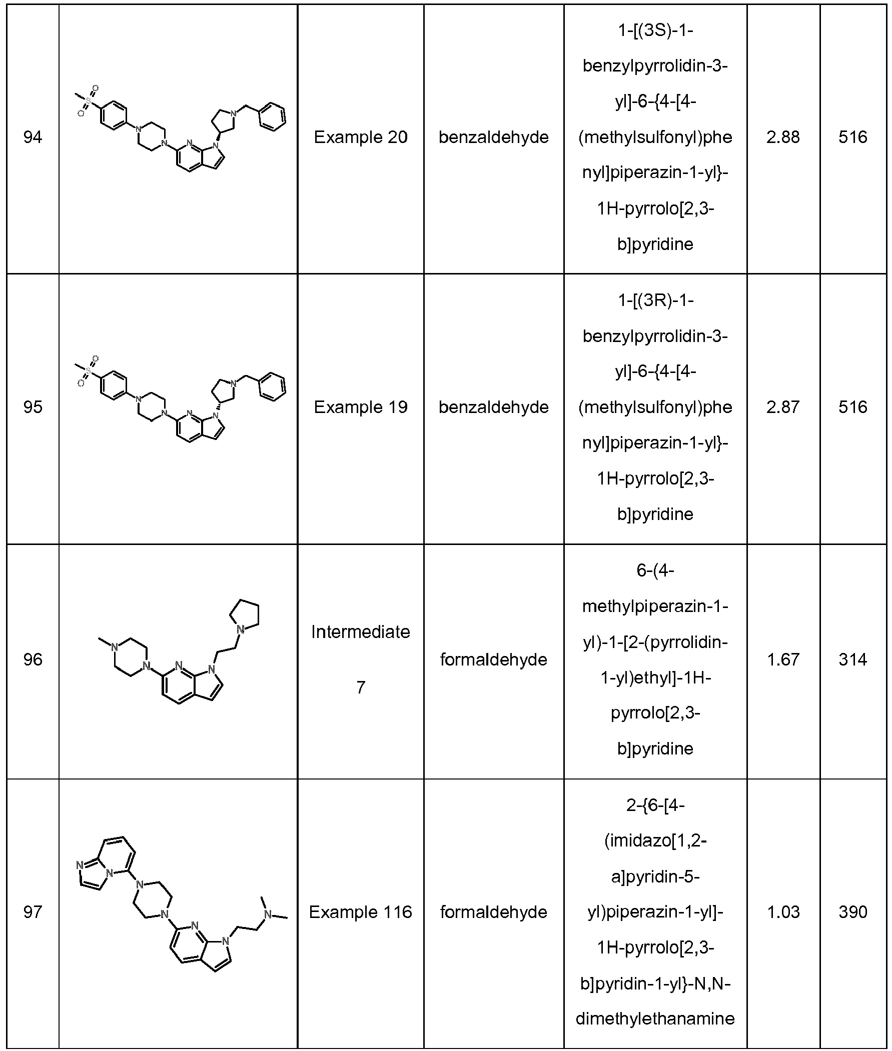

### Example 98

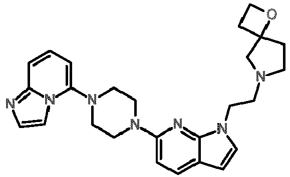

### 5-(4-{1-[2-(1-oxa-6-azaspiro[3.4]oct-6-yl)ethyl]-1H-pyrrolo[2,3-b]pyridin-6-yl}piperazin-1-yl)imidazo[1,2-a]pyridine

Example 98 was prepared following General Procedure 8 using Intermediate 15 (50 mg, 0.09 mmol) and 1-oxa-7-azaspiro[3.4]octane (17 mg, 1.5 eq.). The reaction mixture was then heated at 80 °C overnight in a sealed vial. The reaction was cooled down to ambient temperature and H₂O and DCM added. The layers were separated and the aq. phase was extracted with DCM. The solvent was removed under vacuum and the residue was purified by chromatography gradient elution from DCM to 30% MeOH in DCM to yield Example 98 (5 mg, 12% yield).
LCMS (Method 1, ES⁺) 2.01 min, 458 m/z (M+H)⁺.

### Example 99

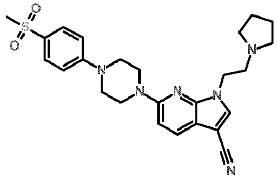

### 6-{4-[4-(methylsulfonyl)phenyl]piperazin-1-yl}-1-[2-(pyrrolidin-1-yl)ethyl]-1H-pyrrolo[2,3-b]pyridine-3-carbonitrile

6-bromo-1-(2-pyrrolidin-1-ylethyl)pyrrolo[2,3-b]pyridine-3-carbonitrile (300 mg, 0.1 mmol) was dissolved in a mixture of DMF:THF (10 mL, 1:1) The pink solution was cooled to 0 °C and sodium hydride (79 mg, 0.11 mmol, 2.2 eq., 60 mass%) added slowly portion-wise over a period of 30 minutes. 1-(2-chloroethyl)pyrrolidine hydrochloride (1.5 eq., 1.4 mmol) was then added carefully portion-wise and the reaction mixture stirred at 80 °C overnight until the starting material was consumed. The crude was dissolved in DCM and H₂O added. The layers were separated and the aq. phase was extracted with DCM. The solvent was removed under vacuum and the resulting residue used in General Procedure 3 with 1-[4-(methylsulfonyl)phenyl]piperazine (1.5 eq., 0.94 mmol). The residue was purified by column chromatography gradient elution from DCM to 30% MeOH in DCM and then re-purified by reverse phase chromatography to yield Example 99 (4 mg, 1% yield).
LCMS (Method 1, ES⁺) 2.62 min, 479 m/z (M+H)⁺.

### Example 100

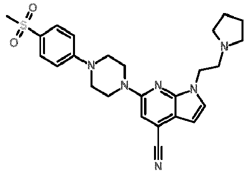

### 6-{4-[4-(methylsulfonyl)phenyl]piperazin-1-yl}-1-[2-(pyrrolidin-1-yl)ethyl]-1H-pyrrolo[2,3-b]pyridine-4-carbonitrile

4-Cyano-6-bromo-7-azaindole (200 mg, 0.9 mmol) reacted with 1-(2-chloroethyl)pyrrolidine hydrochloride (1.5 eq., 1.35 mmol) following General Procedure 2. H₂O and EtOAc were added and the layers separated and the aq. phase was extracted with EtOAc. The solvent was removed under vacuum and the residue used following General Procedure 3 without any further purification using 1-[4-(methylsulfonyl)phenyl]piperazine (1.5 equiv., 1.97 mmol). The residue was purified by column chromatography gradient elution from DCM to 30% MeOH in DCM, and then re-purified by reverse phase column chromatography give Example 100 (5 mg, 7% yield).
LCMS (Method 1, ES⁺) 2.26 min, 479 m/z (M+H)⁺.

### Example 101

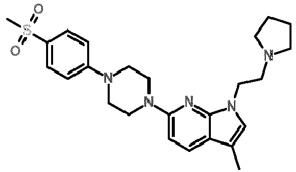

### 3-methyl-6-{4-[4-(methylsulfonyl)phenyl]piperazin-1-yl}-1-[2-(pyrrolidin-1-yl)ethyl]-1H-pyrrolo[2,3-b]pyridine

Intermediate 18 (100 mg, 0.28 mmol), cobalt tetrafluoroborate hexahydrate (0.1 mmol), tris[2-(diphenylphosphino)ethyl]phosphine (0.1 mmol) and K₂CO₃ (1 mmol, 1 mmol) were dissolved in methanol (1 mL). The mixture was heated to 100°C in a sealed vial overnight. The crude reaction mixture was filtered over celite and the volatiles removed under vacuum. The residue was submitted to alkylation following General Procedure 2 using 1-(2-chloroethyl)pyrrolidine hydrochloride (1.5 equiv.). The crude reaction mixture was diluted with EtOAc and H₂O added. The layers were separated and the aq. phase was extracted with EtOAc. Combined organic layers were dried over Na₂SO₄ and the solvent was removed under vacuum. The residue was filtered over celite and rinsed with DCM. After removing the solvent, the residue was purified by reverse phase column chromatography to give Example 101 (2 mg, 2% yield).
LCMS (Method 1, ES⁺) 2.83 min, 468 m/z (M+H)⁺.

### Example 102

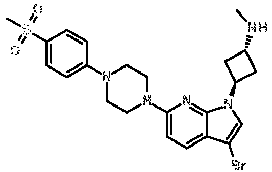

### trans-3-(3-bromo-6-{4-[4-(methylsulfonyl)phenyl]piperazin-1-yl}-1H-pyrrolo[2,3-b]pyridin-1-yl)-N-methylcyclobutanamine

Intermediate 26 (550 mg, 1.02 mmol) was dissolved in DCM (30 mL) and cooled to 0 °C. NBS (165 mg, 0.92 mmol) was added portion-wise. The reaction mixture was stirred in a cooling bath for 10 min then quenched with saturated NaHCO₃ aq. solution (20 mL) and diluted with DCM (20 mL). The aqueous layer was extracted with DCM (2 x 10 mL), the combined organics were dried over Na₂SO₄ and concentrated under reduced pressure. The crude was purified by flash chromatography gradient elution from 20% EtOAc to EtOAc and then to DCM in MeOH giving a mixture of brominated products. The white solid was dissolved in DCM (0.25 M) and TFA (5 mL) and stirred at r.t. until completion of the reaction. The volatiles were removed under vacuum and the residue was dissolved in MeOH and filtered through an SCX column, washing with MeOH and DCM and then eluting with NH₃ (4M) in MeOH. The solvent was removed under vacuum and the residue was purified by preparative HPLC affording Example 102 (7 mg, 1 % yield).
LCMS (Method 1, ES⁺) 1.89 min, 518 & 520 m/z (M+H)⁺.

### Example 103

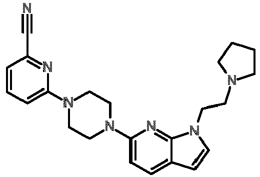

### 6-(4-{1-[2-(pyrrolidin-1-yl)ethyl]-1H-pyrrolo[2,3-b]pyridin-6-yl}piperazin-1-yl)pyridine-2-carbonitrile

A mixture of Intermediate 7 (107 mg, 0.35 mmol), 2-cyano-6-fluoropyridine (44 mg, 0.36 mmol), and potassium carbonate (100 mg, 0.71 mmol) was dissolved in dimethylsulfoxide (2 mL) and heated at 100 °C overnight. The cooled reaction mixture was then treated with an aqueous work up, the organic solvent dried (Na₂SO₄) and concentrated under reduced pressure. A third of the crude residue was purified by reverse phase column chromatography to give the title compound (19.5 mg, 14% yield).
LCMS (Method 1, ES⁺) 2.53 min, 402 m/z (M+H)⁺.

### Example 104

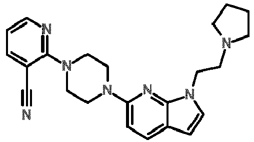

### 2-(4-{1-[2-(pyrrolidin-1-yl)ethyl]-1H-pyrrolo[2,3-b]pyridin-6-yl}piperazin-1-yl)pyridine-3-carbonitrile

A mixture of intermediate 7 (90 mg, 0.30 mmol) and 3-cyano-2-fluoropyridine (45 mg, 0.36 mmol) was dissolved in tetrahydrofuran (2 mL) and triethylamine (0.1 mL, 0.7 mmol) added. The resulting yellow solution was heated at 100 °C overnight. The cooled reaction mixture was then treated with an aqueous work up and the organic layer concentrated under reduced pressure. Half of the crude residue was purified by reverse phase column chromatography to give the title compound (31.2 mg, 25% yield).
LCMS (Method 1, ES⁺) 2.39 min, 402 m/z (M+H)⁺.

### Example 105

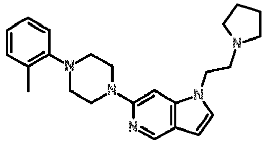

### 6-[4-(2-methylphenyl)piperazin-1-yl]-1-[2-(pyrrolidin-1-yl)ethyl]-1H-pyrrolo[3,2-c]pyridine

Example 105 was prepared according to General Procedure 2 with Intermediate 22 (46 mg, 0.16 mmol) and 1-(2-chloroethyl)pyrrolidine hydrochloride (33 mg, 0.19 mmol). Reverse phase column chromatography for the final purification gave the title compound (16 mg, 26% yield).
LCMS (Method 1, ES⁺) 2.61 min, 390 m/z (M+H)⁺.

### Example 106

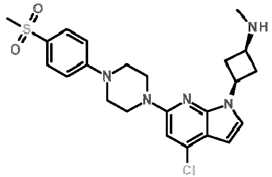

### cis-3-(4-chloro-6-{4-[4-(methylsulfonyl)phenyl]piperazin-1-yl}-1H-pyrrolo[2,3-b]pyridin-1-yl)-N-methylcyclobutanamine

Intermediate 24 (330 mg, 1 eq.) and 1-[4-(methylsulfonyl)phenyl]piperazine (1.1 eq., 0.88 mmol) were reacted following General Procedure 3. The residue was purified by flash column chromatography using gradient elution from hexane to EtOAc. The obtained product was dissolved in DCM (5 mL) and TFA (1 mL) added. The mixture was stirred for 1 hour at ambient temperature. Then the mixture was filtered through an SCX-column and rinsed with DCM and MeOH. The product was eluted with NH₃ (4N) in MeOH. The volatiles were removed under vacuum and then the residue was purified by reverse phase column chromatography to yield the title compound as a white solid (19 mg, 5% yield).
LCMS (Method 1, ES⁺) 1.81 min, 474 m/z (M+H)⁺.

### Example 107

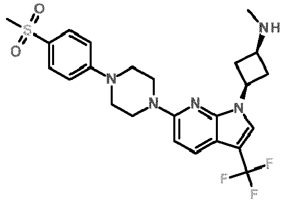

### cis-N-methyl-3-[6-{4-[4-(methylsulfonyl)phenyl]piperazin-1-yl}-3-(trifluoromethyl)-1H-pyrrolo[2,3-b]pyridin-1-yl]cyclobutanamine

Intermediate 25 (160 mg, 0.36 mmol) and 1-[4-(methylsulfonyl)phenyl]piperazine (1.1 eq., 0.4 mmol) were reacted following General Procedure 3. The residue was purified by flash column chromatography using gradient elution from hexane to EtOAc. The resulting residue was dissolved in DCM (5 mL) and TFA (2 mL) was added. The mixture was stirred at ambient temperature for 2 hours and the volatiles removed under vacuum. The residue was purified by flash column chromatography using gradient elution from hexane to EtOAc, and then 20% MeOH in EtOAc to give Example 107 (2 mg, 8% yield).
LCMS (Method 1, ES⁺) 1.92 min, 408 m/z (M+H)⁺.

### Example 108

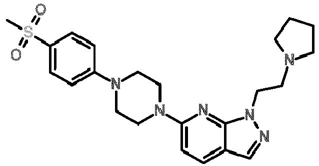

### 6-{4-[4-(methylsulfonyl)phenyl]piperazin-1-yl}-1-[2-(pyrrolidin-1-yl)ethyl]-1H-pyrazolo[3,4-b]pyridine

6-bromo-1H-pyrrolo[3,4-b]pyridine (100 mg, 0.51 mmol) and 1-(2-chloroethyl)pyrrolidine hydrochloride (1.2 eq., 0.61 mmol) were reacted following General Procedure 2. After work-up, the mixture was reacted with 1-[4-(methylsulfonyl)phenyl]piperazine (1.5 eq., 0.76 mmol) following General Procedure 3. The residue was purified by reverse phase column chromatography to yield the title compound as a white solid (8 mg, 3% yield).
LCMS (Method 1, ES⁺) 1.59 min, 455 *m*/*z* (M+H)⁺.

### Example 109

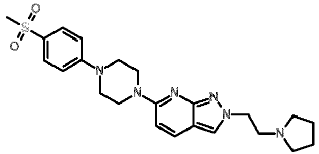

### 6-{4-[4-(methylsulfonyl)phenyl]piperazin-1-yl}-2-[2-(pyrrolidin-1-yl)ethyl]-2H-pyrazolo[3,4-b]pyridine

Example 109 was prepared according to the procedure described for Example 108 and isolated as an alternative regioisomer during purification.
LCMS (Method 1, ES⁺) 1.82 min, 455 *m*/*z* (M+H)⁺.

### Example 110

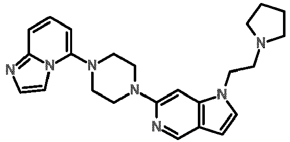

### 5-(4-{1-[2-(pyrrolidin-1-yl)ethyl]-1H-pyrrolo[3,2-c]pyridin-6-yl}piperazin-1-yl)imidazo[1,2-a]pyridine

Intermediate 27 (75 mg, 0.30 mmol) and Intermediate 5 (75 mg, 0.37 mmol) were reacted using General Procedure 3 to give the title compound (63 mg, 50% yield).
LCMS (Method 1, ES⁺) 1.79 min, 416 *m*/*z* (M+H)⁺.

### Example 111

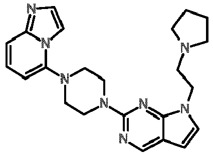

### 2-[4-(imidazo[1,2-a]pyridin-5-yl)piperazin-1-yl]-7-[2-(pyrrolidin-1-yl)ethyl]-7H-pyrrolo[2,3-d]pyrimidine

A mixture of 2-chloro-7H-pyrrolo[2,3-D]pyrimidine (120 mg, 0.76 mmol) and Intermediate 5 (117 mg, 0.58 mmol) were suspended in 1-butanol (3 mL) and trifluoroacetic acid (0.07 mL, 0.9 mmol) added. The reaction mixture was then heated at 120 °C overnight. The reaction mixture was cooled to room temperature and filtered through a SCX cartridge washing first with methanol then eluting with 2 M ammonia in methanol. The ammonia in methanol solution was concentrated under reduced pressure and the residue partially purified using silica column chromatography. The crude mixture was then reacted with 1-(2-chloroethyl)pyrrolidine hydrochloride (102 mg, 0.59 mmol) according to General Procedure 2 to give the title compound (38 mg, 15% yield).
LCMS (Method 1, ES⁺) 1.92 min, 417 *m*/*z* (M+H)⁺.

### Example 112

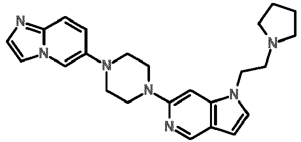

### 6-(4-{1-[2-(pyrrolidin-1-yl)ethyl]-1H-pyrrolo[3,2-c]pyridin-6-yl}piperazin-1-yl)imidazo[1,2-a]pyridine

The title compound was prepared according to General Procedure 3 using intermediate 28 (48 mg, 0.16 mmol) instead of intermediate 7. The crude reaction mixture was directly filtered through celite followed by SCX filtration. The SCX cartridge was washed with methanol and the product eluted with 2 M ammonia in methanol. Final purification of the residue by reverse phase column chromatography gave the title compound (3 mg, 4% yield).
LCMS (Method 1, ES⁺) 1.58 min, 416 *m*/*z* (M+H)⁺.

### Example 113

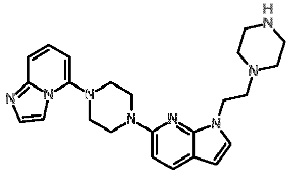

### 5-(4-{1-[2-piperazin-1-yl)ethyl]-1H-pyrrolo[2,3-b]pyridin-6-yl}piperazin-1-yl)imidazo[1,2-a]pyridine

Using General Procedure 3, Intermediate 31 (175 mg, 0.43 mmol) and Intermediate 5 (90 mg, 0.44 mmol) gave tert-butyl 4-[2-[6-(4-imidazo[1,2-a]pyridin-6-ylpiperazin-1-yl)pyrrolo[2,3-b]pyridin-1-yl]ethyl]piperazine-1-carboxylate. This was then dissolved in dichloromethane (3 mL) and treated with trifluoracetic acid (1 mL). The reaction mixture was stirred at room temperature for 36 h then passed through an SCX cartridge, washing first with methanol then eluting with 2 M ammonia in methanol. The ammonia in methanol solution was concentrated under reduced pressure and the residue purified by reverse phase chromatography to give the title compound (10 mg, 8% yield).
LCMS (Method 1, ES⁺) 1.69 min, 431 *m*/*z* (M+H)⁺.

### Example 114

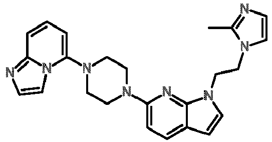

### 5-(4-{1-[2-(2-methyl-1H-imidazol-1-yl)ethyl]-1H-pyrrolo[2,3-b]pyridin-6-yl}piperazin-1-yl)imidazo[1,2-a]pyridine

Intermediate 29 (100 mg, 0.28 mmol) and intermediate 5 (60 mg, 0.29 mmol) were treated according to General Procedure 3 to give the title compound (17 mg, 13% yield).
LCMS (Method 1, ES⁺) 1.82 min, 427 *m*/*z* (M+H)⁺.

### Example 115

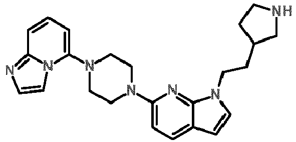

### 5-(4-{1-[2-pyrrolidin-3-yl)ethyl]-1H-pyrrolo[2,3-b]pyridin-6-yl}piperazin-1-yl)imidazo[1,2-a]pyridine

Intermediate 32 (130 mg, 0.28 mmol) and Intermediate 5 (60 mg, 0.29 mmol) were treated according to General Procedure 3 to give tert-butyl 3-[2-[6-(4-imidazo[1,2-a]pyridin-5-ylpiperazin-1-yl)pyrrolo[2,3-b]pyridin-1-yl]ethyl]pyrrolidine-1-carboxylate, which was dissolved in DCM (4 mL) and treated with TFA (1 mL). The reaction mixture was stirred at room temperature for 3 hours then passed through an SCX cartridge, washing first with methanol then eluting with 2 M ammonia in methanol. The ammonia in methanol solution was concentrated under reduced pressure and a fraction of the residue (50 mg) purified by reverse phase chromatography to give the title compound (11 mg).
LCMS (Method 1, ES⁺) 1.62 min, 416 *m*/*z* (M+H)⁺.

### Example 116

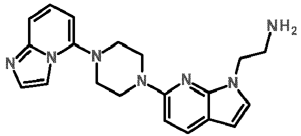

### 2-{6-[4-(imidazo[1,2-a]pyridin-5-yl)piperazin-1-yl]-1H-pyrrolo[2,3-b]pyridin-1-yl}ethanamine

Intermediate 33 (110 mg, 0.23 mmol) was dissolved in dichloromethane (3 mL) and trifluoroacetic acid (0.5 mL) added. The reaction mixture was stirred at room temperature for 3 h then passed through an SCX cartridge eluting with methanol then with 2 M ammonia in methanol. 20% of the resulting residue was purified by reverse phase column chromatography to afford the title compound (6 mg, 7% yield)
LCMS (Method 1, ES⁺) 1.60 min, 362 *m*/*z* (M+H)⁺.

### Examples 117-118

The following compounds were synthesized from Intermediate 7 and the appropriate aryl bromide in accordance with General Procedure 3.

Examples were analysed by LCMS Method 3.

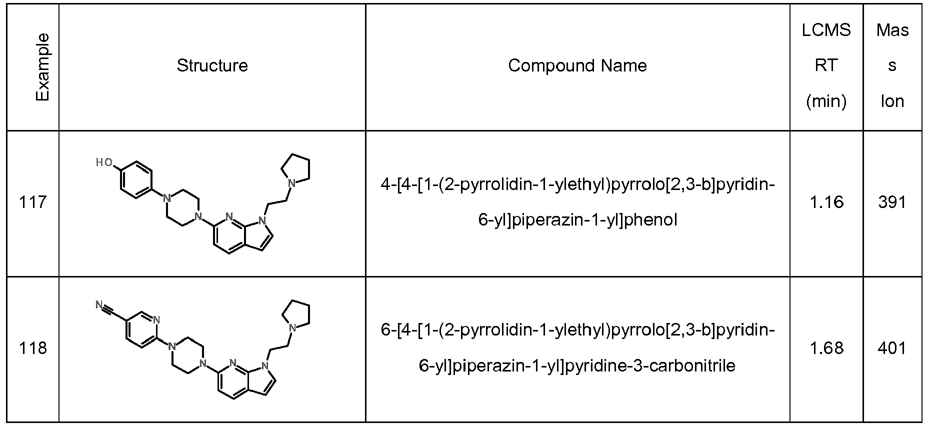

### Example 119

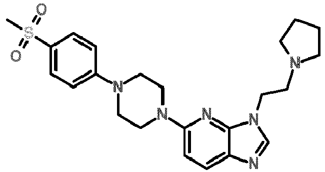

### 5-{4-[4-(methylsulfonyl)phenyl]piperazin-1-yl}-3-[2-(pyrrolidin-1-yl)ethyl]-3H-imidazo[4,5-b]pyridine

5-Bromo-1H-imidazo[4,5,b]pyridine (150 mg, 0.75 mmol) and 1-(2-chloroethyl)pyrrolidine hydrochloride (1.2 eq., 0.8 mmol) were reacted following General Procedure 2. After an aq. work-up, the crude residue was reacted with 1-[4-(methylsulfonyl)phenyl]piperazine (1.5 eq.) following General Procedure 3. The residue was purified by reverse phase column chromatography to yield Example 119 (2 mg, 1% yield).
LCMS (Method 1, ES+) 1.52 min, 455 m/z (M+H)⁺.

### Example 120

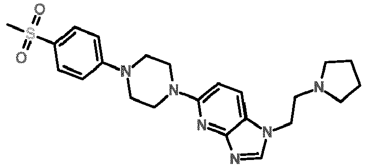

### 5-{4-[4-(methylsulfonyl)phenyl]piperazin-1-yl}-1-[2-(pyrrolidin-1-yl)ethyl]-1H-imidazo[4,5-b]pyridine

Example 120 was prepared according to the procedure described for Example 119 and isolated as an alternative regioisomer during purification.
LCMS (Method 1, ES⁺) 1.61 min, 455 m/z (M+H)⁺.

### Example 121

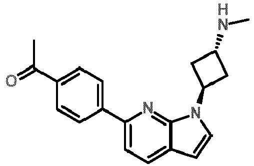

### 1-(4-{1-[trans-3-(methylamino)cyclobutyl]-1H-pyrrolo[2,3-b]pyridin-6-yl}phenyl)ethanone

Intermediate 35 (100 mg, 0.3 mmol) and 4-acetylphenylboronic acid (1.5 eq., 0.4 mmol), were reacted following General Procedure 10. The residue was dissolved in DCM (0.05 M) and TFA (1 mL) was added at 0°C, then the reaction was stirred at ambient for an additional 2 hours. The residue was filtered over a SCX column and washed with DCM and MeOH, then eluted with methanolic NH₃ (4 M). The product was purified by reverse phase chromatography to yield Example 121 (35 mg, 42% yield).
LCMS (Method 1, ES+) 1.71 min, 320 m/z (M+H)⁺.

### Example 122

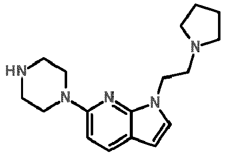

### 6-piperazin-1-yl-1-(2-pyrrolidin-1-ylethyl)pyrrolo[2,3-b]pyridine

Example 122 is also Intermediate 7. Synthesis and characterisation are described above.

### Examples 123-124

Examples 123-124 were prepared in two steps according to General Procedure 3 followed by general procedure 13 and analysed by LCMS Method 1.

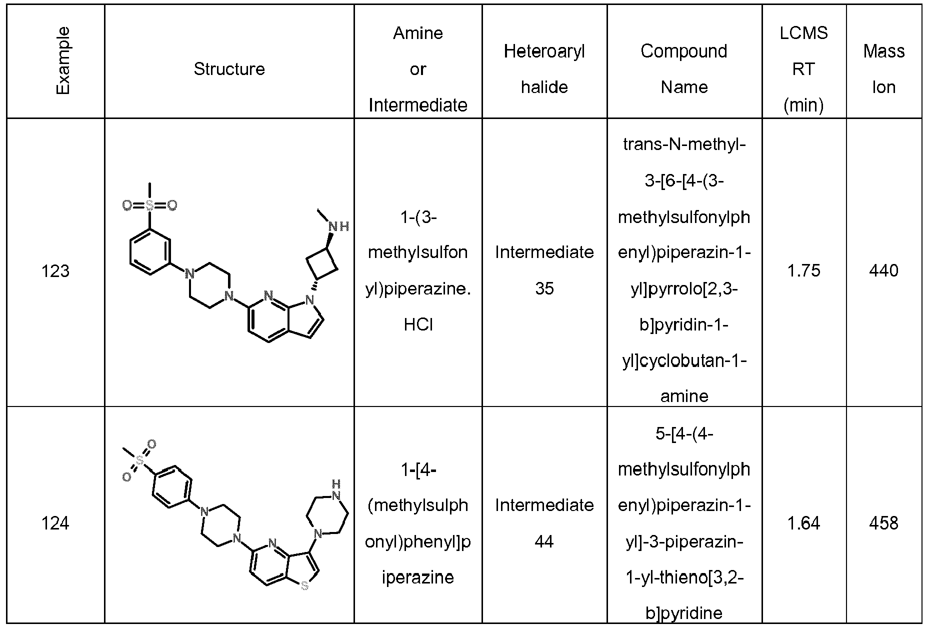

### Example 125

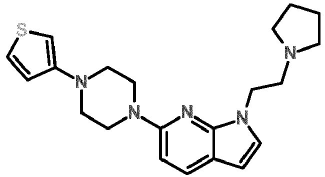

### 1-(2-pyrrolidin-1-ylethyl)-6-[4-(3-thienyl)piperazin-1-yl]pyrrolo[2,3-b]pyridine

Example 125 was prepared from Intermediate 7 and 3-bromothiophene in accordance with General Procedure 3 and analysed by LCMS Method 3.
LCMS (Method 3, ES⁺) 1.73 min, 382 m/z (M+H)⁺

### Example 126

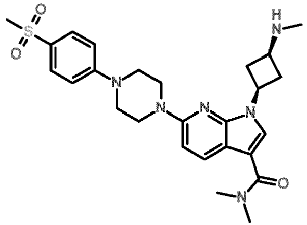

### cis-N,N-dimethyl-1-[3-(methylamino)cyclobutyl]-6-[4-(4-methylsulfonylphenyl)piperazin-1-yl]pyrrolo[2,3-b]pyridine-3-carboxamide

Intermediate 48 (20 mg, 0.05 mmol), dimethylamine (1.2 equiv., 0.06 mmol, 2 mmol/mL) and HATU (25 mg, 0.07 mmol, 1.4 equiv.) were dissolved in DMF (0.25 mL, 0.25 M) and DIPEA (0.04 mL, 0.2 mmol, 4 equiv) was added. The mixture was then stirred at room temperature under N₂ until the reaction was complete (1h). Then, DCM and NH₄Cl aq. sat solution were added to the reaction mixture and the layers were separated. The aq. phase was extracted with DCM, the layers were separated and the volatiles were removed under vacuum. The resulting residue was dissolved in 1,4-dioxane (0.05 M) and was reacted with 1-[4-(methylsulfonyl)phenyl]piperazine (1.5 equiv., 0.07 mmol) following General Procedure 3. The reaction mixture was cooled to r.t. and DCM and H₂O were added. The layers were separated and the aqueous phase was extracted with EtOAc. The combined organic layers were dried over Na₂SO₄ and the solvents were removed under vacuum.
The residue was dissolved in DCM (5 mL) and TFA (1mL) was added. The mixture was stirred at ambient temperature for 2 hours. The reaction crude was filtered through an SCX column and washed with MeOH and DCM. The product was eluted with NH₃ (4N) in MeOH. Volatiles were removed under vacuum and the residue purified by preparative chromatography to give Example 126 (8 mg, 34% yield).
LCMS (Method 1, ES⁺) 1.44 min, 511 m/z (M+H)⁺

### Example 127

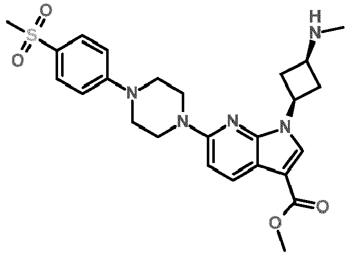

### cis-methyl 1-[3-(methylamino)cyclobutyl]-6-[4-(4-methylsulfonylphenyl)piperazin-1-yl]pyrrolo[2,3-b]pyridine-3-carboxylate

Intermediate 47 (100 mg, 0.17 mmol) was dissolved in DCM (8 mL, 0.02 M) and cooled to 0°C. TFA (2 mL) was added and the mixture stirred at r.t. for 2 h. The mixture was filtered through an SCX column and the column washed with MeOH and then eluted with 4 N NH₃ in MeOH. After removing the volatiles, the residue was purified by preparative column chromatography to afford Example 127 (11 mg, 13% yield).
LCMS (Method 1, ES⁺) 1.67 min, 498 m/z (M+H)⁺

### Examples 128-169

Examples 128-169 were prepared in two steps according to General Procedure 3 then 13.

Examples 128-152 were analysed by LCMS Method 1.

Examples 153-165 and example 168 were analysed by LCMS Method 11.

Examples 166-167 and example 169 were analysed by LCMS Method 13.

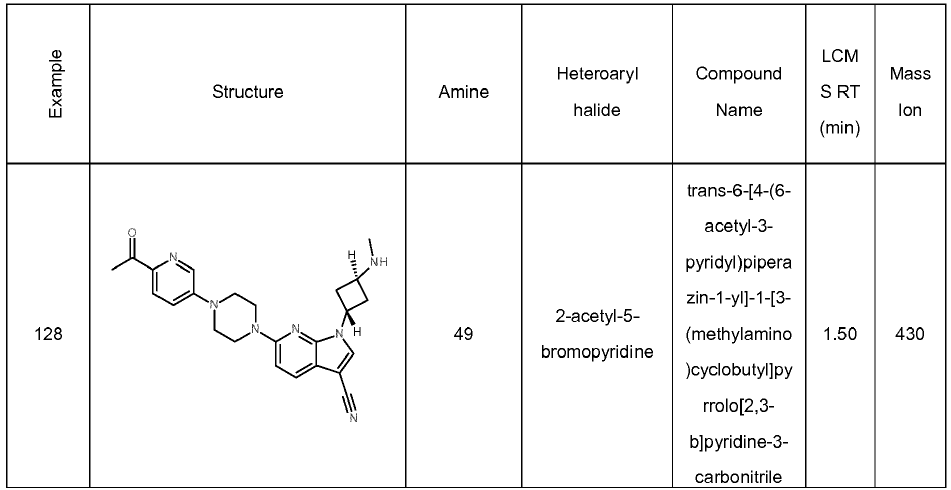

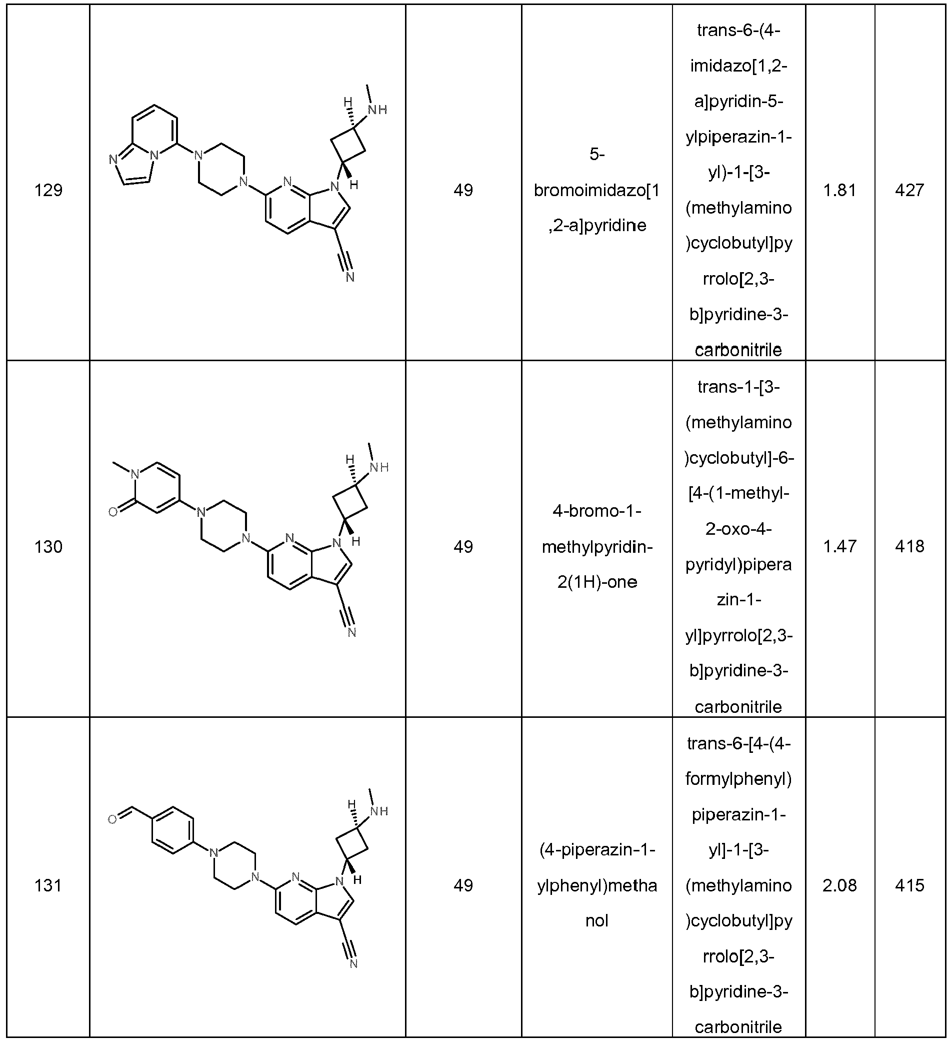

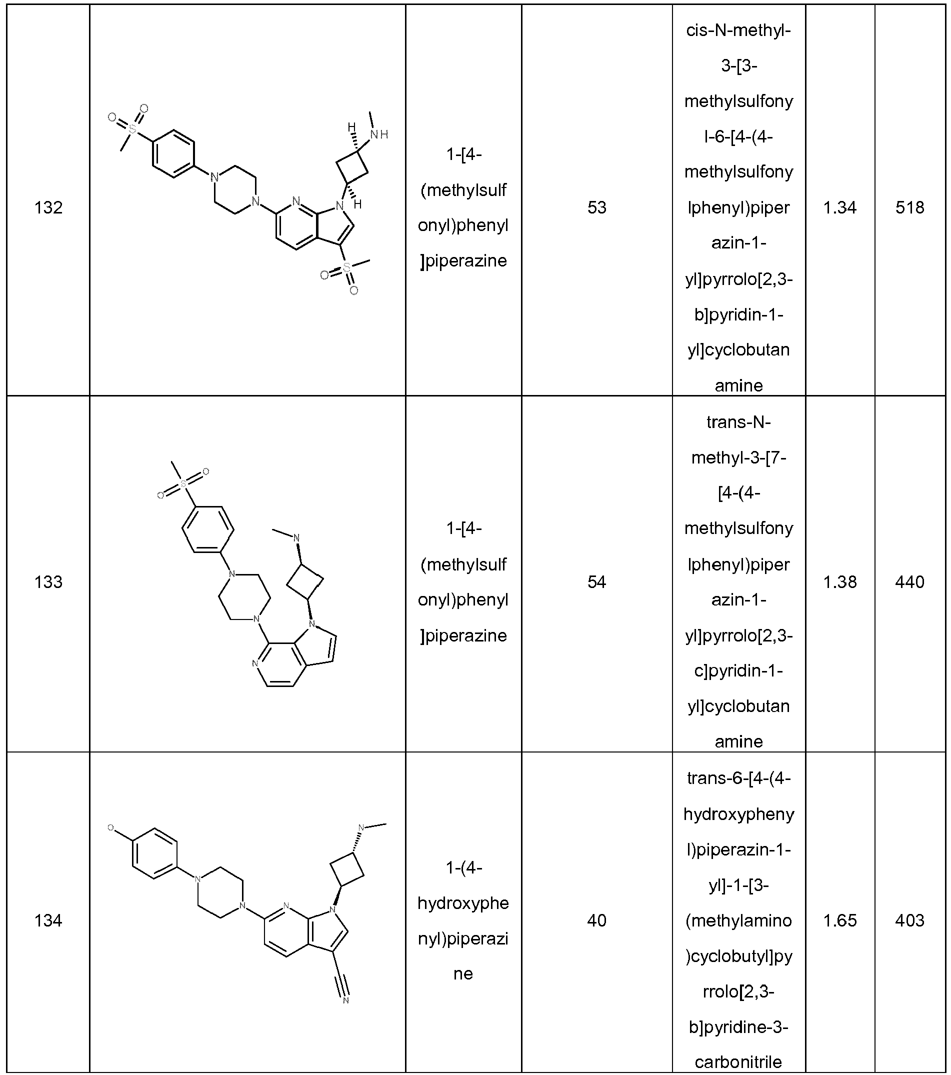

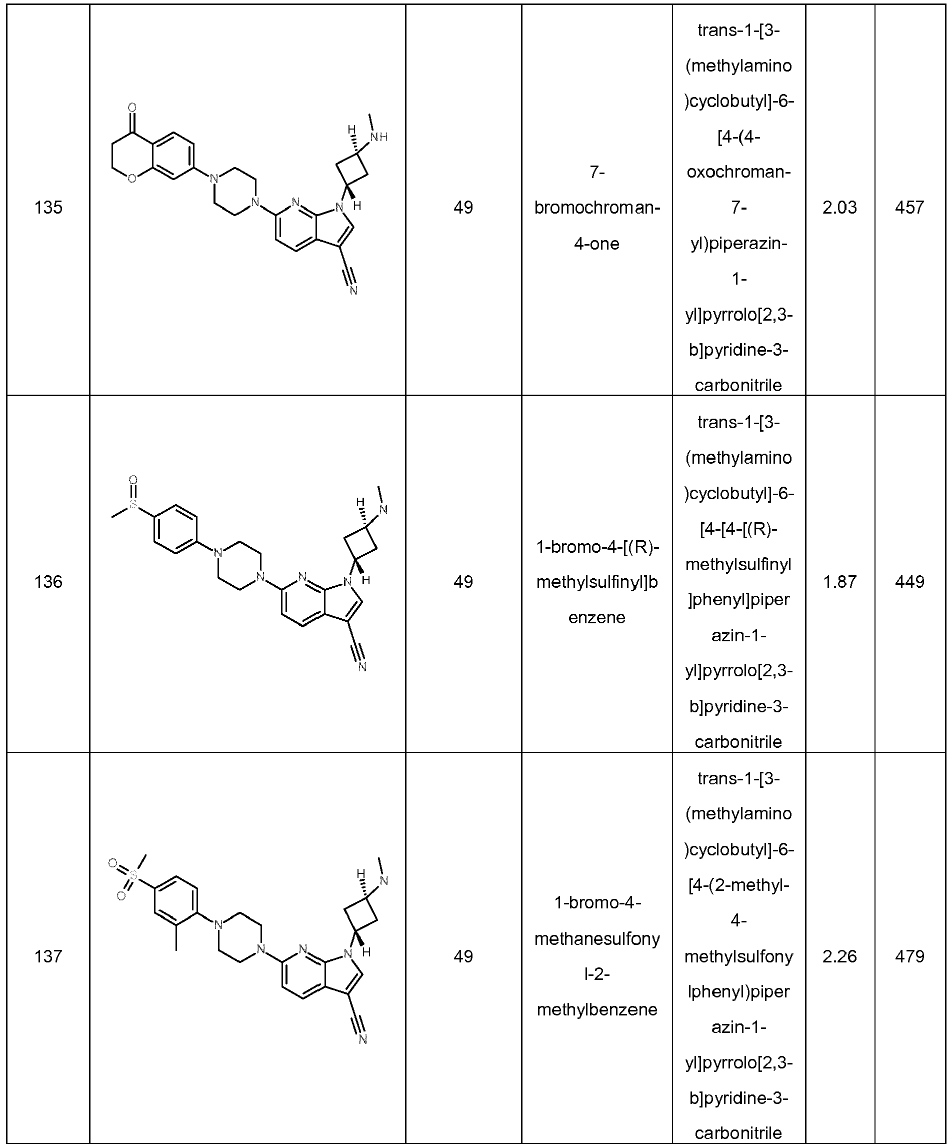

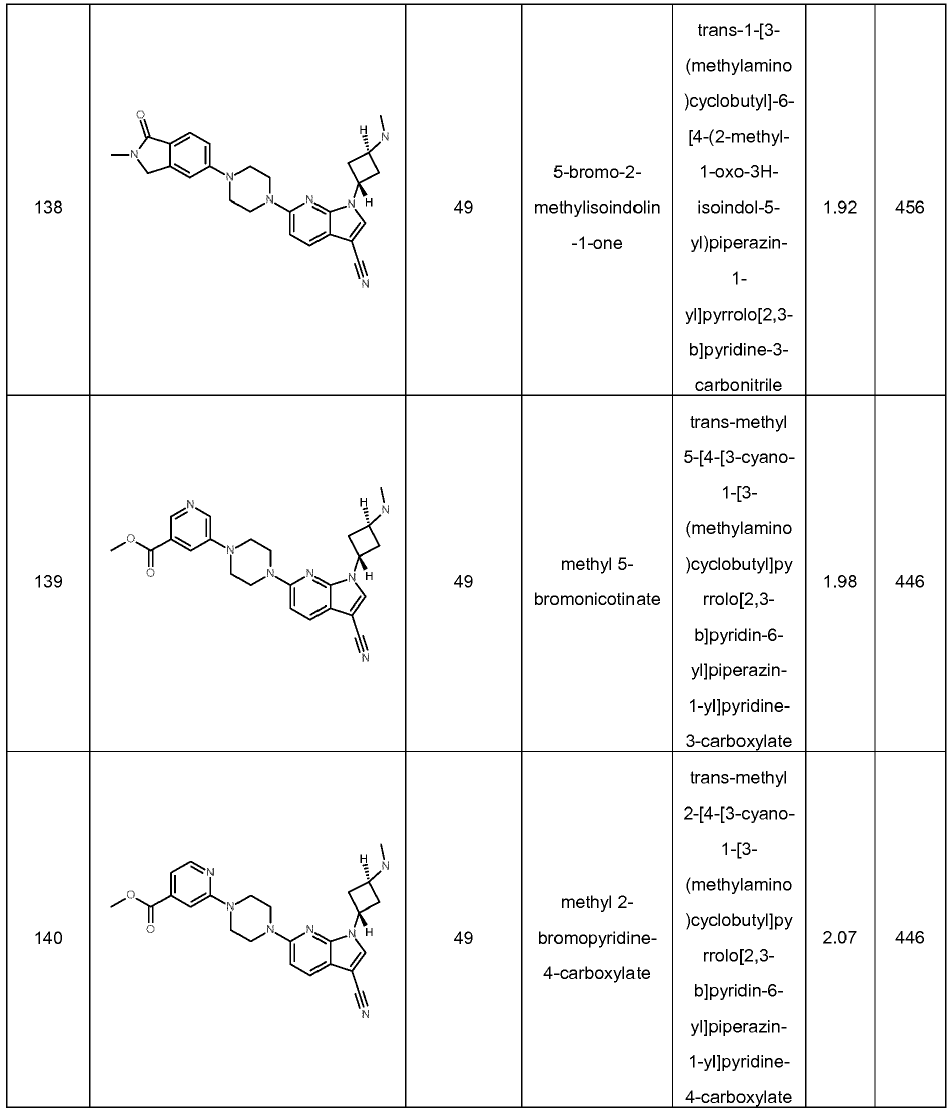

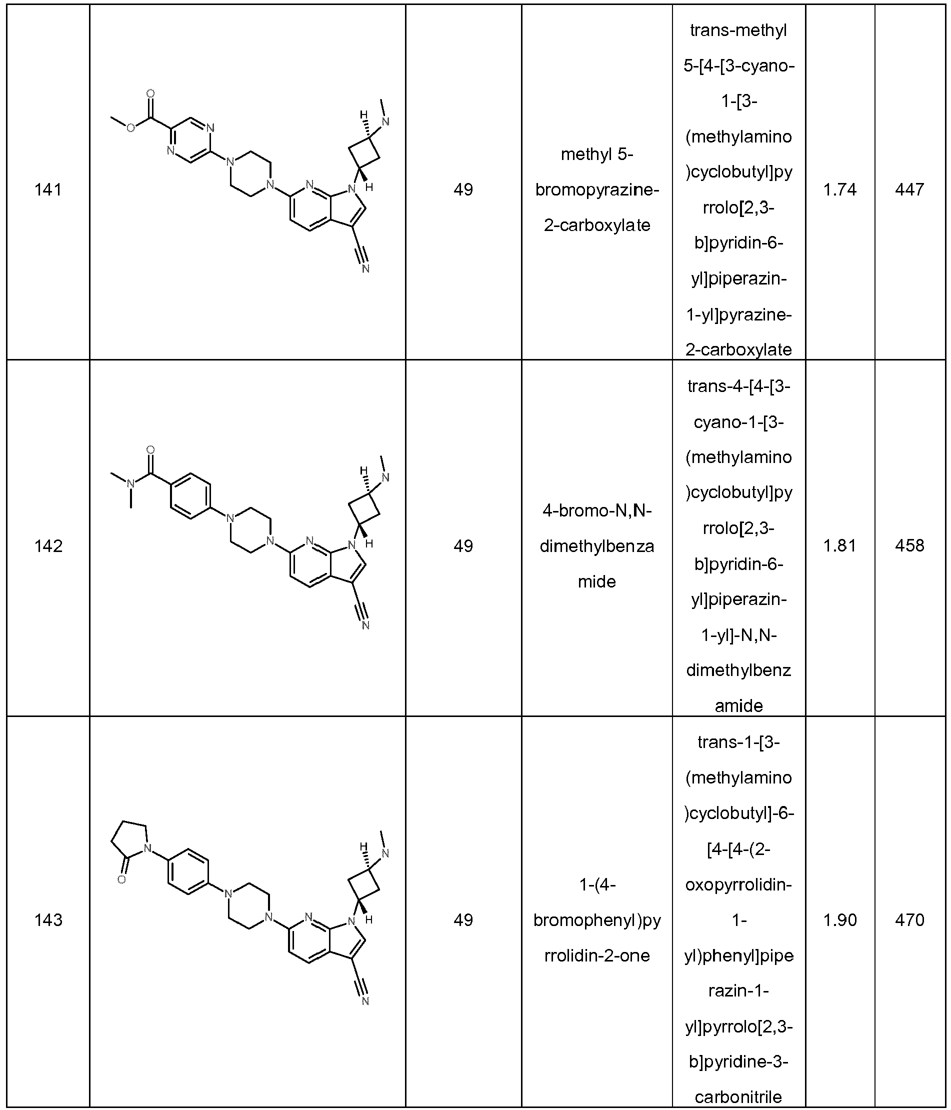

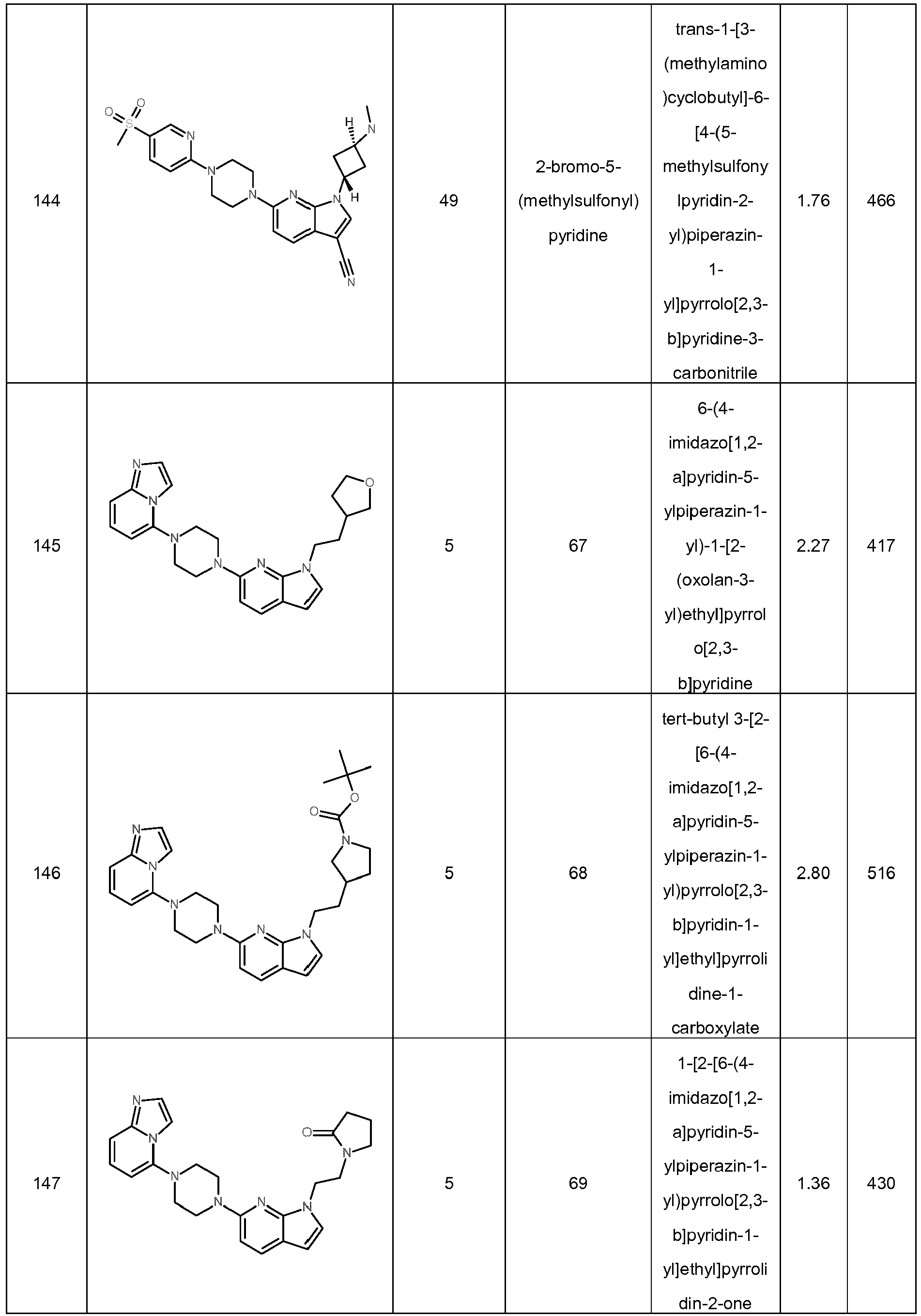

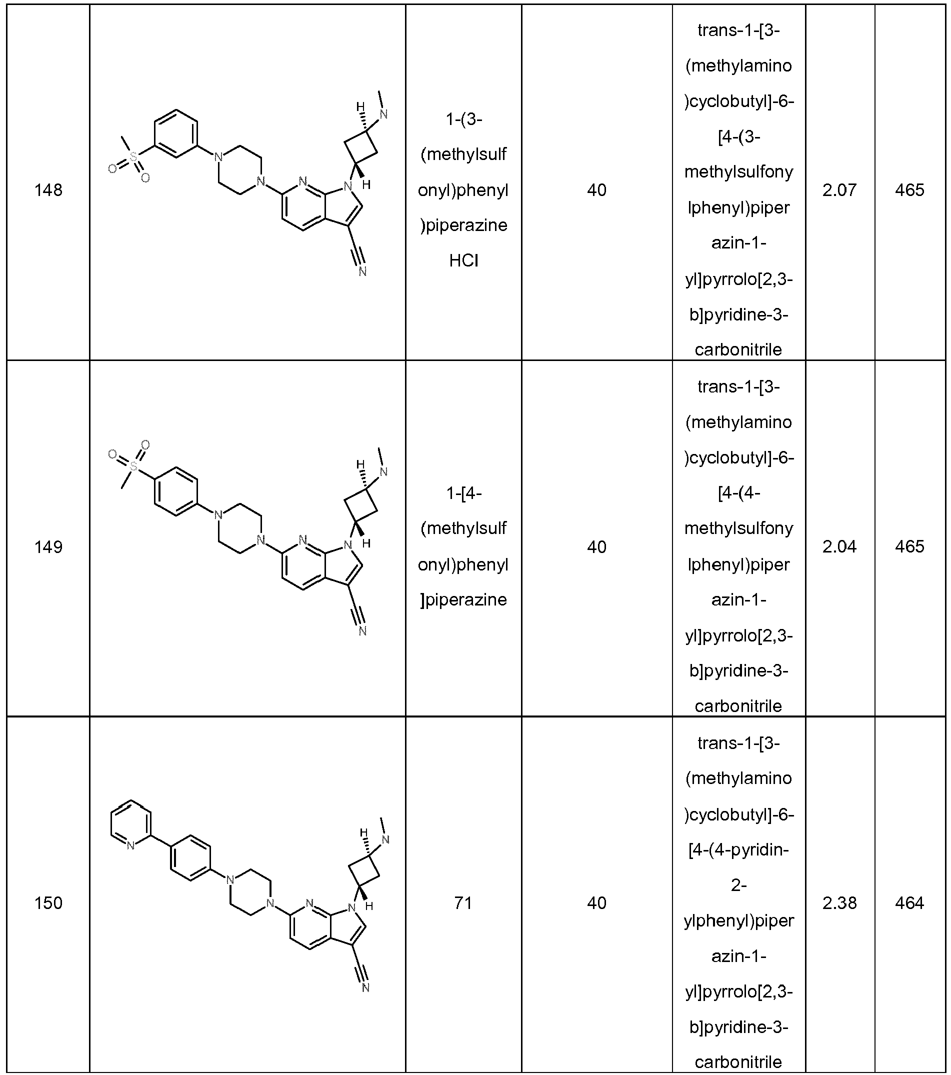

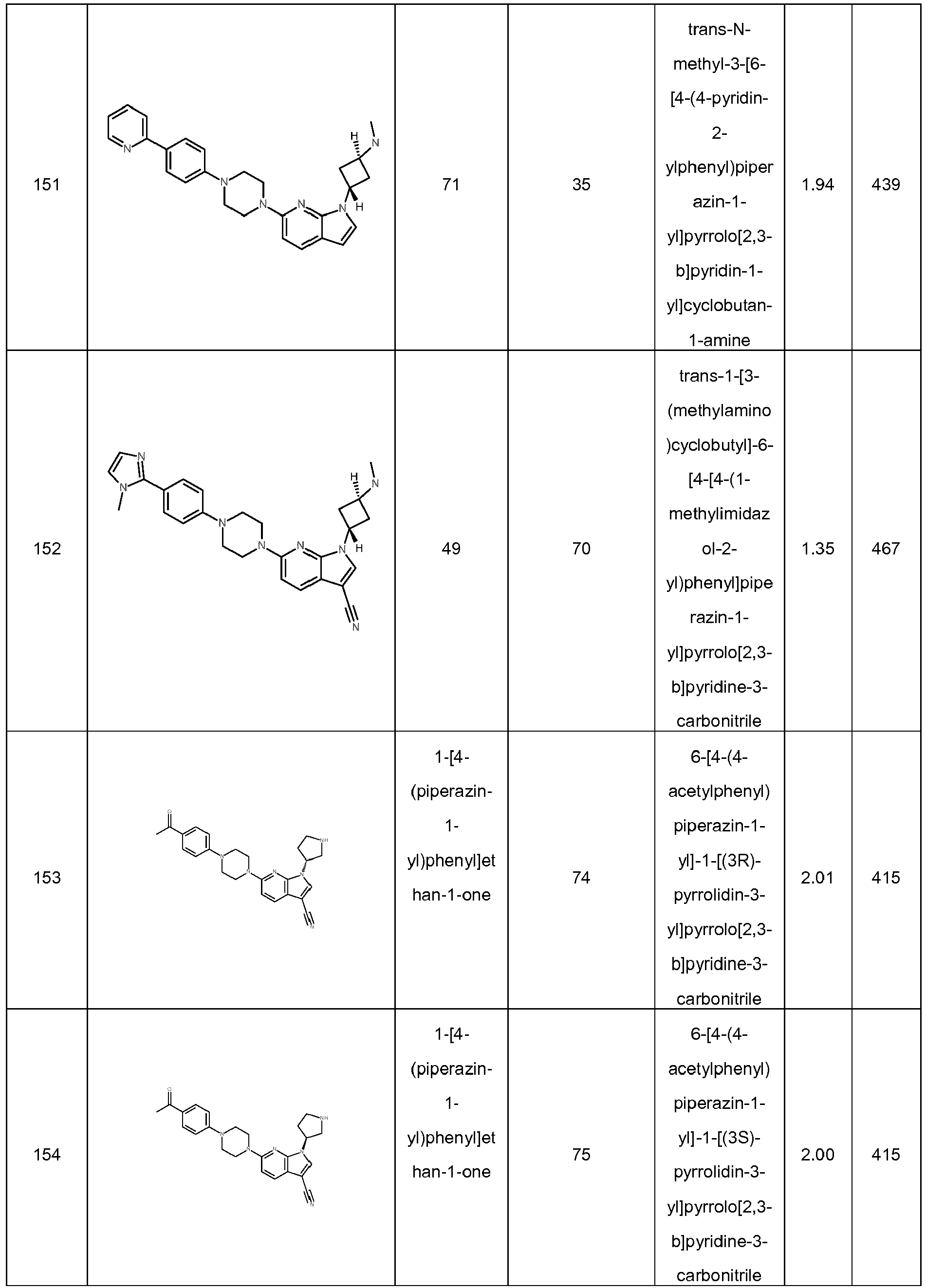

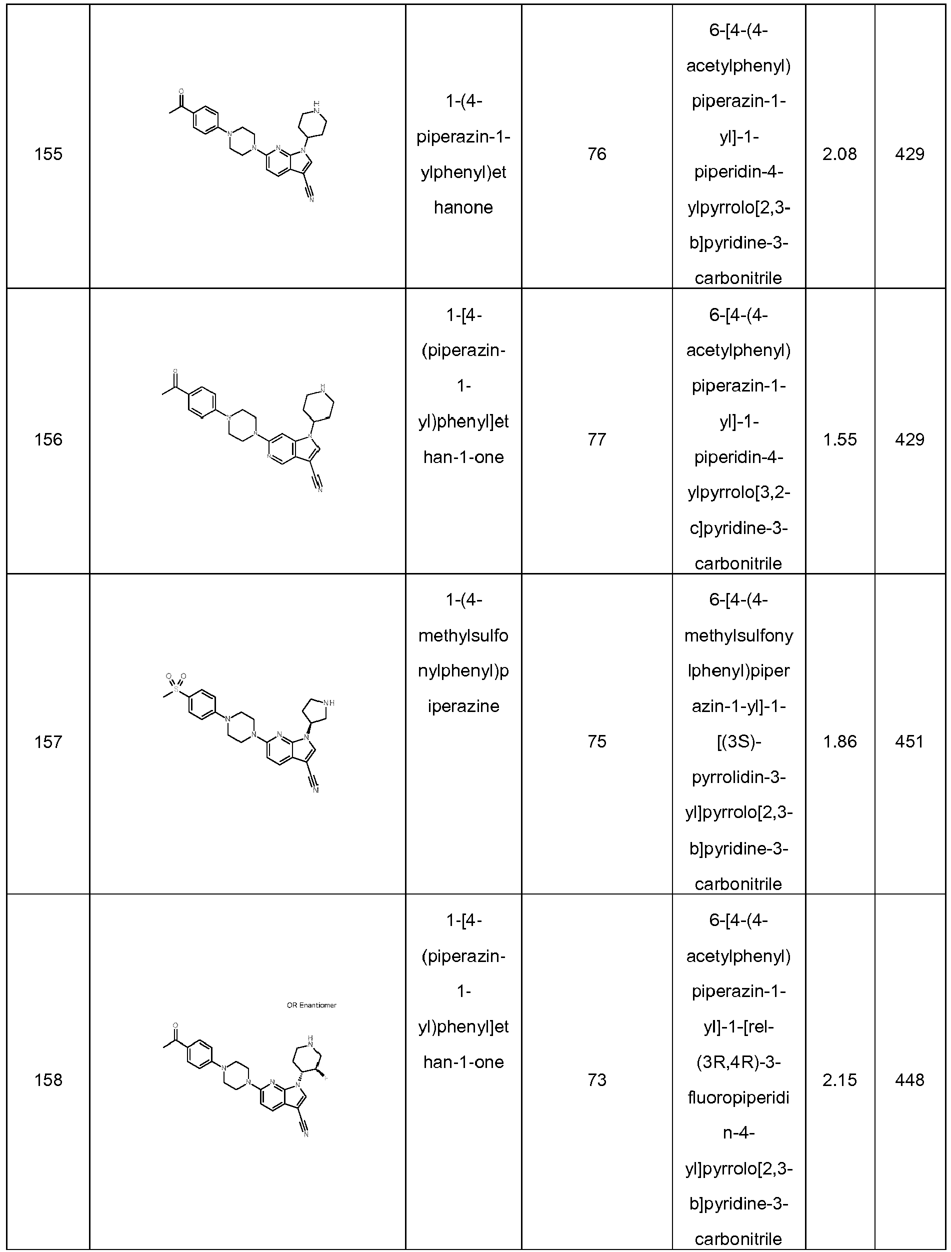

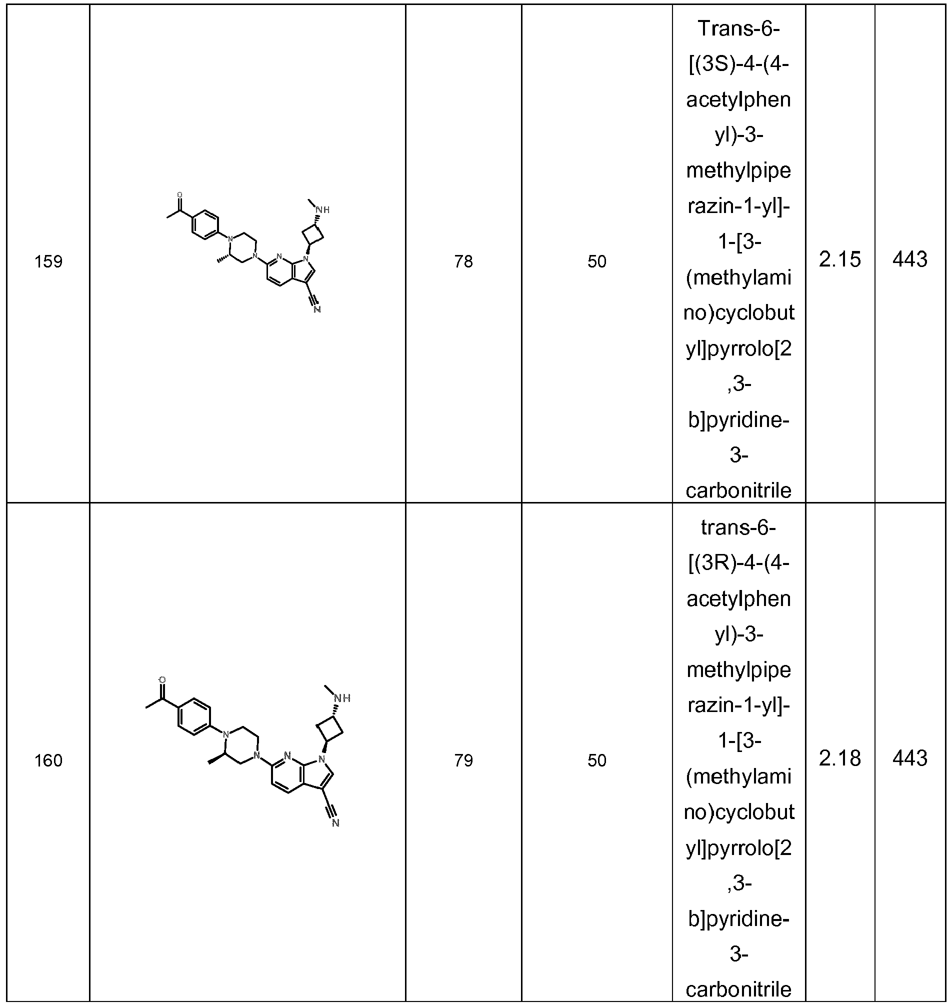

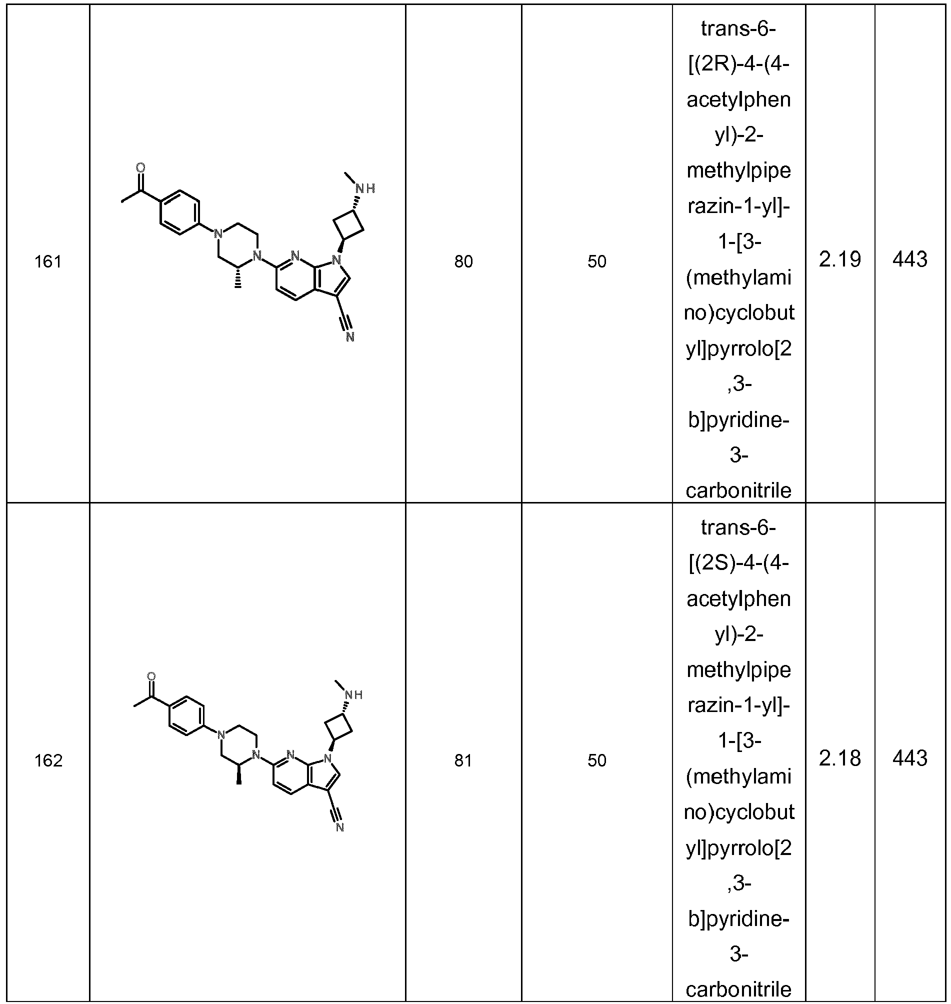

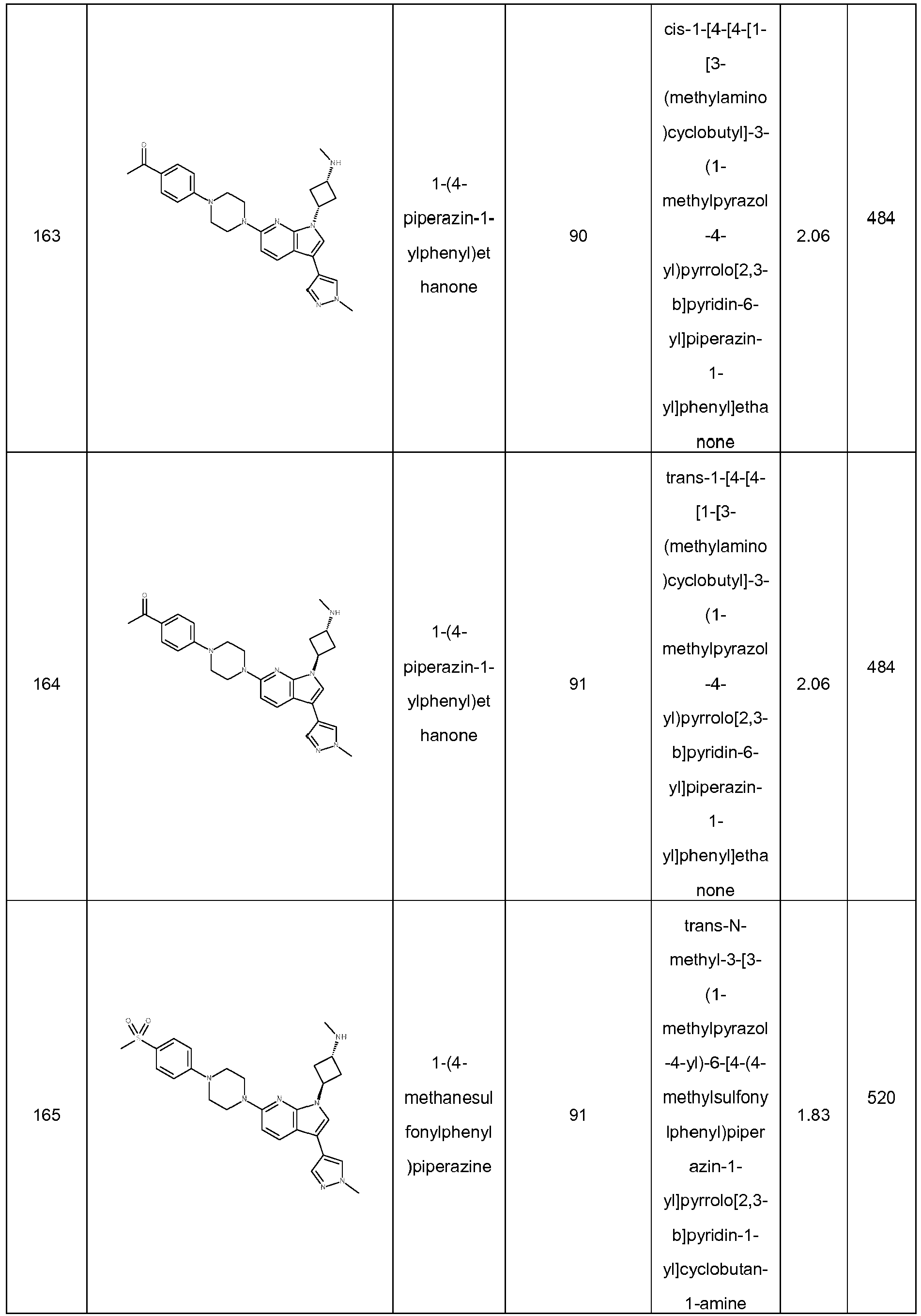

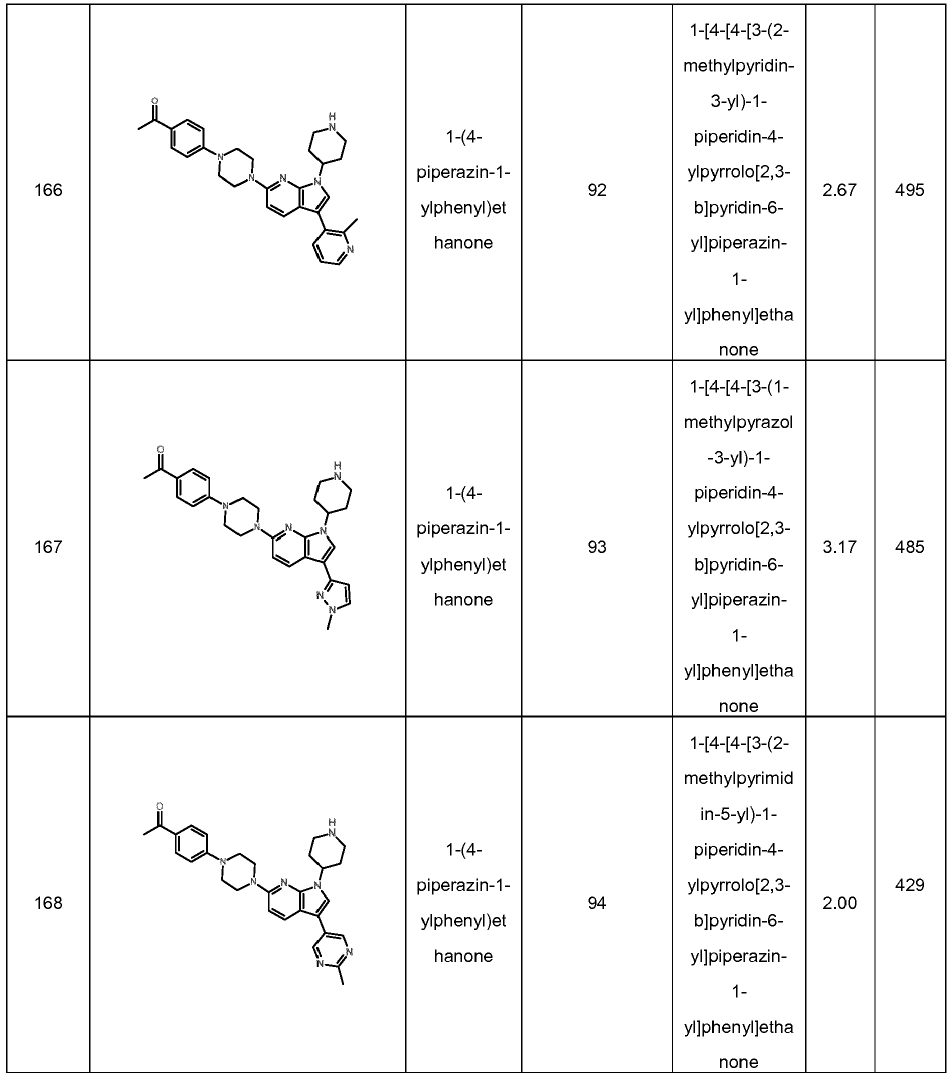

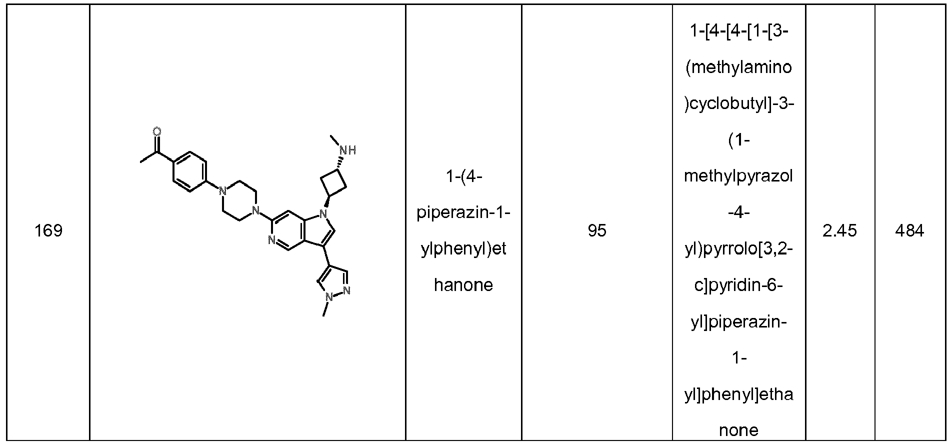

### Examples 170-186

Examples 170-186 were prepared in two steps according to General Procedure 14 then 13.

Examples 170-182 were analysed by LCMS Method 1. Examples 183-186 were analysed by LCMS Method 11.

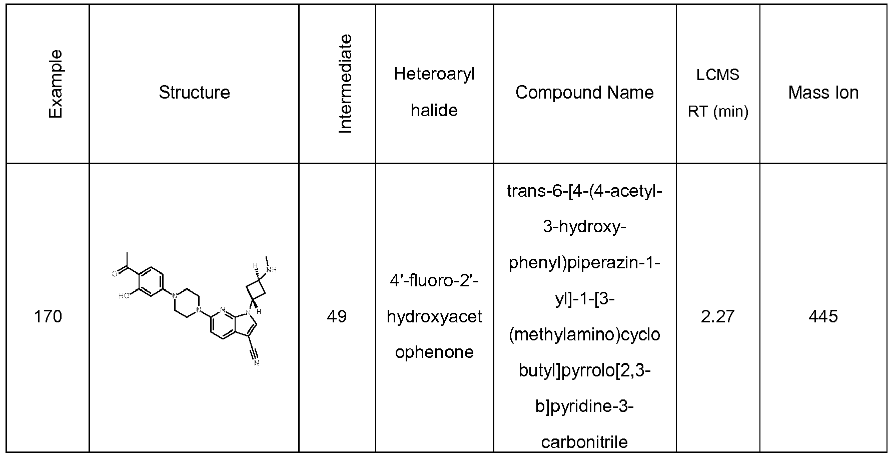

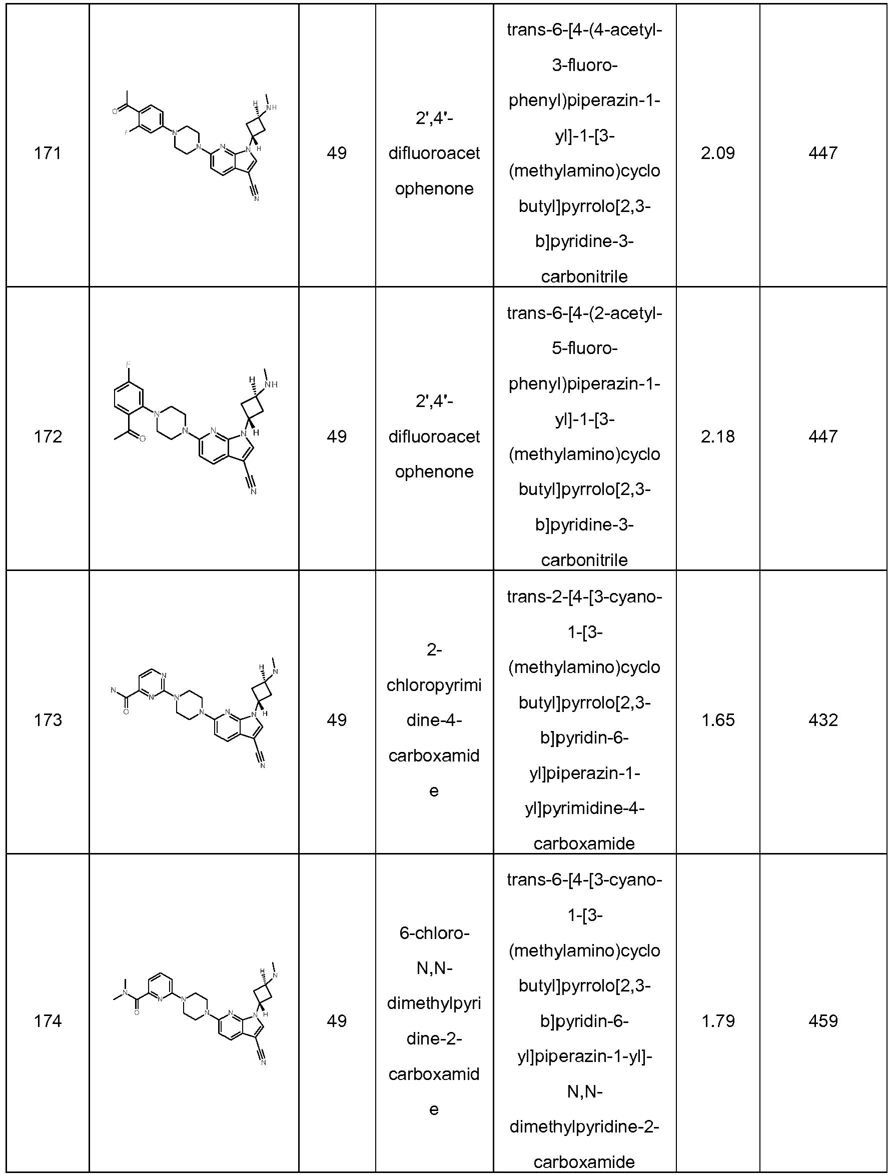

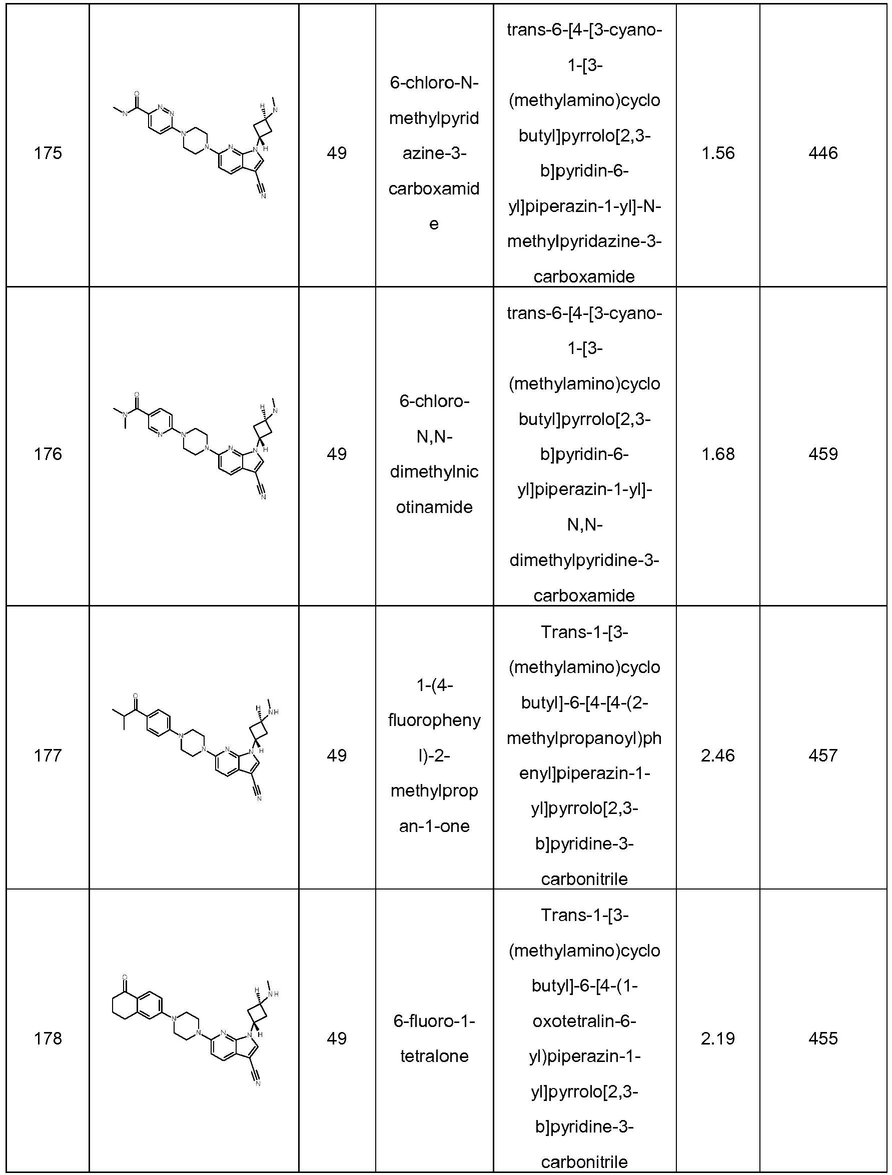

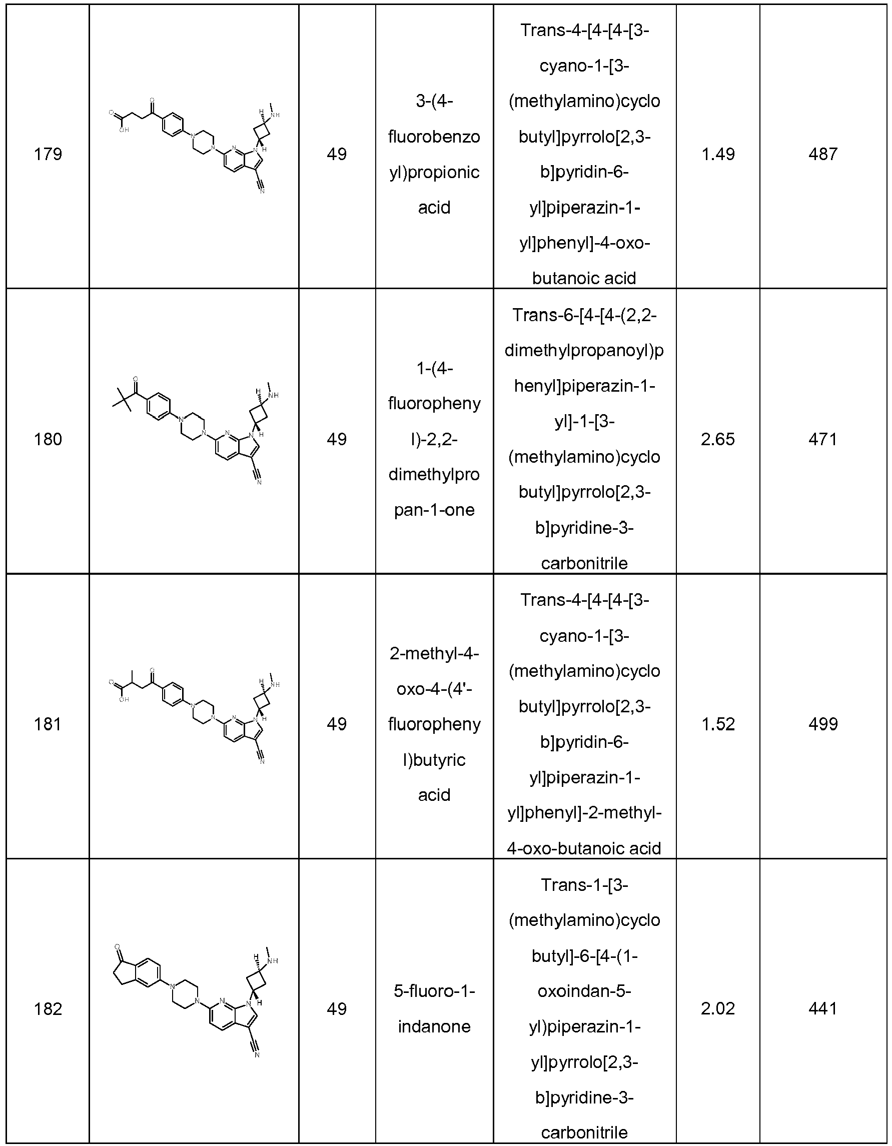

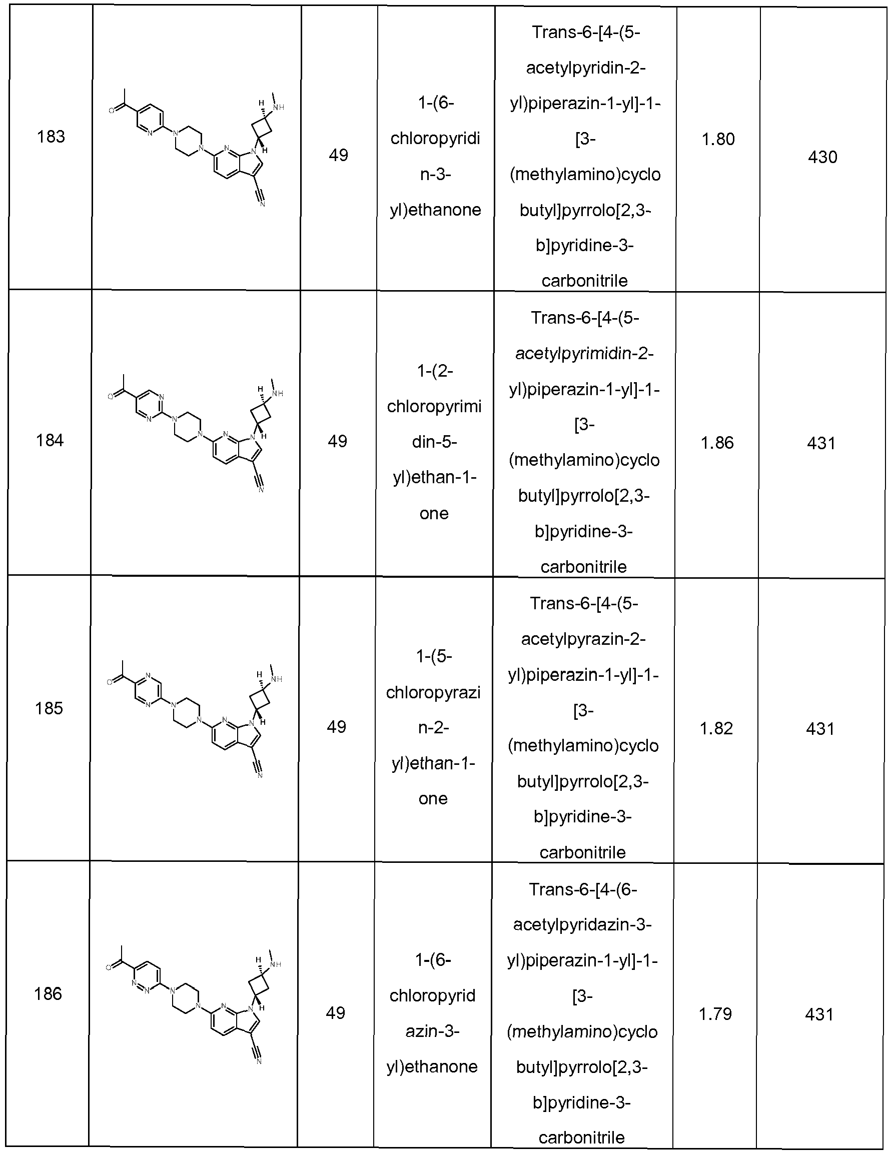

### Examples 187-188

Examples 187-188 were prepared in two steps according to General Procedure 18 then 13 and analysed by LCMS Method 1.

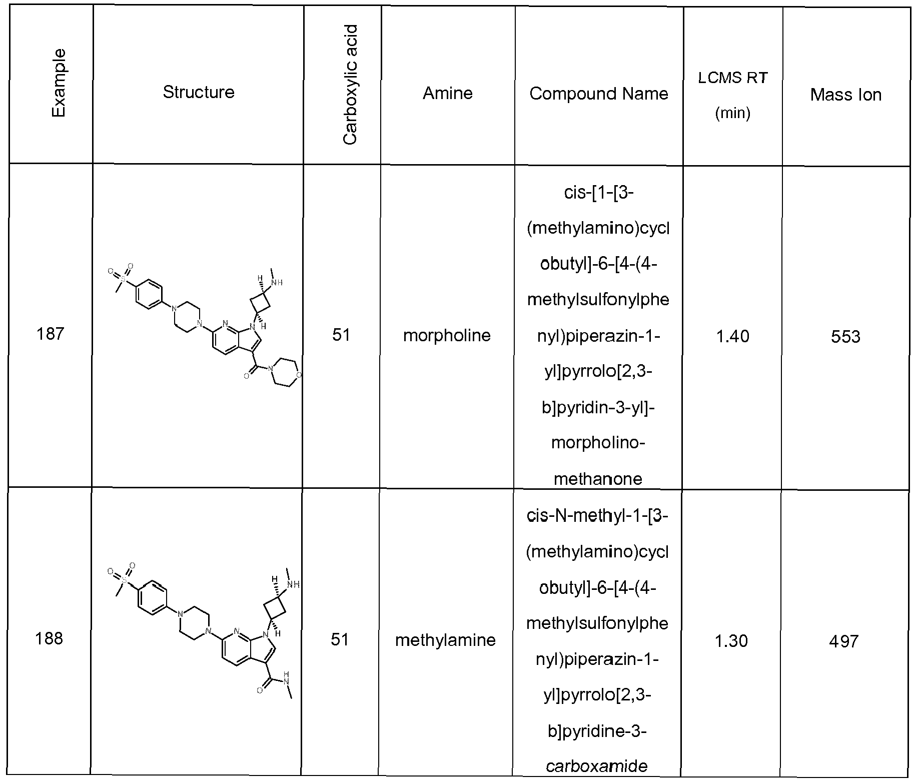

### Example 189

Example 189 was prepared in two steps according to General Procedure 9, 14 then 13 and analysed by LCMS Method 1.

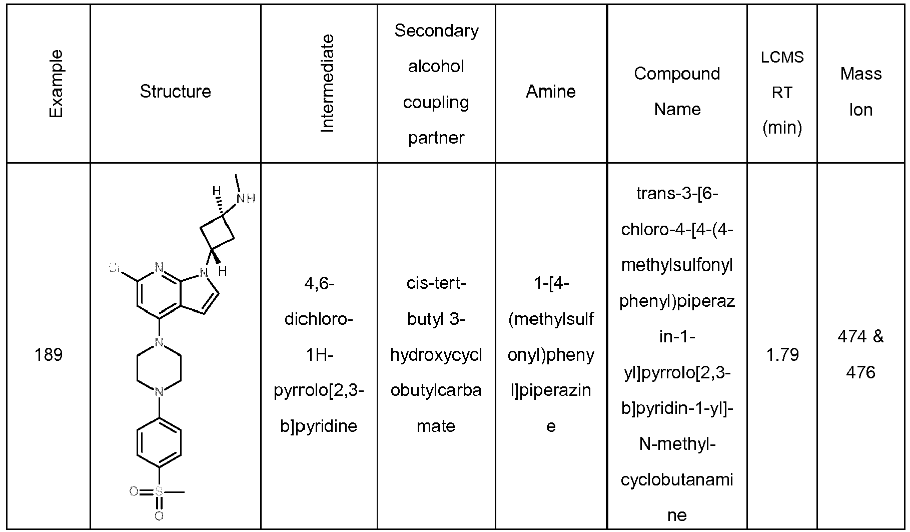

### Examples 190-193

Examples 190-193 were prepared in two steps according to General Procedure 12 then 13 and analysed by LCMS Method 1.

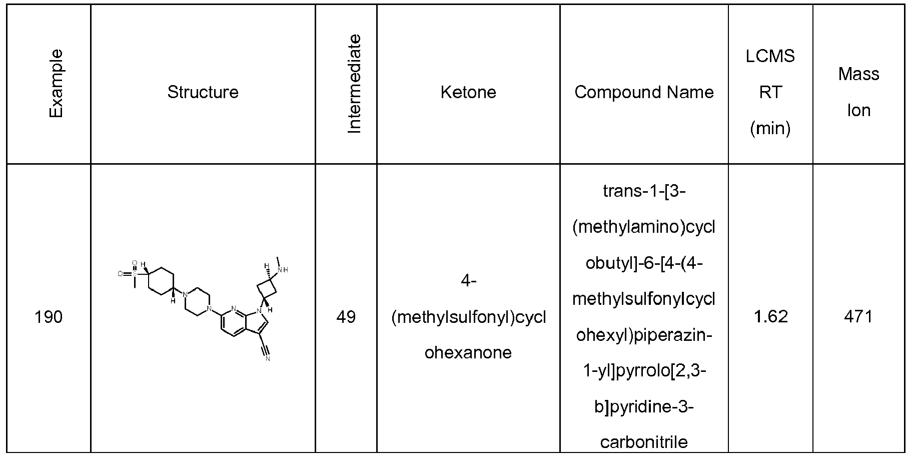

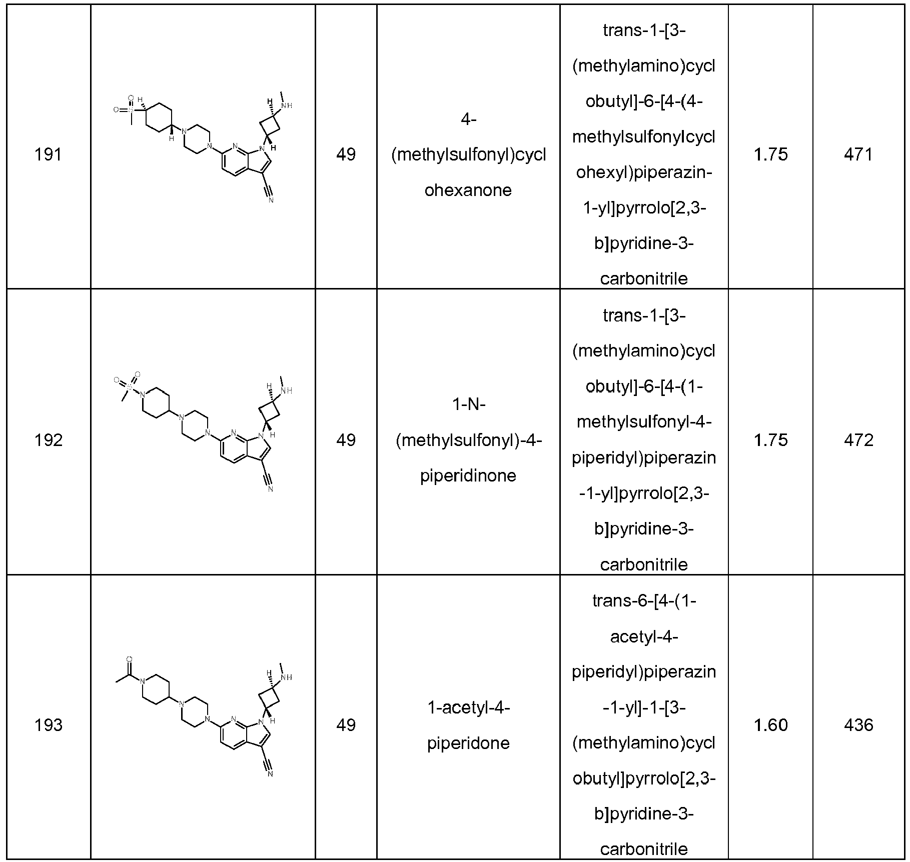

### Examples 194

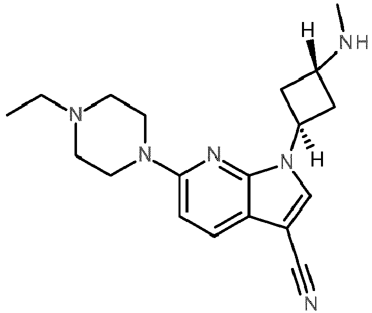

trans-6-(4-ethylpiperazin-1-yl)-1-[3-(methylamino)cyclobutyl]pyrrolo[2,3-b]pyridine-3-carbonitrile

Example 194, was isolated as a side-product in the synthesis of UCB1711019 Example 192
LCMS (Method 1, ES⁺) 1.71 min, 339 *m*/*z* (M+H)⁺

### Examples 195-199

Examples 195-199 were prepared according to General Procedure 12 from the specified starting material example (SM example) and analysed by LCMS Method 11.

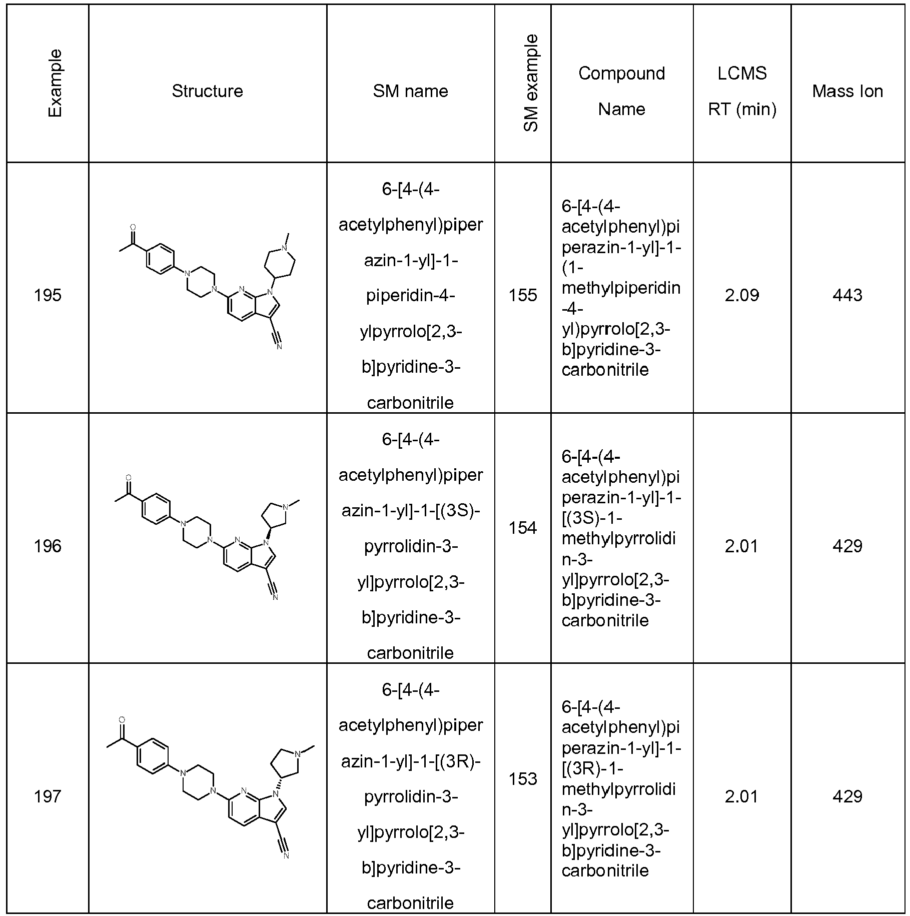

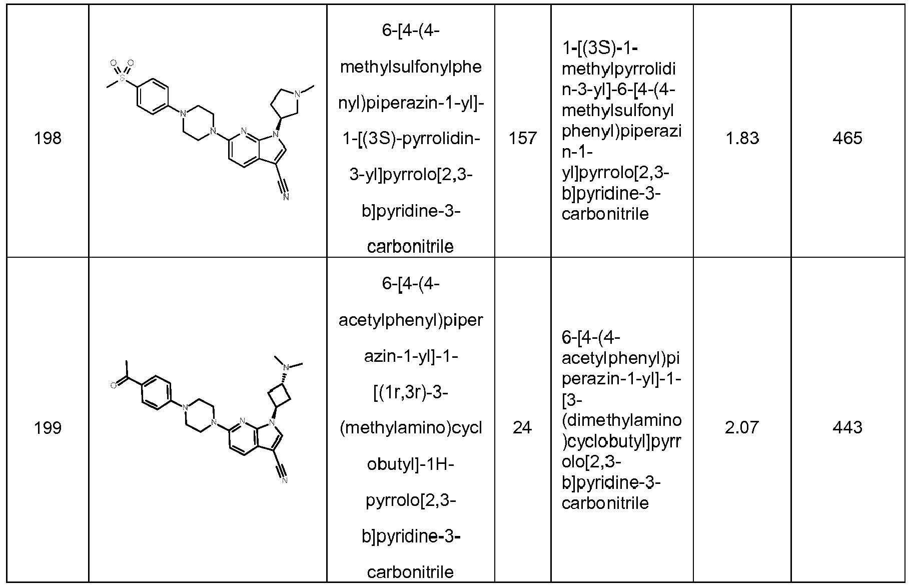

### Examples 200-201

Examples 200-201 were prepared in two steps according to General Procedure 5 (Example 200 used a modified procedure with toluene as solvent) then General Procedure 13.

Example 200 was analysed by LCMS Method 1. Example 201 was analysed by LCMS Method 9.

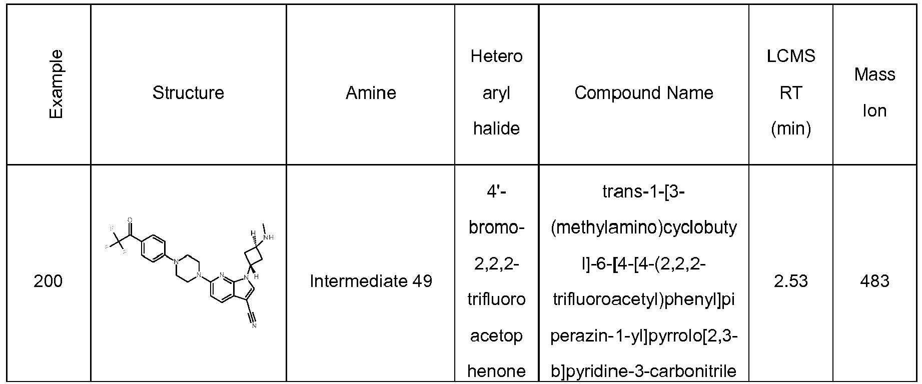

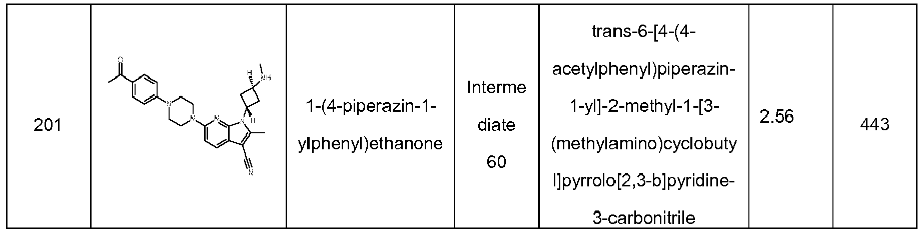

### Examples 202-205

Examples 202-205 were prepared in three steps according to General Procedure 9, 5 then 13. Example 202 was analysed by LCMS Method 1. Examples 203-205 were analysed by LCMS Method 9.

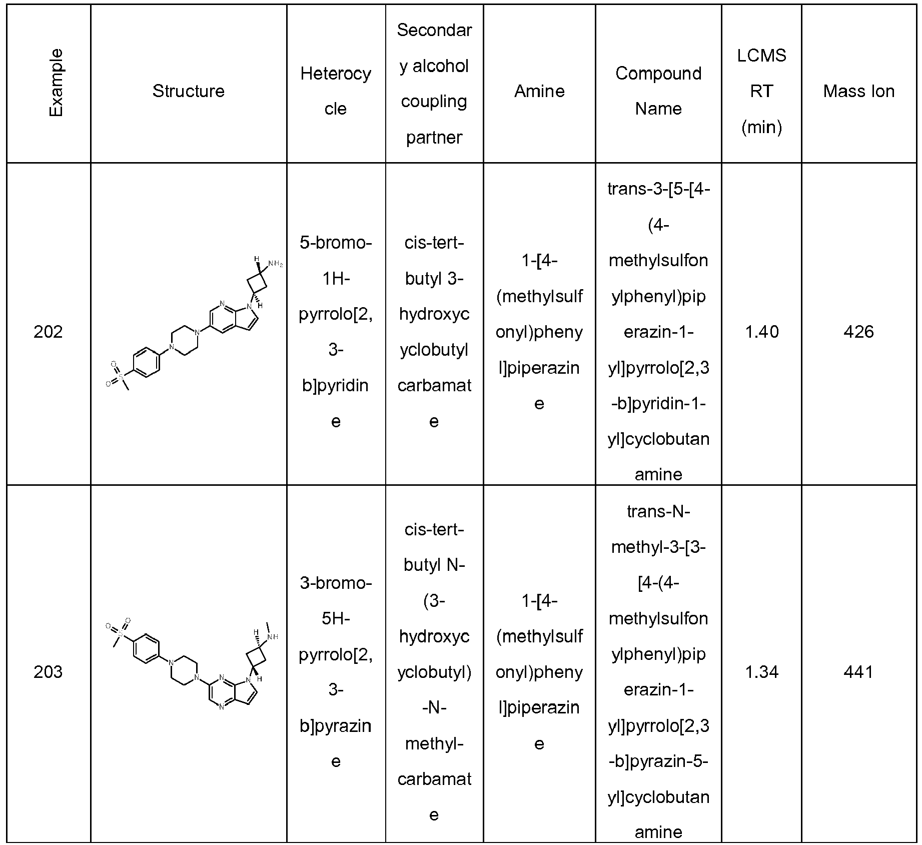

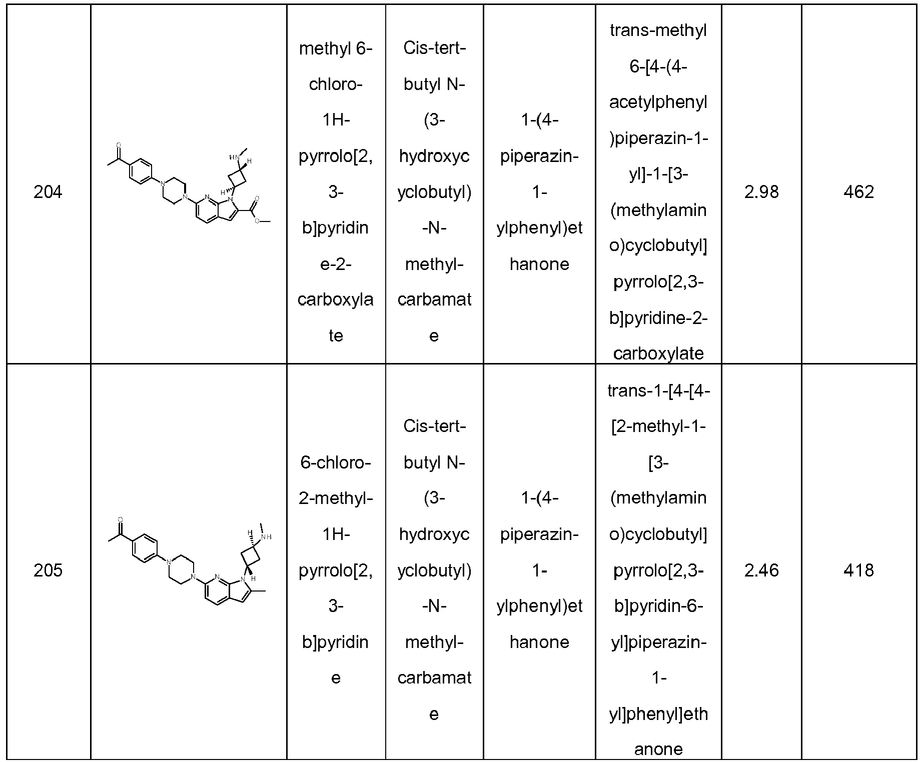

### Examples 206-211

Examples 206-211 were prepared in three steps according to General Procedure 17, 5 then 13 and analysed by LCMS Method 9.

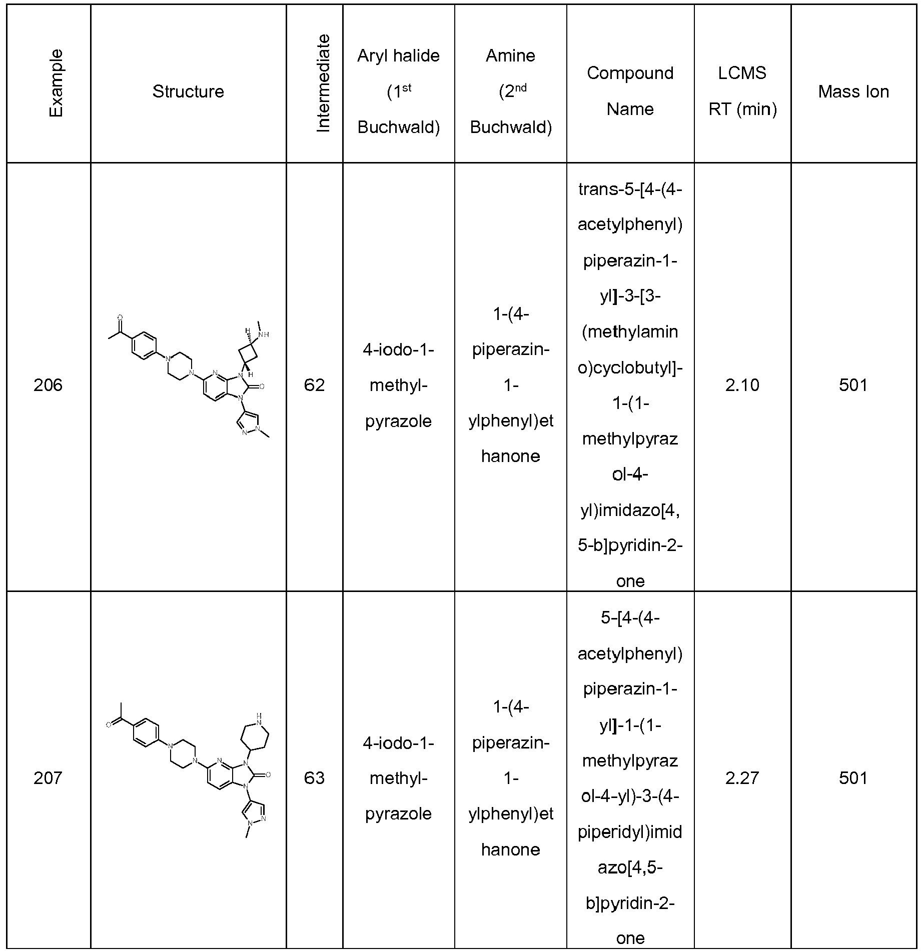

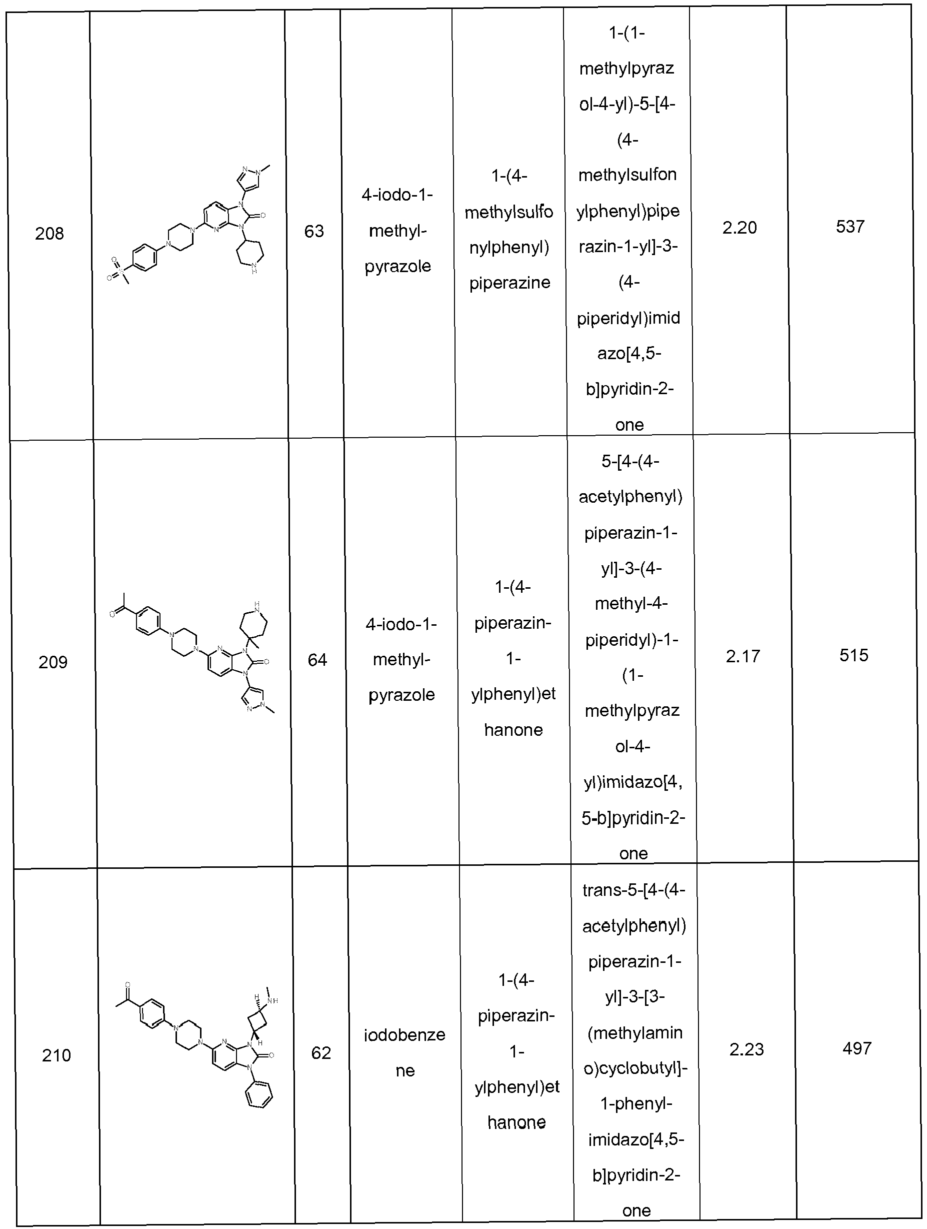

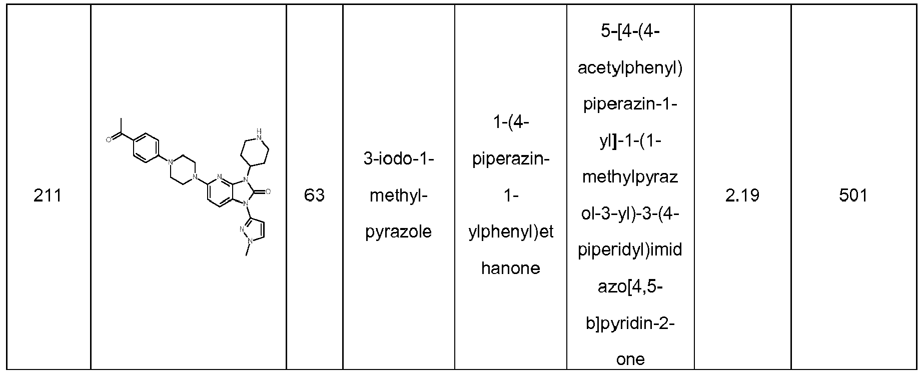

### Examples 212-217

Examples 212-217 were prepared in two steps from intermediate 18 and a secondary alcohol according to General Procedure 9 then 13 and analysed by LCMS Method 3.

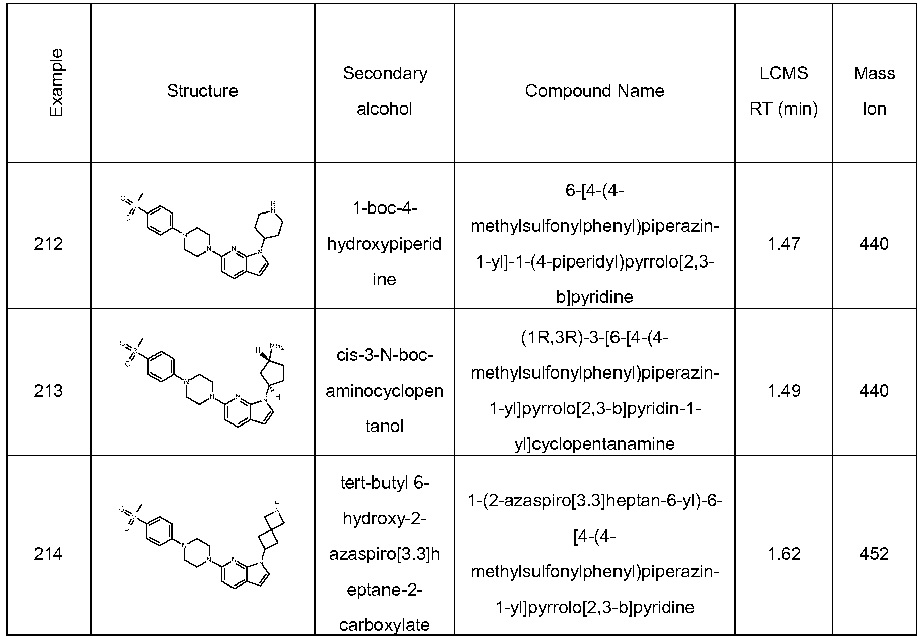

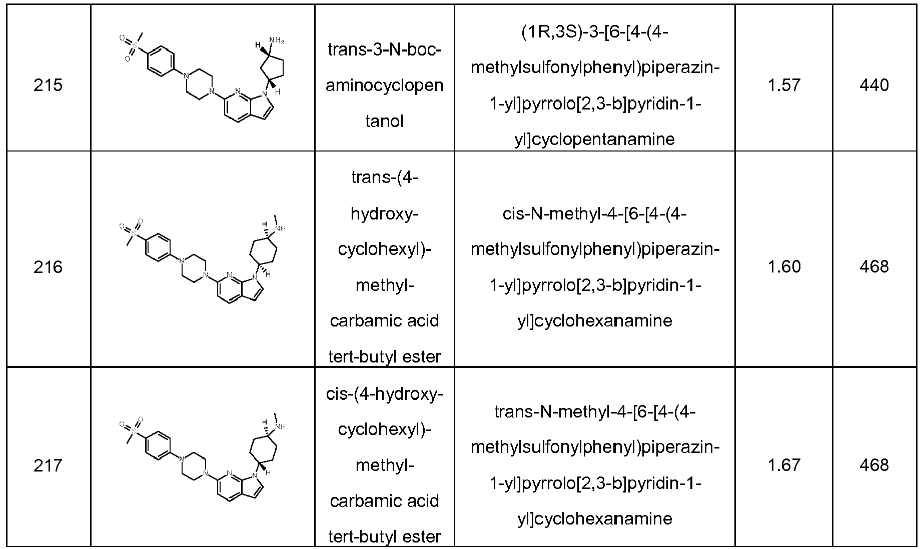

### Examples 218-220

Examples 218-220 were prepared from intermediate 18 and a secondary alcohol according to General Procedure 9 and analysed by LCMS Method 3.

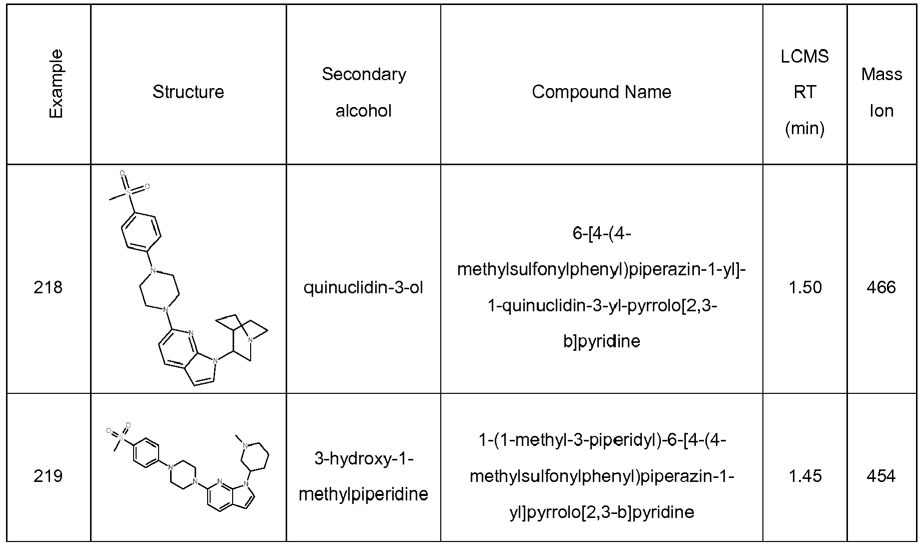

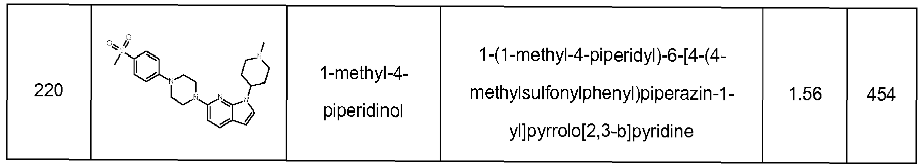

### Examples 221-227

The following compounds were synthesised from stated intermediate and the appropriate amine in accordance with General Procedure 7 or General Procedure 8, depending on the intermediate, and analysed by LCMS Method 3.

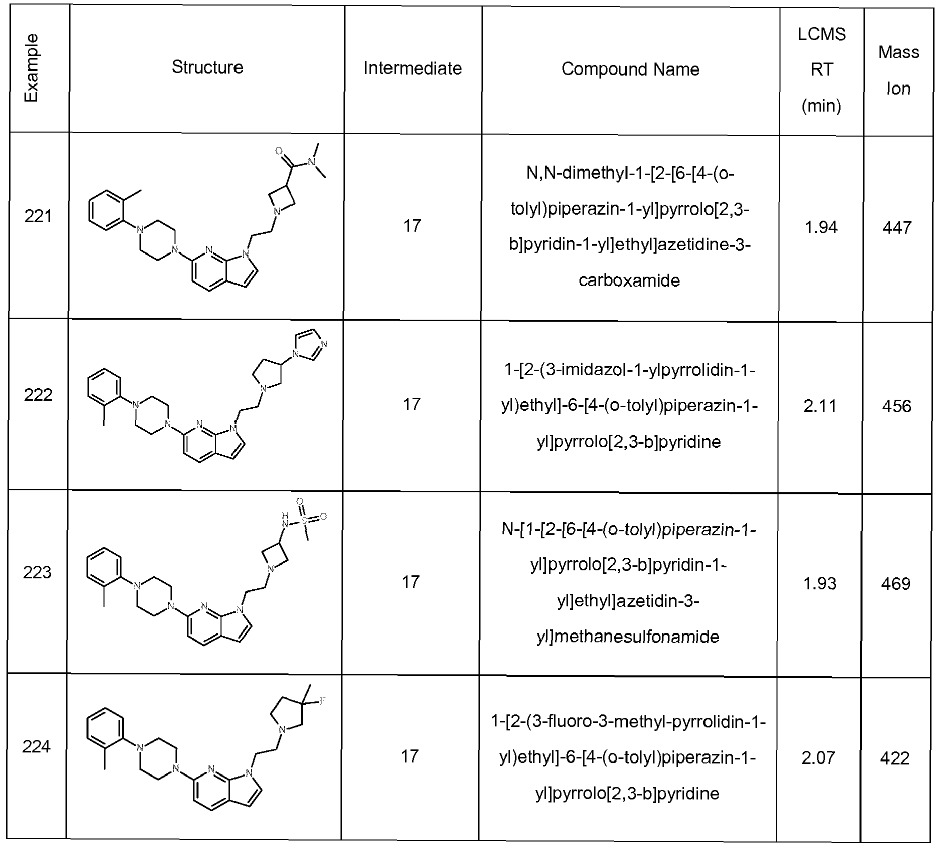

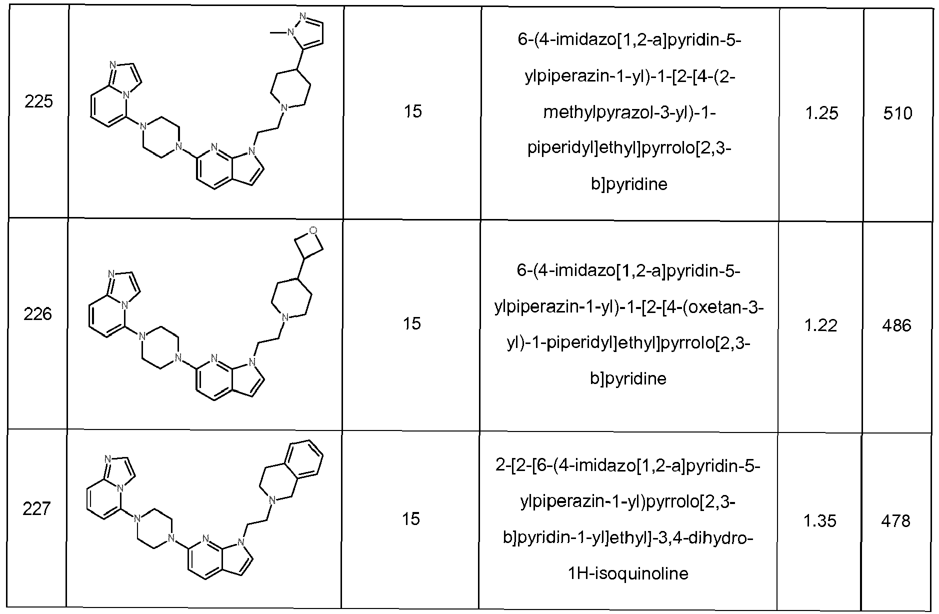

### Examples 228-233

The following compounds were synthesised from Intermediate 7 and the appropriate carboxylic acid in accordance with General Procedure 7 and analysed by LCMS Method 3.

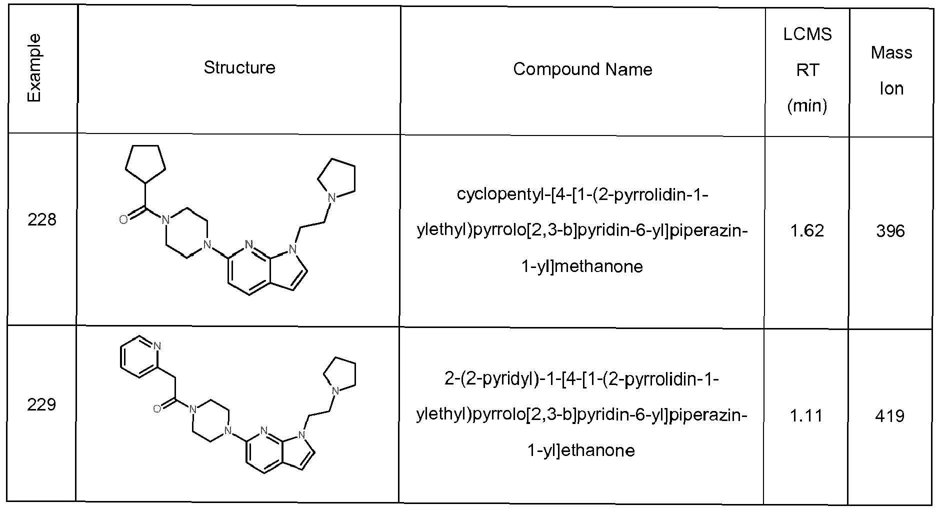

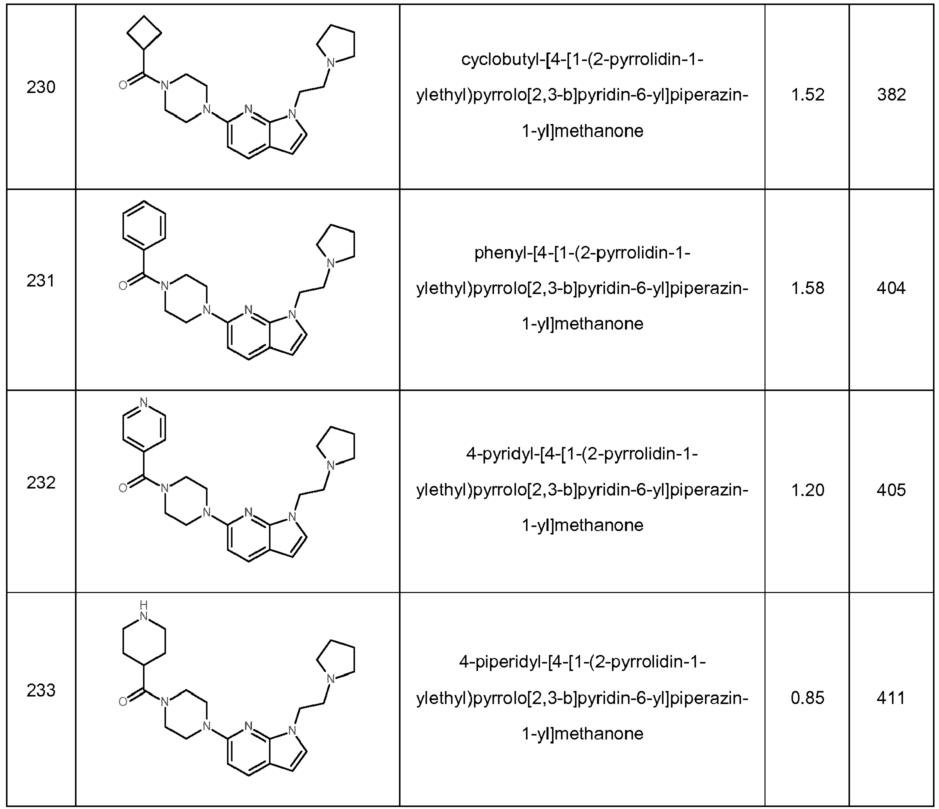

### Example 234

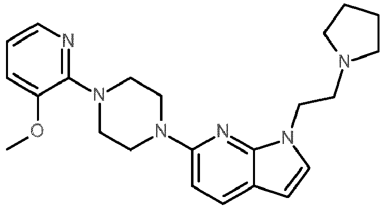

### 6-[4-(3-methoxy-2-pyridyl)piperizin-1-yl]-1-(2-pyrrolidin-1-ylethyl)pyrrolo[2,3-b]pyridine

Prepared using Intermediate 7 and 2-chloro-3-methoxypyridine using General Procedure 3.
LCMS (Method 3, ES⁺) 1.34 min, 407 *m*/*z* (M+H)⁺

### Example 235

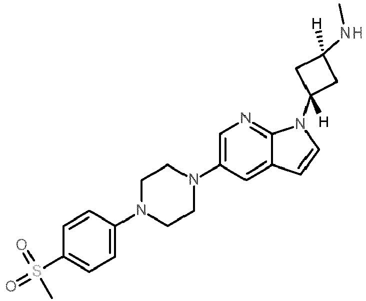

### trans-N-methyl-3-[5-[4-(4-methylsulfonylphenyl)piperazin-1-yl]pyrrolo[2,3-b]pyridin-1-vllcvclobutanamine

General procedure 9 with 5-bromo-1H-pyrrolo[2,3-b]pyridine and cis-tert-butyl 3-hydroxycyclobutylcarbamate followed by general procedure 5 with 1-[4-(methylsulfonyl)phenyl]piperazine.
The intermediate trans-tert-butyl N-[3-[5-[4-(4-methylsulfonylphenyl)piperazin-1-yl]pyrrolo[2,3-b]pyridin-1-yl]cyclobutyl]carbamate (36 mg, 0.07 mmol) was dissolved in DMF (0.7 ml) then NaH (4 mg, 0.1 mmol) was added under nitrogen. After stirring for 5 minutes, Mel (0.006 ml, 0.1 mmol) was added and the mixture was stirred for a further 10 minutes. The mixture was quenched with brine (10 ml) and the product was extracted with DCM (3 x 10 ml). The combined organics were washed with brine (2 x 10 ml), dried (Na₂SO₄) and concentrated in vacuo. The residue was then submitted to general procedure 13 to give the desired product (7 mg).
LCMS (Method 1, ES⁺) 1.45 min, 440 *m*/*z* (M+H)⁺

### Example 236

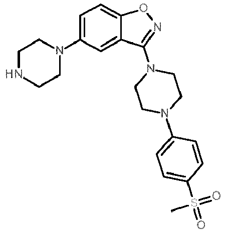

### 3-[4-(4-methylsulphenyl)piperazin-1-yl]-5-piperazin-1-yl-1,2-benzoxazole

A solution of 1-[4-(methylsulfonyl)phenyl]piperazine (216 mg, 0.90 mmol), 5-bromo-3-chlorobenzo[d]isoxazole (200 mg, 0.82 mmol) and 1,8-diazabicyclo[5.4.0]undec-7-ene (0.15 ml, 1.0 mmol) in pyridine (4.1 ml) was heated at 100 °C for 3 days. The mixture was cooled to rt, diluted with water (20 ml) and extracted with CHCl₃ (3 x 30 ml). The combined organics were washed with brine (20 ml), dried (Na₂SO₄) and concentrated in vacuo. The product was purified by column chromatography (hexane to EtOAc over 10 CVs) to give 5-bromo-3-[4-(4-methylsulfonylphenyl)piperazin-1-yl]-1,2-benzoxazole (140 mg, 40%). General procedure 5 with 1-boc-piperazine followed by general procedure 13 gave the desired product (10 mg).
LCMS (Method 1, ES⁺) 1.39 min, 442 *m*/*z* (M+H)⁺

### Example 237

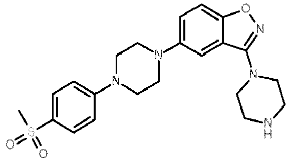

### 5-[4-(4-methylsulfonylphenyl)piperazin-1-yl]-3-piperazin-1-yl-1,2-benzoxazole

A solution of 1-boc-piperazine (171 mg, 0.90 mmol), 5-bromo-3-chlorobenzo[d]isoxazole (200 mg, 0.82 mmol) and 1,8-diazabicyclo[5.4.0]undec-7-ene (0.15 mL, 1.0 mmol) in pyridine (4.1 mL) was heated at 120 °C for 5 days. The mixture was cooled to rt, diluted with water (10 ml) and brine (10 ml) then extracted with CHCl₃ (3 x 20 ml). The combined organics were washed with brine (20 ml), dried (Na₂SO₄), concentrated in vacuo and purified by column chromatography (hexane/EtOAc) to give 152 mg tert-butyl 4-(5-bromo-1,2-benzoxazol-3-yl)piperazine-1-carboxylate.

The intermediate was reacted with 1-[4-(methylsulfonyl)phenyl]piperazine (115 mg) using General Procedure 5, followed by General Procedure 13 to give 23 mg product.
LCMS (Method 1, ES⁺) 1.43 min, 442 *m*/*z* (M+H)⁺

### Example 238

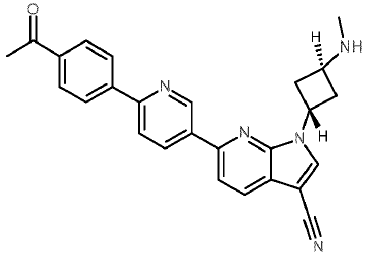

### trans-6-[6-(4-acetylphenyl)-3-pyridyl]-1-[3-(methylamino)cyclobutyl]pyrrolo[2,3-b]pyridine-3-carbonitrile

2-Chloropyridine-5-boronic acid (50 mg, 0.305 mmol), Intermediate 40 (124 mg, 0.306 mmol) and tetrakis(triphenylphosphine)palladium(0) (18 mg, 0.0154 mmol) in degassed N,N-dimethylformamide (1.0 mL) was stirred at rt for 40 mins. A degassed solution of sodium carbonate (48.5 mg, 0.458 mmol) in water (0.5 mL) was added, then the mixture was heated at 80 °C under N₂ for 2 hours. 4-Acetylphenylboronic acid (75 mg, 0.457 mmol) was added, then the reaction mixture was heated to 80 °C for a further 16 hours. The mixture was diluted with EtOAc (20 ml), water (10 ml) and brine (10 ml) and the layers were separated. The aqueous was further extracted with EtOAc (2 x 20 ml) then the combined organics were washed with brine (3 x 10 ml). The brine washings were back-extracted with DCM (2 x 10 ml) then the combined organics were dried (Na₂SO₄) and concentrated in vacuo. The residue was purified by column chromatography (hexane/EtOAc) then subjected to General Procedure 13 to give 2 mg product.
LCMS (Method 1, ES⁺) 2.05 min, 422 *m*/*z* (M+H)⁺

### Example 239

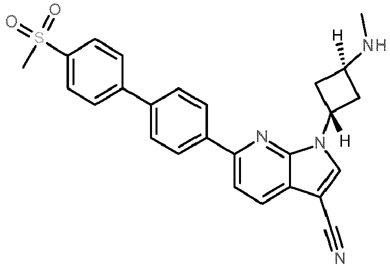

### trans-1-[3-(methylamino)cyclobutyl]-6-[4-(4-methylsulfonylphenyl)phenyl]pyrrolo[2,3-b]pyridine-3-carbonitrile

An oven-dried microwave vial was charged with 4'-bromo-4-methanesulfonyl-biphenyl (85 mg, 0.273 mmol), bis(pinacolato)diboron (69 mg, 0.272 mmol), 2nd generation XPhos precatalyst (19 mg, 0.024 mmol), and potassium acetate (67 mg, 0.683 mmol) before dry 1,4-dioxane (1.2 mL) was added. The resulting mixture was evacuated and backfilled with N₂ three times then stirred at 100 °C. After 1 hour, the mixture was cooled to rt and a freshly prepared solution of intermediate 40 (100 mg, 0.247 mmol) in dry and degassed 1,4-dioxane (0.5 mL) was added followed by a freshly prepared solution and degassed solution of potassium phosphate tribasic (79 mg, 0.372 mmol) in water (0.5 mL). The resulting mixture was stirred at 100 °C under N₂ for 3 hours. The mixture was cooled to rt, diluted with EtOAc (20 ml) and washed with a 1:1 mixture of water (10 ml) and brine (10 ml). The aqueous was further extracted with EtOAc (2 x 20 ml), then the combined organics were dried (Na₂SO₄) and concentrated in vacuo. The residue was purified by column chromatography then subjected to General Procedure 13 to give 44 mg product.
LCMS (Method 1, ES⁺) 2.27 min, 457 *m*/*z* (M+H)⁺

### Example 240

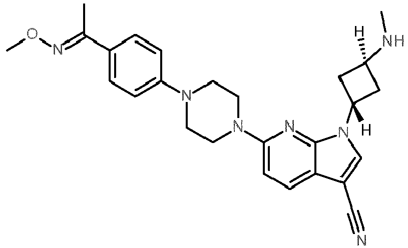

### trans-6-[4-[4-[(E)-N-methoxy-C-methyl-carbonimidoyl]phenyl]piperazin-1-yl]-1-[3-(methylamino)cyclobutyl]pyrrolo[2,3-b]pyridine-3-carbonitrile

Intermediate 40 and 1-(4-acetylphenyl)piperazine were subjected to General Procedure 3. Following this, the resulting trans-tert-butyl N-[3-[6-[4-(4-acetylphenyl)piperazin-1-yl]-3-cyano-pyrrolo[2,3-b]pyridin-1-yl]cyclobutyl]-N-methyl-carbamate (10 mg, 0.0189 mmol) and O-methylhydroxylamine hydrochloride (2 mg, 0.0239 mmol) were dissolved in methanol (1.0 mL) and stirred at 50 °C. After 3 hours the mixture was concentrated in vacuo and subjected to General procedure 13 to give 1 mg product.
LCMS (Method 1, ES⁺) 2.47 min, 458 *m*/*z* (M+H)⁺

### Example 241

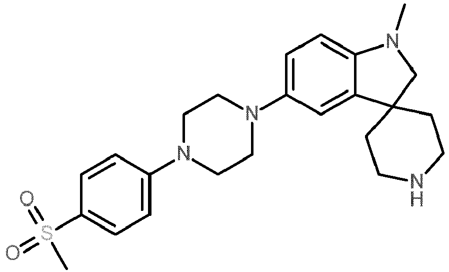

### 1-methyl-5-[4-(4-methylsulfonylphenyl)piperazin-1-yl]spiro[indoline-3,4'-piperidine]

A solution of tert-butyl 5-bromospiro[indoline-3,4'-piperidine]-1'-carboxylate (200 mg, 0.52 mmol) in tetrahydrofuran (5 mL) was degassed then cooled with an ice/brine bath. Sodium hydride (31 mg, 0.78 mmol) was added and the mixture stirred for 30 minutes. Iodomethane (0.04 mL, 0.6 mmol, 1) was then added, the mixture stirred for 5 minutes then removed from the ice bath and heated at 40 °C for 16 hours. The reaction was quenched with water (20 ml) and the product was extracted with DCM (2 x 20 ml). The combined organics were washed with brine (20 ml), dried (Na₂SO₄) and concentrated in vacuo. The product tert-butyl 5-bromo-1-methyl-spiro[indoline-3,4'-piperidine]-1'-carboxylate was purified by column chromatography (hexane/EtOAc) then subjected to General Procedure 5 with 1-[4-(methylsulfonyl)phenyl]piperazine followed by General Procedure 13 to give 17 mg product.
LCMS (Method 1, ES⁺) 1.44 min, 441 *m*/*z* (M+H)⁺

### Example 242

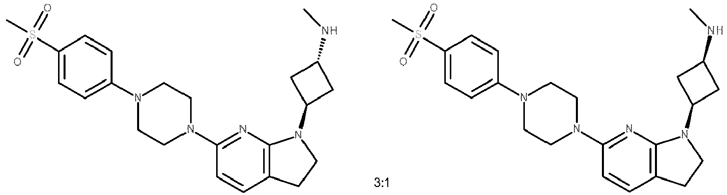

### N-methyl-3-[6-[4-(4-methylsulfonylphenyl)piperazin-1-yl]-2,3-dihydropyrrolo[2,3-b]pyridin-1-vllcvclobutanamine

Tert-butyl n-methyl-n-(3-oxocyclobutyl)carbamate (269 mg, 1.35 mmol), sodium triacetoxyborohydride (603 mg, 2.70 mmol) and acetic acid (0.007 mL) were added to a solution of 6-chloro-1H,2H,3H-pyrrolo[2,3-b]pyridine (200 mg, 1.2 mmol) in dichloromethane (1.2 mL) at 0 °C. After 5 hours, the mixture was diluted with EtOAc (30 ml) and water (10 ml), washed with sat. aq. NaHCO₃ (2 x 10 ml) and brine (10 ml), dried (Na₂SO₄) and concentrated in vacuo to give tert-butyl N-[3-(6-chloro-2,3-dihydropyrrolo[2,3-b]pyridin-1-yl)cyclobutyl]-N-methyl-carbamate (440 mg).

tert-butyl N-[3-(6-chloro-2,3-dihydropyrrolo[2,3-b]pyridin-1-yl)cyclobutyl]-N-methyl-carbamate (200 mg) and 1-[4-(methylsulfonyl)phenyl]piperazine (157 mg) were subjected to General Procedure 3, followed by General Procedure 13 to give 74 mg product as a 3:1 ratio of isomers.
LCMS (Method 1, ES⁺) 1.61 min, 442 *m*/*z* (M+H)⁺ [cis]; LCMS (Method 1, ES⁺) 1.66 min, 442 *m*/*z* (M+H)⁺ [trans]

### Example 243

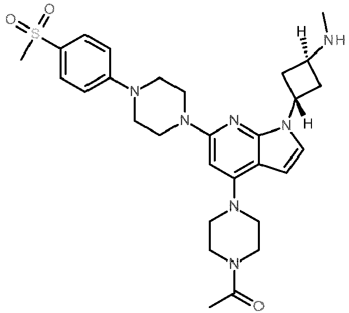

### trans-1-[4-[1-[3-(methylamino)cyclobutyl]-6-[4-(4-methylsulfonylphenyl)piperazin-1-yl]pyrrolo[2,3-b]pyridin-4-yl]piperazin-1-yl]ethenone

4,6-Dichloro-1H-pyrrolo[2,3-b]pyridine and cis-tert-butyl N-(cis-3-hydroxycyclobutyl)-N-methylcarbamate were subjected to General Procedure 9. The resulting product was then reacted with piperazine (5 equiv) using General Procedure 14, followed by General Procedure 3 with 1-[4-(methylsulfonyl)phenyl]piperazine to give trans-tert-butyl N-methyl-N-[3-[6-[4-(4-methylsulfonylphenyl)piperazin-1-yl]-4-piperazin-1-yl-pyrrolo[2,3-b]pyridin-1-yl]cyclobutyl]carbamate.

Acetyl chloride (0.001 mL, 0.01 mmol) was added to a solution of trans-tert-butyl N-methyl-N-[3-[6-[4-(4-methylsulfonylphenyl)piperazin-1-yl]-4-piperazin-1-yl-pyrrolo[2,3-b]pyridin-1-yl]cyclobutyl]carbamate (10 mg, 0.016 mmol) and triethylamine (0.01 mL, 0.07 mmol) in dichloromethane (0.50 mL) and stirred at rt for 5 minutes. Trifluoroacetic acid (0.1 mL) was then added to the reaction mixture. After 30 minutes, the mixture was concentrated, neutralised with NH₃ in MeOH and purified by prep HPLC to give 3 mg product.
LCMS (Method 1, ES⁺) 1.56 min, 566 *m*/*z* (M+H)⁺

### Example 244

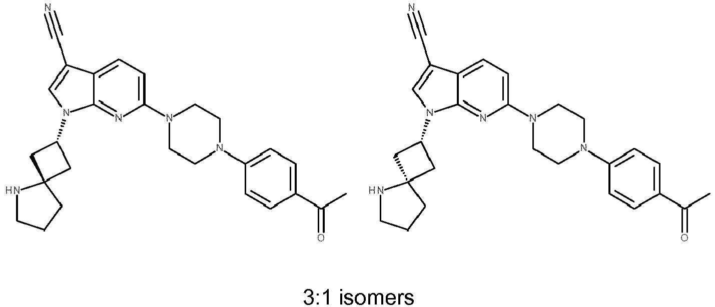

### 6-[4-(4-acetylphenyl)piperazin-1-yl]-1-(5-azaspiro[3.4]octan-2-yl)pyrrolo[2,3-b]pyridine-3-carbonitrile

Sodium borohydride (66 mg, 1.71 mmol) was added to a solution of 5-boc-5-aza-spiro[3.4]octane-2-one (200 mg, 0.86 mmol) in ethanol (10 mL) which was then stirred at rt under N2 for 30 minutes. The reaction was quenched with sat. aq. NH₄Cl (20 ml) and the ethanol was removed in vacuo. The product was then extracted with EtOAc (3 x 20 ml), dried (Na₂SO₄) and concentrated in vacuo to give tert-butyl 2-hydroxy-5-azaspiro[3.4]octane-5-carboxylate (169 mg, 86%).

tert-butyl 2-hydroxy-5-azaspiro[3.4]octane-5-carboxylate (72 mg, 0.32 mmol) was reacted with 6-chloro-1H-pyrrolo[2,3-b]pyridine-3-carbonitrile (55 mg, 0.29 mmol) using General Procedure 9 to give tert-butyl 2-(6-chloro-3-cyano-pyrrolo[2,3-b]pyridin-1-yl)-5-azaspiro[3.4]octane-5-carboxylate (66 mg, 58%).

tert-butyl 2-(6-chloro-3-cyano-pyrrolo[2,3-b]pyridin-1-yl)-5-azaspiro[3.4]octane-5-carboxylate (30 mg, 0.078 mmol) and 1-(4-acetylphenyl)piperazine (18 mg, 0.088 mmol) were subjected to General Procedure 14 followed by General Procedure 13 to give 3 mg product as a 3:1 mixture of isomers.
LCMS (Method 1, ES⁺) 2.28 min, 455 *m*/*z* (M+H)⁺

### Example 245

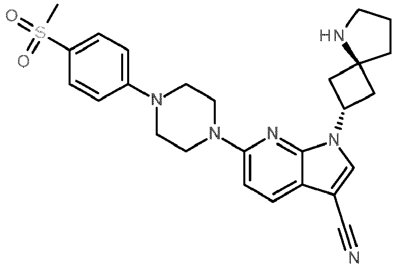

### trans-1-(5-azaspiro[3.4]octan-2-yl)-6-[4-(4-methylsulfonylphenyl)piperazin-1-yl]pyrrolo[2,3-b]pyridine-3-carbonitrile

trans-tert-butyl 2-(6-chloro-3-cyano-pyrrolo[2,3-b]pyridin-1-yl)-5-azaspiro[3.4]octane-5-carboxylate (30 mg, 0.078 mmol, see Example 244) and 1-[4-(methylsulfonyl)phenyl]piperazine (37 mg, 0.15 mmol) were subjected to General Procedure 14 followed by General Procedure 13 to give 7 mg product.
LCMS (Method 1, ES⁺) 2.15 min, 491 *m*/*z* (M+H)⁺

### Example 246

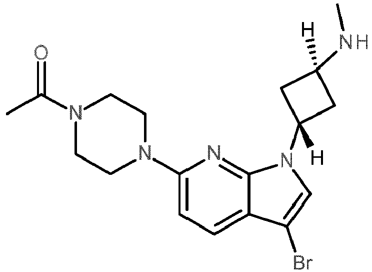

### trans-1-[4-[3-bromo-1-[3-(methylamino)cyclobutyl]pyrrolo[2,3-b]pyridin-6-yl]piperazin-1-yl]ethenone

Intermediate 59 (30 mg, 0.06 mmol) was reacted following General Procedure 13 to give the product as a white solid (22 mg, 91% yield).
LCMS (Method 1, ES⁺) 1.59 min, 406 & 408 *m*/*z* (M+H)⁺.

### Example 247

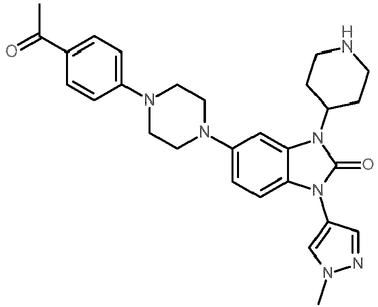

### 5-[4-(4-acetylphenyl)piperazin-1-yl]-1-(1-methylpyrazol-4-yl)-3-(4-piperidyl)benzimidazol-2-one

Sodium carbonate (3.59 g, 34.2 mmol) and tert-butyl 4-aminopiperidine-1-carboxylate (2.74 g, 13.7 mmol) were added to a stirred solution of 4-chloro-2-fluoro-1-nitro-benzene (2.00 g, 11.4 mmol) in DMF (30 mL) at rt under argon. The resulting reaction mixture was heated at 80 °C for 2 h. After complete consumption of starting material, ice cold water (100 mL) was added and extracted with ethyl acetate (100 mL x 3). The organic layer was washed with ice cold water (200 mL x 2) and brine solution (200 mL), dried over sodium sulphate and concentrated in vacuo. The crude material was purified by column chromatography using 15% ethyl acetate in hexane to give tert-butyl 4-(5-chloro-2-nitro-anilino)piperidine-1-carboxylate (3.80 g, 10.7 mmol, 94%).

tert-butyl 4-(5-chloro-2-nitro-anilino)piperidine-1-carboxylate (3.8 g, 10.7 mmol) was submitted to General Procedure 15 then General Procedure 16 to give tert-butyl 4-(6-chloro-2-oxo-3H-benzimidazol-1-yl)piperidine-1-carboxylate (2.50 g, 7.1 mmol, 66% over 2 steps).

The product was prepared in three further steps according to General Procedure 17 with 4-iodo-1-methyl-pyrazole, General Procedure 5 with 1-(4-piperazin-1-ylphenyl)ethanone then General Procedure 13 to give 5-[4-(4-acetylphenyl)piperazin-1-yl]-1-(1-methylpyrazol-4-yl)-3-(4-piperidyl)benzimidazol-2-one (41 mg, 6% over 3 steps).
LCMS (Method 9, ES⁺) 2.03 min, 500 *m*/*z* (M+H)⁺

### Example 248

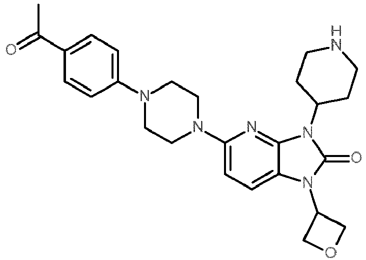

### 5-[4-(4-acetylphenyl)piperazin-1-yl]-1-(oxetan-3-yl)-3-(4-piperidyl)imidazo[4,5-b]pyridin-2-one

Caesium carbonate (1.35 g, 4.14 mmol) was added to a stirred solution of Intermediate 63 (500 mg, 1.38 mmol) and 3-iodooxetane (585 mg, 3.18 mmol) in DMF (10 mL) at rt. The mixture was heated at 100 °C for 4 h. After cooling to rt, the reaction mixture was diluted with water (100 mL) and extracted with ethyl acetate (100 mL x 2). The organic layer was separated, washed with brine (200 mL), dried over sodium sulphate and concentrated under reduced pressure. The crude compound was purified by column chromatography using 2% methanol in DCM to give tert-butyl 4-[5-chloro-1-(oxetan-3-yl)-2-oxo-imidazo[4,5-b]pyridin-3-yl]piperidine-1-carboxylate (400 mg, 0.92 mmol, 66%).

To a stirred solution of tert-butyl 4-[5-chloro-1-(oxetan-3-yl)-2-oxo-imidazo[4,5-b]pyridin-3-yl]piperidine-1-carboxylate (200 mg, 0.46 mmol) in toluene (20 mL) was added 1-(4-piperazin-1-ylphenyl)ethanone (143 mg, 0.687 mmol) and potassium phosphate tribasic (292 mg, 1.37 mmol) at rt. The reaction mixture was degassed using argon for 15 mins. Tris(dibenzylideneacetone)dipalladium(0) (83.8 mg, 0.0916 mmol) and X-Phos (39.7 mg, 0.0916 mmol) were then added, and the mixture was degassed again using argon for 15 mins. After heating at 100 °C for 4 h, the reaction mixture was diluted with ethyl acetate (100 mL) and washed with water (100 mL x 2). The organic layer was separated, washed with brine (150 mL), dried over sodium sulphate and concentrated under reduced pressure to get crude product. Purification by prep HPLC gave tert-butyl 4-[5-[4-(4-acetylphenyl)piperazin-1-yl]-1-(oxetan-3-yl)-2-oxo-imidazo[4,5-b]pyridin-3-yl]piperidine-1-carboxylate (150 mg, 0.26 mmol, 56%).

tert-butyl 4-[5-[4-(4-acetylphenyl)piperazin-1-yl]-1-(oxetan-3-yl)-2-oxo-imidazo[4,5-b]pyridin-3-yl]piperidine-1-carboxylate (150 mg, 0.258 mmol) was submitted to General Procedure 13 to give the product (60 mg, 0.13 mmol, 49%).
LCMS (Method 9, ES⁺) 1.99 min, 477 *m*/*z* (M+H)⁺

### Example 249

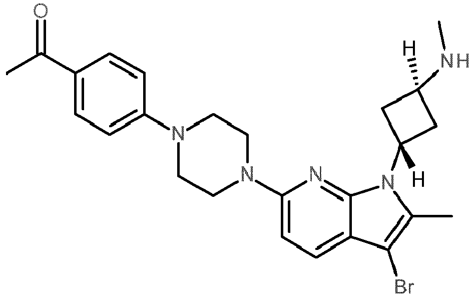

### trans-1-[4-[4-[3-bromo-2-methyl-1-[3-(methylamino)cyclobutyl]pyrrolo[2,3-b]pyridin-6-yl]piperazin-1-yl]phenyl]ethenone

6-chloro-2-methyl-1H-pyrrolo[2,3-b]pyridine (200 mg, 1.20 mmol) and cis-tert-butyl N-(3-hydroxycyclobutyl)-N-methyl-carbamate (266 mg, 1.32 mmol) were reacted using General Procedure 9 to give trans-tert-butyl N-[3-(6-chloro-2-methyl-pyrrolo[2,3-b]pyridin-1-yl)cyclobutyl]-N-methyl-carbamate (400 mg, 1.06 mmol, 88%).

To a stirred solution of trans-tert-butyl N-[3-(6-chloro-2-methyl-pyrrolo[2,3-b]pyridin-1-yl)cyclobutyl]-N-methyl-carbamate (300 mg, 0.796 mmol) in toluene (10 mL) was added 1-(4-piperazin-1-ylphenyl)ethanone (249 mg, 1.19 mmol) and potassium phosphate tribasic (507 mg, 2.39 mmol) at rt. The reaction mixture was degassed using argon for 15 mins. Tris(dibenzylideneacetone)dipalladium(0) (146 mg, 0.159 mmol) and X-Phos (69 mg, 0.159 mmol) were then added, and the mixture was degassed again using argon for 15 mins. After heating at 100 °C for 2 h, the reaction mixture was diluted with water (50 mL) and extracted with ethyl acetate (50 mL x 2). The organic layer was separated, washed with brine (75 mL), dried over sodium sulphate and concentrated under reduced pressure to get the crude product. Purification by prep HPLC gave trans-tert-butyl N-[3-[6-[4-(4-acetylphenyl)piperazin-1-yl]-2-methyl-pyrrolo[2,3-b]pyridin-1-yl]cyclobutyl]-N-methyl-carbamate (130 mg, 0.248 mmol, 31%).

To a stirred solution of trans-tert-butyl N-[3-[6-[4-(4-acetylphenyl)piperazin-1-yl]-2-methyl-pyrrolo[2,3-b]pyridin-1-yl]cyclobutyl]-N-methyl-carbamate (130 mg, 0.248 mmol) in DCM (10 mL) was added NBS (37.5 mg, 0.211 mmol) at -50°C. The resulting reaction mixture was stirred at -50 to -20 °C for 15 mins. After complete consumption of starting material, the mixture was quenched with water (25 mL) and extracted with DCM (25 mL x 2). The collected organic layer was washed with brine (50 mL), dried over sodium sulphate and evaporated under reduced pressure to get crude material. The crude product was purified by column chromatography using 25% EtOAc in hexane, then submitted to General Procedure 13 to give the product (25 mg, 0.047 mmol, 19% over 2 steps).
LCMS (Method 9, ES⁺) 2.76 min, 496 & 498 *m*/*z* (M+H)⁺

### Example 250

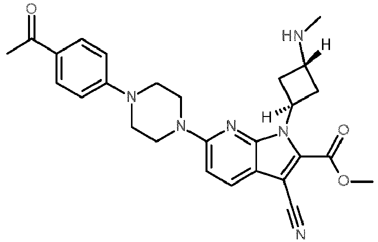

### trans-methyl 6-[4-(4-acetylphenyl)piperazin-1-yl]-3-cyano-1-[3-(methylamino)cyclobutyl]pyrrolo[2,3-b]pyridine-2-carboxylate

Methyl 6-chloro-1H-pyrrolo[2,3-b]pyridine-2-carboxylate (400 mg, 1.90 mmol) and cis-tert-butyl N-(3-hydroxycyclobutyl)-N-methyl-carbamate (459 mg, 2.28 mmol) were reacted using General Procedure 9 to give trans-methyl 1-[3-[tert-butoxycarbonyl(methyl)amino]cyclobutyl]-6-chloro-pyrrolo[2,3-b]pyridine-2-carboxylate (650 mg, 1.61 mmol, 85%).

To a stirred solution of trans-methyl 1-[3-[tert-butoxycarbonyl(methyl)amino]cyclobutyl]-6-chloro-pyrrolo[2,3-b]pyridine-2-carboxylate (300 mg, 0.741 mmol) in toluene (20 mL) was added 1-(4-piperazin-1-ylphenyl)ethanone (232 mg, 1.11 mmol) and potassium phosphate tribasic (472 mg, 2.22 mmol) at rt. The reaction mixture was degassed using argon for 15 mins. Tris(dibenzylideneacetone)dipalladium(0) (136 mg, 0.148 mmol) and X-Phos (64.3 mg, 0.148 mmol) were then added, and the mixture was degassed again using argon for 15 mins. After heating at 100 °C for 2 h, the reaction mixture was diluted with water (75 mL) and extracted with ethyl acetate (75 mL x 2). The organic layer was separated, washed with brine (100 mL), dried over sodium sulphate, concentrated under reduced pressure and purified by column chromatography using 30% ethyl acetate in hexane to give trans-methyl 6-[4-(4-acetylphenyl)piperazin-1-yl]-1-[3-[tert-butoxycarbonyl(methyl)amino]cyclobutyl]pyrrolo[2,3-b]pyridine-2-carboxylate (250 mg, 0.35 mmol, 48%).

To a stirred solution of trans-methyl 6-[4-(4-acetylphenyl)piperazin-1-yl]-1-[3-[tert-butoxycarbonyl(methyl)amino]cyclobutyl]pyrrolo[2,3-b]pyridine-2-carboxylate (250 mg, 0.35 mmol) in DCM (15 mL)was added NBS (53.3 mg, 0.300 mmol) at -50°C. The resulting reaction mixture was stirred at -50 °C for 30 min. After complete consumption of starting material, water (50 mL) was added and the mixture was extracted with DCM (50 mL x 3). The collected organic layer was washed with brine (25 mL), dried over sodium sulphate and purified by column chromatography using 20% EtOAc in hexane to give trans-methyl 6-[4-(4-acetylphenyl)piperazin-1-yl]-3-bromo-1-[3-[tert-butoxycarbonyl(methyl)amino]cyclobutyl]pyrrolo[2,3-b]pyridine-2-carboxylate (100 mg, 0.14 mmol, 39%).

To a stirred solution of trans-methyl 6-[4-(4-acetylphenyl)piperazin-1-yl]-3-bromo-1-[3-[tert-butoxycarbonyl(methyl)amino]cyclobutyl]pyrrolo[2,3-b]pyridine-2-carboxylate (50 mg, 0.069 mmol) in NMP (2 mL) was added copper(I) cyanide (37 mg, 0.41 mmol) at rt. The contents were heated at 130 °C for 16 h. After cooling to rt, the reaction mixture was diluted with ethyl acetate (50 mL) and washed with water (50 mL x 2). The organic layer was separated, washed with brine (30 mL), dried over sodium sulphate, concentrated under reduced pressure and purified by column chromatography using 20% EtOAc in hexane to give trans-methyl 6-[4-(4-acetylphenyl)piperazin-1-yl]-1-[3-[tert-butoxycarbonyl(methyl)amino]cyclobutyl]-3-cyano-pyrrolo[2,3-b]pyridine-2-carboxylate (50 mg, 0.061 mmol, 89%).

trans-methyl 6-[4-(4-acetylphenyl)piperazin-1-yl]-1-[3-[tert-butoxycarbonyl(methyl)amino]cyclobutyl]-3-cyano-pyrrolo[2,3-b]pyridine-2-carboxylate (50.0 mg, 0.0611 mmol) was submitted to General Procedure 13 to give the title compound (30 mg, 0.034 mmol, 55%).
LCMS (Method 9, ES⁺) 2.41 min, 487 *m*/*z* (M+H)⁺

### Example 251

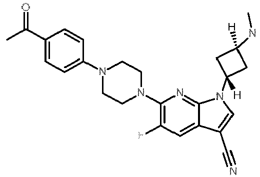

### Trans-6-[4-(4-acetylphenyl)piperazin-1-yl]-5-fluoro-1-[3-(methylamino)cyclobutyl]pyrrolo[2,3-b]pyridine-3-carbonitrile

Intermediate 42 (300 mg, 0.75 mmol) was dissolved in a mixture of DMF (2.5 mL) and acetonitrile (2.5 mL). The solution was cooled to 0 °C and chlorosulfonyl isocyanate (0.07 mL, 0.84 mmol) was added and the reaction stirred in the presence of the ice bath for 30 min then at rt for another 2 hours. The reaction mixture was cooled to 0 °C and a second portion of chlorosulfonyl isocyanate (0.07 mL, 0.84 mmol) added. The ice-bath was removed and the reaction mixture stirred for 15 min. The ice bath was returned and the reaction carefully quenched with water (10 mL). The biphasic solution was stirred for 10 mins then diluted with 50% saturated ammonium chloride (50 mL), diethyl ether (30 mL) and ethyl acetate (10 mL). The organic layer was extracted with water (50 mL), then dried (Na₂SO₄) and concentrated under reduced pressure. A mixture of the resulting residue, 1-(4-acetylphenyl)piperazine (184 mg, 0.90 mmol), RuPhos G3 (62 mg, 0.07 mmol) and sodium tert-butoxide (217 mg, 2.26 mmol) was suspended in degassed 1,4-dioxane (4 mL, 46.9 mmol). The reaction mixture was heated at 90 °C for ∼ 3 hours then left to stand at rt overnight. The reaction mixture was passed through a celite plug eluting with ethyl acetate. The solvent was removed and the crude purified by flash column chromatography to give trans-tert-butyl N-[3-[6-[4-(4-acetylphenyl)piperazin-1-yl]-3-cyano-5-fluoro-pyrrolo[2,3-b]pyridin-1-yl]cyclobutyl]-N-methyl-carbamate (253 mg, 61%).

Trans-tert-butyl N-[3-[6-[4-(4-acetylphenyl)piperazin-1-yl]-3-cyano-5-fluoro-pyrrolo[2,3-b]pyridin-1-yl]cyclobutyl]-N-methyl-carbamate (167 mg, 0.3 mmol) was then treated according to general procedure 13 to give the title compound (110 mg, 80%)
LCMS (Method 1, ES⁺)2.15 min, 447 m/z (M+H)⁺

### Example 252

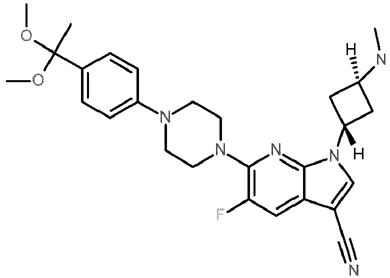

### 6-[4-[4-(1,1-dimethoxyethyl)phenyl]piperazin-1-yl]-5-fluoro-1-[3-(methylamino)cyclobutyl]pyrrolo[2,3-b]pyridine-3-carbonitrile

Example 252 was isolated as a by-product during synthesis of example 251 (42 mg, 28%).
LCMS (Method 1, ES⁺) 2.56 min, 461 m/z (M+H)⁺

### Example 253

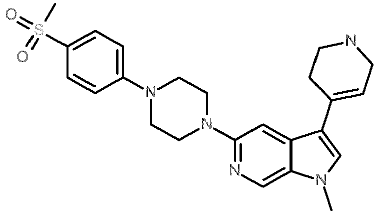

### 1-methyl-5-[4-(4-methylsulfonylphenyl)piperazin-1-yl]-3-(1,2,3,6-tetrahydropyridin-4-yl)pyrrolo[2,3-c]pyridine

N-lodosuccinimide (491 mg, 2.07 mmol) was added to a suspension of Intermediate 66 (641 mg, 1.73 mmol) in acetone (15 mL) and the reaction mixture stirred at rt for 30 min. The reaction mixture was then concentrated under reduced pressure, diluted with DCM and washed with saturated aqueous bicarbonate. The aqueous layer was extracted with DCM, organics combined, dried (Na₂SO₄) and concentrated under reduced pressure. The crude residue was then purified by flash column chromatography to give 3-iodo-1-methyl-5-[4-(4-methylsulfonylphenyl)piperazin-1-yl]pyrrolo[2,3-c]pyridine (350 mg, 40%). A fraction of the iodinated product (100 mg, 0.20 mmol) was mixed with N-boc-1,2,5,6-tetrahydropyridine-4-boronic acid pinacol ester (75 mg, 0.24 mmol), potassium carbonate (60 mg, 0.43 mmol) and 1,1'-bis(diphenylphosphino)ferrocene-palladium(ii)dichloride dichloromethane complex (17 mg, 0.02 mmol) and dissolved in predegassed DMF (1 mL). The mixture was then heated at 100 °C for 1 h and left to stand at rt for 72 h. The crude mixture was passed through an SCX cartridge eluting with MeOH and ethyl acetate and concentrated under reduced pressure. The residue was purified by flash column chromatography to give tert-butyl 4-[1-methyl-5-[4-(4-methylsulfonylphenyl)piperazin-1-yl]pyrrolo[2,3-c]pyridin-3-yl]-3,6-dihydro-2H-pyridine-1-carboxylate (20 mg, 0.04 mmol, 18%) which was then treated according to general procedure 13 to give the title compound (5 mg, 30%).
LCMS (Method 1, ES+) 1.36 min, 452 m/z (M+H)+.

### Example 254

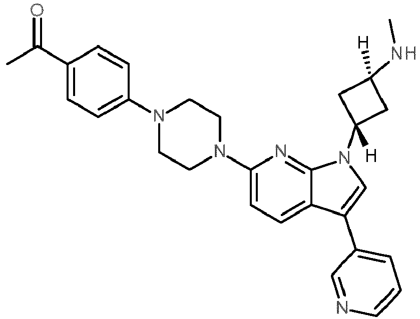

### trans-1-[4-[4-[1-[3-(methylamino)cyclobutyl]-3-(3-pyridyl)pyrrolo[2,3-b]pyridin-6-yl]piperazin-1-yl]phenyl]ethenone trifluoroacetate

To a mixture of Intermediate 65 (21 mg, 0.04 mmol) and pyridine-3-boronic acid (11 mg, 0.09 mmol) in degassed 1,4-dioxane (0.4 mL), Na₂CO₃ (0.1 mL, 2 M in dioxane) and Pd(PPh₃)₄ (7.8 mg, 0.007 mmol) were added. The reaction mixture was heated at 140 °C for 2 h in a microwave apparatus. The mixture was filtered and rinsed with 0.6 mL of MeCN/H₂O (7/3) before injection on prep HPLC and purified using a basic mode. The solvent was removed under vacuum and the yellow solid was dissolved in 1 mL of DCM/TFA (1/1). The mixture was stirred at r.t. for 1 hour until the reaction was complete. The solved was removed under vacuum to give a white solid (4.4 mg, 20% yield over two steps.)
LCMS (Method 3, ES⁺) 2.78 min, 481 m/z (M+H)⁺.

### Examples 255 and 256

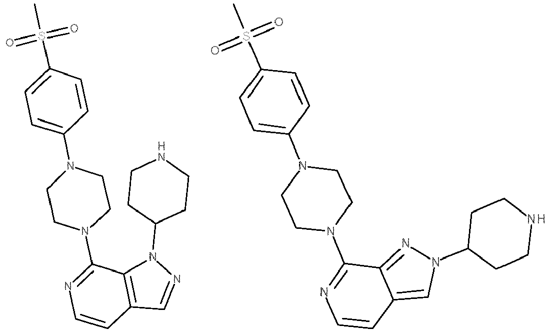

### 7-[4-(4-methylsulfonylphenyl)piperazin-1-yl]-2-piperidin-4-ylpyrazolo[3,4-c]pyridine and 7-[4-(4-methylsulfonylphenyl)piperazin-1-yl]-1-(4-piperidyl)pyrazolo[3,4-c]pyridine

A mixture of 7-bromo-1H-pyrazolo[3,4-c]pyridine (266 mg, 1.34 mmol) and tert-butyl 4-hydroxypiperidine-1-carboxylate (405.52 mg, 2.0149 mmol) was dissolved in THF (13 ml). Triphenylphosphine (422 mg, 1.61 mmol) and diisopropylazodicarboxylate (428 mg, 2.01 mmol) was added and the reaction mixture stirred overnight. Reaction mixture was then partitioned between EtOAc (50 ml) and aqu NaHCO₃ (50 ml). The organic layer was separated, washed with brine and then vac'd down. The crude material was columned with hexane/ethyl acetate 0-100% gradient elution and then the 96:4 mixture of regioisomers was mixed with 1-[4-(methylsulfonyl)phenyl]piperazine (311 mg, 1.3 mmol). This was treated with NaO^{t}Bu (297 mg, 3.1 mmol), RuPhos G3 (102.1 mg, 0.12 mmol) in 1,4-dioxane (12.4 ml), degassed and stirred at 50 °C for 2 h then 90 °C for a further 2 h. The reaction mixture was partitioned between EtOAc (100 ml) and NaHCO₃ (100 ml). The organic layer was separated and the aqu washed with further EtOAc. The combined organics were dried with sodium sulfate and then vac'd down to a brown oil. This was columned with Hex/EtOAc 10-100% to afford a brown oil. Product was treated with DCM (1 ml) and TFA (1ml) then stirred for 30 mins. Solution was vac'd down and partitioned between NaHCO₃ and DCM. The organic layer was separated and vac'd down and the products isolated by reverse phase HPLC.
Example 255: LCMS (Method 1, ES⁺) 1.29 min, 441 *m*/*z* (M+H)⁺
Example 256: LCMS (Method 1, ES⁺) 1.37 min, 441 *m*/*z* (M+H)⁺

### Example 257

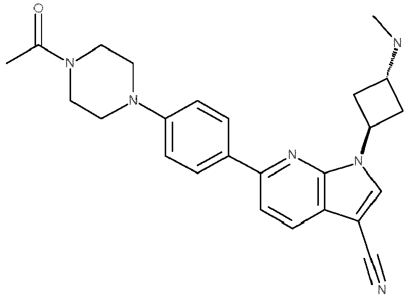

### 6-[4-(4-acetylpiperazin-1-yl)phenyl]-1-[trans-3-(methylamino)cyclobutyl]pyrrolo[2,3-b]pyridine-3-carbonitrile

Intermediate 50 (68 mg, 0.18 mmol), 4-(4-acetyl-1-piperazinyl)phenylboronic acid (70.14 mg, 0.28 mmol) and (XPhos) palladium (II) phenethylamine chloride (28 mg, 0.038 mmol) were dissolved in 1,4-dioxane (3.8 ml) and aqueous Na₂CO₃ (0.40 mmol) then degassed. The reaction was heated at 100 °C for 3 h and allowed to cool to room temperature. The reaction mixture was partitioned between DCM and water then separated and the organic layer was vac'd down. The residue was dissolved in DCM (1.5 ml) and TFA (1.5 ml) was added. The reaction was stirred at r.t. for 1 h. The product was captured on an SCX column and eluted with NH₃/MeOH (7M). The solution was evaporated under reduced pressure and the oil was triturated with MeCN. The solid was filtered off and dried to afford the title compound (40 mg) as a white solid.
LCMS (Method 1, ES⁺) 1.91 min, 429 *m*/*z* (M+H)⁺

### Example 258

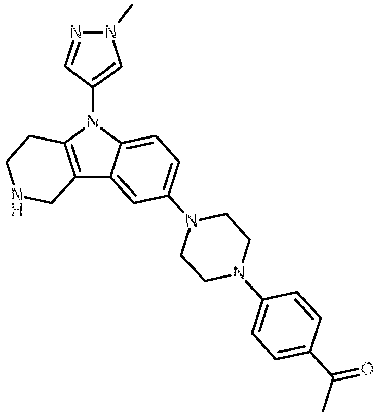

### 1-[4-[4-[5-(1-methylpyrazol-4-yl)-1,2,3,4-tetrahydropyrido[4,3-b]indol-8-yl]piperazin-1-yl]phenyl]ethanone

To a solution of 8-chloro-2,3,4,5-tetrahydro-1H-pyrido[4,3-b]indole (400 mg, 1.94 mmol) in THF (40 mL) was added BOC anhydride (0.534 mL, 2.32 mmol) at 0 °C. The reaction mixture was stirred at rt for 10 mins then diluted with water (50 mL) and extracted with EtOAc (2 x 50 mL). The organic layer was washed with water (50 mL), brine (50 mL), dried over sodium sulfate and evaporated under reduced pressure. The crude material was purified by column chromatography on silica using 30% ethyl acetate in hexane to give tert-butyl 8-chloro-1,3,4,5-tetrahydropyrido[4,3-b]indole-2-carboxylate (420 mg, 1.34 mmol, 70%).

tert-butyl 8-chloro-1,3,4,5-tetrahydropyrido[4,3-b]indole-2-carboxylate (1 equiv) was subjected to a modified General Procedure 17 (with the stated intermediate instead of arylimidazol-2-one) with 4-iodo-1-methyl-pyrazole (1.5 equiv). The resulting product was then subjected to General Procedure 3 with 1-(4-piperazin-1-ylphenyl)ethenone then General Procedure 13 to give the title product (125 mg, 28% over 3 steps).
LCMS (Method 9, ES⁺) 1.83 min, 455 *m*/*z* (M+H)⁺

### Example 259

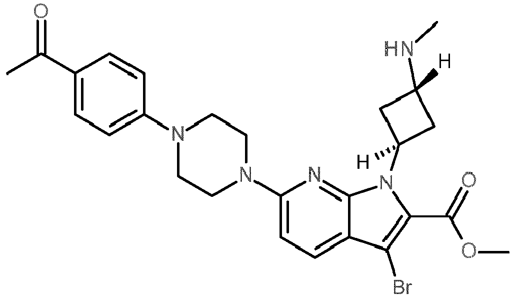

### trans-methyl 6-[4-(4-acetylphenyl)piperazin-1-yl]-3-bromo-1-[3-(methylamino)cyclobutyl]pyrrolo[2,3-b]pyridine-2-carboxylate

Methyl 6-chloro-1H-pyrrolo[2,3-b]pyridine-2-carboxylate (400 mg, 1.90 mmol) and cis-tert-butyl N-(3-hydroxycyclobutyl)-N-methyl-carbamate (459 mg, 2.28 mmol) were reacted using General Procedure 9 to give trans-methyl 1-[3-[tert-butoxycarbonyl(methyl)amino]cyclobutyl]-6-chloro-pyrrolo[2,3-b]pyridine-2-carboxylate (650 mg, 1.61 mmol, 85%).

To a stirred solution of trans-methyl 1-[3-[tert-butoxycarbonyl(methyl)amino]cyclobutyl]-6-chloro-pyrrolo[2,3-b]pyridine-2-carboxylate (300 mg, 0.741 mmol) in toluene (20 mL) was added 1-(4-piperazin-1-ylphenyl)ethanone (232 mg, 1.11 mmol) and potassium phosphate tribasic (472 mg, 2.22 mmol) at rt. The reaction mixture was degassed using argon for 15 mins. Tris(dibenzylideneacetone)dipalladium(0) (136 mg, 0.148 mmol) and X-Phos (64.3 mg, 0.148 mmol) were then added, and the mixture was degassed again using argon for 15 mins. After heating at 100°C for 2 h, the reaction mixture was diluted with water (75 mL) and extracted with ethyl acetate (75 mL x 2). The organic layer was separated, washed with brine (100 mL), dried over sodium sulphate, concentrated under reduced pressure and purified by column chromatography using 30% ethyl acetate in hexane to give trans-methyl 6-[4-(4-acetylphenyl)piperazin-1-yl]-1-[3-[tert-butoxycarbonyl(methyl)amino]cyclobutyl]pyrrolo[2,3-b]pyridine-2-carboxylate (250 mg, 0.35 mmol, 48%).

To a stirred solution of trans-methyl 6-[4-(4-acetylphenyl)piperazin-1-yl]-1-[3-[tert-butoxycarbonyl(methyl)amino]cyclobutyl]pyrrolo[2,3-b]pyridine-2-carboxylate (250 mg, 0.35 mmol) in DCM (15 mL) was added NBS (53.3 mg, 0.300 mmol) at -50°C. The resulting reaction mixture was stirred at -50°C for 30 min. After complete consumption of starting material, water (50 mL) was added and the mixture was extracted with DCM (50 mL x 3). The collected organic layer was washed with brine (25 mL), dried over sodium sulphate and purified by column chromatography using 20% EtOAc in hexane to give trans-methyl 6-[4-(4-acetylphenyl)piperazin-1-yl]-3-bromo-1-[3-[tert-butoxycarbonyl(methyl)amino]cyclobutyl]pyrrolo[2,3-b]pyridine-2-carboxylate (100 mg, 0.14 mmol, 39%).

trans-methyl 6-[4-(4-acetylphenyl)piperazin-1-yl]-3-bromo-1-[3-[tert-butoxycarbonyl(methyl)amino]cyclobutyl]pyrrolo[2,3-b]pyridine-2-carboxylate (45 mg, 0.07 mmol) was subjected to General Procedure 13 to give the title product (28 mg, 0.05 mmol, 74%).
LCMS (Method 9, ES⁺) 2.52 min, 540 & 542 *m*/*z* (M+H)⁺

### Example 260

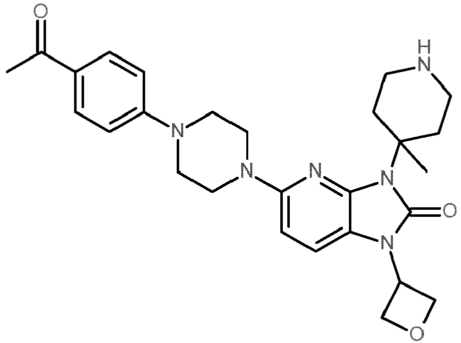

### 5-[4-(4-acetylphenyl)piperazin-1-yl]-3-(4-methyl-4-piperidyl)-1-(oxetan-3-yl)imidazo[4,5-b]pyridin-2-one

To a stirred solution of Intermediate 64 (300 mg, 0.789 mmol) and 3-iodooxetane (218 mg, 1.18 mmol) in DMF (30 mL) was added caesium carbonate (771 mg, 2.37 mmol) at rt. The mixture was heated at 100°C for 5 h then diluted with water (50 mL) and extracted with ethyl acetate (50 mL x 2). The organic layer was separated, washed with brine (50 mL), dried over sodium sulfate and concentrated under reduced pressure. The crude compound was purified by column chromatography using 2% methanol in DCM to give tert-butyl 4-[5-chloro-1-(oxetan-3-yl)-2-oxo-imidazo[4,5-b]pyridin-3-yl]-4-methyl-piperidine-1-carboxylate (280 mg, 0.58 mmol, 73%).

tert-butyl 4-[5-chloro-1-(oxetan-3-yl)-2-oxo-imidazo[4,5-b]pyridin-3-yl]piperidine-1-carboxylate was subjected to General Procedure 3 with 1-(4-piperazin-1-ylphenyl)ethanone then General Procedure 13 to give the title product (27% over two steps).
LCMS (Method 9, ES⁺) 2.19 min, 491 *m*/*z* (M+H)⁺

### Example 261

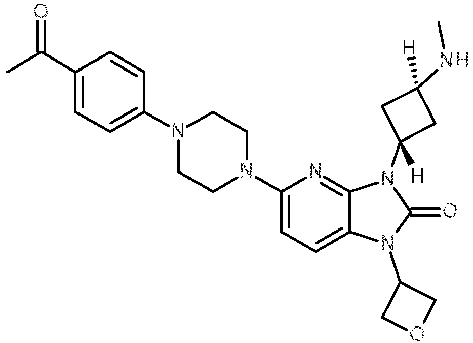

### trans-5-[4-(4-acetylphenyl)piperazin-1-yl1-3-[3-(methylamino)cyclobutyl]-1-(oxetan-3-yl)imidazo[4,5-b]pyridin-2-one

To a stirred solution of Intermediate 62 (250 mg, 0.69 mmol) and 3-iodooxetane (191 mg, 1.04 mmol) in DMF (15 mL) was added caesium carbonate (677 mg, 2.08 mmol) at rt. The mixture was heated at 100°C for 5 h then diluted with water (50 mL) and extracted with ethyl acetate (50 mL x 2). The organic layer was separated, washed with brine (50 mL), dried over sodium sulfate and concentrated under reduced pressure. The crude compound was purified by column chromatography using 2% methanol in DCM to give trans-tert-butyl N-[3-[5-chloro-1-(oxetan-3-yl)-2-oxo-imidazo[4,5-b]pyridin-3-yl]cyclobutyl]-N-methyl-carbamate (240 mg, 0.56 mmol, 80%).

trans-tert-butyl N-[3-[5-chloro-1-(oxetan-3-yl)-2-oxo-imidazo[4,5-b]pyridin-3-yl]cyclobutyl]-N-methyl-carbamate was subjected to General Procedure 3 with 1-(4-piperazin-1-ylphenyl)ethanone then General Procedure 13 to give the title product (58% over two steps).
LCMS (Method 9, ES⁺) 2.03 min, 477 *m*/*z* (M+H)⁺

### Example 262

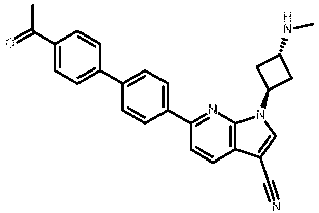

### 6-[4-(4-Acetylphenyl)phenyl]-1-[3-(methylamino)cyclobutyl]pyrrolo[2,3-b]pyridine-3-carbonitrile

The title compound was prepared via boc de-protection of Intermediate 96, according to General Procedure 13.
LCMS (Method 11, ES⁺) 2.42 min, 421 *m*/*z* [M+H]⁺.

### Example 263

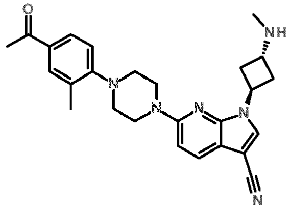

### 6-[4-(4-acetyl-2-methylphenyl)piperazin-1-yl]-1-[3-(methylamino)cyclobutyl]pyrrolo[2,3-b]pyridine-3-carbonitrile

The title compound was prepared by Buchwald amination and de-Boc, according to General procedure 3 and General Procedure 13 from intermediate 50 with 1-(4-bromo-3-methylphenyl)ethanone.
LCMS (Method 11, ES+) 2.30 min, 443 m/z [M+H]⁺

### Example 264

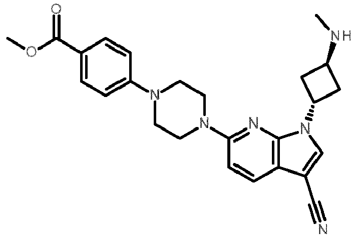

### trans-methyl 4-[4-[3-cyano-1-[3-(methylamino)cyclobutyl]pyrrolo[2,3-b]pyridin-6-yl]piperazin-1-yl]benzoate

The title compound was prepared using intermediate 109 according to General procedure 13.
LCMS (Method 1) 2.16 min, 445 m/z [M+H]⁺

### Example 265

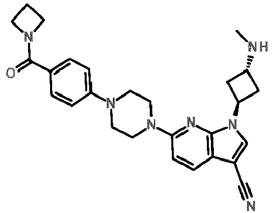

### 6-[4-[4-(azetidine-1-carbonyl)phenyl]piperazin-1-yl]-1-[3-(methylamino)cyclobutyl]pyrrolo[2,3-b]pyridine-3-carbonitrile

To a mixture of intermediate 110 (70 mg, 0.132 mmol) and N-ethyl-N-isopropyl-propan-2-amine (46 uL, 0.264 mmol) in DMF (3 mL) was added HATU (75 mg, 0.198 mmol) at r.t and stirred for 5 min. Then azetidine (18 uL, 0.264 mmol) was added and stirred at r.t for 30 min. The reaction was then quenched with water (0.2 ml). The mixture was then evaporated in vacuo and the residue was purified by Biotage [wet loaded to SNAP KP NH 11 g cartridge, eluent from 0% to 58% (10% MeOH in DCM) in DCM]. The product fractions were evaporated in vacuo to yield the title compound as off-white solid.
LCMS (Method 11, ES+) 3.08 min, 470 m/z [M+H]⁺

### Example 266

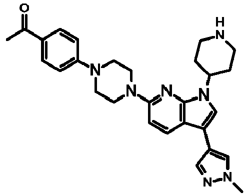

### 1-[4-[4-[3-(1-methylpyrazol-4-yl)-1-piperidin-4-ylpyrrolo[2,3-b]pyridin-6-yl]piperazin-1-yl]phenyl]ethanone

A suspension of 10% Pd/C (50% wet) (41.3 mg, 0.04 mmol) and Intermediate 100 (80 mg, 0.13 mmol) in THF (4 mL) and EtOH (1 mL) was evacuated and back-filled with nitrogen three times and then evacuated and back-filled with hydrogen three times. The suspension was stirred under an atmosphere of hydrogen for 16 h at rt.

The reaction mixture was filtered through a pad of celite and glass fibre filter paper, the filtrate was concentrated in vacuo to afford the crude title compound (65 mg) as a brown gum. The residue was purified by high pH prep HPLC. The product containing fractions were combined and the solvent was removed in vacuo to afford 7 mg (12%) of the title compound as an off white solid.
LCMS (Method 11, ES+) 2.03 min, 484 *m*/*z* [M+H]⁺

### Example 267

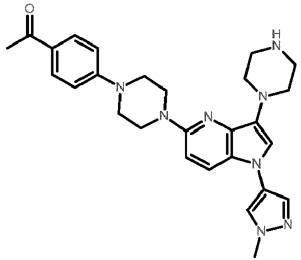

### 1-[4-[4-[1-(1-methylpyrazol-4-yl)-3-piperazin-1-yl-pyrrolo[3,2-b]pyridin-5-yl]piperazin-1-yl]phenyl]ethanone

The title compound was prepared from Intermediate 104 and tert-butyl piperazine-1-carboxylate, in accordance with General Procedure 1.
LCMS (Method 11, ES⁺) 1.70 min, 485 *m*/*z* [M+H]⁺

### Example 268

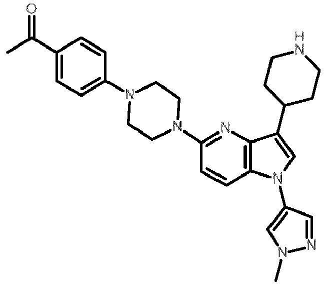

### 1-[4-[4-[1-(1-methylpyrazol-4-yl)-3-piperidin-4-ylpyrrolo[3,2-b]pyridin-5-yl]piperazin-1-yl]phenyl]ethanone

To the solution of Intermediate 108 (40 mg, 0.05 mmol) in DCM (5 mL) / MeOH (2 mL) was added 4 N HCI in dioxane (0.5 mL) and then stirred at r.t overnight. The reaction was then evaporated in vacuo and the residue was purified by low pH preparative HPLC. The desired fraction was basified with 2 N NH₃ in MeOH and evaporated in vacuo to dryness. The residue was then loaded to SCX 2 cartridge (1 g) in DCM / MeOH (1:1), and washed with MeOH (10 mL). The cartridge was then flushed with 2 N NH3 in DCM/MeOH (1:1) to release the product. The desired fraction was evaporated in vacuo to yield the title compound as white solid (13 mg, 52%).
LCMS (Method 11, ES⁺) 1.58 min, 484 *m*/*z* [M+H]⁺.

### - Biological assay

The ability of these compounds encompassed by the present invention to modulate 2'3'-cGAMP stimulated STING signaling as investigated in cell assays. HEK Blue ISG permeabilised cells were pre-incubated with compound for 60 mins at 37 °C, 5% CO₂. The cells were then stimulated with 2'3'-cGAMP. The effect of compound on the 2'3'-cGAMP induced production of Type I interferons is indirectly determined by an ISG54 reporter assay. Type 1 interferons produced by the cells were measured using HEK Blue detection reagent. The ability of compound to inhibit 2'3'-cGAMP stimulated production of Type I interferons from HEK Blue ISG cells was measured as plC₅₀ WT (wild-type).

Compound activity was assayed for STING dependence using HEK Blue ISG KO STING cells (counter-screen). 2'3'-cGAMP does not induce production of Type I interferons from cells that lack the receptor STING.

In the counter-screen, compounds were tested for their ability to modulate the production of Type I interferons by pre-treating permeabilised HEK Blue ISG KO STING cells with compound for 60 mins at 37 °C, 5% CO₂ and then stimulating with interferon. The effect of compound on the interferon induced production of Type I interferons is indirectly determined by an ISG54 reporter assay. Type 1 interferons produced by the cells were measured using HEK Blue detection reagent. The ability of compound to inhibit interferon stimulated production of Type I interferons from HEK Blue ISG KO STING cells was measured as pIC₅₀ KO.
All the pharmaceutically active compounds described herein have pIC₅₀ superior to 4.5 in HEK Blue ISG cells and poorer pIC₅₀ in the HEK Blue ISG KO STING assay.

## Claims

1. A compound of general formula I, or a pharmaceutically acceptable acid addition salt, a racemic mixture or its corresponding enantiomer and/or optical isomers thereof, 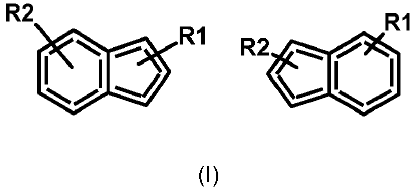 wherein
- the central core A which is the 6,5 heterobicyclic rings contains at least one heteroatom from O,N,S and C atoms can be optionally substituted by halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents;
- R1 represents alkyl or cycloalkyl amines including fused and spirocycloalkyl amines optionally substituted including (C1-3) aminoalkyl, (C3-7) aminocycloalkyl, (C1-3)alkylimidazole, (C1-3)alkyl isoindoline, (C1-3)alkylpiperazine, (C1-3)alkylpiperidine, (C1-3)alkyl imidazopiperazine, (C1-3)alky(C4-7)aminocycloalkyl, (C1-3)alky(C4-7)aminodicycloalkyl; and
- R2 represents aryl, heteroaryl, heterobicyclic, (C4-7) aminocycloalkyl, cycloalkyl, heterocycloalkyl, (C6-8) diaminocycloalkyl, morpholino, (C4-7)cylcoalkylmethyl, piperzinyl, piperdinyl. R2 is optionally substituted with groups including hydroxyl, (C1-6)alkyl, acetyl, halogen, cyano, C1-6 alkyl, trifluoromethyl, difluromethyl, (C2-6) alkenyl, hydroxy, (C1-6) alkoxy, difluoromethoxy, trifluoromethoxy, trifluoroethoxy, (C1-6) alkylthio, (C1-6) alkylsulphonyl, amino, (C1-6) alkylamino, di(C1-6)alkylamino, (C1-6)alkoxy(C1-6)alkyl-amino, N-[(C1-6)alkyl]-N-[hydroxy(C1-6)alkyl]amino, (C2-6) alkylcarbonylamino, (C2-6) alkoxycarbonylamino, (C1-6) alkylsulphonylamino, formyl, (C2-6) alkylcarbonyl, carboxy, (C2-6) alkoxycarbonyl, aminocarbonyl, (C1-6) alkylaminocarbonyl, di(C1-6)alkylaminocarbonyl, aminosulphonyl, (C1-6) alkylaminosulphonyl or di(C1-6)alkylaminosulphonyl; (C3-7)heterocycloalkyl or (C3-7)spiroheterocycloalkyl, either of which groups may be optionally substituted by one or more substituents.

2. A compound according to claim 1, wherein the central core A is selected from the group consisting of

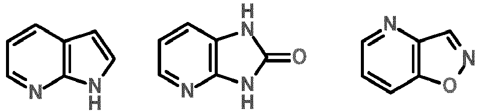

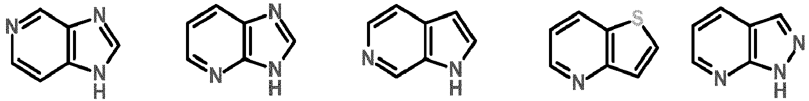

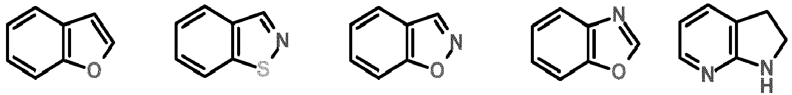

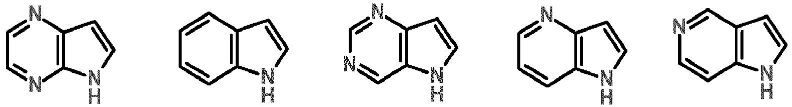

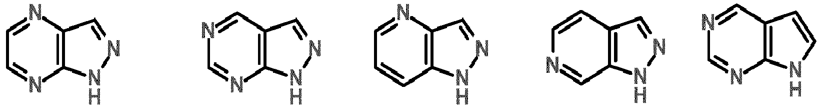

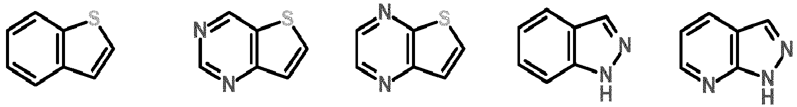

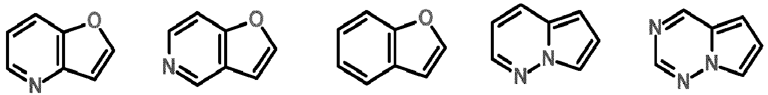

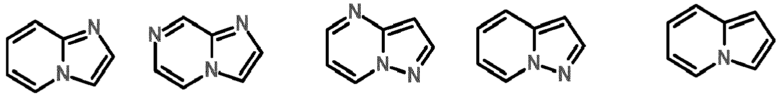

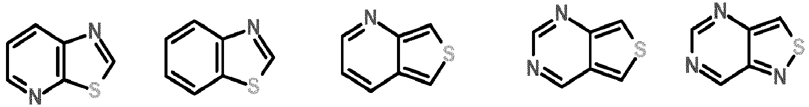

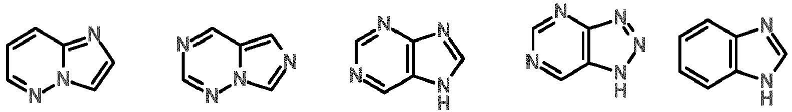

3. A compound according to claim 2, wherein the central core A is selected from the group consisting of

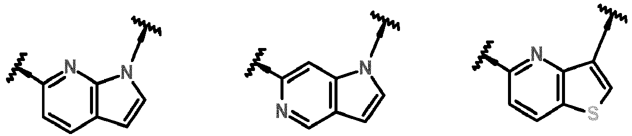

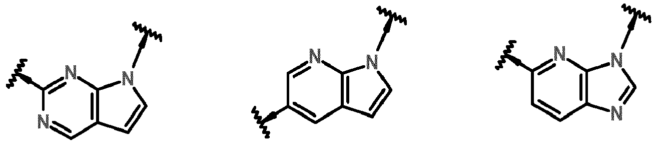

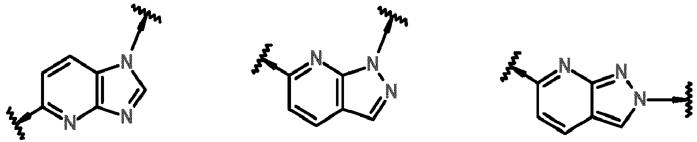

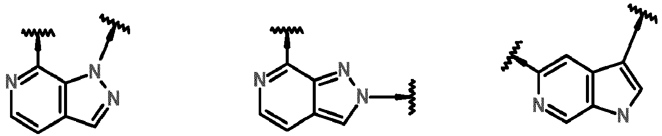

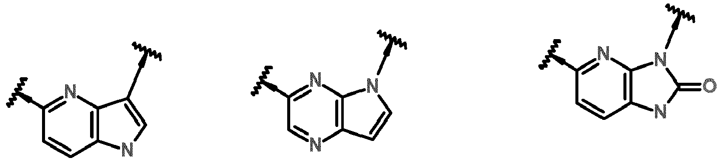

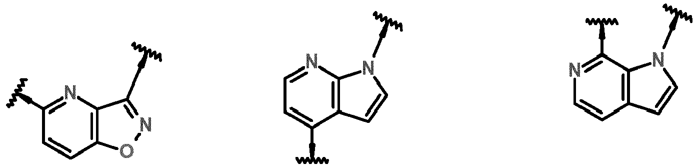

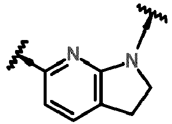

4. A compound according to claim 3, wherein the central core A is

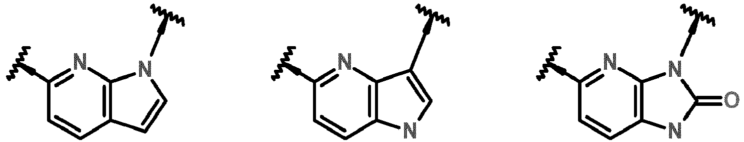

5. A compound according to any of the preceding claims, wherein R1 is ethyl-azabicyclo[3.2.0]heptane; or 2-substituted-5-azaspiro[3.4.]octane; or ethyl-2-methyl-pyrrolidine or (C4-7) cycloalkylamines including 3-substituted-N-methyl-cyclobutanamine and piperidine.

6. A compound according to claim 5, wherein R1 is 3-substituted-N-methyl-cyclobutanamine.

7. A compound according to claim 5, wherein R1 is 2-substituted-5-azaspiro[3.4.]octane.

8. A compound according to claim 5, wherein R1 is (C4-7) cycloalkylamines including 3-substituted-N-methyl-cyclobutanamine and piperidine.

9. A compound according to to any of the preceding claims, wherein R2 is 1-substituted-4-aryl-piperazine or 1-substituted-4-heteroaryl-piperazine or 1-(4-substituted-aryl)piperazine or 1-(4-substituted-heteroaryl)piperazine or 1-substituted-4-aryl-piperidine or 1-substituted4-heteroaryl-piperidine or 1-(4-substituted-aryl)piperidine or 1-(4-substituted-heteroaryl)piperidine with optional substitution on aryl, heteroaryl, piperazine or piperidine groups.

10. A compound according to claim 9, wherein R2 is 1-substituted-4-aryl-piperazine or 1-substituted-4-heteroaryl-piperazine, 1-substituted-4-aryl-piperidine or 1-substituted-4-heteroaryl-piperidine.

11. A compound according to claim 1, wherein
- the central core A is 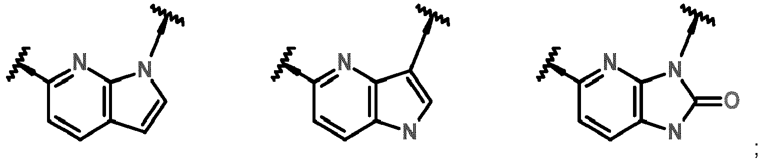
- R1 is ethyl-azabicyclo[3.2.0]heptane ; or 2 -substituted-5-azaspiro[3.4.]octane; or ethyl-2-methyl-pyrrolidine or (C4-7) cycloalkylamines including 3-substituted-N-methyl-cyclobutanamine and piperidine ;
- R2 is 1-substituted-4-aryl-piperazine or 1-substituted-4-heteroaryl-piperazine or 1-(4-substituted-aryl)piperazine or 1-(4-substituted-heteroaryl)piperazine or 1-substituted-4-aryl-piperidine or 1-substituted4-heteroaryl-piperidine or 1-(4-substituted-aryl)piperidine or 1-(4-substituted-heteroaryl)piperidine with optional substitution on aryl, heteroaryl, piperazine or piperidine groups.

12. A compound according to claim 11, wherein
- the central core A is 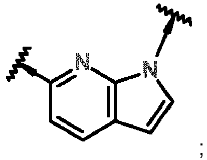
- R1 is 2-substituted-5-azaspiro[3.4.]octane or 3-substituted-N-methyl-cyclobutanamine or 4-substituted piperidine;
- R2 is 1-substituted-4-aryl-piperazine or 1-substituted-4-heteroaryl-piperazine, 1-substituted-4-aryl-piperidine or 1-substituted-4-heteroaryl-piperidine.

13. A compound according to any of the preceding claims selected from the group comprising:
1-[4-[4-[3-(1-methylpyrazol-4-yl)-1-(4-piperidyl)pyrrolo[2,3-b]pyridin-6-yl]piperazin-1-yl]phenyl]ethanone,
1-[4-[4-[3-(2-methyl-3-pyridyl)-1-(4-piperidyl)pyrrolo[2,3-b]pyridin-6-yl]piperazin-1-yl]phenyl]ethanone,
6-[(2S)-2-methyl-4-(2-methylphenyl)piperazin-1-yl]-1-[2-(pyrrolidin-1-yl)ethyl]-1 H-pyrrolo[2,3-b]pyridine,
1-[4-[4-[1-(1-methylpyrazol-4-yl)-3-(4-piperidyl)pyrrolo[3,2-b]pyridin-5-yl]piperazin-1-yl]phenyl]ethanone,
6-[4-(4-acetylphenyl)piperazin-1-yl]-1-(5-azaspiro[3.4]octan-2-yl)pyrrolo[2,3-b]pyridine-3-carbonitrile,
1-[3-(methylamino)cyclobutyl]-6-[4-[4-(2,2,2-trifluoroacetyl)phenyl]piperazin-1-yl]pyrrolo[2,3-b]pyridine-3-carbonitrile,
6-[4-(4-acetylphenyl)piperazin-1-yl]-1-(4-piperidyl)pyrrolo[2,3-b]pyridine-3-carbonitrile, 6-[4-(4-acetyl-3-hydroxy-phenyl)piperazin-1-yl]-1-[3-(methylamino)cyclobutyl]pyrrolo[2,3-b]pyridine-3-carbonitrile,
6-[4-(4-acetyl-3-fluoro-phenyl)piperazin-1-yl]-1-[3-(methylamino)cyclobutyl]pyrrolo[2,3-b]pyridine-3-carbonitrile,
6-[(-4-(4-acetylphenyl)-2-methyl-piperazin-1-yl]-1-[3-(methylamino)cyclobutyl]pyrrolo[2,3-b]pyridine-3-carbonitrile,
6-[4-(4-acetylphenyl)piperazin-1-yl]-1-[3-(methylamino)cyclobutyl]pyrrolo[2,3-b]pyridine-3-carbonitrile,
5-[4-(4-acetylphenyl)piperazin-1-yl]-3-[3-(methylamino)cyclobutyl]-1-(1-methylpyrazol-4-yl)imidazo[4,5-b]pyridin-2-one,
6-[4-(4-acetylphenyl)-3-methyl-piperazin-1-yl]-1-[3-(methylamino)cyclobutyl]pyrrolo[2,3-b]pyridine-3-carbonitrile,
6-[4-(4-acetylphenyl)piperazin-1-yl]-2-methyl-1-[3-(methylamino)cyclobutyl]pyrrolo[2,3-b]pyridine-3-carbonitrile,
6-[4-(5-acetyl-2-pyridyl)piperazin-1-yl]-1-[3-(methylamino)cyclobutyl]pyrrolo[2,3-b]pyridine-3-carbonitrile.

14. A compound according to any of claims 1 to 13 for use as a medicament.

15. A pharmaceutical composition comprising an effective amount of a compound according to claims 1 to 13 in combination with a pharmaceutically acceptable diluent or carrier.

16. A compound according to any of claims 1 to 13 for use in the treatment of inflammatory conditions driven by STING activation.
